(19) Europäisches Patentamt
European Patent Office
Office européen des brevets

(11) EP 3 798 309 A1

(12) EUROPEAN PATENT APPLICATION
published in accordance with Art. 153(4) EPC

(43) Date of publication:
**31.03.2021 Bulletin 2021/13**

(21) Application number: **19807063.3**

(22) Date of filing: **22.05.2019**

(51) Int Cl.:
***C12N 15/31*** *(2006.01)* ***A61K 31/7088*** *(2006.01)*
***A61K 31/711*** *(2006.01)* ***A61K 39/106*** *(2006.01)*
***A61K 48/00*** *(2006.01)* ***A61P 1/04*** *(2006.01)*
***A61P 7/04*** *(2006.01)* ***A61P 31/04*** *(2006.01)*
***A61P 35/00*** *(2006.01)* ***A61P 35/02*** *(2006.01)*
***A61P 37/04*** *(2006.01)* ***C07K 14/205*** *(2006.01)*
***C12N 15/11*** *(2006.01)* ***C12Q 1/686*** *(2018.01)*
***C12Q 1/689*** *(2018.01)* ***G01N 33/569*** *(2006.01)*

(86) International application number:
**PCT/JP2019/020248**

(87) International publication number:
**WO 2019/225639 (28.11.2019 Gazette 2019/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.05.2018 JP 2018098504**
**24.05.2018 JP 2018099903**

(71) Applicant: **The Kitasato Institute**
**Tokyo 108-8641 (JP)**

(72) Inventors:
- **MATSUI Hidenori**
  **Tokyo 108-8641 (JP)**
- **NAKAMURA Masahiko**
  **Tokyo 108-8641 (JP)**
- **MURAYAMA Somay**
  **Tokyo 108-8641 (JP)**
- **TAKAHASHI Tetsufumi**
  **Tokyo 108-8641 (JP)**
- **RIMBARA Emiko**
  **Tokyo 162-8640 (JP)**
- **SUZUKI Masato**
  **Tokyo 162-8640 (JP)**
- **SHIBAYAMA Keigo**
  **Tokyo 162-8640 (JP)**

(74) Representative: **J A Kemp LLP**
**14 South Square**
**Gray's Inn**
**London WC1R 5JJ (GB)**

# (54) METHOD FOR DIAGNOSING INFECTION WITH HELICOBACTER SUIS

(57) One object of the present invention is to provide an *H*. suis-specific diagnosis method. Another object of the present invention is to provide a simple examination method comprising an *H. suis*-specific diagnosis method that is a non-invasive method. The present inventors newly found that an outer membrane protein (HsvA: named by the present inventor) specifically present exclusively in *H. suis* and an antibody against this protein are present in the body of a subject infected with *H. suis.* Further, the present inventors found a diagnostic method using an amino acid sequence that acts as the antigenic site of HsvA protein. Accordingly, the present invention provides a method whereby *H. suis* can be specifically detected with the use of an *H. suis*-specific antigen comprising an HsvA antigen peptide and an *H. suis*-specific DNA derived from hsvA gene.

EP 3 798 309 A1

SEQUENCE LISTING

```
<110>  Kitasato Institute
       National Institute of Infectious Disease

<120>  Diagnosis for H.suis infection

<130>  Y09-70009WO

<150>  JP2018-99903
<151>  2018-05-24

<150>  JP2018-98504
<151>  2018-05-23

<160>  91

<170>  PatentIn version 3.5

<210>  1
<211>  9842
<212>  DNA
<213>  Helicobacter suis


<220>
<221>  CDS
<222>  (420)..(9398)

<220>
<221>  sig_peptide
<222>  (420)..(500)

<400>  1
gttataacct tgaacaaata ggctttcaca gggcaaaaaa tacatgtttt ctctcacatg      60

gaatgcgcct cttgaggctt tcacagataa aaatcagttt tttggaggtg taggagtaga     120

tggggtgtat ctgcatttgc ataaagccca tgaattctta ggcatgcgtg ctctgccaac     180

ttttattgta aatgacatca ttaaaaatcc acagggggaa tcctatctca aagattacag     240

cgcgcattta aaacaggttt ttcataaata ggcttctttc tttttacttc attaacttta     300

aagtttgcta gctggatatt tttaacaggc gtttttataa cgcctcttgt gtatttaaac     360

taaaataagt atctcttaaa ttaatttctt atacatgatt ttatgctaaa gttttctct      419

atg aaa aag ttt agt tct ctc aca ttg aaa ttt ggc cac gct cta gca      467
Met Lys Lys Phe Ser Ser Leu Thr Leu Lys Phe Gly His Ala Leu Ala
1               5                   10                  15

cgg cgc att aaa gct aac caa gct agg cgt gcg ggt act tat gtc ttt      515
Arg Arg Ile Lys Ala Asn Gln Ala Arg Arg Ala Gly Thr Tyr Val Phe
            20                  25                  30

aaa aaa caa ctc ccc caa ccc aag cgt tct gag ttg aaa cct aaa tta      563
Lys Lys Gln Leu Pro Gln Pro Lys Arg Ser Glu Leu Lys Pro Lys Leu
        35                  40                  45

atc aag cag ggg act ttt att tta ggt gta atg agc caa ccg ctt tta      611
Ile Lys Gln Gly Thr Phe Ile Leu Gly Val Met Ser Gln Pro Leu Leu
    50                  55                  60
```

```
gcg tgg tat cct tca tgg att agt ggc aca cac aca ctc aac aca aca        659
Ala Trp Tyr Pro Ser Trp Ile Ser Gly Thr His Thr Leu Asn Thr Thr
65                  70                  75                  80

aat att aag cag tat atg caa aat aac cat aga agc caa aat tgg tta        707
Asn Ile Lys Gln Tyr Met Gln Asn Asn His Arg Ser Gln Asn Trp Leu
                85                  90                  95

tgg act ggt ggc tct aat gtc ttt tat gaa gct agt aat gga agt tat        755
Trp Thr Gly Gly Ser Asn Val Phe Tyr Glu Ala Ser Asn Gly Ser Tyr
            100                 105                 110

ttt tgt act aac tgg aat tgt aac ggc tcg gtt aca tta att ggt agt        803
Phe Cys Thr Asn Trp Asn Cys Asn Gly Ser Val Thr Leu Ile Gly Ser
        115                 120                 125

ggc tct acc act tac acg ctt agc aat ctc aat tac gaa ggc ggt agc        851
Gly Ser Thr Thr Tyr Thr Leu Ser Asn Leu Asn Tyr Glu Gly Gly Ser
    130                 135                 140

ctc aat cta caa att aat ggt aat ggc aca caa ggc gcg ctt aat att        899
Leu Asn Leu Gln Ile Asn Gly Asn Gly Thr Gln Gly Ala Leu Asn Ile
145                 150                 155                 160

agc aat gtc aac atg gat agc tat caa gga agg caa ttt aca gat aca        947
Ser Asn Val Asn Met Asp Ser Tyr Gln Gly Arg Gln Phe Thr Asp Thr
                165                 170                 175

tgg aat gcc caa agt gtt agt atc agc gga aat cta caa gta ggg caa        995
Trp Asn Ala Gln Ser Val Ser Ile Ser Gly Asn Leu Gln Val Gly Gln
            180                 185                 190

aat cat atc act atc aac gca aac cac ggc act aca gcg aac aac gct       1043
Asn His Ile Thr Ile Asn Ala Asn His Gly Thr Thr Ala Asn Asn Ala
        195                 200                 205

acc att aac gcg gat caa ggc aac ggg att tta aat atc aat gat aaa       1091
Thr Ile Asn Ala Asp Gln Gly Asn Gly Ile Leu Asn Ile Asn Asp Lys
    210                 215                 220

aca agc gag agt ttc aca aac acc acc ttt aaa ggt acg ggg cag atc       1139
Thr Ser Glu Ser Phe Thr Asn Thr Thr Phe Lys Gly Thr Gly Gln Ile
225                 230                 235                 240

aat tta cag agc aac aac atc acc ttt aac aat gtt act ttt aat gat       1187
Asn Leu Gln Ser Asn Asn Ile Thr Phe Asn Asn Val Thr Phe Asn Asp
                245                 250                 255

agc aac act ggt tca cat gtt acg gac aac ggc act ctt acc ctt gag       1235
Ser Asn Thr Gly Ser His Val Thr Asp Asn Gly Thr Leu Thr Leu Glu
            260                 265                 270

ggc act gag acc ttt gca caa aac tcg ccc ctt atc aat cta ggc gcc       1283
Gly Thr Glu Thr Phe Ala Gln Asn Ser Pro Leu Ile Asn Leu Gly Ala
        275                 280                 285

aat gtt act atc caa gcc aac acc att ttt aac att aca gaa gat ctt       1331
Asn Val Thr Ile Gln Ala Asn Thr Ile Phe Asn Ile Thr Glu Asp Leu
    290                 295                 300

aca aag acc acc aac gga gcc tat aat ctt gat acc ctt gta agt aca       1379
Thr Lys Thr Thr Asn Gly Ala Tyr Asn Leu Asp Thr Leu Val Ser Thr
```

```
305                 310                 315                 320

agt ggt aat aaa agt atc aac gat agt agc tat gca agc cat ttg tgg        1427
Ser Gly Asn Lys Ser Ile Asn Asp Ser Ser Tyr Ala Ser His Leu Trp
                325                 330                 335

gat ctt atc cgc tat caa ggc caa aca ggt agc ctt ttt aac ggg caa        1475
Asp Leu Ile Arg Tyr Gln Gly Gln Thr Gly Ser Leu Phe Asn Gly Gln
            340                 345                 350

ctc agt aat ggc act act tta agt aat cct agc tct ggc aat ggg att        1523
Leu Ser Asn Gly Thr Thr Leu Ser Asn Pro Ser Ser Gly Asn Gly Ile
        355                 360                 365

tac tat gtg aaa tat act ttt ggc aat ggt gat tgg gat att ctt aaa        1571
Tyr Tyr Val Lys Tyr Thr Phe Gly Asn Gly Asp Trp Asp Ile Leu Lys
    370                 375                 380

gaa gat ttt gaa aat aac tct cta tcg gcg caa ctt gag gct tac gct        1619
Glu Asp Phe Glu Asn Asn Ser Leu Ser Ala Gln Leu Glu Ala Tyr Ala
385                 390                 395                 400

aaa aat cag ggt gat att tgg agt aca atc cac aac att aat tct act        1667
Lys Asn Gln Gly Asp Ile Trp Ser Thr Ile His Asn Ile Asn Ser Thr
                405                 410                 415

tgg aat ttt aat gtc gga gag ggt agt gcc tat atc aac ccc ggg aat        1715
Trp Asn Phe Asn Val Gly Glu Gly Ser Ala Tyr Ile Asn Pro Gly Asn
            420                 425                 430

gga aca gat caa gct tgg gca caa agc gat aaa aat ttt aaa gtt act        1763
Gly Thr Asp Gln Ala Trp Ala Gln Ser Asp Lys Asn Phe Lys Val Thr
        435                 440                 445

tta gat aat ggg agt ggt ggg acg ctt att ctt ggt aat agc aca gaa        1811
Leu Asp Asn Gly Ser Gly Gly Thr Leu Ile Leu Gly Asn Ser Thr Glu
    450                 455                 460

acc ccc agt tca agc ggt aaa att ctc ttt gga ggc aca ggg ggt aat        1859
Thr Pro Ser Ser Ser Gly Lys Ile Leu Phe Gly Gly Thr Gly Gly Asn
465                 470                 475                 480

cct ttt gct tta aat ggc aat tac ggc gga gat ctt ggc tat atg aca        1907
Pro Phe Ala Leu Asn Gly Asn Tyr Gly Gly Asp Leu Gly Tyr Met Thr
                485                 490                 495

ggg gaa ttt gac gcg ggt aaa att tat ctt acc ggt acc ata gaa agc        1955
Gly Glu Phe Asp Ala Gly Lys Ile Tyr Leu Thr Gly Thr Ile Glu Ser
            500                 505                 510

gga aac tct ttt cac gat ggt aac ggc act aac att agc tac aac gct        2003
Gly Asn Ser Phe His Asp Gly Asn Gly Thr Asn Ile Ser Tyr Asn Ala
        515                 520                 525

att aca aac att aca gct aat ggt ttg cac tat ctc aac gat aac gcc        2051
Ile Thr Asn Ile Thr Ala Asn Gly Leu His Tyr Leu Asn Asp Asn Ala
    530                 535                 540

gga gca tgg cat agc aac gct aat ttt aga gct acc aat ggc agt atc        2099
Gly Ala Trp His Ser Asn Ala Asn Phe Arg Ala Thr Asn Gly Ser Ile
545                 550                 555                 560

aac atc tca aac tcc caa ttc caa gat caa agt agc ggg caa ttt acc        2147
```

```
Asn Ile Ser Asn Ser Gln Phe Gln Asp Gln Ser Ser Gly Gln Phe Thr
                565                 570                 575

ttt aac tct caa aat caa act ttt agt agc aca act ttt act ggc aat      2195
Phe Asn Ser Gln Asn Gln Thr Phe Ser Ser Thr Thr Phe Thr Gly Asn
            580                 585                 590

agt cat acg atc act cta cac gct acc aac aac cta acc ctc acc aac      2243
Ser His Thr Ile Thr Leu His Ala Thr Asn Asn Leu Thr Leu Thr Asn
        595                 600                 605

aca agc ttt aat gat tca aat gct agc ttg aca cta gag gca gat aag      2291
Thr Ser Phe Asn Asp Ser Asn Ala Ser Leu Thr Leu Glu Ala Asp Lys
    610                 615                 620

ggt agt tta cga gat acc gat aac caa act agc caa atc aca gct aaa      2339
Gly Ser Leu Arg Asp Thr Asp Asn Gln Thr Ser Gln Ile Thr Ala Lys
625                 630                 635                 640

aac ctc caa gta att gct aat caa gct agt ttt agt aac acg atc ttt      2387
Asn Leu Gln Val Ile Ala Asn Gln Ala Ser Phe Ser Asn Thr Ile Phe
                645                 650                 655

aat gca caa aac agc tct ttt aaa act aac caa ttg acc ctg aca aat      2435
Asn Ala Gln Asn Ser Ser Phe Lys Thr Asn Gln Leu Thr Leu Thr Asn
            660                 665                 670

gat aca ttt aat aat ggt agc tat agc ttt gca cct gaa aac aac ggc      2483
Asp Thr Phe Asn Asn Gly Ser Tyr Ser Phe Ala Pro Glu Asn Asn Gly
        675                 680                 685

aac cac aca acc aca ttt ggc ggt acc acc acg att aat act agc agt      2531
Asn His Thr Thr Thr Phe Gly Gly Thr Thr Thr Ile Asn Thr Ser Ser
    690                 695                 700

agc ccc ttt gct aat tta ggc gga agc att agt tta aac aac ggc gct      2579
Ser Pro Phe Ala Asn Leu Gly Gly Ser Ile Ser Leu Asn Asn Gly Ala
705                 710                 715                 720

att ttt aat ctt aat aat att tta agt tct tta caa att ggt aca act      2627
Ile Phe Asn Leu Asn Asn Ile Leu Ser Ser Leu Gln Ile Gly Thr Thr
                725                 730                 735

tat aac atc tta ggc gga agc ggg gct aat att ctt tac aag aac gat      2675
Tyr Asn Ile Leu Gly Gly Ser Gly Ala Asn Ile Leu Tyr Lys Asn Asp
            740                 745                 750

aca caa tat gcc tca gca ctt tgg caa ctc att aaa atc aac ggg act      2723
Thr Gln Tyr Ala Ser Ala Leu Trp Gln Leu Ile Lys Ile Asn Gly Thr
        755                 760                 765

act att aac tca gaa aca gag ata agt gat aat aac gat acg caa gtt      2771
Thr Ile Asn Ser Glu Thr Glu Ile Ser Asp Asn Asn Asp Thr Gln Val
    770                 775                 780

tgg aat gta gtc ttt aac ata gat ggc atg ccc att aag ata caa gaa      2819
Trp Asn Val Val Phe Asn Ile Asp Gly Met Pro Ile Lys Ile Gln Glu
785                 790                 795                 800

acc ttt gct agt agt gga ctt agc ttt aaa gtt att agc caa gct aaa      2867
Thr Phe Ala Ser Ser Gly Leu Ser Phe Lys Val Ile Ser Gln Ala Lys
                805                 810                 815
```

```
gat att tgg tca gat gtg tat cac atg act act aat tgt gca tac aat      2915
Asp Ile Trp Ser Asp Val Tyr His Met Thr Thr Asn Cys Ala Tyr Asn
            820                 825                 830

gcc tta gcc agc cca ccg ggc tgt tat ggt tat caa aat ggt aca cac      2963
Ala Leu Ala Ser Pro Pro Gly Cys Tyr Gly Tyr Gln Asn Gly Thr His
        835                 840                 845

aac ata ttg aag cac ttt aat aag tac ggc ttt gaa gct tac aat gaa      3011
Asn Ile Leu Lys His Phe Asn Lys Tyr Gly Phe Glu Ala Tyr Asn Glu
    850                 855                 860

agt gta ggc gca gac ggg att gcc tat atc aac cct tta aac tca caa      3059
Ser Val Gly Ala Asp Gly Ile Ala Tyr Ile Asn Pro Leu Asn Ser Gln
865                 870                 875                 880

agc cac gat ggc tat aac gcc tcc acc cac aca ctc caa act tat aca      3107
Ser His Asp Gly Tyr Asn Ala Ser Thr His Thr Leu Gln Thr Tyr Thr
                885                 890                 895

aaa att ggc aat ggc ggt act tat aat gtt ggg gag atg caa aat ggc      3155
Lys Ile Gly Asn Gly Gly Thr Tyr Asn Val Gly Glu Met Gln Asn Gly
            900                 905                 910

aaa tgg gtt aac aat ggt act tta att tta ggt aat aac acc tca caa      3203
Lys Trp Val Asn Asn Gly Thr Leu Ile Leu Gly Asn Asn Thr Ser Gln
        915                 920                 925

ccc gtt aaa ggg ggt aaa ata gaa ttt ggc aaa gtg gac tca gta ggc      3251
Pro Val Lys Gly Gly Lys Ile Glu Phe Gly Lys Val Asp Ser Val Gly
    930                 935                 940

tat atc acc gat gtt ttt aat gcc gga cat att ttt cta act aac agc      3299
Tyr Ile Thr Asp Val Phe Asn Ala Gly His Ile Phe Leu Thr Asn Ser
945                 950                 955                 960

ata gaa gtt ggg gat agt tcc tta agc ggg gcg ggg gca act cta act      3347
Ile Glu Val Gly Asp Ser Ser Leu Ser Gly Ala Gly Ala Thr Leu Thr
                965                 970                 975

ttt aat gct aat aat aat atc acc gcc gac ggt ctt acc tac cac caa      3395
Phe Asn Ala Asn Asn Asn Ile Thr Ala Asp Gly Leu Thr Tyr His Gln
            980                 985                 990

gat gct acc gct atc cct ccc ttc  ggc tct gct atc agc  cag cat agc    3443
Asp Ala Thr Ala Ile Pro Pro Phe  Gly Ser Ala Ile Ser  Gln His Ser
        995                 1000                 1005

tac ggt  aac ttc atc gca caa  caa agc ttt agc gcg  ctc aat tcc       3488
Tyr Gly  Asn Phe Ile Ala Gln  Gln Ser Phe Ser Ala  Leu Asn Ser
    1010                 1015                 1020

tct ttt  gaa gat gat act agc  ggt agt tta gac ttt  acc ggt aaa       3533
Ser Phe  Glu Asp Asp Thr Ser  Gly Ser Leu Asp Phe  Thr Gly Lys
    1025                 1030                 1035

aac agc  att aat ttt aca agc  agc acc gtt ata ggt  agt aaa agt       3578
Asn Ser  Ile Asn Phe Thr Ser  Ser Thr Val Ile Gly  Ser Lys Ser
    1040                 1045                 1050

agt att  acc ctt aac tcc gca  aat acc acc cta aac  aac tct gcc       3623
Ser Ile  Thr Leu Asn Ser Ala  Asn Thr Thr Leu Asn  Asn Ser Ala
    1055                 1060                 1065
```

```
ttt ttt  gtg ggc aat ggt acg  act ctt act ttt ggt  aat gtg atc        3668
Phe Phe  Val Gly Asn Gly Thr  Thr Leu Thr Phe Gly  Asn Val Ile
    1070                 1075                 1080

aca act  aat agt cat agt agc  tat tca tct agc aca  aac acc att        3713
Thr Thr  Asn Ser His Ser Ser  Tyr Ser Ser Ser Thr  Asn Thr Ile
    1085                 1090                 1095

aat aac  tct aca atc aac ctc  aac caa aac tct agc  ttg tat ttg        3758
Asn Asn  Ser Thr Ile Asn Leu  Asn Gln Asn Ser Ser  Leu Tyr Leu
    1100                 1105                 1110

cat ggc  agt acc acc cta gat  aac acc act tcc ttt  tta aac tta        3803
His Gly  Ser Thr Thr Leu Asp  Asn Thr Thr Ser Phe  Leu Asn Leu
    1115                 1120                 1125

agt agt  caa gca act ttt tat  gat tca gct agc cta  agt ggt agc        3848
Ser Ser  Gln Ala Thr Phe Tyr  Asp Ser Ala Ser Leu  Ser Gly Ser
    1130                 1135                 1140

act aca  att aat ctt gat cac  aat agc aag att aca  atg aat agc        3893
Thr Thr  Ile Asn Leu Asp His  Asn Ser Lys Ile Thr  Met Asn Ser
    1145                 1150                 1155

acc act  acg cta aat gat aac  gct aat ttt aat tta  att aac tcc        3938
Thr Thr  Thr Leu Asn Asp Asn  Ala Asn Phe Asn Leu  Ile Asn Ser
    1160                 1165                 1170

gct caa  gct aac ttt caa ggc  aat agt act ttt aat  aat aac tct        3983
Ala Gln  Ala Asn Phe Gln Gly  Asn Ser Thr Phe Asn  Asn Asn Ser
    1175                 1180                 1185

ggg atc  act ctt gct agt ggt  gct aag gct atg ttt  aca cac act        4028
Gly Ile  Thr Leu Ala Ser Gly  Ala Lys Ala Met Phe  Thr His Thr
    1190                 1195                 1200

aca aca  agc acc caa gat att  aca act ttt aat aat  aac tcg ttt        4073
Thr Thr  Ser Thr Gln Asp Ile  Thr Thr Phe Asn Asn  Asn Ser Phe
    1205                 1210                 1215

tta aat  gtc aat ggg agt ttt  aca gat ttt ata ccc  agt aca aat        4118
Leu Asn  Val Asn Gly Ser Phe  Thr Asp Phe Ile Pro  Ser Thr Asn
    1220                 1225                 1230

agt aac  agg aca att agc ggg  gga gga tca agt agt  aat caa aac        4163
Ser Asn  Arg Thr Ile Ser Gly  Gly Gly Ser Ser Ser  Asn Gln Asn
    1235                 1240                 1245

tat agc  gcg cag ttt aat gat  ctc gtg ttt aac aac  tat gct tcc        4208
Tyr Ser  Ala Gln Phe Asn Asp  Leu Val Phe Asn Asn  Tyr Ala Ser
    1250                 1255                 1260

atg ctc  tta aat agc gca gat  att caa agt agc caa  acc acc ttt        4253
Met Leu  Leu Asn Ser Ala Asp  Ile Gln Ser Ser Gln  Thr Thr Phe
    1265                 1270                 1275

gct aat  tct gtg aat atc aat  atg caa aat agc ctt  ttt aat gcg        4298
Ala Asn  Ser Val Asn Ile Asn  Met Gln Asn Ser Leu  Phe Asn Ala
    1280                 1285                 1290

agt acg  ctt aat tta aat ggc  ggt aat ttt tac atg  caa aat agc        4343
Ser Thr  Leu Asn Leu Asn Gly  Gly Asn Phe Tyr Met  Gln Asn Ser
```

```
    1295                1300                1305

cag att  aaa gcg ggg gca gtg  agt gtg gga tct tct  gat tct gtt        4388
Gln Ile  Lys Ala Gly Ala Val  Ser Val Gly Ser Ser  Asp Ser Val
    1310                1315                1320

aat att  aat tct ggg gtc aat  tct ctt aat cgc tcc  ctc atc act        4433
Asn Ile  Asn Ser Gly Val Asn  Ser Leu Asn Arg Ser  Leu Ile Thr
    1325                1330                1335

agc tca  agc ttt aat ctt aat  gga aca tta aac cta  gat ggc ctt        4478
Ser Ser  Ser Phe Asn Leu Asn  Gly Thr Leu Asn Leu  Asp Gly Leu
    1340                1345                1350

tta ctt  gga cct aca act aca  ggt aca acc aaa agt  acg gcc aac        4523
Leu Leu  Gly Pro Thr Thr Thr  Gly Thr Thr Lys Ser  Thr Ala Asn
    1355                1360                1365

ggg caa  cct ctt att tct gta  gga ggg gga aca ttt  agc tta aat        4568
Gly Gln  Pro Leu Ile Ser Val  Gly Gly Gly Thr Phe  Ser Leu Asn
    1370                1375                1380

ggc att  ctt aat atc tct aat  att gat ctc tcc gcc  cac ctc tct        4613
Gly Ile  Leu Asn Ile Ser Asn  Ile Asp Leu Ser Ala  His Leu Ser
    1385                1390                1395

agc caa  aca aat cac act agt  tcc tct gct acc tat  gat att gtg        4658
Ser Gln  Thr Asn His Thr Ser  Ser Ser Ala Thr Tyr  Asp Ile Val
    1400                1405                1410

aat gct  aac aac att aca ggc  atg agt ggg gct aat  ggt tat caa        4703
Asn Ala  Asn Asn Ile Thr Gly  Met Ser Gly Ala Asn  Gly Tyr Gln
    1415                1420                1425

aaa att  gag tat tac ggc att  aaa atc aat aat gct  acc tat tct        4748
Lys Ile  Glu Tyr Tyr Gly Ile  Lys Ile Asn Asn Ala  Thr Tyr Ser
    1430                1435                1440

gat tca  aat tct gat tca aat  aaa acc caa agc tgg  agt ttt aca        4793
Asp Ser  Asn Ser Asp Ser Asn  Lys Thr Gln Ser Trp  Ser Phe Thr
    1445                1450                1455

aac cct  tta aat ggc gca caa  act att act gaa aaa  ata caa agt        4838
Asn Pro  Leu Asn Gly Ala Gln  Thr Ile Thr Glu Lys  Ile Gln Ser
    1460                1465                1470

ggt aaa  ctc act atc acc att  agt aat agc aat cat  ttt gta gct        4883
Gly Lys  Leu Thr Ile Thr Ile  Ser Asn Ser Asn His  Phe Val Ala
    1475                1480                1485

aca gat  tac cac aac atc gcc  ccc gaa ctc ttt ttt  tac aaa caa        4928
Thr Asp  Tyr His Asn Ile Ala  Pro Glu Leu Phe Phe  Tyr Lys Gln
    1490                1495                1500

tcc gca  caa aac ttg cct agt  aca aac tca gca agt  gca gat ata        4973
Ser Ala  Gln Asn Leu Pro Ser  Thr Asn Ser Ala Ser  Ala Asp Ile
    1505                1510                1515

agc agt  tat gat tat tcc agc  gat gaa agc ggg act  ttc ttt tta        5018
Ser Ser  Tyr Asp Tyr Ser Ser  Asp Glu Ser Gly Thr  Phe Phe Leu
    1520                1525                1530

gat ggc  aat tta aaa ggg gtg  tat tat cca aaa tct  agc aca aca        5063
```

```
Asp Gly  Asn Leu Lys Gly Val  Tyr Tyr Pro Lys Ser  Ser Thr Thr
    1535                 1540                 1545

ggc act  acc cca gtt att ccg  ggt act tat aac gct  caa ggc cag         5108
Gly Thr  Thr Pro Val Ile Pro  Gly Thr Tyr Asn Ala  Gln Gly Gln
    1550                 1555                 1560

ccc tta  caa ggt ctt tat att  tct aat aac ggc ctg  ttt aat gaa         5153
Pro Leu  Gln Gly Leu Tyr Ile  Ser Asn Asn Gly Leu  Phe Asn Glu
    1565                 1570                 1575

acc acc  ctt aat aac tta gta  gac att gtc cga agt  att tgg cct         5198
Thr Thr  Leu Asn Asn Leu Val  Asp Ile Val Arg Ser  Ile Trp Pro
    1580                 1585                 1590

cat tta  aaa acc ctt ttg cct  aaa att cta caa gat  tta agc gat         5243
His Leu  Lys Thr Leu Leu Pro  Lys Ile Leu Gln Asp  Leu Ser Asp
    1595                 1600                 1605

cca agt  aat atc att caa gac  tta gaa aac tca ggg  att aaa tta         5288
Pro Ser  Asn Ile Ile Gln Asp  Leu Glu Asn Ser Gly  Ile Lys Leu
    1610                 1615                 1620

act agc  cag caa agc aag gaa  ctt tta agt ttt ata  gac ggg cta         5333
Thr Ser  Gln Gln Ser Lys Glu  Leu Leu Ser Phe Ile  Asp Gly Leu
    1625                 1630                 1635

tca agc  aat atc aac caa act  ttt aat aat ggt acg  ctt gta gtg         5378
Ser Ser  Asn Ile Asn Gln Thr  Phe Asn Asn Gly Thr  Leu Val Val
    1640                 1645                 1650

ggt tcg  cct cag gca gga caa  acc gga agt agt agc  gtg gtg tgg         5423
Gly Ser  Pro Gln Ala Gly Gln  Thr Gly Ser Ser Ser  Val Val Trp
    1655                 1660                 1665

ttt ggt  ggc aat ggt tat act  acg gct tgt acg gct  gca caa act         5468
Phe Gly  Gly Asn Gly Tyr Thr  Thr Ala Cys Thr Ala  Ala Gln Thr
    1670                 1675                 1680

gaa aaa  ggg tgt cag gat tta  aga ggc act tat tta  ggt cag ctt         5513
Glu Lys  Gly Cys Gln Asp Leu  Arg Gly Thr Tyr Leu  Gly Gln Leu
    1685                 1690                 1695

tta gga  tca act tct gct gca  cta ggc tat att gag  gct aat ttt         5558
Leu Gly  Ser Thr Ser Ala Ala  Leu Gly Tyr Ile Glu  Ala Asn Phe
    1700                 1705                 1710

aag gct  aaa gat att tat atc  acc ggc acc gta ggc  agt ggg aat         5603
Lys Ala  Lys Asp Ile Tyr Ile  Thr Gly Thr Val Gly  Ser Gly Asn
    1715                 1720                 1725

gct tgg  ggg ata ggt ggg agc  gca gat gta act ttt  aat agc gcg         5648
Ala Trp  Gly Ile Gly Gly Ser  Ala Asp Val Thr Phe  Asn Ser Ala
    1730                 1735                 1740

act aat  tta acc ctc aat caa  gcc acg att gat gcg  gaa ggc acg         5693
Thr Asn  Leu Thr Leu Asn Gln  Ala Thr Ile Asp Ala  Glu Gly Thr
    1745                 1750                 1755

gat caa  att ttt aat atg cta  ggt gag ggc ggg ata  caa aaa atc         5738
Asp Gln  Ile Phe Asn Met Leu  Gly Glu Gly Gly Ile  Gln Lys Ile
    1760                 1765                 1770
```

```
tta ggc  caa aag ggg cta gcc  cag atg tta ggt aat  tat atc tac        5783
Leu Gly  Gln Lys Gly Leu Ala  Gln Met Leu Gly Asn  Tyr Ile Tyr
    1775                 1780                 1785

gat cta  gcc aat ggc tct agt  att aat ctt aat ccc  att agt agc        5828
Asp Leu  Ala Asn Gly Ser Ser  Ile Asn Leu Asn Pro  Ile Ser Ser
    1790                 1795                 1800

att cca  agc tct atc cag cct  ctg gct aaa gat tta  ggc agg cag        5873
Ile Pro  Ser Ser Ile Gln Pro  Leu Ala Lys Asp Leu  Gly Arg Gln
    1805                 1810                 1815

att ggt  aaa atc aaa ctt agc  gat gtg ctt agc gcc  tca gat gtg        5918
Ile Gly  Lys Ile Lys Leu Ser  Asp Val Leu Ser Ala  Ser Asp Val
    1820                 1825                 1830

agt gca  ctt tta aac atg ccc  ggt atg gat aat gtg  atc aaa aat        5963
Ser Ala  Leu Leu Asn Met Pro  Gly Met Asp Asn Val  Ile Lys Asn
    1835                 1840                 1845

att tta  agt act aag act gtg  agt tct gta tta ggc  agt cgt ggg        6008
Ile Leu  Ser Thr Lys Thr Val  Ser Ser Val Leu Gly  Ser Arg Gly
    1850                 1855                 1860

ctt att  tct agt ctt aac caa  tct gag caa aat aaa  atc tat ggt        6053
Leu Ile  Ser Ser Leu Asn Gln  Ser Glu Gln Asn Lys  Ile Tyr Gly
    1865                 1870                 1875

gct att  gat aaa gag tta cat  ttc tca ggg gga aaa  gtc att gcc        6098
Ala Ile  Asp Lys Glu Leu His  Phe Ser Gly Gly Lys  Val Ile Ala
    1880                 1885                 1890

tcc att  gcc aac ggg gtt tat  ggt aac cac acc ctt  ttg agc ctc        6143
Ser Ile  Ala Asn Gly Val Tyr  Gly Asn His Thr Leu  Leu Ser Leu
    1895                 1900                 1905

att aac  tcc ctc tct cct atg  acg ggt aaa aat gtg  gac aat att        6188
Ile Asn  Ser Leu Ser Pro Met  Thr Gly Lys Asn Val  Asp Asn Ile
    1910                 1915                 1920

tta aac  atg cca agc acc acg  agc gga caa agc cag  atc aaa agt        6233
Leu Asn  Met Pro Ser Thr Thr  Ser Gly Gln Ser Gln  Ile Lys Ser
    1925                 1930                 1935

ctt tta  gag caa acc aca ttt  gag cag gtt ttt caa  gaa ctg ctc        6278
Leu Leu  Glu Gln Thr Thr Phe  Glu Gln Val Phe Gln  Glu Leu Leu
    1940                 1945                 1950

tct aat  caa aac cta ctc aat  aaa acc att gct tgg  tta ggc cca        6323
Ser Asn  Gln Asn Leu Leu Asn  Lys Thr Ile Ala Trp  Leu Gly Pro
    1955                 1960                 1965

caa att  tta gat gaa atg ctt  aaa act gct att gat  gat tta ctc        6368
Gln Ile  Leu Asp Glu Met Leu  Lys Thr Ala Ile Asp  Asp Leu Leu
    1970                 1975                 1980

aac cct  aca aat cag ctt act  gcc gct gaa aaa aat  att ctt gat        6413
Asn Pro  Thr Asn Gln Leu Thr  Ala Ala Glu Lys Asn  Ile Leu Asp
    1985                 1990                 1995

aac att  ctt aac aat gtc ttt  agc tca gaa aaa aag  gca gtc caa        6458
Asn Ile  Leu Asn Asn Val Phe  Ser Ser Glu Lys Lys  Ala Val Gln
    2000                 2005                 2010
```

```
caa atg gaa aat tct aac cct gat gtc aaa cac gtt att gat ggc      6503
Gln Met Glu Asn Ser Asn Pro Asp Val Lys His Val Ile Asp Gly
    2015                2020                2025

tta atg cag gct aaa ggt cta ggg gag gtt tat agc aag ggc ctt      6548
Leu Met Gln Ala Lys Gly Leu Gly Glu Val Tyr Ser Lys Gly Leu
    2030                2035                2040

cag agt att tta tct aat aaa ctg caa ggt caa tta aaa agc atg      6593
Gln Ser Ile Leu Ser Asn Lys Leu Gln Gly Gln Leu Lys Ser Met
    2045                2050                2055

ggc tta ggt tct ttg ctc gcg cct aaa gct cta ggt aat ttc tgg      6638
Gly Leu Gly Ser Leu Leu Ala Pro Lys Ala Leu Gly Asn Phe Trp
    2060                2065                2070

cag aag ggt tat ttt aac ttc cta gcc aat gat gat att tta gtg      6683
Gln Lys Gly Tyr Phe Asn Phe Leu Ala Asn Asp Asp Ile Leu Val
    2075                2080                2085

aac aat agc act ttt agt aat gct tca ggc ggg act tta agt ttt      6728
Asn Asn Ser Thr Phe Ser Asn Ala Ser Gly Gly Thr Leu Ser Phe
    2090                2095                2100

ata gcc ggt aag tct att att ttt gca ggg caa aat aca att aat      6773
Ile Ala Gly Lys Ser Ile Ile Phe Ala Gly Gln Asn Thr Ile Asn
    2105                2110                2115

ttt agt aat aat caa ggt aca cta gaa ttt tta agt aat gat gtg      6818
Phe Ser Asn Asn Gln Gly Thr Leu Glu Phe Leu Ser Asn Asp Val
    2120                2125                2130

tct aat att gat ctg act act ctt aat gcc act gac ggt tta acc      6863
Ser Asn Ile Asp Leu Thr Thr Leu Asn Ala Thr Asp Gly Leu Thr
    2135                2140                2145

att gat gcc ccc ttt aat aat ctt tat gtt cag aaa ggc aat atc      6908
Ile Asp Ala Pro Phe Asn Asn Leu Tyr Val Gln Lys Gly Asn Ile
    2150                2155                2160

acc ctt aat caa tat gag agt ttt agc gtg cag gcg ggc aat ttt      6953
Thr Leu Asn Gln Tyr Glu Ser Phe Ser Val Gln Ala Gly Asn Phe
    2165                2170                2175

gac ttt tta ggc acg gtg caa gca gac ggg gct gtg gat tta tcc      6998
Asp Phe Leu Gly Thr Val Gln Ala Asp Gly Ala Val Asp Leu Ser
    2180                2185                2190

ggt gta acc ggt tta gct gtt tta ggt act ctt aat ctt act gag      7043
Gly Val Thr Gly Leu Ala Val Leu Gly Thr Leu Asn Leu Thr Glu
    2195                2200                2205

gat agc acc ctt aag gct aat aat tta acc aca ata agc gcc ttt      7088
Asp Ser Thr Leu Lys Ala Asn Asn Leu Thr Thr Ile Ser Ala Phe
    2210                2215                2220

aac aac caa tcc caa aac ctg ctt aat atc agc ggg aat ttt aac      7133
Asn Asn Gln Ser Gln Asn Leu Leu Asn Ile Ser Gly Asn Phe Asn
    2225                2230                2235

tcc tat ggc aca ttt agt aca caa ggg gct ggg ata aat atc gga      7178
Ser Tyr Gly Thr Phe Ser Thr Gln Gly Ala Gly Ile Asn Ile Gly
```

```
    2240                2245                2250

ggg ggg  ttt aat agc ata ggg  gct tta act ttt aat  gta gcg ggt         7223
Gly Gly  Phe Asn Ser Ile Gly  Ala Leu Thr Phe Asn  Val Ala Gly
    2255                2260                2265

gca aca  gtc aaa act aca ggc  cct agc tca gat agt  tca agt tca         7268
Ala Thr  Val Lys Thr Thr Gly  Pro Ser Ser Asp Ser  Ser Ser Ser
    2270                2275                2280

agc act  agc tcc tct aca agt  tct agt aat tct aat  agc tct gga         7313
Ser Thr  Ser Ser Ser Thr Ser  Ser Ser Asn Ser Asn  Ser Ser Gly
    2285                2290                2295

ggc tct  agc tcc tcc tct agt  act tca gac tct act  agt tct agc         7358
Gly Ser  Ser Ser Ser Ser Ser  Thr Ser Asp Ser Thr  Ser Ser Ser
    2300                2305                2310

gcg cct  act agt tct aca aca  gct aca gct tta act  cta gct agc         7403
Ala Pro  Thr Ser Ser Thr Thr  Ala Thr Ala Leu Thr  Leu Ala Ser
    2315                2320                2325

acc acc  gtg tct aca act tct  act aca aat tct agc  agt gat aca         7448
Thr Thr  Val Ser Thr Thr Ser  Thr Thr Asn Ser Ser  Ser Asp Thr
    2330                2335                2340

agt aat  agc tca agc tct aat  agc tcc tct tct aat  agt gtg tct         7493
Ser Asn  Ser Ser Ser Ser Asn  Ser Ser Ser Ser Asn  Ser Val Ser
    2345                2350                2355

aat gca  att ccg gtt agc ccc  act cct agt aca caa  att ccg ctt         7538
Asn Ala  Ile Pro Val Ser Pro  Thr Pro Ser Thr Gln  Ile Pro Leu
    2360                2365                2370

atc caa  gta ggt gga ctt gtg  aat tta aat tta ggt  ggt agt gcg         7583
Ile Gln  Val Gly Gly Leu Val  Asn Leu Asn Leu Gly  Gly Ser Ala
    2375                2380                2385

att att  agc ttt aat aca gag  gca caa aca aat agc  acg gga act         7628
Ile Ile  Ser Phe Asn Thr Glu  Ala Gln Thr Asn Ser  Thr Gly Thr
    2390                2395                2400

aca gga  act aca caa ggc aca  gca aac aca ggc agt  acc gga tca         7673
Thr Gly  Thr Thr Gln Gly Thr  Ala Asn Thr Gly Ser  Thr Gly Ser
    2405                2410                2415

agc aca  aac tct agc tct aca  agc aca tca caa aca  aca tct agt         7718
Ser Thr  Asn Ser Ser Ser Thr  Ser Thr Ser Gln Thr  Thr Ser Ser
    2420                2425                2430

agt tct  act agc tcc aca caa  aca aca agc cta agc  tct aat act         7763
Ser Ser  Thr Ser Ser Thr Gln  Thr Thr Ser Leu Ser  Ser Asn Thr
    2435                2440                2445

agt cta  gct act acc agc caa  acg cct aca act tct  agc ggg gct         7808
Ser Leu  Ala Thr Thr Ser Gln  Thr Pro Thr Thr Ser  Ser Gly Ala
    2450                2455                2460

tct ccg  gat agt tca aac cct  aca gcc tct cct ata  acc cct tca         7853
Ser Pro  Asp Ser Ser Asn Pro  Thr Ala Ser Pro Ile  Thr Pro Ser
    2465                2470                2475

aat ggc  gct tat acg cta att  caa act aat agc tgg  att cat tac         7898
```

```
Asn Gly  Ala Tyr Thr Leu Ile  Gln Thr Asn Ser Trp  Ile His Tyr
    2480                 2485                 2490

aac ccc  acc tcc ttt aat cca  aac aac tgg aga caa  tat tta gag       7943
Asn Pro  Thr Ser Phe Asn Pro  Asn Asn Trp Arg Gln  Tyr Leu Glu
    2495                 2500                 2505

ctc tac  acc agt ctt aaa atc  aac ggg aca gct ttc  cag ctt aac       7988
Leu Tyr  Thr Ser Leu Lys Ile  Asn Gly Thr Ala Phe  Gln Leu Asn
    2510                 2515                 2520

gct caa  ggt aca ggt ctt act  tat aat ggt caa gca  gtc aat atc       8033
Ala Gln  Gly Thr Gly Leu Thr  Tyr Asn Gly Gln Ala  Val Asn Ile
    2525                 2530                 2535

tca caa  cga ggc ttg ctt gtc  aat tat caa ggc aca  aat ggc caa       8078
Ser Gln  Arg Gly Leu Leu Val  Asn Tyr Gln Gly Thr  Asn Gly Gln
    2540                 2545                 2550

gaa gtg  agc gcc tct att gat  tat aat aag atg caa  ata ggc ata       8123
Glu Val  Ser Ala Ser Ile Asp  Tyr Asn Lys Met Gln  Ile Gly Ile
    2555                 2560                 2565

ggc caa  agc ttg cat gtt atc  gcg ccc act att aca  caa tac atc       8168
Gly Gln  Ser Leu His Val Ile  Ala Pro Thr Ile Thr  Gln Tyr Ile
    2570                 2575                 2580

acc caa  ata caa ggg cag tct  gtg gtt aat gcg cta  gaa aat gca       8213
Thr Gln  Ile Gln Gly Gln Ser  Val Val Asn Ala Leu  Glu Asn Ala
    2585                 2590                 2595

ggc ggg  ccg ggt gtg atg aat  tgg ttt ggt aag ctt  tta att gag       8258
Gly Gly  Pro Gly Val Met Asn  Trp Phe Gly Lys Leu  Leu Ile Glu
    2600                 2605                 2610

aca aaa  aac acc ccg ctt ttt  gcg ccc tac tat ctt  gaa caa cac       8303
Thr Lys  Asn Thr Pro Leu Phe  Ala Pro Tyr Tyr Leu  Glu Gln His
    2615                 2620                 2625

tcc tta  agc gat cta ctt aaa  att gta aaa gat att  caa aat gcg       8348
Ser Leu  Ser Asp Leu Leu Lys  Ile Val Lys Asp Ile  Gln Asn Ala
    2630                 2635                 2640

act gat  tgg atg ggc gct tct  ggt tta aaa gcc act  agc tct aaa       8393
Thr Asp  Trp Met Gly Ala Ser  Gly Leu Lys Ala Thr  Ser Ser Lys
    2645                 2650                 2655

ttg cta  caa atc agt gta cac  acc aaa cag atg agc  cgt tta gct       8438
Leu Leu  Gln Ile Ser Val His  Thr Lys Gln Met Ser  Arg Leu Ala
    2660                 2665                 2670

aag ctt  tca aat ttt gct tct  aat gat gca tta cca  gat ttt cat       8483
Lys Leu  Ser Asn Phe Ala Ser  Asn Asp Ala Leu Pro  Asp Phe His
    2675                 2680                 2685

gat ttt  tta gtc agt ctt aaa  ggt aaa aaa ttt gct  agc gcg gtg       8528
Asp Phe  Leu Val Ser Leu Lys  Gly Lys Lys Phe Ala  Ser Ala Val
    2690                 2695                 2700

cct aat  gct atg gat att atc  acc gcc tat tct caa  aga gat aaa       8573
Pro Asn  Ala Met Asp Ile Ile  Thr Ala Tyr Ser Gln  Arg Asp Lys
    2705                 2710                 2715
```

```
tta aaa  aat aac cta tgg ata  acc ggt gtg gga gga  gca agt ttt       8618
Leu Lys  Asn Asn Leu Trp Ile  Thr Gly Val Gly Gly  Ala Ser Phe
    2720                 2725                 2730

gta gca  gga ggc aca ggt acg  ctt tat ggg ctg aat  gtg ggt tat       8663
Val Ala  Gly Gly Thr Gly Thr  Leu Tyr Gly Leu Asn  Val Gly Tyr
    2735                 2740                 2745

gat cgc  ttt ata aag ggc gtg  att gtg ggg ggt tat  atg gct tat       8708
Asp Arg  Phe Ile Lys Gly Val  Ile Val Gly Gly Tyr  Met Ala Tyr
    2750                 2755                 2760

ggt tat  agt ggc ttt tat ggc  aat atc aat agc gct  aat tct aat       8753
Gly Tyr  Ser Gly Phe Tyr Gly  Asn Ile Asn Ser Ala  Asn Ser Asn
    2765                 2770                 2775

aat gtc  aat gtg gga ttt tat  agc cgc gcc ttt atc  aag ggt aga       8798
Asn Val  Asn Val Gly Phe Tyr  Ser Arg Ala Phe Ile  Lys Gly Arg
    2780                 2785                 2790

aac gag  att aca gga agt att  aat gaa acc tat ggc  tat aac aaa       8843
Asn Glu  Ile Thr Gly Ser Ile  Asn Glu Thr Tyr Gly  Tyr Asn Lys
    2795                 2800                 2805

acc tac  att gat gca act aat  ccc att ctc acc cct  ctt aac cag       8888
Thr Tyr  Ile Asp Ala Thr Asn  Pro Ile Leu Thr Pro  Leu Asn Gln
    2810                 2815                 2820

caa tac  cac tat ggc act tgg  act acc aat gtc ggc  gct aac tat       8933
Gln Tyr  His Tyr Gly Thr Trp  Thr Thr Asn Val Gly  Ala Asn Tyr
    2825                 2830                 2835

ggc tat  gac ttc ttt ttt aaa  aac aaa cat gtt att  ctc aaa ccc       8978
Gly Tyr  Asp Phe Phe Phe Lys  Asn Lys His Val Ile  Leu Lys Pro
    2840                 2845                 2850

caa att  ggc ctc act tat tat  tat atc ggc ctc tca  ggc ttg caa       9023
Gln Ile  Gly Leu Thr Tyr Tyr  Tyr Ile Gly Leu Ser  Gly Leu Gln
    2855                 2860                 2865

ggc aag  atg aat gat ccg att  tat aac gag ttt aga  gcc aat gcc       9068
Gly Lys  Met Asn Asp Pro Ile  Tyr Asn Glu Phe Arg  Ala Asn Ala
    2870                 2875                 2880

gat cca  gcg cat aaa tct gtt  ttg acg atc aat tta  gcc cta gag       9113
Asp Pro  Ala His Lys Ser Val  Leu Thr Ile Asn Leu  Ala Leu Glu
    2885                 2890                 2895

agt cgc  cac tat ttt agg aaa  aac tcc tat tac tat  gtc att gcc       9158
Ser Arg  His Tyr Phe Arg Lys  Asn Ser Tyr Tyr Tyr  Val Ile Ala
    2900                 2905                 2910

ggc tta  ggg cgg gat tta ttc  gtc cat tct atg ggt  gat aaa ata       9203
Gly Leu  Gly Arg Asp Leu Phe  Val His Ser Met Gly  Asp Lys Ile
    2915                 2920                 2925

gta cgc  ttt att ggt aat gat  atg tta agt tat cgt  tat ggg gga       9248
Val Arg  Phe Ile Gly Asn Asp  Met Leu Ser Tyr Arg  Tyr Gly Gly
    2930                 2935                 2940

atg tat  aat acc ttt gct agc  ctc acc aca ggg ggt  gag gtt cgc       9293
Met Tyr  Asn Thr Phe Ala Ser  Leu Thr Thr Gly Gly  Glu Val Arg
    2945                 2950                 2955
```

```
tta ttt  cga tcc ttt tat gtc  aat gct ggt att gga  gcg cgc ttt      9338
Leu Phe  Arg Ser Phe Tyr Val  Asn Ala Gly Ile Gly  Ala Arg Phe
    2960                 2965                 2970

ggt ttg  gat tat caa gat att  aat att aca ggc aat  gtc ggg atg      9383
Gly Leu  Asp Tyr Gln Asp Ile  Asn Ile Thr Gly Asn  Val Gly Met
    2975                 2980                 2985

cgc tat  gct ttt tag acaaatttt aattctgtgt tgctgtctca ctttatttta     9438
Arg Tyr  Ala Phe
    2990

aaattctttt taagctctgt ttaagttttt gcgtgctact atccaaatcc ccctcctttt   9498

tagtctctgg agtttctcca gggactttcc ttgaataatg ctaaggcttt ctttaagacg   9558

ccaagaacct atttgcgcg tacatgtttt acgcaagcca accctttctt ctgaactctt    9618

gcttacttta gaacaactat taaaagaagc caaaattact tatgatttta aactccctga   9678

aatgtccttg tctcaagaag ttttattgtg ttttggagga gatggcacgc ttctagcggc   9738

cctgcgccac ccttcaaata gcctatgttt tggaatccat gtcgggcatt tggggttttt   9798

aacagccact aatttagaag gcgctcccca ttttttagaa gcgc                    9842


<210>  2
<211>  2992
<212>  PRT
<213>  Helicobacter suis

<400>  2

Met Lys Lys Phe Ser Ser Leu Thr Leu Lys Phe Gly His Ala Leu Ala
1               5                   10                  15

Arg Arg Ile Lys Ala Asn Gln Ala Arg Arg Ala Gly Thr Tyr Val Phe
            20                  25                  30

Lys Lys Gln Leu Pro Gln Pro Lys Arg Ser Glu Leu Lys Pro Lys Leu
        35                  40                  45

Ile Lys Gln Gly Thr Phe Ile Leu Gly Val Met Ser Gln Pro Leu Leu
    50                  55                  60

Ala Trp Tyr Pro Ser Trp Ile Ser Gly Thr His Thr Leu Asn Thr Thr
65                  70                  75                  80

Asn Ile Lys Gln Tyr Met Gln Asn Asn His Arg Ser Gln Asn Trp Leu
                85                  90                  95

Trp Thr Gly Gly Ser Asn Val Phe Tyr Glu Ala Ser Asn Gly Ser Tyr
            100                 105                 110
```

```
Phe Cys Thr Asn Trp Asn Cys Asn Gly Ser Val Thr Leu Ile Gly Ser
        115                 120                 125

Gly Ser Thr Thr Tyr Thr Leu Ser Asn Leu Asn Tyr Glu Gly Gly Ser
    130                 135                 140

Leu Asn Leu Gln Ile Asn Gly Asn Gly Thr Gln Gly Ala Leu Asn Ile
145                 150                 155                 160

Ser Asn Val Asn Met Asp Ser Tyr Gln Gly Arg Gln Phe Thr Asp Thr
                165                 170                 175

Trp Asn Ala Gln Ser Val Ser Ile Ser Gly Asn Leu Gln Val Gly Gln
            180                 185                 190

Asn His Ile Thr Ile Asn Ala Asn His Gly Thr Thr Ala Asn Asn Ala
        195                 200                 205

Thr Ile Asn Ala Asp Gln Gly Asn Gly Ile Leu Asn Ile Asn Asp Lys
    210                 215                 220

Thr Ser Glu Ser Phe Thr Asn Thr Thr Phe Lys Gly Thr Gly Gln Ile
225                 230                 235                 240

Asn Leu Gln Ser Asn Asn Ile Thr Phe Asn Asn Val Thr Phe Asn Asp
                245                 250                 255

Ser Asn Thr Gly Ser His Val Thr Asp Asn Gly Thr Leu Thr Leu Glu
            260                 265                 270

Gly Thr Glu Thr Phe Ala Gln Asn Ser Pro Leu Ile Asn Leu Gly Ala
        275                 280                 285

Asn Val Thr Ile Gln Ala Asn Thr Ile Phe Asn Ile Thr Glu Asp Leu
    290                 295                 300

Thr Lys Thr Thr Asn Gly Ala Tyr Asn Leu Asp Thr Leu Val Ser Thr
305                 310                 315                 320

Ser Gly Asn Lys Ser Ile Asn Asp Ser Ser Tyr Ala Ser His Leu Trp
                325                 330                 335

Asp Leu Ile Arg Tyr Gln Gly Gln Thr Gly Ser Leu Phe Asn Gly Gln
            340                 345                 350

Leu Ser Asn Gly Thr Thr Leu Ser Asn Pro Ser Ser Gly Asn Gly Ile
        355                 360                 365
```

Tyr Tyr Val Lys Tyr Thr Phe Gly Asn Gly Asp Trp Asp Ile Leu Lys
370 375 380

Glu Asp Phe Glu Asn Asn Ser Leu Ser Ala Gln Leu Glu Ala Tyr Ala
385 390 395 400

Lys Asn Gln Gly Asp Ile Trp Ser Thr Ile His Asn Ile Asn Ser Thr
405 410 415

Trp Asn Phe Asn Val Gly Glu Gly Ser Ala Tyr Ile Asn Pro Gly Asn
420 425 430

Gly Thr Asp Gln Ala Trp Ala Gln Ser Asp Lys Asn Phe Lys Val Thr
435 440 445

Leu Asp Asn Gly Ser Gly Gly Thr Leu Ile Leu Gly Asn Ser Thr Glu
450 455 460

Thr Pro Ser Ser Ser Gly Lys Ile Leu Phe Gly Gly Thr Gly Gly Asn
465 470 475 480

Pro Phe Ala Leu Asn Gly Asn Tyr Gly Gly Asp Leu Gly Tyr Met Thr
485 490 495

Gly Glu Phe Asp Ala Gly Lys Ile Tyr Leu Thr Gly Thr Ile Glu Ser
500 505 510

Gly Asn Ser Phe His Asp Gly Asn Gly Thr Asn Ile Ser Tyr Asn Ala
515 520 525

Ile Thr Asn Ile Thr Ala Asn Gly Leu His Tyr Leu Asn Asp Asn Ala
530 535 540

Gly Ala Trp His Ser Asn Ala Asn Phe Arg Ala Thr Asn Gly Ser Ile
545 550 555 560

Asn Ile Ser Asn Ser Gln Phe Gln Asp Gln Ser Ser Gly Gln Phe Thr
565 570 575

Phe Asn Ser Gln Asn Gln Thr Phe Ser Ser Thr Thr Phe Thr Gly Asn
580 585 590

Ser His Thr Ile Thr Leu His Ala Thr Asn Asn Leu Thr Leu Thr Asn
595 600 605

Thr Ser Phe Asn Asp Ser Asn Ala Ser Leu Thr Leu Glu Ala Asp Lys
610 615 620

Gly Ser Leu Arg Asp Thr Asp Asn Gln Thr Ser Gln Ile Thr Ala Lys
625                 630                 635                 640

Asn Leu Gln Val Ile Ala Asn Gln Ala Ser Phe Ser Asn Thr Ile Phe
                645                 650                 655

Asn Ala Gln Asn Ser Ser Phe Lys Thr Asn Gln Leu Thr Leu Thr Asn
            660                 665                 670

Asp Thr Phe Asn Asn Gly Ser Tyr Ser Phe Ala Pro Glu Asn Asn Gly
        675                 680                 685

Asn His Thr Thr Thr Phe Gly Gly Thr Thr Thr Ile Asn Thr Ser Ser
    690                 695                 700

Ser Pro Phe Ala Asn Leu Gly Gly Ser Ile Ser Leu Asn Asn Gly Ala
705                 710                 715                 720

Ile Phe Asn Leu Asn Asn Ile Leu Ser Ser Leu Gln Ile Gly Thr Thr
                725                 730                 735

Tyr Asn Ile Leu Gly Gly Ser Gly Ala Asn Ile Leu Tyr Lys Asn Asp
            740                 745                 750

Thr Gln Tyr Ala Ser Ala Leu Trp Gln Leu Ile Lys Ile Asn Gly Thr
        755                 760                 765

Thr Ile Asn Ser Glu Thr Glu Ile Ser Asp Asn Asn Asp Thr Gln Val
    770                 775                 780

Trp Asn Val Val Phe Asn Ile Asp Gly Met Pro Ile Lys Ile Gln Glu
785                 790                 795                 800

Thr Phe Ala Ser Ser Gly Leu Ser Phe Lys Val Ile Ser Gln Ala Lys
                805                 810                 815

Asp Ile Trp Ser Asp Val Tyr His Met Thr Thr Asn Cys Ala Tyr Asn
            820                 825                 830

Ala Leu Ala Ser Pro Pro Gly Cys Tyr Gly Tyr Gln Asn Gly Thr His
        835                 840                 845

Asn Ile Leu Lys His Phe Asn Lys Tyr Gly Phe Glu Ala Tyr Asn Glu
    850                 855                 860

Ser Val Gly Ala Asp Gly Ile Ala Tyr Ile Asn Pro Leu Asn Ser Gln

865 870 875 880

Ser His Asp Gly Tyr Asn Ala Ser Thr His Thr Leu Gln Thr Tyr Thr
885 890 895

Lys Ile Gly Asn Gly Gly Thr Tyr Asn Val Gly Glu Met Gln Asn Gly
900 905 910

Lys Trp Val Asn Asn Gly Thr Leu Ile Leu Gly Asn Asn Thr Ser Gln
915 920 925

Pro Val Lys Gly Gly Lys Ile Glu Phe Gly Lys Val Asp Ser Val Gly
930 935 940

Tyr Ile Thr Asp Val Phe Asn Ala Gly His Ile Phe Leu Thr Asn Ser
945 950 955 960

Ile Glu Val Gly Asp Ser Ser Leu Ser Gly Ala Gly Ala Thr Leu Thr
965 970 975

Phe Asn Ala Asn Asn Asn Ile Thr Ala Asp Gly Leu Thr Tyr His Gln
980 985 990

Asp Ala Thr Ala Ile Pro Pro Phe Gly Ser Ala Ile Ser Gln His Ser
995 1000 1005

Tyr Gly Asn Phe Ile Ala Gln Gln Ser Phe Ser Ala Leu Asn Ser
1010 1015 1020

Ser Phe Glu Asp Asp Thr Ser Gly Ser Leu Asp Phe Thr Gly Lys
1025 1030 1035

Asn Ser Ile Asn Phe Thr Ser Ser Thr Val Ile Gly Ser Lys Ser
1040 1045 1050

Ser Ile Thr Leu Asn Ser Ala Asn Thr Thr Leu Asn Asn Ser Ala
1055 1060 1065

Phe Phe Val Gly Asn Gly Thr Thr Leu Thr Phe Gly Asn Val Ile
1070 1075 1080

Thr Thr Asn Ser His Ser Ser Tyr Ser Ser Ser Thr Asn Thr Ile
1085 1090 1095

Asn Asn Ser Thr Ile Asn Leu Asn Gln Asn Ser Ser Leu Tyr Leu
1100 1105 1110

His Gly Ser Thr Thr Leu Asp Asn Thr Thr Ser Phe Leu Asn Leu
1115 1120 1125

Ser Ser Gln Ala Thr Phe Tyr Asp Ser Ala Ser Leu Ser Gly Ser
1130 1135 1140

Thr Thr Ile Asn Leu Asp His Asn Ser Lys Ile Thr Met Asn Ser
1145 1150 1155

Thr Thr Thr Leu Asn Asp Asn Ala Asn Phe Asn Leu Ile Asn Ser
1160 1165 1170

Ala Gln Ala Asn Phe Gln Gly Asn Ser Thr Phe Asn Asn Asn Ser
1175 1180 1185

Gly Ile Thr Leu Ala Ser Gly Ala Lys Ala Met Phe Thr His Thr
1190 1195 1200

Thr Thr Ser Thr Gln Asp Ile Thr Thr Phe Asn Asn Asn Ser Phe
1205 1210 1215

Leu Asn Val Asn Gly Ser Phe Thr Asp Phe Ile Pro Ser Thr Asn
1220 1225 1230

Ser Asn Arg Thr Ile Ser Gly Gly Gly Ser Ser Ser Asn Gln Asn
1235 1240 1245

Tyr Ser Ala Gln Phe Asn Asp Leu Val Phe Asn Asn Tyr Ala Ser
1250 1255 1260

Met Leu Leu Asn Ser Ala Asp Ile Gln Ser Ser Gln Thr Thr Phe
1265 1270 1275

Ala Asn Ser Val Asn Ile Asn Met Gln Asn Ser Leu Phe Asn Ala
1280 1285 1290

Ser Thr Leu Asn Leu Asn Gly Gly Asn Phe Tyr Met Gln Asn Ser
1295 1300 1305

Gln Ile Lys Ala Gly Ala Val Ser Val Gly Ser Ser Asp Ser Val
1310 1315 1320

Asn Ile Asn Ser Gly Val Asn Ser Leu Asn Arg Ser Leu Ile Thr
1325 1330 1335

Ser Ser Ser Phe Asn Leu Asn Gly Thr Leu Asn Leu Asp Gly Leu
1340 1345 1350

Leu Leu Gly Pro Thr Thr Thr Gly Thr Thr Lys Ser Thr Ala Asn
1355 1360 1365

Gly Gln Pro Leu Ile Ser Val Gly Gly Gly Thr Phe Ser Leu Asn
1370 1375 1380

Gly Ile Leu Asn Ile Ser Asn Ile Asp Leu Ser Ala His Leu Ser
1385 1390 1395

Ser Gln Thr Asn His Thr Ser Ser Ser Ala Thr Tyr Asp Ile Val
1400 1405 1410

Asn Ala Asn Asn Ile Thr Gly Met Ser Gly Ala Asn Gly Tyr Gln
1415 1420 1425

Lys Ile Glu Tyr Tyr Gly Ile Lys Ile Asn Asn Ala Thr Tyr Ser
1430 1435 1440

Asp Ser Asn Ser Asp Ser Asn Lys Thr Gln Ser Trp Ser Phe Thr
1445 1450 1455

Asn Pro Leu Asn Gly Ala Gln Thr Ile Thr Glu Lys Ile Gln Ser
1460 1465 1470

Gly Lys Leu Thr Ile Thr Ile Ser Asn Ser Asn His Phe Val Ala
1475 1480 1485

Thr Asp Tyr His Asn Ile Ala Pro Glu Leu Phe Phe Tyr Lys Gln
1490 1495 1500

Ser Ala Gln Asn Leu Pro Ser Thr Asn Ser Ala Ser Ala Asp Ile
1505 1510 1515

Ser Ser Tyr Asp Tyr Ser Ser Asp Glu Ser Gly Thr Phe Phe Leu
1520 1525 1530

Asp Gly Asn Leu Lys Gly Val Tyr Tyr Pro Lys Ser Ser Thr Thr
1535 1540 1545

Gly Thr Thr Pro Val Ile Pro Gly Thr Tyr Asn Ala Gln Gly Gln
1550 1555 1560

Pro Leu Gln Gly Leu Tyr Ile Ser Asn Asn Gly Leu Phe Asn Glu
1565 1570 1575

Thr Thr Leu Asn Asn Leu Val Asp Ile Val Arg Ser Ile Trp Pro
1580 1585 1590

His Leu Lys Thr Leu Leu Pro Lys Ile Leu Gln Asp Leu Ser Asp
1595 1600 1605

Pro Ser Asn Ile Ile Gln Asp Leu Glu Asn Ser Gly Ile Lys Leu
1610 1615 1620

Thr Ser Gln Gln Ser Lys Glu Leu Leu Ser Phe Ile Asp Gly Leu
1625 1630 1635

Ser Ser Asn Ile Asn Gln Thr Phe Asn Asn Gly Thr Leu Val Val
1640 1645 1650

Gly Ser Pro Gln Ala Gly Gln Thr Gly Ser Ser Ser Val Val Trp
1655 1660 1665

Phe Gly Gly Asn Gly Tyr Thr Thr Ala Cys Thr Ala Ala Gln Thr
1670 1675 1680

Glu Lys Gly Cys Gln Asp Leu Arg Gly Thr Tyr Leu Gly Gln Leu
1685 1690 1695

Leu Gly Ser Thr Ser Ala Ala Leu Gly Tyr Ile Glu Ala Asn Phe
1700 1705 1710

Lys Ala Lys Asp Ile Tyr Ile Thr Gly Thr Val Gly Ser Gly Asn
1715 1720 1725

Ala Trp Gly Ile Gly Gly Ser Ala Asp Val Thr Phe Asn Ser Ala
1730 1735 1740

Thr Asn Leu Thr Leu Asn Gln Ala Thr Ile Asp Ala Glu Gly Thr
1745 1750 1755

Asp Gln Ile Phe Asn Met Leu Gly Glu Gly Gly Ile Gln Lys Ile
1760 1765 1770

Leu Gly Gln Lys Gly Leu Ala Gln Met Leu Gly Asn Tyr Ile Tyr
1775 1780 1785

Asp Leu Ala Asn Gly Ser Ser Ile Asn Leu Asn Pro Ile Ser Ser
1790 1795 1800

Ile Pro Ser Ser Ile Gln Pro Leu Ala Lys Asp Leu Gly Arg Gln
1805 1810 1815

Ile Gly Lys Ile Lys Leu Ser Asp Val Leu Ser Ala Ser Asp Val

1820 1825 1830

Ser Ala Leu Leu Asn Met Pro Gly Met Asp Asn Val Ile Lys Asn
1835 1840 1845

Ile Leu Ser Thr Lys Thr Val Ser Ser Val Leu Gly Ser Arg Gly
1850 1855 1860

Leu Ile Ser Ser Leu Asn Gln Ser Glu Gln Asn Lys Ile Tyr Gly
1865 1870 1875

Ala Ile Asp Lys Glu Leu His Phe Ser Gly Gly Lys Val Ile Ala
1880 1885 1890

Ser Ile Ala Asn Gly Val Tyr Gly Asn His Thr Leu Leu Ser Leu
1895 1900 1905

Ile Asn Ser Leu Ser Pro Met Thr Gly Lys Asn Val Asp Asn Ile
1910 1915 1920

Leu Asn Met Pro Ser Thr Thr Ser Gly Gln Ser Gln Ile Lys Ser
1925 1930 1935

Leu Leu Glu Gln Thr Thr Phe Glu Gln Val Phe Gln Glu Leu Leu
1940 1945 1950

Ser Asn Gln Asn Leu Leu Asn Lys Thr Ile Ala Trp Leu Gly Pro
1955 1960 1965

Gln Ile Leu Asp Glu Met Leu Lys Thr Ala Ile Asp Asp Leu Leu
1970 1975 1980

Asn Pro Thr Asn Gln Leu Thr Ala Ala Glu Lys Asn Ile Leu Asp
1985 1990 1995

Asn Ile Leu Asn Asn Val Phe Ser Ser Glu Lys Lys Ala Val Gln
2000 2005 2010

Gln Met Glu Asn Ser Asn Pro Asp Val Lys His Val Ile Asp Gly
2015 2020 2025

Leu Met Gln Ala Lys Gly Leu Gly Glu Val Tyr Ser Lys Gly Leu
2030 2035 2040

Gln Ser Ile Leu Ser Asn Lys Leu Gln Gly Gln Leu Lys Ser Met
2045 2050 2055

```
Gly Leu  Gly Ser Leu Leu Ala  Pro Lys Ala Leu Gly  Asn Phe Trp
    2060                 2065                 2070

Gln Lys  Gly Tyr Phe Asn Phe  Leu Ala Asn Asp Asp  Ile Leu Val
    2075                 2080                 2085

Asn Asn  Ser Thr Phe Ser Asn  Ala Ser Gly Gly Thr  Leu Ser Phe
    2090                 2095                 2100

Ile Ala  Gly Lys Ser Ile Ile  Phe Ala Gly Gln Asn  Thr Ile Asn
    2105                 2110                 2115

Phe Ser  Asn Asn Gln Gly Thr  Leu Glu Phe Leu Ser  Asn Asp Val
    2120                 2125                 2130

Ser Asn  Ile Asp Leu Thr Thr  Leu Asn Ala Thr Asp  Gly Leu Thr
    2135                 2140                 2145

Ile Asp  Ala Pro Phe Asn Asn  Leu Tyr Val Gln Lys  Gly Asn Ile
    2150                 2155                 2160

Thr Leu  Asn Gln Tyr Glu Ser  Phe Ser Val Gln Ala  Gly Asn Phe
    2165                 2170                 2175

Asp Phe  Leu Gly Thr Val Gln  Ala Asp Gly Ala Val  Asp Leu Ser
    2180                 2185                 2190

Gly Val  Thr Gly Leu Ala Val  Leu Gly Thr Leu Asn  Leu Thr Glu
    2195                 2200                 2205

Asp Ser  Thr Leu Lys Ala Asn  Asn Leu Thr Thr Ile  Ser Ala Phe
    2210                 2215                 2220

Asn Asn  Gln Ser Gln Asn Leu  Leu Asn Ile Ser Gly  Asn Phe Asn
    2225                 2230                 2235

Ser Tyr  Gly Thr Phe Ser Thr  Gln Gly Ala Gly Ile  Asn Ile Gly
    2240                 2245                 2250

Gly Gly  Phe Asn Ser Ile Gly  Ala Leu Thr Phe Asn  Val Ala Gly
    2255                 2260                 2265

Ala Thr  Val Lys Thr Thr Gly  Pro Ser Ser Asp Ser  Ser Ser Ser
    2270                 2275                 2280

Ser Thr  Ser Ser Ser Thr Ser  Ser Ser Asn Ser Asn  Ser Ser Gly
    2285                 2290                 2295
```

```
Gly Ser  Ser Ser Ser Ser Ser  Thr Ser Asp Ser Thr  Ser Ser Ser
    2300                 2305                 2310

Ala Pro  Thr Ser Ser Thr Thr  Ala Thr Ala Leu Thr  Leu Ala Ser
    2315                 2320                 2325

Thr Thr  Val Ser Thr Thr Ser  Thr Thr Asn Ser Ser  Ser Asp Thr
    2330                 2335                 2340

Ser Asn  Ser Ser Ser Ser Asn  Ser Ser Ser Ser Asn  Ser Val Ser
    2345                 2350                 2355

Asn Ala  Ile Pro Val Ser Pro  Thr Pro Ser Thr Gln  Ile Pro Leu
    2360                 2365                 2370

Ile Gln  Val Gly Gly Leu Val  Asn Leu Asn Leu Gly  Gly Ser Ala
    2375                 2380                 2385

Ile Ile  Ser Phe Asn Thr Glu  Ala Gln Thr Asn Ser  Thr Gly Thr
    2390                 2395                 2400

Thr Gly  Thr Thr Gln Gly Thr  Ala Asn Thr Gly Ser  Thr Gly Ser
    2405                 2410                 2415

Ser Thr  Asn Ser Ser Ser Thr  Ser Thr Ser Gln Thr  Thr Ser Ser
    2420                 2425                 2430

Ser Ser  Thr Ser Ser Thr Gln  Thr Thr Ser Leu Ser  Ser Asn Thr
    2435                 2440                 2445

Ser Leu  Ala Thr Thr Ser Gln  Thr Pro Thr Thr Ser  Ser Gly Ala
    2450                 2455                 2460

Ser Pro  Asp Ser Ser Asn Pro  Thr Ala Ser Pro Ile  Thr Pro Ser
    2465                 2470                 2475

Asn Gly  Ala Tyr Thr Leu Ile  Gln Thr Asn Ser Trp  Ile His Tyr
    2480                 2485                 2490

Asn Pro  Thr Ser Phe Asn Pro  Asn Asn Trp Arg Gln  Tyr Leu Glu
    2495                 2500                 2505

Leu Tyr  Thr Ser Leu Lys Ile  Asn Gly Thr Ala Phe  Gln Leu Asn
    2510                 2515                 2520

Ala Gln  Gly Thr Gly Leu Thr  Tyr Asn Gly Gln Ala  Val Asn Ile
    2525                 2530                 2535
```

Ser Gln Arg Gly Leu Leu Val Asn Tyr Gln Gly Thr Asn Gly Gln
2540 2545 2550

Glu Val Ser Ala Ser Ile Asp Tyr Asn Lys Met Gln Ile Gly Ile
2555 2560 2565

Gly Gln Ser Leu His Val Ile Ala Pro Thr Ile Thr Gln Tyr Ile
2570 2575 2580

Thr Gln Ile Gln Gly Gln Ser Val Val Asn Ala Leu Glu Asn Ala
2585 2590 2595

Gly Gly Pro Gly Val Met Asn Trp Phe Gly Lys Leu Leu Ile Glu
2600 2605 2610

Thr Lys Asn Thr Pro Leu Phe Ala Pro Tyr Tyr Leu Glu Gln His
2615 2620 2625

Ser Leu Ser Asp Leu Leu Lys Ile Val Lys Asp Ile Gln Asn Ala
2630 2635 2640

Thr Asp Trp Met Gly Ala Ser Gly Leu Lys Ala Thr Ser Ser Lys
2645 2650 2655

Leu Leu Gln Ile Ser Val His Thr Lys Gln Met Ser Arg Leu Ala
2660 2665 2670

Lys Leu Ser Asn Phe Ala Ser Asn Asp Ala Leu Pro Asp Phe His
2675 2680 2685

Asp Phe Leu Val Ser Leu Lys Gly Lys Lys Phe Ala Ser Ala Val
2690 2695 2700

Pro Asn Ala Met Asp Ile Ile Thr Ala Tyr Ser Gln Arg Asp Lys
2705 2710 2715

Leu Lys Asn Asn Leu Trp Ile Thr Gly Val Gly Gly Ala Ser Phe
2720 2725 2730

Val Ala Gly Gly Thr Gly Thr Leu Tyr Gly Leu Asn Val Gly Tyr
2735 2740 2745

Asp Arg Phe Ile Lys Gly Val Ile Val Gly Gly Tyr Met Ala Tyr
2750 2755 2760

Gly Tyr Ser Gly Phe Tyr Gly Asn Ile Asn Ser Ala Asn Ser Asn

```
        2765                2770                2775

Asn Val  Asn Val Gly Phe Tyr  Ser Arg Ala Phe Ile  Lys Gly Arg
    2780                2785                2790

Asn Glu  Ile Thr Gly Ser Ile  Asn Glu Thr Tyr Gly  Tyr Asn Lys
    2795                2800                2805

Thr Tyr  Ile Asp Ala Thr Asn  Pro Ile Leu Thr Pro  Leu Asn Gln
    2810                2815                2820

Gln Tyr  His Tyr Gly Thr Trp  Thr Thr Asn Val Gly  Ala Asn Tyr
    2825                2830                2835

Gly Tyr  Asp Phe Phe Phe Lys  Asn Lys His Val Ile  Leu Lys Pro
    2840                2845                2850

Gln Ile  Gly Leu Thr Tyr Tyr  Tyr Ile Gly Leu Ser  Gly Leu Gln
    2855                2860                2865

Gly Lys  Met Asn Asp Pro Ile  Tyr Asn Glu Phe Arg  Ala Asn Ala
    2870                2875                2880

Asp Pro  Ala His Lys Ser Val  Leu Thr Ile Asn Leu  Ala Leu Glu
    2885                2890                2895

Ser Arg  His Tyr Phe Arg Lys  Asn Ser Tyr Tyr Tyr  Val Ile Ala
    2900                2905                2910

Gly Leu  Gly Arg Asp Leu Phe  Val His Ser Met Gly  Asp Lys Ile
    2915                2920                2925

Val Arg  Phe Ile Gly Asn Asp  Met Leu Ser Tyr Arg  Tyr Gly Gly
    2930                2935                2940

Met Tyr  Asn Thr Phe Ala Ser  Leu Thr Thr Gly Gly  Glu Val Arg
    2945                2950                2955

Leu Phe  Arg Ser Phe Tyr Val  Asn Ala Gly Ile Gly  Ala Arg Phe
    2960                2965                2970

Gly Leu  Asp Tyr Gln Asp Ile  Asn Ile Thr Gly Asn  Val Gly Met
    2975                2980                2985

Arg Tyr  Ala Phe
    2990
```

<210> 3
<211> 14
<212> PRT
<213> Artificial Sequence

<220>
<223> antigen peptide No.11(SNTW101, HS1, HS5)

<400> 3

Glu Lys Lys Ala Val Gln Gln Met Glu Asn Ser Asn Pro Asp
1 5 10

<210> 4
<211> 14
<212> PRT
<213> Artificial Sequence

<220>
<223> antigen peptide No.11(TKY)

<400> 4

Glu Lys Lys Ala Val Glu Gln Met Glu Asn Ser Asn Pro Asp
1 5 10

<210> 5
<211> 14
<212> PRT
<213> Artificial Sequence

<220>
<223> antigen peptide No.11 (SH8)

<400> 5

Glu Lys Asp Ala Val Thr Ser Leu Lys Asn Ser Asn Ser Gly
1 5 10

<210> 6
<211> 14
<212> PRT
<213> Artificial Sequence

<220>
<223> antigen peptide No.11 (SH10)

<400> 6

Glu Lys Asp Ala Val Thr Ser Leu Glu Asn Ser Asn Ser Gly
1 5 10

<210> 7
<211> 14
<212> PRT
<213> Artificial Sequence

<220>
<223> antigen peptide No.19 (SNTW101, HS1, HS5, SH8)

<400> 7

Asn Gln Gly Thr Leu Glu Phe Leu Ser Asn Asp Val Ser Asn
1               5                   10

<210> 8
<211> 14
<212> PRT
<213> Artificial Sequence

<220>
<223> antigen peptide No.19 (TKY, SH10)

<400> 8

Asn Gln Gly Thr Leu Glu Phe Leu Ser Asn Asp Val Ser Thr
1               5                   10

<210> 9
<211> 14
<212> PRT
<213> Artificial Sequence

<220>
<223> antigen peptide No.33 (SNTW101, HS1, HS5)

<400> 9

Leu Ser Asn Lys Leu Gln Gly Gln Leu Lys Ser Met Gly Leu
1               5                   10

<210> 10
<211> 14
<212> PRT
<213> Artificial Sequence

<220>
<223> antigen peptide No.33 (TKY, SH8)

<400> 10

Leu Ser Asn Lys Leu Gln Asp Gln Leu Lys Ser Met Gly Leu
1               5                   10

<210> 11
<211> 14
<212> PRT
<213> Artificial Sequence

<220>
<223> antigen peptide No.33 (SH10)

<400> 11

Phe Ser Asp Lys Leu Gln Asn Met Leu Lys Ser Leu Asn Met
1               5                   10

<210> 12
<211> 14
<212> PRT
<213> Artificial Sequence

<220>
<223> antigen peptide No.16

<400> 12

Thr Asn Gly Gln Glu Val Ser Ala Ser Ile Asp Tyr Asn Lys
1 5 10

<210> 13
<211> 14
<212> PRT
<213> Artificial Sequence

<220>
<223> antigen peptide No.23

<400> 13

Ala Lys Leu Ser Asn Phe Ala Ser Asn Asp Ala Leu Pro Asp
1 5 10

<210> 14
<211> 14
<212> PRT
<213> Artificial Sequence

<220>
<223> antigen peptide No.10

<400> 14

Pro Thr Thr Ser Ser Gly Ala Ser Pro Asp Ser Ser Asn Pro
1 5 10

<210> 15
<211> 14
<212> PRT
<213> Artificial Sequence

<220>
<223> antigen peptide No.21

<400> 15

Gly Leu Gly Arg Asp Leu Phe Val His Ser Met Gly Asp Lys
1 5 10

<210> 16
<211> 14
<212> PRT
<213> Artificial Sequence

<220>
<223> antigen peptide No.15

<400> 16

Gln Ile Gly Lys Ile Lys Leu Ser Asp Val Leu Ser Ala Ser
1 5 10

<210> 17
<211> 14
<212> PRT
<213> Artificial Sequence

<220>
<223> antigen peptide No.34

<400> 17

Tyr Gly Ala Ile Asp Lys Glu Leu His Phe Ser Gly Gly Lys
1 5 10

<210> 18
<211> 14
<212> PRT
<213> Artificial Sequence

<220>
<223> antigen peptide No.20

<400> 18

Asn Val Asp Asn Ile Leu Asn Met Pro Ser Thr Thr Ser Gly
1 5 10

<210> 19
<211> 14
<212> PRT
<213> Artificial Sequence

<220>
<223> antigen peptide No.22

<400> 19

Gly Asn Leu Lys Gly Val Tyr Tyr Pro Lys Ser Ser Thr Thr
1 5 10

<210> 20
<211> 14
<212> PRT
<213> Artificial Sequence

<220>
<223> antigen peptide No.14

<400> 20

Ile Thr Glu Lys Ile Gln Ser Gly Lys Leu Thr Ile Thr Ile
1 5 10

<210> 21
<211> 14
<212> PRT
<213> Artificial Sequence

<220>
<223> antigen peptide No.26

<400> 21

Phe His Asp Phe Leu Val Ser Leu Lys Gly Lys Lys Phe Ala
1 5 10

<210> 22
<211> 14
<212> PRT
<213> Artificial Sequence

<220>
<223> antigen peptide No.31

<400> 22

Thr Thr Gly Gly Glu Val Arg Leu Phe Arg Ser Phe Tyr Val
1 5 10

<210> 23
<211> 14
<212> PRT
<213> Artificial Sequence

<220>
<223> antigen peptide No.35

<400> 23

Ile Gly Ala Arg Phe Gly Leu Asp Tyr Gln Asp Ile Asn Ile
1 5 10

<210> 24
<211> 14
<212> PRT
<213> Artificial Sequence

<220>
<223> antigen peptide No.11(mix)

<220>
<221> MISC_FEATURE
<222> (3)..(3)
<223> Xaa is K or D

<220>
<221> MISC_FEATURE
<222> (6)..(6)
<223> Xaa is Q,E or T

<220>
<221> MISC_FEATURE

<222> (7)..(7)
<223> Xaa is Q or S

<220>
<221> MISC_FEATURE
<222> (8)..(8)
<223> Xaa is M or L

<220>
<221> MISC_FEATURE
<222> (9)..(9)
<223> Xaa is E or K

<220>
<221> MISC_FEATURE
<222> (13)..(13)
<223> Xaa is P or S

<220>
<221> MISC_FEATURE
<222> (14)..(14)
<223> Xaa is D or G

<400> 24

```
Glu Lys Xaa Ala Val Xaa Xaa Xaa Xaa Asn Ser Asn Xaa Xaa
1               5                   10
```

<210> 25
<211> 14
<212> PRT
<213> Artificial Sequence

<220>
<223> antigen peptide No.19 (mix)

<220>
<221> MISC_FEATURE
<222> (14)..(14)
<223> Xaa is N or T

<400> 25

```
Asn Gln Gly Thr Leu Glu Phe Leu Ser Asn Asp Val Ser Xaa
1               5                   10
```

<210> 26
<211> 14
<212> PRT
<213> Artificial Sequence

<220>
<223> antigen peptide No.33 (mix)

<220>
<221> MISC_FEATURE
<222> (1)..(1)
<223> Xaa is L or F

<220>
<221> MISC_FEATURE
<222> (3)..(3)
<223> Xaa is N or D

<220>
<221> MISC_FEATURE
<222> (7)..(7)
<223> Xaa is G, D or N

<220>
<221> MISC_FEATURE
<222> (8)..(8)
<223> Xaa is Q or M

<220>
<221> MISC_FEATURE
<222> (12)..(12)
<223> Xaa is M or L

<220>
<221> MISC_FEATURE
<222> (13)..(13)
<223> Xaa is G or N

<220>
<221> MISC_FEATURE
<222> (14)..(14)
<223> Xaa is L or M

<400> 26

```
Xaa Ser Xaa Lys Leu Gln Xaa Xaa Leu Lys Ser Xaa Xaa Xaa
1               5                   10
```

<210> 27
<211> 9009
<212> DNA
<213> Helicobacter suis

<220>
<221> CDS
<222> (1)..(9009)

<400> 27

```
atg aaa aag ttt agt tct ctc aca ttg aaa ttt ggc cac gct cta gca        48
Met Lys Lys Phe Ser Ser Leu Thr Leu Lys Phe Gly His Ala Leu Ala
1               5                   10                  15

cgg cgc att aaa gct aac caa gct agg cgt gcg ggt act tat gtc ttt        96
Arg Arg Ile Lys Ala Asn Gln Ala Arg Arg Ala Gly Thr Tyr Val Phe
            20                  25                  30

aaa aaa caa ctc ccc caa ccc aag cgt tct gag ttg aaa cct aaa tta       144
Lys Lys Gln Leu Pro Gln Pro Lys Arg Ser Glu Leu Lys Pro Lys Leu
        35                  40                  45

atc aag cag ggg act ttt att tta ggt gta atg agc caa ccg ctt tta       192
Ile Lys Gln Gly Thr Phe Ile Leu Gly Val Met Ser Gln Pro Leu Leu
    50                  55                  60
```

```
gcg tgg tat cct tca tgg att agt ggc aca cac aca ctc aac aca aca        240
Ala Trp Tyr Pro Ser Trp Ile Ser Gly Thr His Thr Leu Asn Thr Thr
65                  70                  75                  80

aat att aag cag tat atg caa aat aac cat aga agc caa aat ttg tta        288
Asn Ile Lys Gln Tyr Met Gln Asn Asn His Arg Ser Gln Asn Leu Leu
                85                  90                  95

tgg act ggt ggc tct aat gtc ttt tat gaa gct agt aat gga agt tat        336
Trp Thr Gly Gly Ser Asn Val Phe Tyr Glu Ala Ser Asn Gly Ser Tyr
            100                 105                 110

ttt tgt act aac tgg aat tgt aac ggc tcg gtt aca tta att ggt aat        384
Phe Cys Thr Asn Trp Asn Cys Asn Gly Ser Val Thr Leu Ile Gly Asn
        115                 120                 125

ggc tct acc gct tac acg ctt agc aat ctc aat tac gag ggc ggt agc        432
Gly Ser Thr Ala Tyr Thr Leu Ser Asn Leu Asn Tyr Glu Gly Gly Ser
    130                 135                 140

ctt aat tta caa att aat ggc aat ggt aca caa ggc acg ctt aat att        480
Leu Asn Leu Gln Ile Asn Gly Asn Gly Thr Gln Gly Thr Leu Asn Ile
145                 150                 155                 160

agc aat gtc aac atg gat agc tat aaa gga agg caa ttt aca gat aca        528
Ser Asn Val Asn Met Asp Ser Tyr Lys Gly Arg Gln Phe Thr Asp Thr
                165                 170                 175

tgg aat gcc caa agt gtt agt atc agc gga aat cta caa gta ggg caa        576
Trp Asn Ala Gln Ser Val Ser Ile Ser Gly Asn Leu Gln Val Gly Gln
            180                 185                 190

aat cat atc act atc aac gca aac cac ggc act aca gcg aac aac gct        624
Asn His Ile Thr Ile Asn Ala Asn His Gly Thr Thr Ala Asn Asn Ala
        195                 200                 205

acc att aac gcg gat caa ggc aac ggg att tta aat atc aat gat aaa        672
Thr Ile Asn Ala Asp Gln Gly Asn Gly Ile Leu Asn Ile Asn Asp Lys
    210                 215                 220

aca agc gag agt ttc aca aac acc acc ttt aaa ggt acg ggg cag atc        720
Thr Ser Glu Ser Phe Thr Asn Thr Thr Phe Lys Gly Thr Gly Gln Ile
225                 230                 235                 240

aat tta cag agc aac aac atc acc ttt aag aat gtt act ttt aat gat        768
Asn Leu Gln Ser Asn Asn Ile Thr Phe Lys Asn Val Thr Phe Asn Asp
                245                 250                 255

agc aac act ggt tca cat gtt acg gac aac ggc act ctt acc ctt gag        816
Ser Asn Thr Gly Ser His Val Thr Asp Asn Gly Thr Leu Thr Leu Glu
            260                 265                 270

ggc act gag acc ttt gca caa aac tcg ccc ctt atc aat cta ggc gcc        864
Gly Thr Glu Thr Phe Ala Gln Asn Ser Pro Leu Ile Asn Leu Gly Ala
        275                 280                 285

aat gtt act atc caa gcc aac acc att ttt aac att aca gaa gat ctt        912
Asn Val Thr Ile Gln Ala Asn Thr Ile Phe Asn Ile Thr Glu Asp Leu
    290                 295                 300

aca aag acc acc aac gga gcc tat aat ctt gat acc ctt gta agt aca        960
Thr Lys Thr Thr Asn Gly Ala Tyr Asn Leu Asp Thr Leu Val Ser Thr
305                 310                 315                 320
```

```
agt ggt aat aaa agt atc aac gat agt agc tat gca agc cat ttg tgg        1008
Ser Gly Asn Lys Ser Ile Asn Asp Ser Ser Tyr Ala Ser His Leu Trp
                325                 330                 335

gat ctt atc cgc tat caa ggc caa aca ggt agc ctt ttt aac ggg caa        1056
Asp Leu Ile Arg Tyr Gln Gly Gln Thr Gly Ser Leu Phe Asn Gly Gln
            340                 345                 350

ctc agt aat ggc act act tta agt aat cct agc tct ggc aat ggg att        1104
Leu Ser Asn Gly Thr Thr Leu Ser Asn Pro Ser Ser Gly Asn Gly Ile
        355                 360                 365

tac tat gtg aaa tat act ttt ggc aat ggt gat tgg gat att ctt aaa        1152
Tyr Tyr Val Lys Tyr Thr Phe Gly Asn Gly Asp Trp Asp Ile Leu Lys
    370                 375                 380

gaa gat ttt gaa aat aac tct cta tcg gcg caa ctt gag gct tac gct        1200
Glu Asp Phe Glu Asn Asn Ser Leu Ser Ala Gln Leu Glu Ala Tyr Ala
385                 390                 395                 400

aaa aat cag ggt gat att tgg agt aca atc cac aac att aat tct act        1248
Lys Asn Gln Gly Asp Ile Trp Ser Thr Ile His Asn Ile Asn Ser Thr
                405                 410                 415

tgg aat ttt aat gtc gga gag ggt agt gcc tat atc aac ccc ggg aat        1296
Trp Asn Phe Asn Val Gly Glu Gly Ser Ala Tyr Ile Asn Pro Gly Asn
            420                 425                 430

gga aca gat caa gct tgg gca caa aga gat aaa aat ttt aaa gtt act        1344
Gly Thr Asp Gln Ala Trp Ala Gln Arg Asp Lys Asn Phe Lys Val Thr
        435                 440                 445

tta gat aat ggg agt ggt ggg acg ctt att ctt ggt aat agc aca gaa        1392
Leu Asp Asn Gly Ser Gly Gly Thr Leu Ile Leu Gly Asn Ser Thr Glu
    450                 455                 460

acc ccc agt tca agc ggt aaa att ctc ttt gga ggc aca ggg ggt aat        1440
Thr Pro Ser Ser Ser Gly Lys Ile Leu Phe Gly Gly Thr Gly Gly Asn
465                 470                 475                 480

cct ttt gct tta aat ggc aat tac ggc gga gat ctt ggc tat atg aca        1488
Pro Phe Ala Leu Asn Gly Asn Tyr Gly Gly Asp Leu Gly Tyr Met Thr
                485                 490                 495

ggg gaa ttt gac gcg ggt aaa att tat ctt acc ggt acc ata gaa agc        1536
Gly Glu Phe Asp Ala Gly Lys Ile Tyr Leu Thr Gly Thr Ile Glu Ser
            500                 505                 510

gga aac tct ttt cac gat ggt aac ggc act aac att agc tac aac gct        1584
Gly Asn Ser Phe His Asp Gly Asn Gly Thr Asn Ile Ser Tyr Asn Ala
        515                 520                 525

att aca aac att aca gct aat ggt ttg cac tat ctc aac gat aac gcc        1632
Ile Thr Asn Ile Thr Ala Asn Gly Leu His Tyr Leu Asn Asp Asn Ala
    530                 535                 540

gga gta tgg cat agc aac gct aat ttt aga gct acc aat ggc agt atc        1680
Gly Val Trp His Ser Asn Ala Asn Phe Arg Ala Thr Asn Gly Ser Ile
545                 550                 555                 560

aac atc tca aac tcc caa ttc caa gat caa agt agc ggg caa ttt acc        1728
Asn Ile Ser Asn Ser Gln Phe Gln Asp Gln Ser Ser Gly Gln Phe Thr
```

```
                565                 570                 575

ttt aac tct caa aat caa act ttt agt agc aca act ttt act ggc aat        1776
Phe Asn Ser Gln Asn Gln Thr Phe Ser Ser Thr Thr Phe Thr Gly Asn
            580                 585                 590

agt cat acg atc act cta cac gct acc aac aac cta acc ctc acc aac        1824
Ser His Thr Ile Thr Leu His Ala Thr Asn Asn Leu Thr Leu Thr Asn
        595                 600                 605

aca agc ttt aat gat tca aat gct agc ttg aca cta gag gca gat aag        1872
Thr Ser Phe Asn Asp Ser Asn Ala Ser Leu Thr Leu Glu Ala Asp Lys
    610                 615                 620

ggt agt tta caa gat acc gat aac caa act agc caa atc aca gct aaa        1920
Gly Ser Leu Gln Asp Thr Asp Asn Gln Thr Ser Gln Ile Thr Ala Lys
625                 630                 635                 640

aac ctc caa gta att gct aat caa gct agt ttt agt aac acg atc ttt        1968
Asn Leu Gln Val Ile Ala Asn Gln Ala Ser Phe Ser Asn Thr Ile Phe
                645                 650                 655

aat gca caa aac agc tct ttt aaa act aac caa ttg acc ctg aca aat        2016
Asn Ala Gln Asn Ser Ser Phe Lys Thr Asn Gln Leu Thr Leu Thr Asn
            660                 665                 670

gat aca ttt aat aat ggt agc tat agc ttt gca cct gaa aac aac ggc        2064
Asp Thr Phe Asn Asn Gly Ser Tyr Ser Phe Ala Pro Glu Asn Asn Gly
        675                 680                 685

aac cac aca act aca ttt agc ggt acc acc acg att aat act agc agt        2112
Asn His Thr Thr Thr Phe Ser Gly Thr Thr Thr Ile Asn Thr Ser Ser
    690                 695                 700

agt cct ttt gct aat tta ggt ggg gcg atc aag ttt aat agt ggc gct        2160
Ser Pro Phe Ala Asn Leu Gly Gly Ala Ile Lys Phe Asn Ser Gly Ala
705                 710                 715                 720

acc ttt aac ctg aat aat att tta agt tct ttg caa att ggt aca act        2208
Thr Phe Asn Leu Asn Asn Ile Leu Ser Ser Leu Gln Ile Gly Thr Thr
                725                 730                 735

tac agc atc tta ggc gga agt ggg gcc aac att aat tat agt agt gat        2256
Tyr Ser Ile Leu Gly Gly Ser Gly Ala Asn Ile Asn Tyr Ser Ser Asp
            740                 745                 750

acg cag tat gcc aac aat ctt tgg aat tta atc cgt att agc ggg gct        2304
Thr Gln Tyr Ala Asn Asn Leu Trp Asn Leu Ile Arg Ile Ser Gly Ala
        755                 760                 765

agt atc agc tca gaa aca gaa atg agt gat agc aac ggc acg cag gtt        2352
Ser Ile Ser Ser Glu Thr Glu Met Ser Asp Ser Asn Gly Thr Gln Val
    770                 775                 780

tgg gat gtg gtc ttt ggt ata aat ggc atg ccc att aag ata caa gaa        2400
Trp Asp Val Val Phe Gly Ile Asn Gly Met Pro Ile Lys Ile Gln Glu
785                 790                 795                 800

acc ttt gct agt agt gga ctt agc tta aaa gtt att agc caa gct aaa        2448
Thr Phe Ala Ser Ser Gly Leu Ser Leu Lys Val Ile Ser Gln Ala Lys
                805                 810                 815

gat att tgg tca gat gtg tat aac atg act act aat tgt tca tac aat        2496
```

```
Asp Ile Trp Ser Asp Val Tyr Asn Met Thr Thr Asn Cys Ser Tyr Asn
            820                 825                 830

gcc tta gcc agc cca ccg ggc tgt tat ggt tat caa aat ggt aca tac      2544
Ala Leu Ala Ser Pro Pro Gly Cys Tyr Gly Tyr Gln Asn Gly Thr Tyr
        835                 840                 845

aac ata tgg aag cac ttt aat aag tac ggc ttt gaa gct tac aat gaa      2592
Asn Ile Trp Lys His Phe Asn Lys Tyr Gly Phe Glu Ala Tyr Asn Glu
    850                 855                 860

agt gta ggc gca gac ggg att gcc tat atc aac cct tta aac tca caa      2640
Ser Val Gly Ala Asp Gly Ile Ala Tyr Ile Asn Pro Leu Asn Ser Gln
865                 870                 875                 880

agc cac gat ggc tat aac gcc tcc acc cac aca ctc caa act tat aca      2688
Ser His Asp Gly Tyr Asn Ala Ser Thr His Thr Leu Gln Thr Tyr Thr
                885                 890                 895

aaa ctt ggc aat ggc ggt act tat aat gtt gga gaa aaa aaa aat agc      2736
Lys Leu Gly Asn Gly Gly Thr Tyr Asn Val Gly Glu Lys Lys Asn Ser
            900                 905                 910

caa tgg gtt aac gat ggc act tta att tta ggt aac aat acc cta aaa      2784
Gln Trp Val Asn Asp Gly Thr Leu Ile Leu Gly Asn Asn Thr Leu Lys
        915                 920                 925

gcc gct aca ggg ggt aaa ata gaa ttt ggc gca gtg ggc tca gta ggc      2832
Ala Ala Thr Gly Gly Lys Ile Glu Phe Gly Ala Val Gly Ser Val Gly
    930                 935                 940

tat atc acc gat gtt ttt aat gcc ggt aat att ttt cta act aat act      2880
Tyr Ile Thr Asp Val Phe Asn Ala Gly Asn Ile Phe Leu Thr Asn Thr
945                 950                 955                 960

ata gaa gtt gga gat agc tcg cta agc ggg gca ggg gcg act cta act      2928
Ile Glu Val Gly Asp Ser Ser Leu Ser Gly Ala Gly Ala Thr Leu Thr
                965                 970                 975

ttt aat gct aat aac aat att acc gcc gat ggc ctc acc tac cac caa      2976
Phe Asn Ala Asn Asn Asn Ile Thr Ala Asp Gly Leu Thr Tyr His Gln
            980                 985                 990

gat gct acc gct atc cct ccc ttt  ggt tct act atc agc  cag cat agc    3024
Asp Ala Thr Ala Ile Pro Pro Phe  Gly Ser Thr Ile Ser  Gln His Ser
        995                 1000                 1005

cac ggt  aac ttc atc gca caa  caa agc ttt agc gcg  ctc aat tcc      3069
His Gly  Asn Phe Ile Ala Gln  Gln Ser Phe Ser Ala  Leu Asn Ser
    1010                 1015                 1020

tct ttt  gaa gat gat act agc  ggt agt tta gac ttt  acc ggt aaa      3114
Ser Phe  Glu Asp Asp Thr Ser  Gly Ser Leu Asp Phe  Thr Gly Lys
    1025                 1030                 1035

aac agc  att aag ttt aca agc  agc acc gtt ata ggt  agt aaa agt      3159
Asn Ser  Ile Lys Phe Thr Ser  Ser Thr Val Ile Gly  Ser Lys Ser
    1040                 1045                 1050

agt att  acc ctt aac tcc gca  aat acc acc cta aac  aac tct gcc      3204
Ser Ile  Thr Leu Asn Ser Ala  Asn Thr Thr Leu Asn  Asn Ser Ala
    1055                 1060                 1065
```

```
ttt ttt  gtg ggc aat ggt acg  act ctt att ttt ggt  aat gtg atc        3249
Phe Phe  Val Gly Asn Gly Thr  Thr Leu Ile Phe Gly  Asn Val Ile
    1070                 1075                 1080

aca act  aat agt cat agt agc  tat tca tct agc aca  aac acc att        3294
Thr Thr  Asn Ser His Ser Ser  Tyr Ser Ser Ser Thr  Asn Thr Ile
    1085                 1090                 1095

aat aac  tct aca atc aac ctc  aac caa aac tct atc  ttg tat ttg        3339
Asn Asn  Ser Thr Ile Asn Leu  Asn Gln Asn Ser Ile  Leu Tyr Leu
    1100                 1105                 1110

cat ggc  agt acc acc cta gat  aac acc act tcc ttt  tta aac tta        3384
His Gly  Ser Thr Thr Leu Asp  Asn Thr Thr Ser Phe  Leu Asn Leu
    1115                 1120                 1125

ggt agt  caa gca act ttt tat  gat tca gct agc cta  agt ggt agc        3429
Gly Ser  Gln Ala Thr Phe Tyr  Asp Ser Ala Ser Leu  Ser Gly Ser
    1130                 1135                 1140

act aca  att aat ctt gat cac  aat agc aag att ata  atg aat agc        3474
Thr Thr  Ile Asn Leu Asp His  Asn Ser Lys Ile Ile  Met Asn Ser
    1145                 1150                 1155

acc acg  acg cta aat gat aac  gct aat ttt aat tta  att aac tcc        3519
Thr Thr  Thr Leu Asn Asp Asn  Ala Asn Phe Asn Leu  Ile Asn Ser
    1160                 1165                 1170

gct caa  gct aac ttt caa ggc  aat agt act ttt aat  aat agc tct        3564
Ala Gln  Ala Asn Phe Gln Gly  Asn Ser Thr Phe Asn  Asn Ser Ser
    1175                 1180                 1185

ggg atc  act ctt gct agt ggt  gct aag gct atg ttt  aca cac act        3609
Gly Ile  Thr Leu Ala Ser Gly  Ala Lys Ala Met Phe  Thr His Thr
    1190                 1195                 1200

aca aca  agc acc caa gat att  aca act ttt aat aat  aac tcg ttt        3654
Thr Thr  Ser Thr Gln Asp Ile  Thr Thr Phe Asn Asn  Asn Ser Phe
    1205                 1210                 1215

tta aat  gta aac ggg agt ttt  aca gat ttt ata cca  aat aca agt        3699
Leu Asn  Val Asn Gly Ser Phe  Thr Asp Phe Ile Pro  Asn Thr Ser
    1220                 1225                 1230

agc ggg  agc att agc ggg ggg  gga tca agt agt aat  caa aac tat        3744
Ser Gly  Ser Ile Ser Gly Gly  Gly Ser Ser Ser Asn  Gln Asn Tyr
    1235                 1240                 1245

agc gcg  cag ttt aat aat cta  gtg ttt aac aac tat  gcc tcc atg        3789
Ser Ala  Gln Phe Asn Asn Leu  Val Phe Asn Asn Tyr  Ala Ser Met
    1250                 1255                 1260

ctc tta  aat agc gca gat att  caa agt agc caa acc  acc ttt gct        3834
Leu Leu  Asn Ser Ala Asp Ile  Gln Ser Ser Gln Thr  Thr Phe Ala
    1265                 1270                 1275

aat tct  gtg aat atc aat atg  caa aat agc ctt ttt  aat gcg ggt        3879
Asn Ser  Val Asn Ile Asn Met  Gln Asn Ser Leu Phe  Asn Ala Gly
    1280                 1285                 1290

acg ctt  aat tta aat ggc ggt  aat ttt tac atg caa  aat agc cag        3924
Thr Leu  Asn Leu Asn Gly Gly  Asn Phe Tyr Met Gln  Asn Ser Gln
    1295                 1300                 1305
```

```
att aaa gcg gga gct gta aat gta gga tct tct gat tct gtt aat     3969
Ile Lys Ala Gly Ala Val Asn Val Gly Ser Ser Asp Ser Val Asn
    1310                1315                1320

atc aat tct ggg gtc aat tct ctt aat cgc tcg ctt att act agc     4014
Ile Asn Ser Gly Val Asn Ser Leu Asn Arg Ser Leu Ile Thr Ser
    1325                1330                1335

tca agc ttt aat tta aac ggt act tta aat tta gat ggt ctt tta     4059
Ser Ser Phe Asn Leu Asn Gly Thr Leu Asn Leu Asp Gly Leu Leu
    1340                1345                1350

ctt gaa ccc aca act agt aca ggt gca agc tca agc gca gct aac     4104
Leu Glu Pro Thr Thr Ser Thr Gly Ala Ser Ser Ser Ala Ala Asn
    1355                1360                1365

agc caa cct ctt att tct gtt tct aat aat agc tct agt agc acg     4149
Ser Gln Pro Leu Ile Ser Val Ser Asn Asn Ser Ser Ser Ser Thr
    1370                1375                1380

gga ggg ggg aca ttt gat tta agt ggt att ctt aat atc tct aat     4194
Gly Gly Gly Thr Phe Asp Leu Ser Gly Ile Leu Asn Ile Ser Asn
    1385                1390                1395

att gat ctc tcc gcg ccc ttt tct agc caa act aat aat aca agt     4239
Ile Asp Leu Ser Ala Pro Phe Ser Ser Gln Thr Asn Asn Thr Ser
    1400                1405                1410

tcc tcc gcc act tat aat att gta aat gct agt aag att aca ggt     4284
Ser Ser Ala Thr Tyr Asn Ile Val Asn Ala Ser Lys Ile Thr Gly
    1415                1420                1425

atg agt ggg gct aat ggc tat caa aaa att gag tac tac ggc att     4329
Met Ser Gly Ala Asn Gly Tyr Gln Lys Ile Glu Tyr Tyr Gly Ile
    1430                1435                1440

aaa att aat aac gct acc tat tct gat tca aat aaa acc caa agc     4374
Lys Ile Asn Asn Ala Thr Tyr Ser Asp Ser Asn Lys Thr Gln Ser
    1445                1450                1455

tgg agt ttt aca aac cct tta aat ggc gca caa act att act gaa     4419
Trp Ser Phe Thr Asn Pro Leu Asn Gly Ala Gln Thr Ile Thr Glu
    1460                1465                1470

aaa ata caa aat ggt aaa ctc act atc acc att agt aat agc aat     4464
Lys Ile Gln Asn Gly Lys Leu Thr Ile Thr Ile Ser Asn Ser Asn
    1475                1480                1485

cat ttt gta gct aca gat tac cac aac atc gcc ccc gaa ctc ttt     4509
His Phe Val Ala Thr Asp Tyr His Asn Ile Ala Pro Glu Leu Phe
    1490                1495                1500

ttt tac aaa caa tcc gca caa aac ttg cct agt aca aac tca gca     4554
Phe Tyr Lys Gln Ser Ala Gln Asn Leu Pro Ser Thr Asn Ser Ala
    1505                1510                1515

agt gca gat ata agc agt tat gat tat tcc agc gat gaa agc ggg     4599
Ser Ala Asp Ile Ser Ser Tyr Asp Tyr Ser Ser Asp Glu Ser Gly
    1520                1525                1530

act ttc ttt tta gac ggt aat tta aaa ggg gtg tat gat cca aag     4644
Thr Phe Phe Leu Asp Gly Asn Leu Lys Gly Val Tyr Asp Pro Lys
```

```
        1535                1540                1545

gct aat  act tca agt agt aaa  act tcc tca aca ccg  gtt att ccg        4689
Ala Asn  Thr Ser Ser Ser Lys  Thr Ser Ser Thr Pro  Val Ile Pro
        1550                1555                1560

ggc act  tat aat gct caa ggc  cag ccc tta caa ggt  ctt tat att        4734
Gly Thr  Tyr Asn Ala Gln Gly  Gln Pro Leu Gln Gly  Leu Tyr Ile
        1565                1570                1575

tct aat  aac ggc ctg ttt aat  gaa acc acc ctt aat  aac tta gta        4779
Ser Asn  Asn Gly Leu Phe Asn  Glu Thr Thr Leu Asn  Asn Leu Val
        1580                1585                1590

ggc att  gtc caa agt att tgg  cct cat tta aaa acc  ctt ttg cct        4824
Gly Ile  Val Gln Ser Ile Trp  Pro His Leu Lys Thr  Leu Leu Pro
        1595                1600                1605

caa att  cta caa gat tta agc  gat cca agt aat atc  att caa gac        4869
Gln Ile  Leu Gln Asp Leu Ser  Asp Pro Ser Asn Ile  Ile Gln Asp
        1610                1615                1620

tta gaa  aac tca ggg att aaa  tta act agc cag caa  agc aag gaa        4914
Leu Glu  Asn Ser Gly Ile Lys  Leu Thr Ser Gln Gln  Ser Lys Glu
        1625                1630                1635

ctt tta  agt ttt ata gac ggg  cta tca agc aat atc  aac caa act        4959
Leu Leu  Ser Phe Ile Asp Gly  Leu Ser Ser Asn Ile  Asn Gln Thr
        1640                1645                1650

ttt aat  aat ggt acg ctt gta  gtg ggt tcg cct cag  gca gga caa        5004
Phe Asn  Asn Gly Thr Leu Val  Val Gly Ser Pro Gln  Ala Gly Gln
        1655                1660                1665

acc gga  agt agt agc gtg gtg  tgg ttt ggt ggc aat  ggt tat act        5049
Thr Gly  Ser Ser Ser Val Val  Trp Phe Gly Gly Asn  Gly Tyr Thr
        1670                1675                1680

acg gct  tgt acg gct gca caa  act aaa aaa gga tgt  cag gat tta        5094
Thr Ala  Cys Thr Ala Ala Gln  Thr Lys Lys Gly Cys  Gln Asp Leu
        1685                1690                1695

aga ggc  act tat tta ggt gag  ctt cta ggc tca act  tct gct gca        5139
Arg Gly  Thr Tyr Leu Gly Glu  Leu Leu Gly Ser Thr  Ser Ala Ala
        1700                1705                1710

cta ggc  tat att gag gct aat  ttt aag gct aaa gat  att tat atc        5184
Leu Gly  Tyr Ile Glu Ala Asn  Phe Lys Ala Lys Asp  Ile Tyr Ile
        1715                1720                1725

acc ggc  acc gta ggc agt ggg  gat gct tgg ggg ata  ggt ggg agt        5229
Thr Gly  Thr Val Gly Ser Gly  Asp Ala Trp Gly Ile  Gly Gly Ser
        1730                1735                1740

gca gat  gta act ttt aat agc  gcg act aat tta acc  ctt aat cag        5274
Ala Asp  Val Thr Phe Asn Ser  Ala Thr Asn Leu Thr  Leu Asn Gln
        1745                1750                1755

gcc acg  att gat gca gaa ggc  acg gat caa att ttt  aat atg cta        5319
Ala Thr  Ile Asp Ala Glu Gly  Thr Asp Gln Ile Phe  Asn Met Leu
        1760                1765                1770

ggt gag  ggc ggg ata caa aaa  atc tta ggc caa aaa  ggg cta gcc        5364
```

```
Gly Glu  Gly Gly Ile Gln Lys  Ile Leu Gly Gln Lys  Gly Leu Ala
    1775                 1780                 1785

cag atg  tta ggt aat tat atc  tac gat cta gcc aat  ggc tct agt      5409
Gln Met  Leu Gly Asn Tyr Ile  Tyr Asp Leu Ala Asn  Gly Ser Ser
    1790                 1795                 1800

att aat  ctt aat ccc act agt  agc att cca agc tct  atc aag cct      5454
Ile Asn  Leu Asn Pro Thr Ser  Ser Ile Pro Ser Ser  Ile Lys Pro
    1805                 1810                 1815

cta gct  aaa gat tta ggc ggg  cag att ggt aaa atc  aaa ctt agc      5499
Leu Ala  Lys Asp Leu Gly Gly  Gln Ile Gly Lys Ile  Lys Leu Ser
    1820                 1825                 1830

gat gta  ttg aat gcc tca gat  gtg agt gct ctt tta  aac atg ccc      5544
Asp Val  Leu Asn Ala Ser Asp  Val Ser Ala Leu Leu  Asn Met Pro
    1835                 1840                 1845

ggt atg  gat aat gtg att aaa  aat att tta agt act  aag act gtg      5589
Gly Met  Asp Asn Val Ile Lys  Asn Ile Leu Ser Thr  Lys Thr Val
    1850                 1855                 1860

agt tct  gta tta ggc ggt gga  ggg ctt att tct agt  ttg gat cag      5634
Ser Ser  Val Leu Gly Gly Gly  Gly Leu Ile Ser Ser  Leu Asp Gln
    1865                 1870                 1875

gct gag  caa aat aaa atc tat  aat gct att gat aaa  gag tta cat      5679
Ala Glu  Gln Asn Lys Ile Tyr  Asn Ala Ile Asp Lys  Glu Leu His
    1880                 1885                 1890

ttc tca  ggg gga aaa gcc att  gcc tcc att gcc aac  ggg gtt tat      5724
Phe Ser  Gly Gly Lys Ala Ile  Ala Ser Ile Ala Asn  Gly Val Tyr
    1895                 1900                 1905

ggt aag  cac acc ctt tta agt  ctc att aac tct tta  tcc cct atg      5769
Gly Lys  His Thr Leu Leu Ser  Leu Ile Asn Ser Leu  Ser Pro Met
    1910                 1915                 1920

acg ggt  aaa gat gtg gat aat  att tta aac atg cca  aat act agt      5814
Thr Gly  Lys Asp Val Asp Asn  Ile Leu Asn Met Pro  Asn Thr Ser
    1925                 1930                 1935

agc ggc  caa agt cag gta aaa  aat ttt tta aac aag  acc act ttt      5859
Ser Gly  Gln Ser Gln Val Lys  Asn Phe Leu Asn Lys  Thr Thr Phe
    1940                 1945                 1950

ggg caa  att ttt gaa gaa cta  tta tct aat caa aac  cta ctc aat      5904
Gly Gln  Ile Phe Glu Glu Leu  Leu Ser Asn Gln Asn  Leu Leu Asn
    1955                 1960                 1965

aaa acc  att gct tgg tta ggc  cca caa att tta gat  gaa atg ctt      5949
Lys Thr  Ile Ala Trp Leu Gly  Pro Gln Ile Leu Asp  Glu Met Leu
    1970                 1975                 1980

aaa act  gct att gat gat tta  ctc aac cct aca aat  cag ctt act      5994
Lys Thr  Ala Ile Asp Asp Leu  Leu Asn Pro Thr Asn  Gln Leu Thr
    1985                 1990                 1995

gcc gct  gaa aaa aat att ctt  gat aac att ctt aac  aat gtc ttt      6039
Ala Ala  Glu Lys Asn Ile Leu  Asp Asn Ile Leu Asn  Asn Val Phe
    2000                 2005                 2010
```

```
agc tca  gaa aaa aag gca gtc  caa caa atg gaa aat  tct aac cct        6084
Ser Ser  Glu Lys Lys Ala Val  Gln Gln Met Glu Asn  Ser Asn Pro
    2015                 2020                 2025

gat gtc  aaa cac gtt att gat  ggc tta atg cag gct  aaa ggt cta        6129
Asp Val  Lys His Val Ile Asp  Gly Leu Met Gln Ala  Lys Gly Leu
    2030                 2035                 2040

ggg gag  gtt tat agc aag ggc  ctt cag agt att tta  tct aat aaa        6174
Gly Glu  Val Tyr Ser Lys Gly  Leu Gln Ser Ile Leu  Ser Asn Lys
    2045                 2050                 2055

ctg caa  ggt caa tta aaa agc  atg ggc tta ggt tct  ttg ctc gcg        6219
Leu Gln  Gly Gln Leu Lys Ser  Met Gly Leu Gly Ser  Leu Leu Ala
    2060                 2065                 2070

cct aaa  gct cta ggt aat ttc  tgg cag aag ggt tat  ttt aac ttc        6264
Pro Lys  Ala Leu Gly Asn Phe  Trp Gln Lys Gly Tyr  Phe Asn Phe
    2075                 2080                 2085

cta gcc  aat gat gat att tta  gtg aac aat agc act  ttt agt aat        6309
Leu Ala  Asn Asp Asp Ile Leu  Val Asn Asn Ser Thr  Phe Ser Asn
    2090                 2095                 2100

gct tca  ggc ggg act tta agt  ttt ata gcc ggt aag  tct att att        6354
Ala Ser  Gly Gly Thr Leu Ser  Phe Ile Ala Gly Lys  Ser Ile Ile
    2105                 2110                 2115

ttt gca  ggg caa aat aca att  aat ttt agt aat aat  caa ggt aca        6399
Phe Ala  Gly Gln Asn Thr Ile  Asn Phe Ser Asn Asn  Gln Gly Thr
    2120                 2125                 2130

cta gaa  ttt tta agt aat gat  gtg tct aat att gat  ctg act act        6444
Leu Glu  Phe Leu Ser Asn Asp  Val Ser Asn Ile Asp  Leu Thr Thr
    2135                 2140                 2145

ctt aat  gct acc gat ggc cta  acc att gat gcc ccc  ttt aat aat        6489
Leu Asn  Ala Thr Asp Gly Leu  Thr Ile Asp Ala Pro  Phe Asn Asn
    2150                 2155                 2160

ctt tat  gtc cag aaa ggc aat  atc acc ctt gct tcg  tat gaa ggg        6534
Leu Tyr  Val Gln Lys Gly Asn  Ile Thr Leu Ala Ser  Tyr Glu Gly
    2165                 2170                 2175

ctc aca  gta cgc gca aat aat  ttt gac ttt tta ggc  acg gtg caa        6579
Leu Thr  Val Arg Ala Asn Asn  Phe Asp Phe Leu Gly  Thr Val Gln
    2180                 2185                 2190

gca gat  ggg gct gtg gat tta  tcc ggt gta acc ggt  tta gct gtt        6624
Ala Asp  Gly Ala Val Asp Leu  Ser Gly Val Thr Gly  Leu Ala Val
    2195                 2200                 2205

tta ggt  act ctt aat ctt act  gag gat agc acc ctt  aag gct aat        6669
Leu Gly  Thr Leu Asn Leu Thr  Glu Asp Ser Thr Leu  Lys Ala Asn
    2210                 2215                 2220

aat tta  acc aca ata agc gcc  ttt aac aac caa tcc  caa aac ctg        6714
Asn Leu  Thr Thr Ile Ser Ala  Phe Asn Asn Gln Ser  Gln Asn Leu
    2225                 2230                 2235

ctt aat  atc agc ggg aat ttt  aac tcc tat ggc aca  ttt agt aca        6759
Leu Asn  Ile Ser Gly Asn Phe  Asn Ser Tyr Gly Thr  Phe Ser Thr
    2240                 2245                 2250
```

```
caa ggg  gct ggg gtc aat atc  ggg ggc ggg ttt aat  agt aca ggg        6804
Gln Gly  Ala Gly Val Asn Ile  Gly Gly Gly Phe Asn  Ser Thr Gly
    2255                 2260                 2265

gct tta  act ttt aat gta gcg  ggt gca acg att aaa  acc acc ggc        6849
Ala Leu  Thr Phe Asn Val Ala  Gly Ala Thr Ile Lys  Thr Thr Gly
    2270                 2275                 2280

cct agt  tca gat aat tca acc  tca agc tct agc gcc  tct aca agt        6894
Pro Ser  Ser Asp Asn Ser Thr  Ser Ser Ser Ser Ala  Ser Thr Ser
    2285                 2290                 2295

tct agt  aat gct aat agc tct  gga ggt tct agc tcc  tcc cct agc        6939
Ser Ser  Asn Ala Asn Ser Ser  Gly Gly Ser Ser Ser  Ser Pro Ser
    2300                 2305                 2310

act tct  aca ccc tct act aac  tct agc gcc tct act  agt tct aaa        6984
Thr Ser  Thr Pro Ser Thr Asn  Ser Ser Ala Ser Thr  Ser Ser Lys
    2315                 2320                 2325

aca gct  aca gct tta act cta  gct agc atc acc gtg  tct aca agt        7029
Thr Ala  Thr Ala Leu Thr Leu  Ala Ser Ile Thr Val  Ser Thr Ser
    2330                 2335                 2340

tct aca  act tct agt acc gct  agc tct agc act tct  aca agt tcc        7074
Ser Thr  Thr Ser Ser Thr Ala  Ser Ser Ser Thr Ser  Thr Ser Ser
    2345                 2350                 2355

tct aac  acc tcc tcc agt agc  gat aat agc cca agc  tct aat agt        7119
Ser Asn  Thr Ser Ser Ser Ser  Asp Asn Ser Pro Ser  Ser Asn Ser
    2360                 2365                 2370

ggg tct  aat gta aat ccg gtt  agc cca acg cct agt  acg caa att        7164
Gly Ser  Asn Val Asn Pro Val  Ser Pro Thr Pro Ser  Thr Gln Ile
    2375                 2380                 2385

ccg cta  att caa gtg ggt gga  ctt gtg aat tta aat  tta ggt ggt        7209
Pro Leu  Ile Gln Val Gly Gly  Leu Val Asn Leu Asn  Leu Gly Gly
    2390                 2395                 2400

agt gcg  att att agc ttt aat  aca gag gca caa aca  aat aat gca        7254
Ser Ala  Ile Ile Ser Phe Asn  Thr Glu Ala Gln Thr  Asn Asn Ala
    2405                 2410                 2415

gga tca  agc gcg ggt tct agt  tct aca agt aca cca  caa aca aca        7299
Gly Ser  Ser Ala Gly Ser Ser  Ser Thr Ser Thr Pro  Gln Thr Thr
    2420                 2425                 2430

act agc  agt tct act agt tct  act caa aca gca agt  aca gca caa        7344
Thr Ser  Ser Ser Thr Ser Ser  Thr Gln Thr Ala Ser  Thr Ala Gln
    2435                 2440                 2445

agt acc  aca tta agc cta agc  tct aat act agt cta  gct act acc        7389
Ser Thr  Thr Leu Ser Leu Ser  Ser Asn Thr Ser Leu  Ala Thr Thr
    2450                 2455                 2460

agc caa  acg cct aca act tct  agc ggg gct tct ccg  gat agt tca        7434
Ser Gln  Thr Pro Thr Thr Ser  Ser Gly Ala Ser Pro  Asp Ser Ser
    2465                 2470                 2475

aac cct  aca gcc tct cct ata  acc cct tca aat ggc  gct tat acg        7479
Asn Pro  Thr Ala Ser Pro Ile  Thr Pro Ser Asn Gly  Ala Tyr Thr
```

```
        2480                2485                2490

ctg att  caa act aat agc tgg  att cat tac aac ccc  acc tct ttt        7524
Leu Ile  Gln Thr Asn Ser Trp  Ile His Tyr Asn Pro  Thr Ser Phe
    2495                2500                2505

aat cct  aat aac tgg aga caa  tat tta gag ctc tac  act agc ctt        7569
Asn Pro  Asn Asn Trp Arg Gln  Tyr Leu Glu Leu Tyr  Thr Ser Leu
    2510                2515                2520

aaa atc  aac ggg aca gct ttc  cag ctt aac gct caa  ggt aca gga        7614
Lys Ile  Asn Gly Thr Ala Phe  Gln Leu Asn Ala Gln  Gly Thr Gly
    2525                2530                2535

ctt act  tat aat ggt caa gca  gtc aat atc tca caa  cga ggc ttg        7659
Leu Thr  Tyr Asn Gly Gln Ala  Val Asn Ile Ser Gln  Arg Gly Leu
    2540                2545                2550

ctt gtc  aat tat caa ggc aca  aat ggc caa gaa gtg  agc gcc tct        7704
Leu Val  Asn Tyr Gln Gly Thr  Asn Gly Gln Glu Val  Ser Ala Ser
    2555                2560                2565

att gat  tat aat aag atg caa  ata ggc ata ggc caa  agc ttg cat        7749
Ile Asp  Tyr Asn Lys Met Gln  Ile Gly Ile Gly Gln  Ser Leu His
    2570                2575                2580

gtt atc  gcg ccc act att aca  caa tac atc acc caa  ata caa ggg        7794
Val Ile  Ala Pro Thr Ile Thr  Gln Tyr Ile Thr Gln  Ile Gln Gly
    2585                2590                2595

cag tct  gtg gtt aat gcg cta  gaa aat gca ggc ggg  ccg ggt gtg        7839
Gln Ser  Val Val Asn Ala Leu  Glu Asn Ala Gly Gly  Pro Gly Val
    2600                2605                2610

atg aat  tgg ttt ggt aag ctt  tta att gag aca aaa  aac acc ccg        7884
Met Asn  Trp Phe Gly Lys Leu  Leu Ile Glu Thr Lys  Asn Thr Pro
    2615                2620                2625

ctt ttt  gcg ccc tac tat ctt  gaa caa cac tcc tta  agc gat cta        7929
Leu Phe  Ala Pro Tyr Tyr Leu  Glu Gln His Ser Leu  Ser Asp Leu
    2630                2635                2640

ctt aaa  att gta aaa gat att  caa aat gcg act gat  tgg atg ggc        7974
Leu Lys  Ile Val Lys Asp Ile  Gln Asn Ala Thr Asp  Trp Met Gly
    2645                2650                2655

gct tct  ggt tta aaa gcc act  agc tct aaa ttg cta  caa atc agt        8019
Ala Ser  Gly Leu Lys Ala Thr  Ser Ser Lys Leu Leu  Gln Ile Ser
    2660                2665                2670

gta cac  acc aaa cag atg agc  cgt tta gct aag ctt  tca aat ttt        8064
Val His  Thr Lys Gln Met Ser  Arg Leu Ala Lys Leu  Ser Asn Phe
    2675                2680                2685

gct tct  aat gat gca tta cca  gat ttt cat gat ttt  tta gtc agt        8109
Ala Ser  Asn Asp Ala Leu Pro  Asp Phe His Asp Phe  Leu Val Ser
    2690                2695                2700

ctt aaa  ggt aaa aaa ttt gct  agc gcg gtg cct aat  gct atg gat        8154
Leu Lys  Gly Lys Lys Phe Ala  Ser Ala Val Pro Asn  Ala Met Asp
    2705                2710                2715

att atc  acc gcc tac tcc caa  aga gat aaa tta aaa  aac aac cta        8199
```

```
Ile Ile  Thr Ala Tyr Ser Gln  Arg Asp Lys Leu Lys  Asn Asn Leu
    2720                 2725                 2730

tgg gta  acc ggt gtg gga gga  gca agt ttt gta gca  gga ggc aca      8244
Trp Val  Thr Gly Val Gly Gly  Ala Ser Phe Val Ala  Gly Gly Thr
    2735                 2740                 2745

ggt acg  ctt tat ggg ctg aat  gtg ggt tat gat cgc  ttt ata aag      8289
Gly Thr  Leu Tyr Gly Leu Asn  Val Gly Tyr Asp Arg  Phe Ile Lys
    2750                 2755                 2760

ggc gtg  att gtg ggg ggt tat  atg gct tat ggt tat  agt ggc ttt      8334
Gly Val  Ile Val Gly Gly Tyr  Met Ala Tyr Gly Tyr  Ser Gly Phe
    2765                 2770                 2775

tat ggc  aat atc aat agc gct  aat tct aat aat gtc  aat gtg gga      8379
Tyr Gly  Asn Ile Asn Ser Ala  Asn Ser Asn Asn Val  Asn Val Gly
    2780                 2785                 2790

ttt tat  agc cgc gcc ttt atc  aag ggt aga aac gag  att aca gga      8424
Phe Tyr  Ser Arg Ala Phe Ile  Lys Gly Arg Asn Glu  Ile Thr Gly
    2795                 2800                 2805

agt att  aat gaa acc tat ggc  tat aac aaa acc tac  att gat gca      8469
Ser Ile  Asn Glu Thr Tyr Gly  Tyr Asn Lys Thr Tyr  Ile Asp Ala
    2810                 2815                 2820

act aat  ccc att ctc acc cct  ctt aac cag caa tac  cac tat ggc      8514
Thr Asn  Pro Ile Leu Thr Pro  Leu Asn Gln Gln Tyr  His Tyr Gly
    2825                 2830                 2835

act tgg  act acc aat gtc ggc  gct aac tat ggc tat  gac ttc ttt      8559
Thr Trp  Thr Thr Asn Val Gly  Ala Asn Tyr Gly Tyr  Asp Phe Phe
    2840                 2845                 2850

ttt aaa  aac aaa cat gtt att  ctc aaa ccc caa att  ggc ctc act      8604
Phe Lys  Asn Lys His Val Ile  Leu Lys Pro Gln Ile  Gly Leu Thr
    2855                 2860                 2865

tat tat  tat atc ggc ctc tca  ggc ttg caa ggc aag  atg aat gat      8649
Tyr Tyr  Tyr Ile Gly Leu Ser  Gly Leu Gln Gly Lys  Met Asn Asp
    2870                 2875                 2880

ccg att  tat aac gag ttt aga  gcc aat gcc gat cca  gcg cat aaa      8694
Pro Ile  Tyr Asn Glu Phe Arg  Ala Asn Ala Asp Pro  Ala His Lys
    2885                 2890                 2895

tct att  ttg acg atc aat tta  gcc cta gag agt cgc  cac tat ttt      8739
Ser Ile  Leu Thr Ile Asn Leu  Ala Leu Glu Ser Arg  His Tyr Phe
    2900                 2905                 2910

agg aaa  aac tcc tat tac tat  gtc att gcc ggc tta  ggg cgg gat      8784
Arg Lys  Asn Ser Tyr Tyr Tyr  Val Ile Ala Gly Leu  Gly Arg Asp
    2915                 2920                 2925

tta ttc  gtc cat tct atg ggt  gat aaa atg gta cgc  ttt att ggt      8829
Leu Phe  Val His Ser Met Gly  Asp Lys Met Val Arg  Phe Ile Gly
    2930                 2935                 2940

aat gat  atg tta agt tat cgt  tat ggg gga atg tat  aat acc ttt      8874
Asn Asp  Met Leu Ser Tyr Arg  Tyr Gly Gly Met Tyr  Asn Thr Phe
    2945                 2950                 2955
```

```
gct agc  ctc acc aca ggg ggt  gag gtt cgc tta ttt  cga tcc ttt        8919
Ala Ser  Leu Thr Thr Gly Gly  Glu Val Arg Leu Phe  Arg Ser Phe
    2960                 2965                 2970

tat gtc  aat gct ggt att gga  gcg cgc ttt ggt ttg  gat tat caa        8964
Tyr Val  Asn Ala Gly Ile Gly  Ala Arg Phe Gly Leu  Asp Tyr Gln
    2975                 2980                 2985

gat att  aat att aca ggc aat  gtc ggg atg cgc tat  gct ttt tag        9009
Asp Ile  Asn Ile Thr Gly Asn  Val Gly Met Arg Tyr  Ala Phe
    2990                 2995                 3000


<210>  28
<211>  3002
<212>  PRT
<213>  Helicobacter suis

<400>  28

Met Lys Lys Phe Ser Ser Leu Thr Leu Lys Phe Gly His Ala Leu Ala
1               5                   10                  15

Arg Arg Ile Lys Ala Asn Gln Ala Arg Arg Ala Gly Thr Tyr Val Phe
            20                  25                  30

Lys Lys Gln Leu Pro Gln Pro Lys Arg Ser Glu Leu Lys Pro Lys Leu
        35                  40                  45

Ile Lys Gln Gly Thr Phe Ile Leu Gly Val Met Ser Gln Pro Leu Leu
    50                  55                  60

Ala Trp Tyr Pro Ser Trp Ile Ser Gly Thr His Thr Leu Asn Thr Thr
65                  70                  75                  80

Asn Ile Lys Gln Tyr Met Gln Asn Asn His Arg Ser Gln Asn Leu Leu
                85                  90                  95

Trp Thr Gly Gly Ser Asn Val Phe Tyr Glu Ala Ser Asn Gly Ser Tyr
            100                 105                 110

Phe Cys Thr Asn Trp Asn Cys Asn Gly Ser Val Thr Leu Ile Gly Asn
        115                 120                 125

Gly Ser Thr Ala Tyr Thr Leu Ser Asn Leu Asn Tyr Glu Gly Gly Ser
    130                 135                 140

Leu Asn Leu Gln Ile Asn Gly Asn Gly Thr Gln Gly Thr Leu Asn Ile
145                 150                 155                 160

Ser Asn Val Asn Met Asp Ser Tyr Lys Gly Arg Gln Phe Thr Asp Thr
                165                 170                 175
```

```
Trp Asn Ala Gln Ser Val Ser Ile Ser Gly Asn Leu Gln Val Gly Gln
            180                 185                 190

Asn His Ile Thr Ile Asn Ala Asn His Gly Thr Thr Ala Asn Asn Ala
        195                 200                 205

Thr Ile Asn Ala Asp Gln Gly Asn Gly Ile Leu Asn Ile Asn Asp Lys
    210                 215                 220

Thr Ser Glu Ser Phe Thr Asn Thr Thr Phe Lys Gly Thr Gly Gln Ile
225                 230                 235                 240

Asn Leu Gln Ser Asn Asn Ile Thr Phe Lys Asn Val Thr Phe Asn Asp
                245                 250                 255

Ser Asn Thr Gly Ser His Val Thr Asp Asn Gly Thr Leu Thr Leu Glu
            260                 265                 270

Gly Thr Glu Thr Phe Ala Gln Asn Ser Pro Leu Ile Asn Leu Gly Ala
        275                 280                 285

Asn Val Thr Ile Gln Ala Asn Thr Ile Phe Asn Ile Thr Glu Asp Leu
    290                 295                 300

Thr Lys Thr Thr Asn Gly Ala Tyr Asn Leu Asp Thr Leu Val Ser Thr
305                 310                 315                 320

Ser Gly Asn Lys Ser Ile Asn Asp Ser Ser Tyr Ala Ser His Leu Trp
                325                 330                 335

Asp Leu Ile Arg Tyr Gln Gly Gln Thr Gly Ser Leu Phe Asn Gly Gln
            340                 345                 350

Leu Ser Asn Gly Thr Thr Leu Ser Asn Pro Ser Ser Gly Asn Gly Ile
        355                 360                 365

Tyr Tyr Val Lys Tyr Thr Phe Gly Asn Gly Asp Trp Asp Ile Leu Lys
    370                 375                 380

Glu Asp Phe Glu Asn Asn Ser Leu Ser Ala Gln Leu Glu Ala Tyr Ala
385                 390                 395                 400

Lys Asn Gln Gly Asp Ile Trp Ser Thr Ile His Asn Ile Asn Ser Thr
                405                 410                 415

Trp Asn Phe Asn Val Gly Glu Gly Ser Ala Tyr Ile Asn Pro Gly Asn
            420                 425                 430
```

```
Gly Thr Asp Gln Ala Trp Ala Gln Arg Asp Lys Asn Phe Lys Val Thr
        435                 440                 445

Leu Asp Asn Gly Ser Gly Gly Thr Leu Ile Leu Gly Asn Ser Thr Glu
    450                 455                 460

Thr Pro Ser Ser Ser Gly Lys Ile Leu Phe Gly Gly Thr Gly Gly Asn
465                 470                 475                 480

Pro Phe Ala Leu Asn Gly Asn Tyr Gly Gly Asp Leu Gly Tyr Met Thr
                485                 490                 495

Gly Glu Phe Asp Ala Gly Lys Ile Tyr Leu Thr Gly Thr Ile Glu Ser
            500                 505                 510

Gly Asn Ser Phe His Asp Gly Asn Gly Thr Asn Ile Ser Tyr Asn Ala
        515                 520                 525

Ile Thr Asn Ile Thr Ala Asn Gly Leu His Tyr Leu Asn Asp Asn Ala
    530                 535                 540

Gly Val Trp His Ser Asn Ala Asn Phe Arg Ala Thr Asn Gly Ser Ile
545                 550                 555                 560

Asn Ile Ser Asn Ser Gln Phe Gln Asp Gln Ser Ser Gly Gln Phe Thr
                565                 570                 575

Phe Asn Ser Gln Asn Gln Thr Phe Ser Ser Thr Thr Phe Thr Gly Asn
            580                 585                 590

Ser His Thr Ile Thr Leu His Ala Thr Asn Asn Leu Thr Leu Thr Asn
        595                 600                 605

Thr Ser Phe Asn Asp Ser Asn Ala Ser Leu Thr Leu Glu Ala Asp Lys
    610                 615                 620

Gly Ser Leu Gln Asp Thr Asp Asn Gln Thr Ser Gln Ile Thr Ala Lys
625                 630                 635                 640

Asn Leu Gln Val Ile Ala Asn Gln Ala Ser Phe Ser Asn Thr Ile Phe
                645                 650                 655

Asn Ala Gln Asn Ser Ser Phe Lys Thr Asn Gln Leu Thr Leu Thr Asn
            660                 665                 670

Asp Thr Phe Asn Asn Gly Ser Tyr Ser Phe Ala Pro Glu Asn Asn Gly
```

675 680 685

Asn His Thr Thr Thr Phe Ser Gly Thr Thr Thr Ile Asn Thr Ser Ser
690 695 700

Ser Pro Phe Ala Asn Leu Gly Gly Ala Ile Lys Phe Asn Ser Gly Ala
705 710 715 720

Thr Phe Asn Leu Asn Asn Ile Leu Ser Ser Leu Gln Ile Gly Thr Thr
725 730 735

Tyr Ser Ile Leu Gly Gly Ser Gly Ala Asn Ile Asn Tyr Ser Ser Asp
740 745 750

Thr Gln Tyr Ala Asn Asn Leu Trp Asn Leu Ile Arg Ile Ser Gly Ala
755 760 765

Ser Ile Ser Ser Glu Thr Glu Met Ser Asp Ser Asn Gly Thr Gln Val
770 775 780

Trp Asp Val Val Phe Gly Ile Asn Gly Met Pro Ile Lys Ile Gln Glu
785 790 795 800

Thr Phe Ala Ser Ser Gly Leu Ser Leu Lys Val Ile Ser Gln Ala Lys
805 810 815

Asp Ile Trp Ser Asp Val Tyr Asn Met Thr Thr Asn Cys Ser Tyr Asn
820 825 830

Ala Leu Ala Ser Pro Pro Gly Cys Tyr Gly Tyr Gln Asn Gly Thr Tyr
835 840 845

Asn Ile Trp Lys His Phe Asn Lys Tyr Gly Phe Glu Ala Tyr Asn Glu
850 855 860

Ser Val Gly Ala Asp Gly Ile Ala Tyr Ile Asn Pro Leu Asn Ser Gln
865 870 875 880

Ser His Asp Gly Tyr Asn Ala Ser Thr His Thr Leu Gln Thr Tyr Thr
885 890 895

Lys Leu Gly Asn Gly Gly Thr Tyr Asn Val Gly Glu Lys Lys Asn Ser
900 905 910

Gln Trp Val Asn Asp Gly Thr Leu Ile Leu Gly Asn Asn Thr Leu Lys
915 920 925

```
Ala Ala Thr Gly Gly Lys Ile Glu Phe Gly Ala Val Gly Ser Val Gly
    930                 935                 940

Tyr Ile Thr Asp Val Phe Asn Ala Gly Asn Ile Phe Leu Thr Asn Thr
945                 950                 955                 960

Ile Glu Val Gly Asp Ser Ser Leu Ser Gly Ala Gly Ala Thr Leu Thr
                965                 970                 975

Phe Asn Ala Asn Asn Asn Ile Thr Ala Asp Gly Leu Thr Tyr His Gln
            980                 985                 990

Asp Ala Thr Ala Ile Pro Pro Phe  Gly Ser Thr Ile Ser  Gln His Ser
        995                 1000                 1005

His Gly  Asn Phe Ile Ala Gln  Gln Ser Phe Ser Ala  Leu Asn Ser
    1010                 1015                 1020

Ser Phe  Glu Asp Asp Thr Ser  Gly Ser Leu Asp Phe  Thr Gly Lys
    1025                 1030                 1035

Asn Ser  Ile Lys Phe Thr Ser  Ser Thr Val Ile Gly  Ser Lys Ser
    1040                 1045                 1050

Ser Ile  Thr Leu Asn Ser Ala  Asn Thr Thr Leu Asn  Asn Ser Ala
    1055                 1060                 1065

Phe Phe  Val Gly Asn Gly Thr  Thr Leu Ile Phe Gly  Asn Val Ile
    1070                 1075                 1080

Thr Thr  Asn Ser His Ser Ser  Tyr Ser Ser Ser Thr  Asn Thr Ile
    1085                 1090                 1095

Asn Asn  Ser Thr Ile Asn Leu  Asn Gln Asn Ser Ile  Leu Tyr Leu
    1100                 1105                 1110

His Gly  Ser Thr Thr Leu Asp  Asn Thr Thr Ser Phe  Leu Asn Leu
    1115                 1120                 1125

Gly Ser  Gln Ala Thr Phe Tyr  Asp Ser Ala Ser Leu  Ser Gly Ser
    1130                 1135                 1140

Thr Thr  Ile Asn Leu Asp His  Asn Ser Lys Ile Ile  Met Asn Ser
    1145                 1150                 1155

Thr Thr  Thr Leu Asn Asp Asn  Ala Asn Phe Asn Leu  Ile Asn Ser
    1160                 1165                 1170
```

Ala Gln Ala Asn Phe Gln Gly Asn Ser Thr Phe Asn Asn Ser Ser
1175 1180 1185

Gly Ile Thr Leu Ala Ser Gly Ala Lys Ala Met Phe Thr His Thr
1190 1195 1200

Thr Thr Ser Thr Gln Asp Ile Thr Thr Phe Asn Asn Asn Ser Phe
1205 1210 1215

Leu Asn Val Asn Gly Ser Phe Thr Asp Phe Ile Pro Asn Thr Ser
1220 1225 1230

Ser Gly Ser Ile Ser Gly Gly Gly Ser Ser Ser Asn Gln Asn Tyr
1235 1240 1245

Ser Ala Gln Phe Asn Asn Leu Val Phe Asn Asn Tyr Ala Ser Met
1250 1255 1260

Leu Leu Asn Ser Ala Asp Ile Gln Ser Ser Gln Thr Thr Phe Ala
1265 1270 1275

Asn Ser Val Asn Ile Asn Met Gln Asn Ser Leu Phe Asn Ala Gly
1280 1285 1290

Thr Leu Asn Leu Asn Gly Gly Asn Phe Tyr Met Gln Asn Ser Gln
1295 1300 1305

Ile Lys Ala Gly Ala Val Asn Val Gly Ser Ser Asp Ser Val Asn
1310 1315 1320

Ile Asn Ser Gly Val Asn Ser Leu Asn Arg Ser Leu Ile Thr Ser
1325 1330 1335

Ser Ser Phe Asn Leu Asn Gly Thr Leu Asn Leu Asp Gly Leu Leu
1340 1345 1350

Leu Glu Pro Thr Thr Ser Thr Gly Ala Ser Ser Ser Ala Ala Asn
1355 1360 1365

Ser Gln Pro Leu Ile Ser Val Ser Asn Asn Ser Ser Ser Ser Thr
1370 1375 1380

Gly Gly Gly Thr Phe Asp Leu Ser Gly Ile Leu Asn Ile Ser Asn
1385 1390 1395

Ile Asp Leu Ser Ala Pro Phe Ser Ser Gln Thr Asn Asn Thr Ser
1400 1405 1410

Ser Ser Ala Thr Tyr Asn Ile Val Asn Ala Ser Lys Ile Thr Gly
1415 1420 1425

Met Ser Gly Ala Asn Gly Tyr Gln Lys Ile Glu Tyr Tyr Gly Ile
1430 1435 1440

Lys Ile Asn Asn Ala Thr Tyr Ser Asp Ser Asn Lys Thr Gln Ser
1445 1450 1455

Trp Ser Phe Thr Asn Pro Leu Asn Gly Ala Gln Thr Ile Thr Glu
1460 1465 1470

Lys Ile Gln Asn Gly Lys Leu Thr Ile Thr Ile Ser Asn Ser Asn
1475 1480 1485

His Phe Val Ala Thr Asp Tyr His Asn Ile Ala Pro Glu Leu Phe
1490 1495 1500

Phe Tyr Lys Gln Ser Ala Gln Asn Leu Pro Ser Thr Asn Ser Ala
1505 1510 1515

Ser Ala Asp Ile Ser Ser Tyr Asp Tyr Ser Ser Asp Glu Ser Gly
1520 1525 1530

Thr Phe Phe Leu Asp Gly Asn Leu Lys Gly Val Tyr Asp Pro Lys
1535 1540 1545

Ala Asn Thr Ser Ser Ser Lys Thr Ser Ser Thr Pro Val Ile Pro
1550 1555 1560

Gly Thr Tyr Asn Ala Gln Gly Gln Pro Leu Gln Gly Leu Tyr Ile
1565 1570 1575

Ser Asn Asn Gly Leu Phe Asn Glu Thr Thr Leu Asn Asn Leu Val
1580 1585 1590

Gly Ile Val Gln Ser Ile Trp Pro His Leu Lys Thr Leu Leu Pro
1595 1600 1605

Gln Ile Leu Gln Asp Leu Ser Asp Pro Ser Asn Ile Ile Gln Asp
1610 1615 1620

Leu Glu Asn Ser Gly Ile Lys Leu Thr Ser Gln Gln Ser Lys Glu
1625 1630 1635

Leu Leu Ser Phe Ile Asp Gly Leu Ser Ser Asn Ile Asn Gln Thr

1640 1645 1650

Phe Asn Asn Gly Thr Leu Val Val Gly Ser Pro Gln Ala Gly Gln
1655 1660 1665

Thr Gly Ser Ser Ser Val Val Trp Phe Gly Gly Asn Gly Tyr Thr
1670 1675 1680

Thr Ala Cys Thr Ala Ala Gln Thr Lys Lys Gly Cys Gln Asp Leu
1685 1690 1695

Arg Gly Thr Tyr Leu Gly Glu Leu Leu Gly Ser Thr Ser Ala Ala
1700 1705 1710

Leu Gly Tyr Ile Glu Ala Asn Phe Lys Ala Lys Asp Ile Tyr Ile
1715 1720 1725

Thr Gly Thr Val Gly Ser Gly Asp Ala Trp Gly Ile Gly Gly Ser
1730 1735 1740

Ala Asp Val Thr Phe Asn Ser Ala Thr Asn Leu Thr Leu Asn Gln
1745 1750 1755

Ala Thr Ile Asp Ala Glu Gly Thr Asp Gln Ile Phe Asn Met Leu
1760 1765 1770

Gly Glu Gly Gly Ile Gln Lys Ile Leu Gly Gln Lys Gly Leu Ala
1775 1780 1785

Gln Met Leu Gly Asn Tyr Ile Tyr Asp Leu Ala Asn Gly Ser Ser
1790 1795 1800

Ile Asn Leu Asn Pro Thr Ser Ser Ile Pro Ser Ser Ile Lys Pro
1805 1810 1815

Leu Ala Lys Asp Leu Gly Gly Gln Ile Gly Lys Ile Lys Leu Ser
1820 1825 1830

Asp Val Leu Asn Ala Ser Asp Val Ser Ala Leu Leu Asn Met Pro
1835 1840 1845

Gly Met Asp Asn Val Ile Lys Asn Ile Leu Ser Thr Lys Thr Val
1850 1855 1860

Ser Ser Val Leu Gly Gly Gly Gly Leu Ile Ser Ser Leu Asp Gln
1865 1870 1875

Ala Glu Gln Asn Lys Ile Tyr Asn Ala Ile Asp Lys Glu Leu His
1880 1885 1890

Phe Ser Gly Gly Lys Ala Ile Ala Ser Ile Ala Asn Gly Val Tyr
1895 1900 1905

Gly Lys His Thr Leu Leu Ser Leu Ile Asn Ser Leu Ser Pro Met
1910 1915 1920

Thr Gly Lys Asp Val Asp Asn Ile Leu Asn Met Pro Asn Thr Ser
1925 1930 1935

Ser Gly Gln Ser Gln Val Lys Asn Phe Leu Asn Lys Thr Thr Phe
1940 1945 1950

Gly Gln Ile Phe Glu Glu Leu Leu Ser Asn Gln Asn Leu Leu Asn
1955 1960 1965

Lys Thr Ile Ala Trp Leu Gly Pro Gln Ile Leu Asp Glu Met Leu
1970 1975 1980

Lys Thr Ala Ile Asp Asp Leu Leu Asn Pro Thr Asn Gln Leu Thr
1985 1990 1995

Ala Ala Glu Lys Asn Ile Leu Asp Asn Ile Leu Asn Asn Val Phe
2000 2005 2010

Ser Ser Glu Lys Lys Ala Val Gln Gln Met Glu Asn Ser Asn Pro
2015 2020 2025

Asp Val Lys His Val Ile Asp Gly Leu Met Gln Ala Lys Gly Leu
2030 2035 2040

Gly Glu Val Tyr Ser Lys Gly Leu Gln Ser Ile Leu Ser Asn Lys
2045 2050 2055

Leu Gln Gly Gln Leu Lys Ser Met Gly Leu Gly Ser Leu Leu Ala
2060 2065 2070

Pro Lys Ala Leu Gly Asn Phe Trp Gln Lys Gly Tyr Phe Asn Phe
2075 2080 2085

Leu Ala Asn Asp Asp Ile Leu Val Asn Asn Ser Thr Phe Ser Asn
2090 2095 2100

Ala Ser Gly Gly Thr Leu Ser Phe Ile Ala Gly Lys Ser Ile Ile
2105 2110 2115

Phe Ala Gly Gln Asn Thr Ile Asn Phe Ser Asn Asn Gln Gly Thr
2120 2125 2130

Leu Glu Phe Leu Ser Asn Asp Val Ser Asn Ile Asp Leu Thr Thr
2135 2140 2145

Leu Asn Ala Thr Asp Gly Leu Thr Ile Asp Ala Pro Phe Asn Asn
2150 2155 2160

Leu Tyr Val Gln Lys Gly Asn Ile Thr Leu Ala Ser Tyr Glu Gly
2165 2170 2175

Leu Thr Val Arg Ala Asn Asn Phe Asp Phe Leu Gly Thr Val Gln
2180 2185 2190

Ala Asp Gly Ala Val Asp Leu Ser Gly Val Thr Gly Leu Ala Val
2195 2200 2205

Leu Gly Thr Leu Asn Leu Thr Glu Asp Ser Thr Leu Lys Ala Asn
2210 2215 2220

Asn Leu Thr Thr Ile Ser Ala Phe Asn Asn Gln Ser Gln Asn Leu
2225 2230 2235

Leu Asn Ile Ser Gly Asn Phe Asn Ser Tyr Gly Thr Phe Ser Thr
2240 2245 2250

Gln Gly Ala Gly Val Asn Ile Gly Gly Gly Phe Asn Ser Thr Gly
2255 2260 2265

Ala Leu Thr Phe Asn Val Ala Gly Ala Thr Ile Lys Thr Thr Gly
2270 2275 2280

Pro Ser Ser Asp Asn Ser Thr Ser Ser Ser Ser Ala Ser Thr Ser
2285 2290 2295

Ser Ser Asn Ala Asn Ser Ser Gly Gly Ser Ser Ser Ser Pro Ser
2300 2305 2310

Thr Ser Thr Pro Ser Thr Asn Ser Ser Ala Ser Thr Ser Ser Lys
2315 2320 2325

Thr Ala Thr Ala Leu Thr Leu Ala Ser Ile Thr Val Ser Thr Ser
2330 2335 2340

Ser Thr Thr Ser Ser Thr Ala Ser Ser Ser Thr Ser Thr Ser Ser
2345 2350 2355

Ser Asn Thr Ser Ser Ser Ser Asp Asn Ser Pro Ser Ser Asn Ser
2360 2365 2370

Gly Ser Asn Val Asn Pro Val Ser Pro Thr Pro Ser Thr Gln Ile
2375 2380 2385

Pro Leu Ile Gln Val Gly Gly Leu Val Asn Leu Asn Leu Gly Gly
2390 2395 2400

Ser Ala Ile Ile Ser Phe Asn Thr Glu Ala Gln Thr Asn Asn Ala
2405 2410 2415

Gly Ser Ser Ala Gly Ser Ser Ser Thr Ser Thr Pro Gln Thr Thr
2420 2425 2430

Thr Ser Ser Ser Thr Ser Ser Thr Gln Thr Ala Ser Thr Ala Gln
2435 2440 2445

Ser Thr Thr Leu Ser Leu Ser Ser Asn Thr Ser Leu Ala Thr Thr
2450 2455 2460

Ser Gln Thr Pro Thr Thr Ser Ser Gly Ala Ser Pro Asp Ser Ser
2465 2470 2475

Asn Pro Thr Ala Ser Pro Ile Thr Pro Ser Asn Gly Ala Tyr Thr
2480 2485 2490

Leu Ile Gln Thr Asn Ser Trp Ile His Tyr Asn Pro Thr Ser Phe
2495 2500 2505

Asn Pro Asn Asn Trp Arg Gln Tyr Leu Glu Leu Tyr Thr Ser Leu
2510 2515 2520

Lys Ile Asn Gly Thr Ala Phe Gln Leu Asn Ala Gln Gly Thr Gly
2525 2530 2535

Leu Thr Tyr Asn Gly Gln Ala Val Asn Ile Ser Gln Arg Gly Leu
2540 2545 2550

Leu Val Asn Tyr Gln Gly Thr Asn Gly Gln Glu Val Ser Ala Ser
2555 2560 2565

Ile Asp Tyr Asn Lys Met Gln Ile Gly Ile Gly Gln Ser Leu His
2570 2575 2580

Val Ile Ala Pro Thr Ile Thr Gln Tyr Ile Thr Gln Ile Gln Gly

2585 2590 2595

Gln Ser Val Val Asn Ala Leu Glu Asn Ala Gly Gly Pro Gly Val
2600 2605 2610

Met Asn Trp Phe Gly Lys Leu Leu Ile Glu Thr Lys Asn Thr Pro
2615 2620 2625

Leu Phe Ala Pro Tyr Tyr Leu Glu Gln His Ser Leu Ser Asp Leu
2630 2635 2640

Leu Lys Ile Val Lys Asp Ile Gln Asn Ala Thr Asp Trp Met Gly
2645 2650 2655

Ala Ser Gly Leu Lys Ala Thr Ser Ser Lys Leu Leu Gln Ile Ser
2660 2665 2670

Val His Thr Lys Gln Met Ser Arg Leu Ala Lys Leu Ser Asn Phe
2675 2680 2685

Ala Ser Asn Asp Ala Leu Pro Asp Phe His Asp Phe Leu Val Ser
2690 2695 2700

Leu Lys Gly Lys Lys Phe Ala Ser Ala Val Pro Asn Ala Met Asp
2705 2710 2715

Ile Ile Thr Ala Tyr Ser Gln Arg Asp Lys Leu Lys Asn Asn Leu
2720 2725 2730

Trp Val Thr Gly Val Gly Gly Ala Ser Phe Val Ala Gly Gly Thr
2735 2740 2745

Gly Thr Leu Tyr Gly Leu Asn Val Gly Tyr Asp Arg Phe Ile Lys
2750 2755 2760

Gly Val Ile Val Gly Gly Tyr Met Ala Tyr Gly Tyr Ser Gly Phe
2765 2770 2775

Tyr Gly Asn Ile Asn Ser Ala Asn Ser Asn Asn Val Asn Val Gly
2780 2785 2790

Phe Tyr Ser Arg Ala Phe Ile Lys Gly Arg Asn Glu Ile Thr Gly
2795 2800 2805

Ser Ile Asn Glu Thr Tyr Gly Tyr Asn Lys Thr Tyr Ile Asp Ala
2810 2815 2820

Thr Asn Pro Ile Leu Thr Pro Leu Asn Gln Gln Tyr His Tyr Gly
2825 2830 2835

Thr Trp Thr Thr Asn Val Gly Ala Asn Tyr Gly Tyr Asp Phe Phe
2840 2845 2850

Phe Lys Asn Lys His Val Ile Leu Lys Pro Gln Ile Gly Leu Thr
2855 2860 2865

Tyr Tyr Tyr Ile Gly Leu Ser Gly Leu Gln Gly Lys Met Asn Asp
2870 2875 2880

Pro Ile Tyr Asn Glu Phe Arg Ala Asn Ala Asp Pro Ala His Lys
2885 2890 2895

Ser Ile Leu Thr Ile Asn Leu Ala Leu Glu Ser Arg His Tyr Phe
2900 2905 2910

Arg Lys Asn Ser Tyr Tyr Tyr Val Ile Ala Gly Leu Gly Arg Asp
2915 2920 2925

Leu Phe Val His Ser Met Gly Asp Lys Met Val Arg Phe Ile Gly
2930 2935 2940

Asn Asp Met Leu Ser Tyr Arg Tyr Gly Gly Met Tyr Asn Thr Phe
2945 2950 2955

Ala Ser Leu Thr Thr Gly Gly Glu Val Arg Leu Phe Arg Ser Phe
2960 2965 2970

Tyr Val Asn Ala Gly Ile Gly Ala Arg Phe Gly Leu Asp Tyr Gln
2975 2980 2985

Asp Ile Asn Ile Thr Gly Asn Val Gly Met Arg Tyr Ala Phe
2990 2995 3000

<210> 29
<211> 4593
<212> DNA
<213> Helicobacter suis

<220>
<221> CDS
<222> (1)..(4593)

<400> 29
att act gaa aaa ata caa aat ggt aaa ctc act atc acc att agt aat 48
Ile Thr Glu Lys Ile Gln Asn Gly Lys Leu Thr Ile Thr Ile Ser Asn
1 5 10 15

```
agc aat cat ttt gta gct aca gat tac cac aac atc gcc ccc gaa ctc          96
Ser Asn His Phe Val Ala Thr Asp Tyr His Asn Ile Ala Pro Glu Leu
            20                  25                  30

ttt ttt tac aaa caa tcc gca caa aac ttg cct agt aca gac tca gta         144
Phe Phe Tyr Lys Gln Ser Ala Gln Asn Leu Pro Ser Thr Asp Ser Val
        35                  40                  45

agt gca gat ata agc agt tat gat tat tcc agc gat gaa agc ggg act         192
Ser Ala Asp Ile Ser Ser Tyr Asp Tyr Ser Ser Asp Glu Ser Gly Thr
    50                  55                  60

ttc ttt tta gat ggc aat tta aaa ggg gtg tat tat cca aaa tct agc         240
Phe Phe Leu Asp Gly Asn Leu Lys Gly Val Tyr Tyr Pro Lys Ser Ser
65                  70                  75                  80

aca aca ggc act acc tca acc cca gtt att ccg ggt act tat aac gct         288
Thr Thr Gly Thr Thr Ser Thr Pro Val Ile Pro Gly Thr Tyr Asn Ala
                85                  90                  95

caa ggc cag ccc tta caa ggt ctt tat att tct aat aac ggc ctg ttt         336
Gln Gly Gln Pro Leu Gln Gly Leu Tyr Ile Ser Asn Asn Gly Leu Phe
            100                 105                 110

aat gaa acc acc ctt aat aac tta gta ggc att gtc caa agt att tgg         384
Asn Glu Thr Thr Leu Asn Asn Leu Val Gly Ile Val Gln Ser Ile Trp
        115                 120                 125

cct cat tta aaa acc ctt ttg cct caa att cta caa gat tta agc gat         432
Pro His Leu Lys Thr Leu Leu Pro Gln Ile Leu Gln Asp Leu Ser Asp
    130                 135                 140

cca agt aat atc att caa gac tta gaa aac tca agg att aaa tta act         480
Pro Ser Asn Ile Ile Gln Asp Leu Glu Asn Ser Arg Ile Lys Leu Thr
145                 150                 155                 160

agc cag caa agc aag gaa ctt tta agt ttt ata gac ggg cta tca agc         528
Ser Gln Gln Ser Lys Glu Leu Leu Ser Phe Ile Asp Gly Leu Ser Ser
                165                 170                 175

aat atc aac caa act ttt aat aat ggt acg ctt gta gtg ggt tcg cct         576
Asn Ile Asn Gln Thr Phe Asn Asn Gly Thr Leu Val Val Gly Ser Pro
            180                 185                 190

cag gca gga caa acc gga agt agt agc gtg gtg tgg ttt ggt ggc aat         624
Gln Ala Gly Gln Thr Gly Ser Ser Ser Val Val Trp Phe Gly Gly Asn
        195                 200                 205

ggt tat act acg gct tgt acg gct gca caa act gaa aaa ggg tgt cag         672
Gly Tyr Thr Thr Ala Cys Thr Ala Ala Gln Thr Glu Lys Gly Cys Gln
    210                 215                 220

gat tta aga ggc act tat tta ggt cag ctt tta gga tca act tct gct         720
Asp Leu Arg Gly Thr Tyr Leu Gly Gln Leu Leu Gly Ser Thr Ser Ala
225                 230                 235                 240

gca cta ggc tat att gag gct aat ttt aag gct aaa gat att tat atc         768
Ala Leu Gly Tyr Ile Glu Ala Asn Phe Lys Ala Lys Asp Ile Tyr Ile
                245                 250                 255

acc ggc acc gta ggc agt ggg aat gct tgg ggg ata ggt ggg agt gca         816
Thr Gly Thr Val Gly Ser Gly Asn Ala Trp Gly Ile Gly Gly Ser Ala
            260                 265                 270
```

```
gat gta act ttt aat agc gcg act aat tta acc ctc aat caa gcc acg        864
Asp Val Thr Phe Asn Ser Ala Thr Asn Leu Thr Leu Asn Gln Ala Thr
        275                 280                 285

att gat gcg gaa ggc acg gat caa att ttt aat atg cta ggt gag ggc        912
Ile Asp Ala Glu Gly Thr Asp Gln Ile Phe Asn Met Leu Gly Glu Gly
    290                 295                 300

ggg ata caa aaa atc tta ggc caa aag ggg cta gcc cag atg tta ggt        960
Gly Ile Gln Lys Ile Leu Gly Gln Lys Gly Leu Ala Gln Met Leu Gly
305                 310                 315                 320

aat tat atc tac gat cta gcc aat ggc tct agt att aat ctt aat ccc       1008
Asn Tyr Ile Tyr Asp Leu Ala Asn Gly Ser Ser Ile Asn Leu Asn Pro
                325                 330                 335

act agt agc att cca agc tct atc cag cct cta gct aaa gat tta ggc       1056
Thr Ser Ser Ile Pro Ser Ser Ile Gln Pro Leu Ala Lys Asp Leu Gly
            340                 345                 350

ggg cag att ggt aaa atc aaa ctt agc gat gta ttg aat gcc tca gat       1104
Gly Gln Ile Gly Lys Ile Lys Leu Ser Asp Val Leu Asn Ala Ser Asp
        355                 360                 365

gtg agt gct ctt tta aac atg ccc ggt atg gat aat gtg att aaa aat       1152
Val Ser Ala Leu Leu Asn Met Pro Gly Met Asp Asn Val Ile Lys Asn
    370                 375                 380

att tta agt act aag act gtg agc tct gtg cta ggc agt gga gga ctt       1200
Ile Leu Ser Thr Lys Thr Val Ser Ser Val Leu Gly Ser Gly Gly Leu
385                 390                 395                 400

att tct agt ttg aat caa gcc gag caa aat aaa atc tat gat gct att       1248
Ile Ser Ser Leu Asn Gln Ala Glu Gln Asn Lys Ile Tyr Asp Ala Ile
                405                 410                 415

gat aaa gag tta cat ttc tca ggg gga aaa gcc att gcc tcc att gcc       1296
Asp Lys Glu Leu His Phe Ser Gly Gly Lys Ala Ile Ala Ser Ile Ala
            420                 425                 430

aac ggg gtt tat ggt aag cac acc ctt tta agt ctc att aac tct tta       1344
Asn Gly Val Tyr Gly Lys His Thr Leu Leu Ser Leu Ile Asn Ser Leu
        435                 440                 445

tcc cct atg acg ggt aaa gat gtg gat aat att tta aac atg cca aat       1392
Ser Pro Met Thr Gly Lys Asp Val Asp Asn Ile Leu Asn Met Pro Asn
    450                 455                 460

act agt agc ggc caa aat cag gta caa aat ttt tta aag aac acc act       1440
Thr Ser Ser Gly Gln Asn Gln Val Gln Asn Phe Leu Lys Asn Thr Thr
465                 470                 475                 480

ttt ggg caa att ttt gaa gaa cta tta tct aat caa aac cta ctc aat       1488
Phe Gly Gln Ile Phe Glu Glu Leu Leu Ser Asn Gln Asn Leu Leu Asn
                485                 490                 495

aaa acc att gct tgg tta ggc cca caa att tta gat gaa atg ctt aaa       1536
Lys Thr Ile Ala Trp Leu Gly Pro Gln Ile Leu Asp Glu Met Leu Lys
            500                 505                 510

act gct att gat gat tta ctc aac cct aca aat cag ctt act gcc gct       1584
Thr Ala Ile Asp Asp Leu Leu Asn Pro Thr Asn Gln Leu Thr Ala Ala
```

```
        515                 520                 525

gaa aaa aat att ctt gat aac att ctt aac aat gtc ttt agc tca gaa      1632
Glu Lys Asn Ile Leu Asp Asn Ile Leu Asn Asn Val Phe Ser Ser Glu
    530                 535                 540

aaa aag gca gtc caa caa atg gaa aat tct aac cct gat gtc aaa cac      1680
Lys Lys Ala Val Gln Gln Met Glu Asn Ser Asn Pro Asp Val Lys His
545                 550                 555                 560

gtt att gat ggc tta atg cag gct aaa ggt cta ggg gag gtt tat agc      1728
Val Ile Asp Gly Leu Met Gln Ala Lys Gly Leu Gly Glu Val Tyr Ser
                565                 570                 575

aag ggc ctt cag agt att tta tct aat aaa ctg caa ggt caa tta aaa      1776
Lys Gly Leu Gln Ser Ile Leu Ser Asn Lys Leu Gln Gly Gln Leu Lys
            580                 585                 590

agc atg ggc tta ggt tct ttg ctc gcg cct aaa gct cta ggt aat ttc      1824
Ser Met Gly Leu Gly Ser Leu Leu Ala Pro Lys Ala Leu Gly Asn Phe
        595                 600                 605

tgg cag aag ggt tat ttt aac ttc cta gct aat gat gat att tta gtg      1872
Trp Gln Lys Gly Tyr Phe Asn Phe Leu Ala Asn Asp Asp Ile Leu Val
    610                 615                 620

aac aat agc act ttt agt aat gct tca ggc ggg act tta agt ttt ata      1920
Asn Asn Ser Thr Phe Ser Asn Ala Ser Gly Gly Thr Leu Ser Phe Ile
625                 630                 635                 640

gcc ggt aag tct att att ttt gca ggg caa aat aca att aat ttt agt      1968
Ala Gly Lys Ser Ile Ile Phe Ala Gly Gln Asn Thr Ile Asn Phe Ser
                645                 650                 655

aat aat caa ggt aca cta gaa ttt tta agt aat gat gtg tct aat att      2016
Asn Asn Gln Gly Thr Leu Glu Phe Leu Ser Asn Asp Val Ser Asn Ile
            660                 665                 670

gat cta act act ctt aat gct acc gat ggc cta acc att gat gcc ccc      2064
Asp Leu Thr Thr Leu Asn Ala Thr Asp Gly Leu Thr Ile Asp Ala Pro
        675                 680                 685

ttt aat aat ctt tat gtc cag aaa ggc aat atc acc ctt aat caa tac      2112
Phe Asn Asn Leu Tyr Val Gln Lys Gly Asn Ile Thr Leu Asn Gln Tyr
    690                 695                 700

gag agt ttt agc gtg cag gcg ggc aat ttt gac ttt tta ggc acg gtg      2160
Glu Ser Phe Ser Val Gln Ala Gly Asn Phe Asp Phe Leu Gly Thr Val
705                 710                 715                 720

caa gca gac ggg gct gtg gat tta tcc ggt gta acc ggt tta gct gtt      2208
Gln Ala Asp Gly Ala Val Asp Leu Ser Gly Val Thr Gly Leu Ala Val
                725                 730                 735

tta ggt act ctt aat ctt act gag gat agc acc ctt aag gct aat aat      2256
Leu Gly Thr Leu Asn Leu Thr Glu Asp Ser Thr Leu Lys Ala Asn Asn
            740                 745                 750

tta acc aca ata agc gcc ttt aat aac cag tct caa aac tta ctt aac      2304
Leu Thr Thr Ile Ser Ala Phe Asn Asn Gln Ser Gln Asn Leu Leu Asn
        755                 760                 765

atc agc ggg aat ttt aac tcc tat ggc act ttt agc acg caa ggg gct      2352
```

```
Ile Ser Gly Asn Phe Asn Ser Tyr Gly Thr Phe Ser Thr Gln Gly Ala
    770                 775                 780

ggg gta aat atc ggg ggc ggg ttt aat agt aca ggg gct tta act ttt      2400
Gly Val Asn Ile Gly Gly Gly Phe Asn Ser Thr Gly Ala Leu Thr Phe
785                 790                 795                 800

aat gta gcg ggt gca aca gtc aaa act aca ggc cct agc tca gat agt      2448
Asn Val Ala Gly Ala Thr Val Lys Thr Thr Gly Pro Ser Ser Asp Ser
                805                 810                 815

tca agt tca agc act agc tcc tct aca agt tct agt aat tct aat agc      2496
Ser Ser Ser Ser Thr Ser Ser Ser Thr Ser Ser Ser Asn Ser Asn Ser
            820                 825                 830

tct gga ggc tct agc tcc tcc cct agc act tct aca ccc tct act aac      2544
Ser Gly Gly Ser Ser Ser Ser Pro Ser Thr Ser Thr Pro Ser Thr Asn
        835                 840                 845

tct agc gcc tct act agt tct aaa aca gct aca gct tta act cta gct      2592
Ser Ser Ala Ser Thr Ser Ser Lys Thr Ala Thr Ala Leu Thr Leu Ala
    850                 855                 860

agc atc acc gtg tct aca agt tct aca act tct agt acc gct agc tct      2640
Ser Ile Thr Val Ser Thr Ser Ser Thr Thr Ser Ser Thr Ala Ser Ser
865                 870                 875                 880

agc act tct aca agt tcc tct aac acc tcc tcc agt agc gat aat agc      2688
Ser Thr Ser Thr Ser Ser Ser Asn Thr Ser Ser Ser Ser Asp Asn Ser
                885                 890                 895

cca agc tct aat agt ggg tct aat gta aat ccg gtt agc cca acg cct      2736
Pro Ser Ser Asn Ser Gly Ser Asn Val Asn Pro Val Ser Pro Thr Pro
            900                 905                 910

agt acg caa att ccg cta att caa gtg ggt gga ctt gtg aat tta aat      2784
Ser Thr Gln Ile Pro Leu Ile Gln Val Gly Gly Leu Val Asn Leu Asn
        915                 920                 925

tta ggt ggt agt gcg att att agc ttt aat aca gag gca caa aca aat      2832
Leu Gly Gly Ser Ala Ile Ile Ser Phe Asn Thr Glu Ala Gln Thr Asn
    930                 935                 940

aat gca gga tca agc gcg ggt tct agt tct aca agt aca cca caa aca      2880
Asn Ala Gly Ser Ser Ala Gly Ser Ser Ser Thr Ser Thr Pro Gln Thr
945                 950                 955                 960

aca act agc agt tct act agt tct act caa aca gca agt aca gca caa      2928
Thr Thr Ser Ser Ser Thr Ser Ser Thr Gln Thr Ala Ser Thr Ala Gln
                965                 970                 975

agt acc aca tta agc cta agc tct aat act agt cta gct act acc agc      2976
Ser Thr Thr Leu Ser Leu Ser Ser Asn Thr Ser Leu Ala Thr Thr Ser
            980                 985                 990

caa acg cct aca act tct agc ggg  gct tct ccg gat agt  tca aac cct    3024
Gln Thr Pro Thr Thr Ser Ser Gly  Ala Ser Pro Asp Ser  Ser Asn Pro
        995                 1000                 1005

aca gcc  tct cct ata acc cct  tca aat ggc gct tat  acg ctg att       3069
Thr Ala  Ser Pro Ile Thr Pro  Ser Asn Gly Ala Tyr  Thr Leu Ile
    1010                 1015                 1020
```

```
caa act  aat agc tgg att cat  tac aac ccc acc tct  ttt aat cct      3114
Gln Thr  Asn Ser Trp Ile His  Tyr Asn Pro Thr Ser  Phe Asn Pro
    1025                 1030                 1035

aat aac  tgg aga caa tat tta  gag ctc tac act agc  ctt aaa atc      3159
Asn Asn  Trp Arg Gln Tyr Leu  Glu Leu Tyr Thr Ser  Leu Lys Ile
    1040                 1045                 1050

aac ggg  aca gct ttc cag ctt  aac gct caa ggt aca  gga ctt act      3204
Asn Gly  Thr Ala Phe Gln Leu  Asn Ala Gln Gly Thr  Gly Leu Thr
    1055                 1060                 1065

tat aat  ggt caa gca gtc aat  atc tca caa cga ggc  ttg ctt gtc      3249
Tyr Asn  Gly Gln Ala Val Asn  Ile Ser Gln Arg Gly  Leu Leu Val
    1070                 1075                 1080

aat tat  caa ggc aca aat ggc  caa gaa gtg agc gcc  tct att gat      3294
Asn Tyr  Gln Gly Thr Asn Gly  Gln Glu Val Ser Ala  Ser Ile Asp
    1085                 1090                 1095

tat aat  aag atg caa ata ggc  ata ggc caa agc ttg  cat gtt atc      3339
Tyr Asn  Lys Met Gln Ile Gly  Ile Gly Gln Ser Leu  His Val Ile
    1100                 1105                 1110

gcg ccc  act att aca caa tac  atc acc caa ata caa  ggg cag tct      3384
Ala Pro  Thr Ile Thr Gln Tyr  Ile Thr Gln Ile Gln  Gly Gln Ser
    1115                 1120                 1125

gtg gtt  aat gcg cta gaa aat  gca ggc ggg ccg ggt  gtg atg aat      3429
Val Val  Asn Ala Leu Glu Asn  Ala Gly Gly Pro Gly  Val Met Asn
    1130                 1135                 1140

tgg ttt  ggt aag ctt tta att  gag aca aaa aac acc  ccg ctt ttt      3474
Trp Phe  Gly Lys Leu Leu Ile  Glu Thr Lys Asn Thr  Pro Leu Phe
    1145                 1150                 1155

gcg ccc  tac tat ctt gaa caa  cac tcc tta agc gat  cta ctt aaa      3519
Ala Pro  Tyr Tyr Leu Glu Gln  His Ser Leu Ser Asp  Leu Leu Lys
    1160                 1165                 1170

att gta  aaa gat att caa aat  gcg act gat tgg atg  ggc gct tct      3564
Ile Val  Lys Asp Ile Gln Asn  Ala Thr Asp Trp Met  Gly Ala Ser
    1175                 1180                 1185

ggt tta  aaa gcc act agc tct  aaa ttg cta caa atc  agt gta cac      3609
Gly Leu  Lys Ala Thr Ser Ser  Lys Leu Leu Gln Ile  Ser Val His
    1190                 1195                 1200

acc aaa  cag atg agc cgt tta  gct aag ctt tca aat  ttt gct tct      3654
Thr Lys  Gln Met Ser Arg Leu  Ala Lys Leu Ser Asn  Phe Ala Ser
    1205                 1210                 1215

aat gat  gca tta cca gat ttt  cat gat ttt tta gtc  agt ctt aaa      3699
Asn Asp  Ala Leu Pro Asp Phe  His Asp Phe Leu Val  Ser Leu Lys
    1220                 1225                 1230

ggt aaa  aaa ttt gct agc gcg  gtg cct aat gct atg  gat att atc      3744
Gly Lys  Lys Phe Ala Ser Ala  Val Pro Asn Ala Met  Asp Ile Ile
    1235                 1240                 1245

acc gcc  tac tcc caa aga gat  aaa tta aaa aac aac  cta tgg gta      3789
Thr Ala  Tyr Ser Gln Arg Asp  Lys Leu Lys Asn Asn  Leu Trp Val
    1250                 1255                 1260
```

```
acc ggt  gtg gga gga gca agt  ttt gta gca gga ggc  aca ggt acg        3834
Thr Gly  Val Gly Gly Ala Ser  Phe Val Ala Gly Gly  Thr Gly Thr
    1265                 1270                 1275

ctt tat  ggg ctg aat gtg ggt  tat gat cgc ttt ata  aag ggc atg        3879
Leu Tyr  Gly Leu Asn Val Gly  Tyr Asp Arg Phe Ile  Lys Gly Met
    1280                 1285                 1290

att gtg  ggg ggt tat atg gct  tat ggt tat agt ggc  ttt tat ggc        3924
Ile Val  Gly Gly Tyr Met Ala  Tyr Gly Tyr Ser Gly  Phe Tyr Gly
    1295                 1300                 1305

aat atc  aat agc gct aat tct  aat aat gtc aat gtg  gga ttt tat        3969
Asn Ile  Asn Ser Ala Asn Ser  Asn Asn Val Asn Val  Gly Phe Tyr
    1310                 1315                 1320

agc cgc  gcc ttt atc aag ggt  aga aac gag att aca  gga agt att        4014
Ser Arg  Ala Phe Ile Lys Gly  Arg Asn Glu Ile Thr  Gly Ser Ile
    1325                 1330                 1335

aat gaa  acc tat ggc tat aac  aaa acc tac att gat  gca act aat        4059
Asn Glu  Thr Tyr Gly Tyr Asn  Lys Thr Tyr Ile Asp  Ala Thr Asn
    1340                 1345                 1350

ccc att  ctc acc cct ctt aac  cag caa tac cac tat  ggc act tgg        4104
Pro Ile  Leu Thr Pro Leu Asn  Gln Gln Tyr His Tyr  Gly Thr Trp
    1355                 1360                 1365

act acc  aat gtc ggt gct aac  tat ggc tat gac ttc  ttt ttt aaa        4149
Thr Thr  Asn Val Gly Ala Asn  Tyr Gly Tyr Asp Phe  Phe Phe Lys
    1370                 1375                 1380

aac aaa  cat gtt att ctc aaa  ccc caa att ggc ctc  act tat tat        4194
Asn Lys  His Val Ile Leu Lys  Pro Gln Ile Gly Leu  Thr Tyr Tyr
    1385                 1390                 1395

tat atc  ggc ctc tca ggc ttg  caa ggc aag atg aat  gat ccg att        4239
Tyr Ile  Gly Leu Ser Gly Leu  Gln Gly Lys Met Asn  Asp Pro Ile
    1400                 1405                 1410

tat aac  gag ttt aga gcc aat  gcc gat cca gcg cat  aaa tct gtt        4284
Tyr Asn  Glu Phe Arg Ala Asn  Ala Asp Pro Ala His  Lys Ser Val
    1415                 1420                 1425

ttg acg  atc aat tta gcc cta  gag agt cgc cac tat  ttt agg aaa        4329
Leu Thr  Ile Asn Leu Ala Leu  Glu Ser Arg His Tyr  Phe Arg Lys
    1430                 1435                 1440

aac tcc  tat tac tat gtc att  gct gga ttg ggt cgg  gat tta ttc        4374
Asn Ser  Tyr Tyr Tyr Val Ile  Ala Gly Leu Gly Arg  Asp Leu Phe
    1445                 1450                 1455

gtc cat  tct atg ggc gat aaa  atg gta cgc ttt att  ggt aat gat        4419
Val His  Ser Met Gly Asp Lys  Met Val Arg Phe Ile  Gly Asn Asp
    1460                 1465                 1470

atg tta  agt tat agc tat ggg  gga atg tat aat acc  ttt gct agc        4464
Met Leu  Ser Tyr Ser Tyr Gly  Gly Met Tyr Asn Thr  Phe Ala Ser
    1475                 1480                 1485

ctc acc  aca ggg ggt gag gtt  cgc tta ttt cga tcc  ttt tat gtc        4509
Leu Thr  Thr Gly Gly Glu Val  Arg Leu Phe Arg Ser  Phe Tyr Val
```

```
    1490                1495                1500

aat gct  ggt att gga gcg cgc  ttt ggt ttg gat tat  caa gat att        4554
Asn Ala  Gly Ile Gly Ala Arg  Phe Gly Leu Asp Tyr  Gln Asp Ile
    1505                1510                1515

aat att  aca ggc aat gtc ggg  atg cgc tat gct ttt  tag                4593
Asn Ile  Thr Gly Asn Val Gly  Met Arg Tyr Ala Phe
    1520                1525                1530
```

<210> 30
<211> 1530
<212> PRT
<213> Helicobacter suis

<400> 30

```
Ile Thr Glu Lys Ile Gln Asn Gly Lys Leu Thr Ile Thr Ile Ser Asn
1               5                   10                  15

Ser Asn His Phe Val Ala Thr Asp Tyr His Asn Ile Ala Pro Glu Leu
            20                  25                  30

Phe Phe Tyr Lys Gln Ser Ala Gln Asn Leu Pro Ser Thr Asp Ser Val
        35                  40                  45

Ser Ala Asp Ile Ser Ser Tyr Asp Tyr Ser Ser Asp Glu Ser Gly Thr
    50                  55                  60

Phe Phe Leu Asp Gly Asn Leu Lys Gly Val Tyr Tyr Pro Lys Ser Ser
65                  70                  75                  80

Thr Thr Gly Thr Thr Ser Thr Pro Val Ile Pro Gly Thr Tyr Asn Ala
                85                  90                  95

Gln Gly Gln Pro Leu Gln Gly Leu Tyr Ile Ser Asn Asn Gly Leu Phe
            100                 105                 110

Asn Glu Thr Thr Leu Asn Asn Leu Val Gly Ile Val Gln Ser Ile Trp
        115                 120                 125

Pro His Leu Lys Thr Leu Leu Pro Gln Ile Leu Gln Asp Leu Ser Asp
    130                 135                 140

Pro Ser Asn Ile Ile Gln Asp Leu Glu Asn Ser Arg Ile Lys Leu Thr
145                 150                 155                 160

Ser Gln Gln Ser Lys Glu Leu Leu Ser Phe Ile Asp Gly Leu Ser Ser
                165                 170                 175

Asn Ile Asn Gln Thr Phe Asn Asn Gly Thr Leu Val Val Gly Ser Pro
```

180 185 190

Gln Ala Gly Gln Thr Gly Ser Ser Ser Val Val Trp Phe Gly Gly Asn
195 200 205

Gly Tyr Thr Thr Ala Cys Thr Ala Ala Gln Thr Glu Lys Gly Cys Gln
210 215 220

Asp Leu Arg Gly Thr Tyr Leu Gly Gln Leu Leu Gly Ser Thr Ser Ala
225 230 235 240

Ala Leu Gly Tyr Ile Glu Ala Asn Phe Lys Ala Lys Asp Ile Tyr Ile
245 250 255

Thr Gly Thr Val Gly Ser Gly Asn Ala Trp Gly Ile Gly Gly Ser Ala
260 265 270

Asp Val Thr Phe Asn Ser Ala Thr Asn Leu Thr Leu Asn Gln Ala Thr
275 280 285

Ile Asp Ala Glu Gly Thr Asp Gln Ile Phe Asn Met Leu Gly Glu Gly
290 295 300

Gly Ile Gln Lys Ile Leu Gly Gln Lys Gly Leu Ala Gln Met Leu Gly
305 310 315 320

Asn Tyr Ile Tyr Asp Leu Ala Asn Gly Ser Ser Ile Asn Leu Asn Pro
325 330 335

Thr Ser Ser Ile Pro Ser Ser Ile Gln Pro Leu Ala Lys Asp Leu Gly
340 345 350

Gly Gln Ile Gly Lys Ile Lys Leu Ser Asp Val Leu Asn Ala Ser Asp
355 360 365

Val Ser Ala Leu Leu Asn Met Pro Gly Met Asp Asn Val Ile Lys Asn
370 375 380

Ile Leu Ser Thr Lys Thr Val Ser Ser Val Leu Gly Ser Gly Gly Leu
385 390 395 400

Ile Ser Ser Leu Asn Gln Ala Glu Gln Asn Lys Ile Tyr Asp Ala Ile
405 410 415

Asp Lys Glu Leu His Phe Ser Gly Gly Lys Ala Ile Ala Ser Ile Ala
420 425 430

Asn Gly Val Tyr Gly Lys His Thr Leu Leu Ser Leu Ile Asn Ser Leu
435 440 445

Ser Pro Met Thr Gly Lys Asp Val Asp Asn Ile Leu Asn Met Pro Asn
450 455 460

Thr Ser Ser Gly Gln Asn Gln Val Gln Asn Phe Leu Lys Asn Thr Thr
465 470 475 480

Phe Gly Gln Ile Phe Glu Glu Leu Leu Ser Asn Gln Asn Leu Leu Asn
485 490 495

Lys Thr Ile Ala Trp Leu Gly Pro Gln Ile Leu Asp Glu Met Leu Lys
500 505 510

Thr Ala Ile Asp Asp Leu Leu Asn Pro Thr Asn Gln Leu Thr Ala Ala
515 520 525

Glu Lys Asn Ile Leu Asp Asn Ile Leu Asn Asn Val Phe Ser Ser Glu
530 535 540

Lys Lys Ala Val Gln Gln Met Glu Asn Ser Asn Pro Asp Val Lys His
545 550 555 560

Val Ile Asp Gly Leu Met Gln Ala Lys Gly Leu Gly Glu Val Tyr Ser
565 570 575

Lys Gly Leu Gln Ser Ile Leu Ser Asn Lys Leu Gln Gly Gln Leu Lys
580 585 590

Ser Met Gly Leu Gly Ser Leu Leu Ala Pro Lys Ala Leu Gly Asn Phe
595 600 605

Trp Gln Lys Gly Tyr Phe Asn Phe Leu Ala Asn Asp Asp Ile Leu Val
610 615 620

Asn Asn Ser Thr Phe Ser Asn Ala Ser Gly Gly Thr Leu Ser Phe Ile
625 630 635 640

Ala Gly Lys Ser Ile Ile Phe Ala Gly Gln Asn Thr Ile Asn Phe Ser
645 650 655

Asn Asn Gln Gly Thr Leu Glu Phe Leu Ser Asn Asp Val Ser Asn Ile
660 665 670

Asp Leu Thr Thr Leu Asn Ala Thr Asp Gly Leu Thr Ile Asp Ala Pro
675 680 685

Phe Asn Asn Leu Tyr Val Gln Lys Gly Asn Ile Thr Leu Asn Gln Tyr
    690                 695                 700

Glu Ser Phe Ser Val Gln Ala Gly Asn Phe Asp Phe Leu Gly Thr Val
705                 710                 715                 720

Gln Ala Asp Gly Ala Val Asp Leu Ser Gly Val Thr Gly Leu Ala Val
                725                 730                 735

Leu Gly Thr Leu Asn Leu Thr Glu Asp Ser Thr Leu Lys Ala Asn Asn
            740                 745                 750

Leu Thr Thr Ile Ser Ala Phe Asn Asn Gln Ser Gln Asn Leu Leu Asn
        755                 760                 765

Ile Ser Gly Asn Phe Asn Ser Tyr Gly Thr Phe Ser Thr Gln Gly Ala
    770                 775                 780

Gly Val Asn Ile Gly Gly Gly Phe Asn Ser Thr Gly Ala Leu Thr Phe
785                 790                 795                 800

Asn Val Ala Gly Ala Thr Val Lys Thr Thr Gly Pro Ser Ser Asp Ser
                805                 810                 815

Ser Ser Ser Ser Thr Ser Ser Ser Thr Ser Ser Ser Asn Ser Asn Ser
            820                 825                 830

Ser Gly Gly Ser Ser Ser Ser Pro Ser Thr Ser Thr Pro Ser Thr Asn
        835                 840                 845

Ser Ser Ala Ser Thr Ser Ser Lys Thr Ala Thr Ala Leu Thr Leu Ala
    850                 855                 860

Ser Ile Thr Val Ser Thr Ser Ser Thr Thr Ser Ser Thr Ala Ser Ser
865                 870                 875                 880

Ser Thr Ser Thr Ser Ser Ser Asn Thr Ser Ser Ser Ser Asp Asn Ser
                885                 890                 895

Pro Ser Ser Asn Ser Gly Ser Asn Val Asn Pro Val Ser Pro Thr Pro
            900                 905                 910

Ser Thr Gln Ile Pro Leu Ile Gln Val Gly Gly Leu Val Asn Leu Asn
        915                 920                 925

Leu Gly Gly Ser Ala Ile Ile Ser Phe Asn Thr Glu Ala Gln Thr Asn
    930                 935                 940

```
Asn Ala Gly Ser Ser Ala Gly Ser Ser Ser Thr Ser Thr Pro Gln Thr
945                 950                 955                 960

Thr Thr Ser Ser Ser Thr Ser Ser Thr Gln Thr Ala Ser Thr Ala Gln
                965                 970                 975

Ser Thr Thr Leu Ser Leu Ser Ser Asn Thr Ser Leu Ala Thr Thr Ser
            980                 985                 990

Gln Thr Pro Thr Thr Ser Ser Gly  Ala Ser Pro Asp Ser  Ser Asn Pro
        995                 1000                 1005

Thr Ala  Ser Pro Ile Thr Pro  Ser Asn Gly Ala Tyr  Thr Leu Ile
    1010                 1015                 1020

Gln Thr  Asn Ser Trp Ile His  Tyr Asn Pro Thr Ser  Phe Asn Pro
    1025                 1030                 1035

Asn Asn  Trp Arg Gln Tyr Leu  Glu Leu Tyr Thr Ser  Leu Lys Ile
    1040                 1045                 1050

Asn Gly  Thr Ala Phe Gln Leu  Asn Ala Gln Gly Thr  Gly Leu Thr
    1055                 1060                 1065

Tyr Asn  Gly Gln Ala Val Asn  Ile Ser Gln Arg Gly  Leu Leu Val
    1070                 1075                 1080

Asn Tyr  Gln Gly Thr Asn Gly  Gln Glu Val Ser Ala  Ser Ile Asp
    1085                 1090                 1095

Tyr Asn  Lys Met Gln Ile Gly  Ile Gly Gln Ser Leu  His Val Ile
    1100                 1105                 1110

Ala Pro  Thr Ile Thr Gln Tyr  Ile Thr Gln Ile Gln  Gly Gln Ser
    1115                 1120                 1125

Val Val  Asn Ala Leu Glu Asn  Ala Gly Gly Pro Gly  Val Met Asn
    1130                 1135                 1140

Trp Phe  Gly Lys Leu Leu Ile  Glu Thr Lys Asn Thr  Pro Leu Phe
    1145                 1150                 1155

Ala Pro  Tyr Tyr Leu Glu Gln  His Ser Leu Ser Asp  Leu Leu Lys
    1160                 1165                 1170

Ile Val  Lys Asp Ile Gln Asn  Ala Thr Asp Trp Met  Gly Ala Ser
```

1175 1180 1185

Gly Leu Lys Ala Thr Ser Ser Lys Leu Leu Gln Ile Ser Val His
1190 1195 1200

Thr Lys Gln Met Ser Arg Leu Ala Lys Leu Ser Asn Phe Ala Ser
1205 1210 1215

Asn Asp Ala Leu Pro Asp Phe His Asp Phe Leu Val Ser Leu Lys
1220 1225 1230

Gly Lys Lys Phe Ala Ser Ala Val Pro Asn Ala Met Asp Ile Ile
1235 1240 1245

Thr Ala Tyr Ser Gln Arg Asp Lys Leu Lys Asn Asn Leu Trp Val
1250 1255 1260

Thr Gly Val Gly Gly Ala Ser Phe Val Ala Gly Gly Thr Gly Thr
1265 1270 1275

Leu Tyr Gly Leu Asn Val Gly Tyr Asp Arg Phe Ile Lys Gly Met
1280 1285 1290

Ile Val Gly Gly Tyr Met Ala Tyr Gly Tyr Ser Gly Phe Tyr Gly
1295 1300 1305

Asn Ile Asn Ser Ala Asn Ser Asn Asn Val Asn Val Gly Phe Tyr
1310 1315 1320

Ser Arg Ala Phe Ile Lys Gly Arg Asn Glu Ile Thr Gly Ser Ile
1325 1330 1335

Asn Glu Thr Tyr Gly Tyr Asn Lys Thr Tyr Ile Asp Ala Thr Asn
1340 1345 1350

Pro Ile Leu Thr Pro Leu Asn Gln Gln Tyr His Tyr Gly Thr Trp
1355 1360 1365

Thr Thr Asn Val Gly Ala Asn Tyr Gly Tyr Asp Phe Phe Phe Lys
1370 1375 1380

Asn Lys His Val Ile Leu Lys Pro Gln Ile Gly Leu Thr Tyr Tyr
1385 1390 1395

Tyr Ile Gly Leu Ser Gly Leu Gln Gly Lys Met Asn Asp Pro Ile
1400 1405 1410

Tyr Asn Glu Phe Arg Ala Asn Ala Asp Pro Ala His Lys Ser Val
1415 1420 1425

Leu Thr Ile Asn Leu Ala Leu Glu Ser Arg His Tyr Phe Arg Lys
1430 1435 1440

Asn Ser Tyr Tyr Tyr Val Ile Ala Gly Leu Gly Arg Asp Leu Phe
1445 1450 1455

Val His Ser Met Gly Asp Lys Met Val Arg Phe Ile Gly Asn Asp
1460 1465 1470

Met Leu Ser Tyr Ser Tyr Gly Gly Met Tyr Asn Thr Phe Ala Ser
1475 1480 1485

Leu Thr Thr Gly Gly Glu Val Arg Leu Phe Arg Ser Phe Tyr Val
1490 1495 1500

Asn Ala Gly Ile Gly Ala Arg Phe Gly Leu Asp Tyr Gln Asp Ile
1505 1510 1515

Asn Ile Thr Gly Asn Val Gly Met Arg Tyr Ala Phe
1520 1525 1530

<210> 31
<211> 20
<212> DNA
<213> Artificial Sequence

<220>
<223> primer FW1

<400> 31
cttttagcgt ggtatccttc 20

<210> 32
<211> 20
<212> DNA
<213> Artificial Sequence

<220>
<223> primer FW2

<400> 32
ttacaaagac caccaacgga 20

<210> 33
<211> 19
<212> DNA
<213> Artificial Sequence

<220>
<223> primer FW3

<400> 33
taccatagaa agcggaaac 19

<210> 34
<211> 20
<212> DNA
<213> Artificial Sequence

<220>
<223> primer FW4

<400> 34
agctatagct ttgcacctga 20

<210> 35
<211> 23
<212> DNA
<213> Artificial Sequence

<220>
<223> primer FW5

<400> 35
actaacagca tagaagttgg gga 23

<210> 36
<211> 20
<212> DNA
<213> Artificial Sequence

<220>
<223> primer FW6

<400> 36
aacaacatta caggcatgag 20

<210> 37
<211> 20
<212> DNA
<213> Artificial Sequence

<220>
<223> primer FW7

<400> 37
caaatagtaa caggacaatt 20

<210> 38
<211> 20
<212> DNA
<213> Artificial Sequence

<220>
<223> primer FW8

<400> 38
caggatttaa gaggcactta 20

<210> 39
<211> 19
<212> DNA
<213> Artificial Sequence

<220>
<223> primer FW9

<400> 39
tcttaaccaa tctgagcaa 19

<210> 40
<211> 23
<212> DNA
<213> Artificial Sequence

<220>
<223> primer FW10

<400> 40
gtcaaacacg ttattgatgg ctt 23

<210> 41
<211> 19
<212> DNA
<213> Artificial Sequence

<220>
<223> primer FW11

<400> 41
ggggtttaat agcataggg 19

<210> 42
<211> 20
<212> DNA
<213> Artificial Sequence

<220>
<223> primer FW12

<400> 42
tagagctcta caccagtctt 20

<210> 43
<211> 35
<212> DNA
<213> Artificial Sequence

<220>
<223> primer FW13

<400> 43
ataaagccca tgaattctta ggcatgcgtg ctctg 35

<210> 44

<211> 30
<212> DNA
<213> Artificial Sequence

<220>
<223> primer FW14

<400> 44
tgctattgat aaagagttac atttctcagg 30

<210> 45
<211> 19
<212> DNA
<213> Artificial Sequence

<220>
<223> primer FW14-1

<400> 45
atttctcagg gggaaagtc 19

<210> 46
<211> 33
<212> DNA
<213> Artificial Sequence

<220>
<223> primer FW15

<400> 46
ggctaataat ttaaccacaa taagcgcctt taa 33

<210> 47
<211> 19
<212> DNA
<213> Artificial Sequence

<220>
<223> primer forward

<400> 47
gatgggcgct tctggttta 19

<210> 48
<211> 25
<212> DNA
<213> Artificial Sequence

<220>
<223> primer HSVAFW1

<400> 48
atgaattctt aggcatgcgt gctct 25

<210> 49
<211> 22
<212> DNA
<213> Artificial Sequence

<220>
<223> primer HSVAFW2

<400> 49
ttttggaggt gtaggagtag at 22

<210> 50
<211> 20
<212> DNA
<213> Artificial Sequence

<220>
<223> primer HSVAFW3

<400> 50
agcgcgcatt taaaacaggt 20

<210> 51
<211> 20
<212> DNA
<213> Artificial Sequence

<220>
<223> primer HSVANOFW-2

<400> 51
agaaacgaga ttacaggaag 20

<210> 52
<211> 20
<212> DNA
<213> Artificial Sequence

<220>
<223> primer HSVANOFW-3

<400> 52
aggagcaagt tttgtagcag 20

<210> 53
<211> 20
<212> DNA
<213> Artificial Sequence

<220>
<223> primer HSVANOFW-5

<400> 53
aaaatgcgac tgattggatg 20

<210> 54
<211> 18
<212> DNA
<213> Artificial Sequence

<220>
<223> primer FWNO3

<400>  54
ttgaaatttg gccacgct                                                    18

<210>  55
<211>  20
<212>  DNA
<213>  Artificial Sequence

<220>
<223>  primer RV1

<400>  55
tcacccatag aatggacgaa                                                  20

<210>  56
<211>  22
<212>  DNA
<213>  Artificial Sequence

<220>
<223>  primer RV2

<400>  56
ctagcgcatt aaccacagac tg                                               22

<210>  57
<211>  19
<212>  DNA
<213>  Artificial Sequence

<220>
<223>  primer RV3

<400>  57
tagaagttgt agacacggt                                                   19

<210>  58
<211>  20
<212>  DNA
<213>  Artificial Sequence

<220>
<223>  primer RV4

<400>  58
gtgatattgc ctttctgaac                                                  20

<210>  59
<211>  18
<212>  DNA
<213>  Artificial Sequence

<220>
<223>  primer RV5

<400>  59
gcaagtttttg tgcggatt                                                    18

<210> 60
<211> 19
<212> DNA
<213> Artificial Sequence

<220>
<223> primer RV7

<400> 60
atgtgataca catctgacc 19

<210> 61
<211> 20
<212> DNA
<213> Artificial Sequence

<220>
<223> primer RV8

<400> 61
agtgccgtta ccatcgtgaa 20

<210> 62
<211> 35
<212> DNA
<213> Artificial Sequence

<220>
<223> primer RV9

<400> 62
tattcaagga aagtccctgg agaaactcca gagac 35

<210> 63
<211> 33
<212> DNA
<213> Artificial Sequence

<220>
<223> primer RV10

<400> 63
ttaaaggcgc ttattgtggt taaattatta gcc 33

<210> 64
<211> 21
<212> DNA
<213> Artificial Sequence

<220>
<223> primer RV10-1

<400> 64
attagcctta agggtgctat c 21

<210> 65

<211>  25
<212>  DNA
<213>  Artificial Sequence

<220>
<223>  realtime-PCR Reverse

<400>  65
ctggtaatgc atcattagaa gcaaa                                        25


<210>  66
<211>  20
<212>  DNA
<213>  Artificial Sequence

<220>
<223>  primer HSAVRV

<400>  66
tacgcgcaaa ataggttctt                                              20


<210>  67
<211>  20
<212>  DNA
<213>  Artificial Sequence

<220>
<223>  primer HSAVRV2

<400>  67
tggagaaact ccagagacta                                              20


<210>  68
<211>  21
<212>  DNA
<213>  Artificial Sequence

<220>
<223>  primer HSAVRV3

<400>  68
ttgttcgctg tagtgccgtg g                                            21


<210>  69
<211>  20
<212>  DNA
<213>  Artificial Sequence

<220>
<223>  primer RVNO4

<400>  69
gaaggatacc acgctaaaag                                              20


<210>  70
<211>  20
<212>  DNA
<213>  Artificial Sequence

<220>
<223> primer RVNO5

<400> 70
aagctagagt tttggttgag 20

<210> 71
<211> 19
<212> DNA
<213> Artificial Sequence

<220>
<223> primer RVNO6

<400> 71
ttgctcagat tggttaaga 19

<210> 72
<211> 20
<212> DNA
<213> Artificial Sequence

<220>
<223> primer HSVANORV-1

<400> 72
atcgaaataa gcgaacctca 20

<210> 73
<211> 20
<212> DNA
<213> Artificial Sequence

<220>
<223> primer HSVANORV-3

<400> 73
ttgaaagctt agctaaacgg 20

<210> 74
<211> 20
<212> DNA
<213> Artificial Sequence

<220>
<223> primer HSVANORV-4

<400> 74
tggtattgct ggttaagagg 20

<210> 75
<211> 16
<212> DNA
<213> Artificial Sequence

<220>
<223> realtime PCR probe

<400> 75
caaacagatg agccgt 16

<210> 76
<211> 43
<212> DNA
<213> Artificial Sequence

<220>
<223> primer HSVASTART

<400> 76
atgaaaaagt ttagttctct cacattgaaa tttggccacg ctc 43

<210> 77
<211> 45
<212> DNA
<213> Artificial Sequence

<220>
<223> primer HSVASTOP

<400> 77
ctaaaaagca tagcgcatcc cgacattgcc tgtaatatta atatc 45

<210> 78
<211> 257
<212> PRT
<213> Artificial Sequence

<220>
<223> SNTW101 partial peptide

<400> 78

Leu Glu Gln Thr Thr Phe Glu Gln Val Phe Gln Glu Leu Leu Ser Asn
1 5 10 15

Gln Asn Leu Leu Asn Lys Thr Ile Ala Trp Leu Gly Pro Gln Ile Leu
20 25 30

Asp Glu Met Leu Lys Thr Ala Ile Asp Asp Leu Leu Asn Pro Thr Asn
35 40 45

Gln Leu Thr Ala Ala Glu Lys Asn Ile Leu Asp Asn Ile Leu Asn Asn
50 55 60

Val Phe Ser Ser Glu Lys Lys Ala Val Gln Gln Met Glu Asn Ser Asn
65 70 75 80

Pro Asp Val Lys His Val Ile Asp Gly Leu Met Gln Ala Lys Gly Leu
85 90 95

Gly Glu Val Tyr Ser Lys Gly Leu Gln Ser Ile Leu Ser Asn Lys Leu
            100                 105                 110

Gln Gly Gln Leu Lys Ser Met Gly Leu Gly Ser Leu Leu Ala Pro Lys
        115                 120                 125

Ala Leu Gly Asn Phe Trp Gln Lys Gly Tyr Phe Asn Phe Leu Ala Asn
    130                 135                 140

Asp Asp Ile Leu Val Asn Asn Ser Thr Phe Ser Asn Ala Ser Gly Gly
145                 150                 155                 160

Thr Leu Ser Phe Ile Ala Gly Lys Ser Ile Ile Phe Ala Gly Gln Asn
                165                 170                 175

Thr Ile Asn Phe Ser Asn Asn Gln Gly Thr Leu Glu Phe Leu Ser Asn
            180                 185                 190

Asp Val Ser Asn Ile Asp Leu Thr Thr Leu Asn Ala Thr Asp Gly Leu
        195                 200                 205

Thr Ile Asp Ala Pro Phe Asn Asn Leu Tyr Val Gln Lys Gly Asn Ile
    210                 215                 220

Thr Leu Asn Gln Tyr Glu Ser Phe Ser Val Gln Ala Gly Asn Phe Asp
225                 230                 235                 240

Phe Leu Gly Thr Val Gln Ala Asp Gly Ala Val Asp Leu Ser Gly Val
                245                 250                 255

Thr

<210> 79
<211> 257
<212> PRT
<213> Artificial Sequence

<220>
<223> HS1 partial peptide

<400> 79

Leu Asn Lys Thr Thr Phe Gly Gln Ile Phe Glu Glu Leu Leu Ser Asn
1               5                   10                  15

Gln Asn Leu Leu Asn Lys Thr Ile Ala Trp Leu Gly Pro Gln Ile Leu
            20                  25                  30

Asp Glu Met Leu Lys Thr Ala Ile Asp Asp Leu Leu Asn Pro Thr Asn

```
        35                  40                  45

Gln Leu Thr Ala Ala Glu Lys Asn Ile Leu Asp Asn Ile Leu Asn Asn
    50                  55                  60

Val Phe Ser Ser Glu Lys Lys Ala Val Gln Gln Met Glu Asn Ser Asn
65                  70                  75                  80

Pro Asp Val Lys His Val Ile Asp Gly Leu Met Gln Ala Lys Gly Leu
                85                  90                  95

Gly Glu Val Tyr Ser Lys Gly Leu Gln Ser Ile Leu Ser Asn Lys Leu
            100                 105                 110

Gln Gly Gln Leu Lys Ser Met Gly Leu Gly Ser Leu Leu Ala Pro Lys
        115                 120                 125

Ala Leu Gly Asn Phe Trp Gln Lys Gly Tyr Phe Asn Phe Leu Ala Asn
    130                 135                 140

Asp Asp Ile Leu Val Asn Asn Ser Thr Phe Ser Asn Ala Ser Gly Gly
145                 150                 155                 160

Thr Leu Ser Phe Ile Ala Gly Lys Ser Ile Ile Phe Ala Gly Gln Asn
                165                 170                 175

Thr Ile Asn Phe Ser Asn Asn Gln Gly Thr Leu Glu Phe Leu Ser Asn
            180                 185                 190

Asp Val Ser Asn Ile Asp Leu Thr Thr Leu Asn Ala Thr Asp Gly Leu
        195                 200                 205

Thr Ile Asp Ala Pro Phe Asn Asn Leu Tyr Val Gln Lys Gly Asn Ile
    210                 215                 220

Thr Leu Ala Ser Tyr Glu Gly Leu Thr Val Arg Ala Asn Asn Phe Asp
225                 230                 235                 240

Phe Leu Gly Thr Val Gln Ala Asp Gly Ala Val Asp Leu Ser Gly Val
                245                 250                 255

Thr


<210>  80
<211>  257
<212>  PRT
<213>  Artificial Sequence
```

<220>
<223>  HS5 partial peptide

<400>  80

Leu Lys Asn Thr Thr Phe Gly Gln Ile Phe Glu Glu Leu Leu Ser Asn
1               5                   10                  15

Gln Asn Leu Leu Asn Lys Thr Ile Ala Trp Leu Gly Pro Gln Ile Leu
            20                  25                  30

Asp Glu Met Leu Lys Thr Ala Ile Asp Asp Leu Leu Asn Pro Thr Asn
        35                  40                  45

Gln Leu Thr Ala Ala Glu Lys Asn Ile Leu Asp Asn Ile Leu Asn Asn
    50                  55                  60

Val Phe Ser Ser Glu Lys Lys Ala Val Gln Gln Met Glu Asn Ser Asn
65                  70                  75                  80

Pro Asp Val Lys His Val Ile Asp Gly Leu Met Gln Ala Lys Gly Leu
                85                  90                  95

Gly Glu Val Tyr Ser Lys Gly Leu Gln Ser Ile Leu Ser Asn Lys Leu
            100                 105                 110

Gln Gly Gln Leu Lys Ser Met Gly Leu Gly Ser Leu Leu Ala Pro Lys
        115                 120                 125

Ala Leu Gly Asn Phe Trp Gln Lys Gly Tyr Phe Asn Phe Leu Ala Asn
    130                 135                 140

Asp Asp Ile Leu Val Asn Asn Ser Thr Phe Ser Asn Ala Ser Gly Gly
145                 150                 155                 160

Thr Leu Ser Phe Ile Ala Gly Lys Ser Ile Ile Phe Ala Gly Gln Asn
                165                 170                 175

Thr Ile Asn Phe Ser Asn Asn Gln Gly Thr Leu Glu Phe Leu Ser Asn
            180                 185                 190

Asp Val Ser Asn Ile Asp Leu Thr Thr Leu Asn Ala Thr Asp Gly Leu
        195                 200                 205

Thr Ile Asp Ala Pro Phe Asn Asn Leu Tyr Val Gln Lys Gly Asn Ile
    210                 215                 220

Thr Leu Asn Gln Tyr Glu Ser Phe Ser Val Gln Ala Gly Asn Phe Asp

225 230 235 240

Phe Leu Gly Thr Val Gln Ala Asp Gly Ala Val Asp Leu Ser Gly Val
245 250 255

Thr

<210> 81
<211> 257
<212> PRT
<213> Artificial Sequence

<220>
<223> TKY partial peptide

<400> 81

Leu Asn Asn Thr Thr Phe Gly Gln Val Leu Glu Glu Leu Leu Ser Asn
1 5 10 15

Gln Asn Leu Leu Asn Lys Thr Ile Ala Trp Leu Gly Pro Gln Ile Leu
20 25 30

Asp Glu Met Leu Lys Thr Ala Ile Asp Asp Leu Leu Asn Pro Thr Ser
35 40 45

Gln Leu Thr Ala Ala Glu Lys Asn Ile Leu Asp Asn Ile Leu Asn Asn
50 55 60

Val Phe Gly Ser Glu Lys Lys Ala Val Glu Gln Met Glu Asn Ser Asn
65 70 75 80

Pro Asp Val Lys Gln Val Ile Asp Gly Leu Met Gln Ala Lys Gly Leu
85 90 95

Gly Glu Val Tyr Ser Lys Gly Leu Gln Ser Ile Leu Ser Asn Lys Leu
100 105 110

Gln Asp Gln Leu Lys Ser Met Gly Leu Gly Ser Leu Leu Ala Pro Lys
115 120 125

Ala Leu Gly Asn Phe Trp Gln Lys Gly Tyr Phe Asn Phe Leu Ala Asn
130 135 140

Asp Asp Ile Leu Val Ser Asn Ser Thr Phe Asp Asn Ala Ser Gly Gly
145 150 155 160

Thr Leu Ser Phe Ile Ala Gly Lys Ser Ile Ile Phe Ala Gly Gln Asn
165 170 175

Thr Ile Asn Phe Ser Asn Asn Gln Gly Thr Leu Glu Phe Leu Ser Asn
180 185 190

Asp Val Ser Thr Ile Asp Leu Thr Thr Leu Asn Ala Thr Asp Gly Leu
195 200 205

Thr Ile Asp Ala Pro Phe Asn Asn Leu Tyr Val Gln Lys Gly Asn Ile
210 215 220

Thr Leu Asn Gln Tyr Glu Ser Phe Ser Val Gln Ser Gly Asn Phe Asp
225 230 235 240

Phe Leu Gly Thr Val Gln Ala Asp Gly Ala Val Asp Leu Ser Gly Val
245 250 255

Thr

<210> 82
<211> 257
<212> PRT
<213> Artificial Sequence

<220>
<223> SH8 partial paptide

<400> 82

Leu Lys Gln Thr Asn Phe Thr Gln Ala Phe Gln Ala Leu Glu Ser Asn
1 5 10 15

Gln Asn Leu Leu Asn Lys Thr Ile Ala Trp Val Gly Pro Gln Ile Leu
20 25 30

Asp Glu Met Leu Gln Thr Ala Ile Asn Asp Leu Leu Asn Pro Thr Gln
35 40 45

Glu Leu Thr Lys Ala Glu Thr Asp Ile Leu Asn Asn Ile Leu Asn Asn
50 55 60

Val Phe Lys Lys Glu Lys Asp Ala Val Thr Ser Leu Lys Asn Ser Asn
65 70 75 80

Ser Gly Ile Lys Thr Met Ile Glu Gly Leu Ile Asn Asn Lys Gly Leu
85 90 95

Gly Glu Val Tyr Ser Lys Gly Leu Gln Ser Ile Leu Ser Asn Lys Leu
100 105 110

Gln Asp Gln Leu Lys Ser Met Gly Leu Gly Ser Leu Leu Ala Pro Lys
115 120 125

Ala Leu Gly Asn Phe Trp Gln Lys Gly Tyr Phe Asn Phe Leu Ala Asn
130 135 140

Asp Asp Ile Leu Val Ser Asn Ser Thr Phe Asp Asn Ala Ser Gly Gly
145 150 155 160

Thr Leu Ser Phe Ile Ala Gly Lys Ser Ile Ile Phe Ala Gly Gln Asn
165 170 175

Thr Ile Asn Phe Ser Asn Asn Gln Gly Thr Leu Glu Phe Leu Ser Asn
180 185 190

Asp Val Ser Asn Ile Asp Leu Thr Thr Leu Asn Ala Thr Asp Gly Leu
195 200 205

Thr Ile Asp Ala Pro Phe Asn Asn Leu Tyr Val Gln Lys Gly Asn Ile
210 215 220

Thr Leu Asn Gln Tyr Glu Ser Phe Ser Val Gln Ser Gly Asn Phe Asp
225 230 235 240

Phe Leu Gly Thr Val Gln Ala Asp Gly Ala Val Asp Leu Ser Gly Val
245 250 255

Thr

<210> 83
<211> 257
<212> PRT
<213> Artificial Sequence

<220>
<223> SH10 partial peptide

<400> 83

Leu Lys Gln Thr Asn Phe Ser Gln Ala Phe Gln Ala Leu Val Ser Asn
1 5 10 15

Gln Asn Leu Leu Asn Lys Thr Ile Ala Trp Leu Gly Pro Gln Ile Leu
20 25 30

Asp Glu Met Leu Gln Thr Ala Ile Asn Asp Leu Leu Asn Pro Thr Gln
35 40 45

```
Glu Leu Thr Lys Ala Glu Thr Asp Ile Leu Asn Asn Ile Leu Asn Asn
    50                  55                  60

Val Phe Lys Lys Glu Lys Asp Ala Val Thr Ser Leu Glu Asn Ser Asn
65                  70                  75                  80

Ser Gly Ile Lys Thr Met Ile Glu Gly Leu Ile Asn Asn Lys Gly Leu
                85                  90                  95

Gly Gly Val Tyr Thr Lys Gly Leu Gln Ser Ile Phe Ser Asp Lys Leu
            100                 105                 110

Gln Asn Met Leu Lys Ser Leu Asn Met Gly Ser Leu Leu Glu Pro Lys
        115                 120                 125

Ala Leu Gly Lys Phe Trp Glu Lys Gly Tyr Phe Asn Phe Leu Ala Asn
    130                 135                 140

Asp Asn Val Leu Val Ser Asn Ser Thr Phe Lys Asn Ala Ser Gly Gly
145                 150                 155                 160

Thr Leu Ser Phe Ile Ala Gly Lys Ser Ile Ile Phe Ser Gly Gln Asn
                165                 170                 175

Thr Ile Asn Phe Ser Asn Asn Gln Gly Thr Leu Glu Phe Leu Ser Asn
            180                 185                 190

Asp Val Ser Thr Ile Asp Leu Thr Thr Leu Asn Ala Thr Asp Gly Leu
        195                 200                 205

Thr Ile Asp Ala Pro Phe Asn Asn Leu Tyr Val Gln Lys Gly Asn Ile
    210                 215                 220

Thr Leu Asn Gln Tyr Glu Ser Phe Ser Val Gln Ser Gly Asn Phe Asp
225                 230                 235                 240

Phe Leu Gly Thr Val Gln Ala Asp Gly Ala Val Asp Leu Ser Gly Val
                245                 250                 255

Thr
```

<210> 84
<211> 20
<212> DNA
<213> Artificial Sequence

<220>
<223> primer VAC3624F

<400> 84
gagcgagcta tggttatgac 20

<210> 85
<211> 21
<212> DNA
<213> Artificial Sequence

<220>
<223> primer VAC4041R

<400> 85
cattcctaaa ttggaagcga a 21

<210> 86
<211> 14
<212> PRT
<213> Artificial Sequence

<220>
<223> antigen peptide No.8

<400> 86

Lys Gln Leu Pro Gln Pro Lys Arg Ser Glu Leu Lys Pro Lys
1 5 10

<210> 87
<211> 14
<212> PRT
<213> Artificial Sequence

<220>
<223> antigen peptide No. 31N

<400> 87

Thr Asn Ile Lys Gln Tyr Met Gln Asn Asn His Arg Ser Gln
1 5 10

<210> 88
<211> 14
<212> PRT
<213> Artificial Sequence

<220>
<223> antigen peptide No. 81

<400> 88

Thr Leu Thr Leu Glu Gly Thr Glu Thr Phe Ala Gln Asn Ser
1 5 10

<210> 89
<211> 14
<212> PRT
<213> Artificial Sequence

```
<220>
<223>  antigen peptide No.63

<400>  89

Glu Ala Tyr Ala Lys Asn Gln Gly Asp Ile Trp Ser Thr Ile
1               5                   10


<210>  90
<211>  14
<212>  PRT
<213>  Artificial Sequence

<220>
<223>  antigen peptide No. 73

<400>  90

Val Ile Gly Ser Lys Ser Ser Ile Thr Leu Asn Ser Ala Asn
1               5                   10


<210>  91
<211>  14
<212>  PRT
<213>  Artificial Sequence

<220>
<223>  antigen peptide No. 61

<400>  91

Ala Asp Ile Gln Ser Ser Gln Thr Thr Phe Ala Asn Ser Val
1               5                   10
```

## Description

### Technical Field

The present invention relates to a newly found outer membrane protein gene specific for *Helicobacter suis* and a gene product (protein) thereof, and to a method and a drug for diagnosing *Helicobacter suis* infection using an antibody thereagainst.

### Background Art

It is currently known that infection with *Helicobacter pylori* (a microaerophilic gram-negative spiral bacterium parasitic in the human stomach; hereinafter, referred to as *"H. pylori")* is involved in chronic gastritis, gastric ulcer, duodenal ulcer, stomach cancer, gastric mucosa-associated lymphoid tissue (MALT) lymphoma, and diffuse large B-cell lymphoma. An isolation culture method, a urea breath test, measurement of *H. pylori* antibody titers in serum or urine (ELISA and latex agglutination method), and measurement of *H. pylori* antigens in feces (immunochromatography) are typically adopted as methods for testing for *H. pylori* infection.

However, as diagnostic techniques and disinfectants for *H. pylori* have become widely available, *Helicobacter heilmannii* in a broad sense has come to be recognized as a species of *Helicobacter,* other than *H. pylori,* causing severe gastric diseases in humans. The *Helicobacter heilmannii* in a broad sense *(Helicobacter heilmannii sensu lato;* hereinafter, referred to as *"H. heilmannii*") includes *Helicobacter heilmannii* in a narrow sense *(H. heilmannii sensu stricto), H. suis, H. bizzozeronnii, H. felis,* and H. *salmonis.* Among them, *Helicobacter suis* (hereinafter, referred to as *"H. suis")* is a species of *Helicobacter* often found in the human stomach. Nonetheless, a rapid diagnosis method has not yet been established therefor (Non Patent Literature 1).

*H. pylori* infects only primates, which presumably get infection from close relatives only in childhood, whereas *H. suis* is transmitted through animals such as pigs, cats, and dogs, irrespective of age. *H. pylori* is 2.5 to 5.0 μm in the whole length, while *H. heilmannii* is 5 to 10 μm (Non Patent Literature 2). *H. heilmannii* including *H. suis* lacks primary pathogenic factors of *H. pylori,* i.e., a gene of a type IV secretion apparatus-related protein called CagPAI (pathogenicity island), a gene cluster including genes of protein inducing cancer through injection into hosts, and a gene of a protein toxin, called VacA (vacuolating cytotoxin A), exhibiting a wide variety of effects such as development of erosion or ulcer in gastric mucosa, killing of cultured cells by vacuolar degeneration, and induction of apoptosis (Non Patent Literature 3).

It has also been reported that *H. suis* infection highly frequently causes development of gastric MALT lymphoma composed mainly of accumulated lymphocytes. Infection experiments using mice have shown that the infectivity of *H. suis* is much stronger than that of *H. pylori* (Non Patent Literature 4). In addition, it has been reported that *H. suis* has flagella at both ends, is highly motile, invades a deep part of a cavity of a gland or parietal cells of the stomach, and is resistant to antibiotics (Non Patent Literature 5).

In fact, approximately 60% or more of patients manifesting symptoms of gastritis or gastric diseases despite being negative for *H. pylori* are considered to be positive for *H. suis* infection (Non Patent Literature 6). Also, 25 to 50% of MALT lymphoma cases are considered to be caused by *H. suis* infection (Non Patent Literature 5).

However, *H. pylori* is urease-positive, while *H. suis* separated from humans often exhibits negativity in urease tests or urea breath tests despite having the urease gene *(H. suis* separated from animals is positive in urease tests or urea breath tests). Accordingly, infection with *H. suis* cannot be diagnosed by urease tests or urea breath tests which are used for *H. pylori.* Although there is a report on successful *in vitro* culture of a strain separated from pigs (Patent Literature 1), there is no report on the colony formation of *H. suis* in an isolation agar medium. A strain separated from humans cannot be cultured *in vitro,* and *H. suis* contained in gastric biopsy of human patients still cannot be purely cultured *in vitro* (Non Patent Literature 7). Hence, diagnosis by an isolation culture method which is used for *H. pylori* cannot be used for *H. suis.* Accordingly, *H. suis* found in humans cannot be detected by usual testing methods for *H. pylori* because of being poorly culturable and having weak urease activity.

Meanwhile, the outline of the whole genomes (draft genomes) of an *H. heilmannii sensu stricto* ASB1 strain isolated from a cat with severe gastritis (Non Patent Literature 8), an *H. bizzozeronnii* CIII-1 strain isolated from human gastric mucosa (Non Patent Literature 9), *H. suis* H1 and H5 strains isolated from pig gastric mucosa (Non Patent Literature 10), an *H. felis* CS1 strain (Non Patent Literature 11), and an *H. suis* SNTW101 strain isolated from a Japanese patient with nodular gastritis (Non Patent Literature 12) have been analyzed and reported. PCR testing targeting *H. heilmannii-specific* 16S rRNA gene after preparation of DNA from gastric biopsy is currently a general diagnosis method for infection with *H. heilmannii* including *H. suis.*

However, existing diagnosis methods for *H. heilmannii* require tissue testing and fail to discriminate *H. suis* from other species of *H. heilmannii* for diagnosis (Patent Literature 2). Thus, neither a method capable of diagnosing *H. suis* infection exclusively nor a non-invasive method has yet been established (Non Patent Literature 13). Accordingly, the development of a simple and highly sensitive testing method for *H. suis* has been desired.

The present inventors have previously reported a method for diagnosing *H. suis* infection, targeting an F2R2 protein and a gene sequence thereof, based on genome analysis information on *H. suis* isolated from cynomolgus monkeys (Patent Literature 3).

Citation List

Patent Literature

Patent Literature 1: Japanese Patent Laid-Open No. 2011-15664
Patent Literature 2: U.S. Patent Publication No. 2006-0078919
Patent Literature 3: Japanese Patent Laid-Open No. 2016-10331

Non Patent Literature

Non Patent Literature 1: Blaecher C et al., Helicobacter. (2016) 22 (3): e12369
Non Patent Literature 2: Overby A et al., Digestion. (2016) 93 (4) : 260-5
Non Patent Literature 3: Vermoote M et al., Vet Res. (2011) 42: 51
Non Patent Literature 4: Nakamura M et al., Infect Immun. (2007) 75 (3): 1214-22
Non Patent Literature 5: Overby A et al., Digestion. (2017) 95 (1) : 61-6
Non Patent Literature 6: Masahiko Nakamura et al., Modern Medical Laboratory (2016) 44 (4): 278-84
Non Patent Literature 7: Matsui H et al., Helicobacter. (2014) 19 (4) : 260-71
Non Patent Literature 8: Smet A et al., Genome Announcements (2013) 1 (1): e00033-12
Non Patent Literature 9: Schott T et al., Journal of Bacteriology (2011) 193 (17): 4565-6
Non Patent Literature 10: Vermoote M et al., Veterinary Research (2011) 42: 51
Non Patent Literature 11: Arnold I C et al., Genome Biol. Evol. (2011) 3: 302-8
Non Patent Literature 12: Matsui H et al., Genome Announcements (2016) 4 (5): e00934-16
Non Patent Literature 13: Bento-Miranda M et al., World J Gastroenterol (2014) 20 (47): 17779-87

Summary of Invention

Technical Problem

An object of the present invention is to provide an H. suis-specific diagnosis method. Another object of the present invention is to provide a simple and rapid testing method including an H. suis-specific diagnosis method that is a non-invasive method.

Solution to Problem

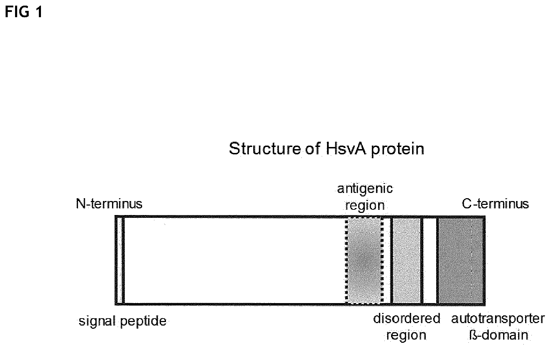

The present inventors newly found this time that an outer membrane protein (HsvA; named by the present inventor) specifically present exclusively in *H. suis* and an antibody against this protein are present in the body of a subject infected with *H. suis.* Further, the present inventors found a diagnosis method using an amino acid sequence that acts as the antigenic site of the HsvA protein. The F2R2 protein lucks structures such as an amino-terminal signal peptide region common in bacterial cell outer membrane proteins, a carboxy-terminal autotransporter (outer membrane transport mechanism of a protein unique to gram-negative bacteria), and a disordered region involved in the exertion of functions, which are present in the HsvA protein targeted by the present invention (see Figure 1). Thus, subjects infected with *H. suis* can be expected to have a higher antibody titer against the HsvA protein, a putative outer membrane protein, than that against the F2R2 protein.

The present inventors have conducted various studies on a method capable of specifically detecting H. *suis,* and consequently found that an antibody against an *H.* suis-specific outer membrane protein HsvA is present in the blood of a patient infected with *H. suis.* The present inventors have revealed that *H. suis* infection can be detected while discriminating from *H. pylori* infection, by using an amino acid sequence of the antigenic site of the HsvA protein.

The hsvA gene is contained in an *H. suis* SNTW101 strain (see Matsui H et al., Genome Announcements (2016) 4 (5): e00934-16) separated from a patient with nodular gastritis. Its nucleotide sequence (hsvA gene: SEQ ID NO: 1) largely differs in molecular weight from and has no identity with that of an *H. pylori* vacuolating cytotoxin protein VacA (Cover TL et al., Nat Rev Microbiol. (2005) 3 (4): 320-32). However, the HsvA protein has an amino-terminal signal peptide region and a carboxy-terminal autotransporter (outer membrane transport mechanism of a protein unique to gram-negative bacteria) β-domain characteristic of an *H. pylori* outer membrane protein. This protein also has a disordered region involved in the exertion of functions (see Figure 1). The hsvA gene is also contained in the draft genomes of *H. suis* HS1 and HS5 strains separated from a pig (Vermoote M et al., Vet Res. (2011) 42: 51). However, the HsvA protein derived from these strains has no identity with the *H. pylori* VacA protein. Thus, the hsvA gene had been considered not only to lack the functions of *H. pylori* VacA but to be not expressed in the first place. However, the present inventors have newly found the presence of the HsvA protein and an antibody thereagainst in the body of a subject infected with H. *suis* and conducted further studies on HsvA. As a result, the present inventors have found for the first time that the HsvA protein is expressed as an outer membrane protein in *H. suis.*

The present invention is based on these findings. Accordingly, the present invention relates to the following aspects.

(1) An HsvA antigen peptide or an immunologically equivalent mutant thereof.

(2) The antigen peptide or the immunologically equivalent mutant thereof of (1), derived from *H. suis.*

(3) The antigen peptide or the immunologically equivalent mutant thereof of (1), wherein the HsvA antigen peptide has a sequence consisting of 5 to 50 amino acids contained in any one of the amino acid sequences set forth in SEQ ID NO: 2, SEQ ID NO: 28, SEQ ID NO: 30, and SEQ ID NOs: 78 to 83.

(4) The peptide or the immunologically equivalent mutant thereof of (3), wherein the HsvA antigen peptide has a sequence consisting of 5 to 50 amino acids contained in any one of the amino acid sequences set forth in SEQ ID NOs: 78 to 83.

(5) The peptide or the immunologically equivalent mutant thereof of (1), wherein the HsvA antigen peptide is a peptide having any one amino acid sequence selected from the group of the following:

EKX1AVX2X3X4X5NSNX6X7 (peptide No. 11: SEQ ID NO: 24);
NQGTLEFLSNDVSX8 (peptide No. 19: SEQ ID NO: 25);
X9SX10KLQX11X12LKSX13X14X15 (peptide No. 33: SEQ ID NO: 26) ;
TNGQEVSASIDYNK (peptide No. 16: SEQ ID NO: 12);
AKLSNFASNDALPD (peptide No. 23: SEQ ID NO: 13);
PTTSSGASPDSSNP (peptide No. 10: SEQ ID NO: 14);
GLGRDLFVHSMGDK (peptide No. 21: SEQ ID NO: 15);
QIGKIKLSDVLSAS (peptide No. 15: SEQ ID NO: 16);
YGAIDKELHFSGGK (peptide No. 34: SEQ ID NO: 17);
NVDNILNMPSTTSG (peptide No. 20: SEQ ID NO: 18);
GNLKGVYYPKSSTT (peptide No. 22: SEQ ID NO: 19);
ITEKIQSGKLTITI (peptide No. 14: SEQ ID NO: 20);
FHDFLVSLKGKKFA (peptide No. 26: SEQ ID NO: 21);
TTGGEVRLFRSFYV (peptide No. 31: SEQ ID NO: 22); and
IGARFGLDYQDINI (peptide No. 35: SEQ ID NO: 23), or an
immunologically equivalent mutant thereof, wherein

X1 is K or D, X2 is Q, E or T, X3 is Q or S, X4 is M or L, X5 is E or K, X6 is P or S, X7 is D or G, X8 is N or T, X9 is L or F, X10 is N or D, X11 is G, D or N, X12 is Q or M, X13 is M or L, X14 is G or N, and X15 is L or M.

(6) The peptide or the immunologically equivalent mutant thereof of (5), wherein the HsvA antigen peptide is a peptide having any one amino acid sequence selected from the group of the following:

EKKAVQQMENSNPD (peptide No. 11: SEQ ID NO: 3);
EKKAVEQMENSNPD (peptide No. 11 (TKY): SEQ ID NO: 4);
EKDAVTSLKNSNSG (peptide No. 11 (SH8): SEQ ID NO: 5);
EKDAVTSLENSNSG (peptide No. 11 (SH10): SEQ ID NO: 6);
NQGTLEFLSNDVSN (peptide No. 19: SEQ ID NO: 7);
NQGTLEFLSNDVST (peptide No. 19 (TKY): SEQ ID NO: 8);
LSNKLQGQLKSMGL (peptide No. 33: SEQ ID NO: 9);
LSNKLQDQLKSMGL (peptide No. 33 (TKY): SEQ ID NO: 10);
FSDKLQNMLKSLNM (peptide No. 33 (SH10): SEQ ID NO: 11);
TNGQEVSASIDYNK (peptide No. 16: SEQ ID NO: 12);
AKLSNFASNDALPD (peptide No. 23: SEQ ID NO: 13);
PTTSSGASPDSSNP (peptide No. 10: SEQ ID NO: 14);

GLGRDLFVHSMGDK (peptide No. 21: SEQ ID NO: 15);
QIGKIKLSDVLSAS (peptide No. 15: SEQ ID NO: 16);
YGAIDKELHFSGGK (peptide No. 34: SEQ ID NO: 17);
NVDNILNMPSTTSG (peptide No. 20: SEQ ID NO: 18);
GNLKGVYYPKSSTT (peptide No. 22: SEQ ID NO: 19);
ITEKIQSGKLTITI (peptide No. 14: SEQ ID NO: 20);
FHDFLVSLKGKKFA (peptide No. 26: SEQ ID NO: 21);
TTGGEVRLFRSFYV (peptide No. 31: SEQ ID NO: 22);
IGARFGLDYQDINI (peptide No. 35: SEQ ID NO: 23);
KQLPQPKRSELKPK (peptide No. 8: SEQ ID NO: 86);
TNIKQYMQNNHRSQ (peptide No. 31N: SEQ ID NO: 87);
TLTLEGTETFAQNS (peptide No. 81: SEQ ID NO: 88);
EAYAKNQGDIWSTI (peptide No. 63: SEQ ID NO: 89);
VIGSKSSITLNSAN (peptide No. 73: SEQ ID NO: 90); and
ADIQSSQTTFANSV (peptide No. 61: SEQ ID NO: 91); or an immunologically equivalent mutant thereof.

(7) An antibody that specifically binds to a peptide or an immunologically equivalent mutant thereof of any one of (1) to (6), or an immunoreactive fragment thereof.
(8) A nucleic acid molecule consisting of 5 to 40 nucleotides, the nucleic acid molecule being capable of binding under stringent conditions to hsvA gene.
(9) The nucleic acid molecule of (8), wherein the hsvA gene has the nucleotide sequence set forth in SEQ ID NO: 1, SEQ ID NO: 27 or SEQ ID NO: 29.
(10) The nucleic acid molecule of (9) having any one nucleotide sequence selected from the group of the following:

CTTTTAGCGTGGTATCCTTC (SEQ ID NO: 31);
TTACAAAGACCACCAACGGA (SEQ ID NO: 32); TACCATAGAAAGCGGAAAC (SEQ ID NO: 33); AGCTATAGCTTTGCACCTGA (SEQ ID NO: 34);
ACTAACAGCATAGAAGTTGGGGA (SEQ ID NO: 35);
AACAACATTACAGGCATGAG (SEQ ID NO: 36);
CAAATAGTAACAGGACAATT (SEQ ID NO: 37);
CAGGATTTAAGAGGCACTTA (SEQ ID NO: 38); TCTTAACCAATCTGAGCAA (SEQ ID NO: 39); GTCAAACACGTTATTGATGGCTT (SEQ ID NO: 40); GGGGTTTAATAGCATAGGG (SEQ ID NO: 41); TAGAGCTCTACACCAGTCTT (SEQ ID NO: 42); ATAAAGCCCATGAATTCTTAGGCATGCGTGCTCTG (SEQ ID NO: 43); TGCTATTGATAAAGAGTTACATTTCTCAGG (SEQ ID NO: 44); ATTTCTCAGGGGGAAAGTC (SEQ ID NO: 45);
GGCTAATAATTTAACCACAATAAGCGCCTTTAA (SEQ ID NO: 46);
GATGGGCGCTTCTGGTTTA (SEQ ID NO: 47);
ATGAATTCTTAGGCATGCGTGCTCT (SEQ ID NO: 48);
TTTTGGAGGTGTAGGAGTAGAT (SEQ ID NO: 49);
AGCGCGCATTTAAAACAGGT (SEQ ID NO: 50);
AGAAACGAGATTACAGGAAG (SEQ ID NO: 51);
AGGAGCAAGTTTTGTAGCAG (SEQ ID NO: 52);
AAAATGCGACTGATTGGATG (SEQ ID NO: 53); TTGAAATTTGGCCACGCT (SEQ ID NO: 54); TCACCCATAGAATGGACGAA (SEQ ID NO: 55); CTAGCGCATTAACCACAGACTG (SEQ ID NO: 56);
TAGAAGTTGTAGACACGGT (SEQ ID NO: 57); GTGATATTGCCTTTCTGAAC (SEQ ID NO: 58); GCAAGTTTTGTGCGGATT (SEQ ID NO: 59);
ATGTGATACACATCTGACC (SEQ ID NO: 60); AGTGCCGTTACCATCGTGAA (SEQ ID NO: 61); TATTCAAGGAAAGTCCCTGGAGAAACTCCAGAGAC (SEQ ID NO: 62); TTAAAGGCGCTTATTGTGGTTAAATTATTAGCC (SEQ ID NO: 63); ATTAGCCTTAAGGGTGCTATC (SEQ ID NO: 64);
CTGGTAATGCATCATTAGAAGCAAA (SEQ ID NO: 65);
TACGCGCAAAATAGGTTCTT (SEQ ID NO: 66);
TGGAGAAACTCCAGAGACTA (SEQ ID NO: 67);
TTGTTCGCTGTAGTGCCGTGG (SEQ ID NO: 68);
GAAGGATACCACGCTAAAAG (SEQ ID NO: 69);
AAGCTAGAGTTTTGGTTGAG (SEQ ID NO: 70); TTGCTCAGATTGGTTAAGA (SEQ ID NO: 71); ATCGAAATAAGCGAACCTCA (SEQ ID NO: 72); TTGAAAGCTTAGCTAAACGG (SEQ ID NO: 73);
TGGTATTGCTGGTTAAGAGG (SEQ ID NO: 74), CAAACAGATGAGCCGT (SEQ ID NO: 75);
ATGAAAAAGTTTAGTTCTCTCACATTGAAATTTGGCCACGCTC (SEQ ID NO: 76); and CTAAAAAGCATAGCGCATCCCGACATTGCCTGTAATATTAATATC (SEQ ID NO: 77).

(11) The nucleic acid molecule of any one of (8) to (10), wherein the nucleic acid molecule is a primer capable of amplifying the whole or a portion of the hsvA gene.
(12) The nucleic acid molecule of any one of (8) to (10), wherein the nucleic acid molecule is a probe for detecting the hsvA gene.
(13) A method for determining the presence of *H. suis,* comprising detecting the whole or a portion of hsvA gene in a sample, wherein the sample in which the whole or a portion of the hsvA gene has been detected is determined to have *H. suis.*
(14) The method for determining the presence of *H. suis* of (13), comprising:

amplifying the whole or a portion of the hsvA gene using DNA in the sample as a template and using a primer of (11);
detecting the amplified DNA; and
determining the sample in which the DNA amplification has been detected, to have *H. suis.*

(15) The method for determining the presence of *H. suis* of (13), comprising:

contacting DNA in the sample with a probe of (12);
detecting DNA bound with the probe of (12); and
determining the sample in which the DNA bound with the probe of (11) has been detected, to have *H. suis.*

(16) A method for determining the presence of *H. suis,* comprising: detecting HsvA protein or a fragment thereof in a sample; and determining the sample in which the HsvA protein or the fragment thereof has been detected, to have *H. suis.*
(17) The method for determining the presence of *H. suis* of (16), comprising:

contacting the sample with an antibody or an immunoreactive fragment thereof of (7);
detecting the HsvA protein or the fragment thereof bound with the antibody or the immunoreactive fragment thereof, in the sample; and
determining the sample in which the HsvA protein or the fragment thereof bound with the antibody or the immunoreactive fragment thereof has been detected, as a sample having *H. suis.*

(18) A method for determining infection of a subject with *H. suis,* comprising:

performing a method of any one of (12) to (17) using a sample derived from the subject as a sample; and
determining the subject from which the sample determined to have *H. suis* by the method is derived, to be infected with *H. suis.*

(19) A method for determining infection of a subject with *H. suis,* comprising:

detecting an antibody that binds to an HsvA antigen peptide, in a blood sample derived from the subject; and
determining the subject in which the antibody that binds to the peptide has been detected, to be infected with *H. suis.*

(20) The method for determining infection with *H. suis* of (19), comprising the steps of:

contacting the sample derived from the subject with a peptide of any one of (1) to (6);
detecting an antibody bound with the peptide, in the blood sample; and
determining the subject in which the antibody bound with the peptide has been detected, to be infected with *H. suis.*

(21) A composition for determination of *H. suis* infection, comprising any one member selected from a peptide of any one of (1) to (6), a nucleic acid molecule of any one of (8) to (10), and an antibody or an immunoreactive fragment thereof of (7).
(22) A pharmaceutical composition comprising any one member selected from a peptide of any one of (1) to (6), a nucleic acid molecule of any one of (8) to (10), and an antibody or an immunoreactive fragment thereof of (7).
(23) The pharmaceutical composition of (22) for *H. suis* eradication therapy.

(24) The pharmaceutical composition of (22) for treatment or prevention of gastritis, gastric ulcer, duodenal ulcer, stomach cancer, chronic gastritis, gastric MALT lymphoma, nodular gastritis, idiopathic thrombocytopenic purpura, functional dyspepsia, or diffuse large B-cell lymphoma.

*(H. suis)*

The term *"H. suis"* herein means a strain that is included in *H. heilmannii* (in a broad sense) and belongs to *H. heilmannii* type 1. *H. suis* is known to infect the stomachs of humans as well as dogs, cats, pigs, monkeys, and the like. An infected mammal herein from which the *H. suis* has been isolated is not particularly limited and can be, for example, a human, a monkey, or a pig. Preferably, *H. suis* is *H. suis* isolated from a human (e.g., an SNTW101 strain).

(hsvA gene and HsvA protein)

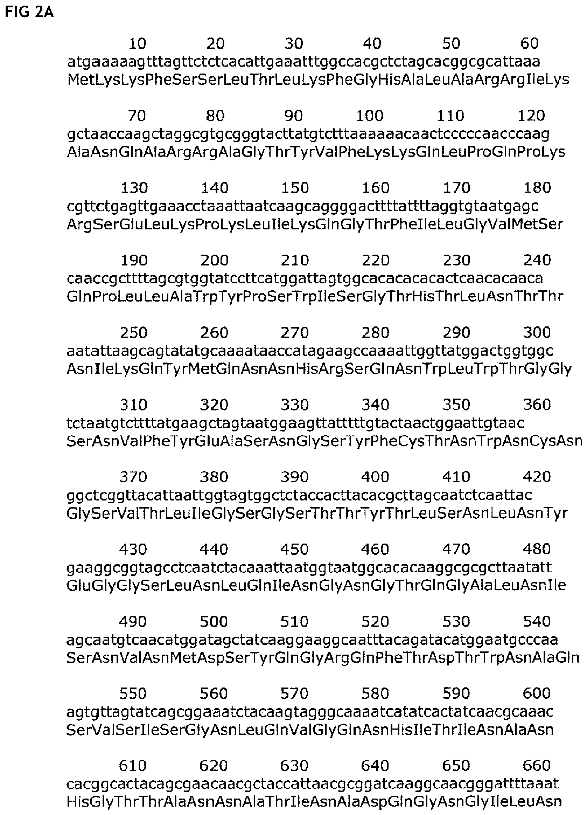

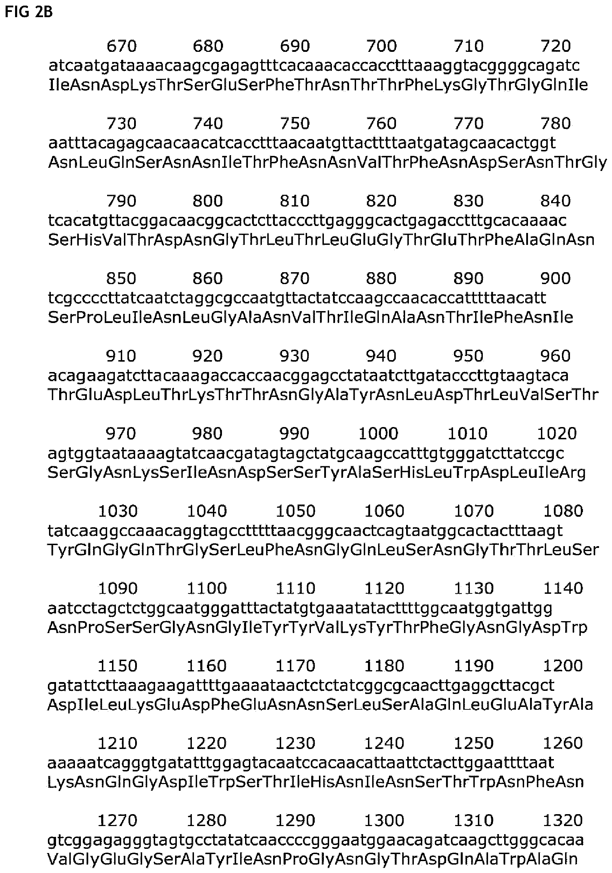

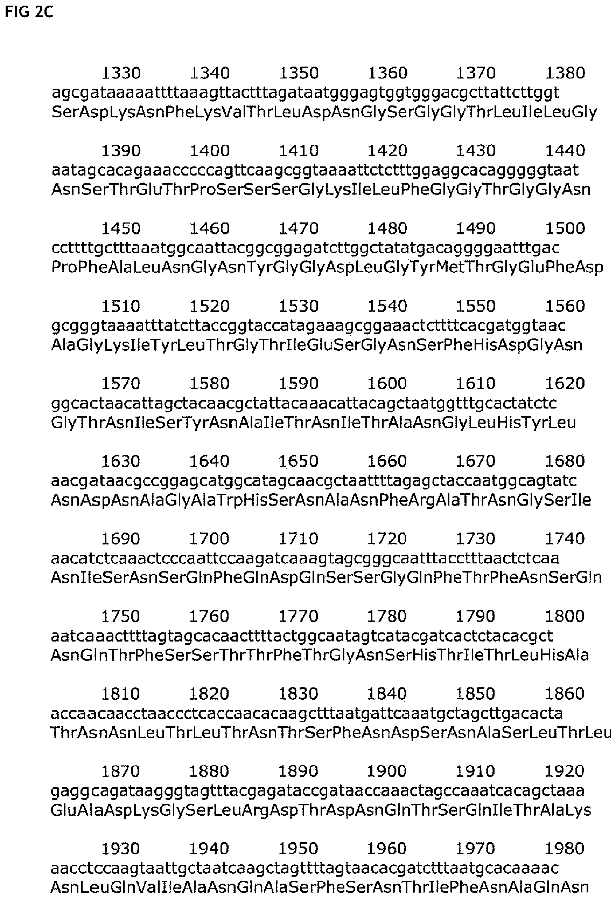

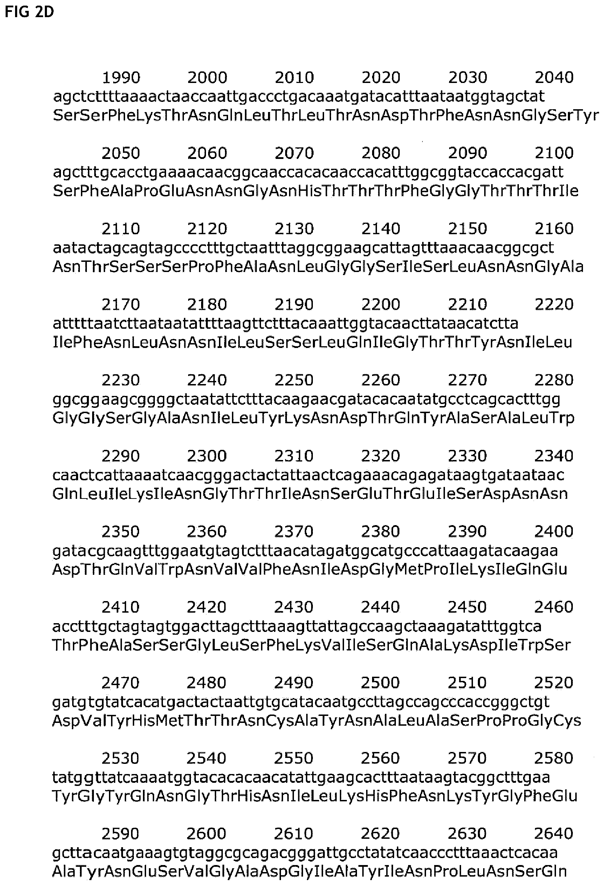

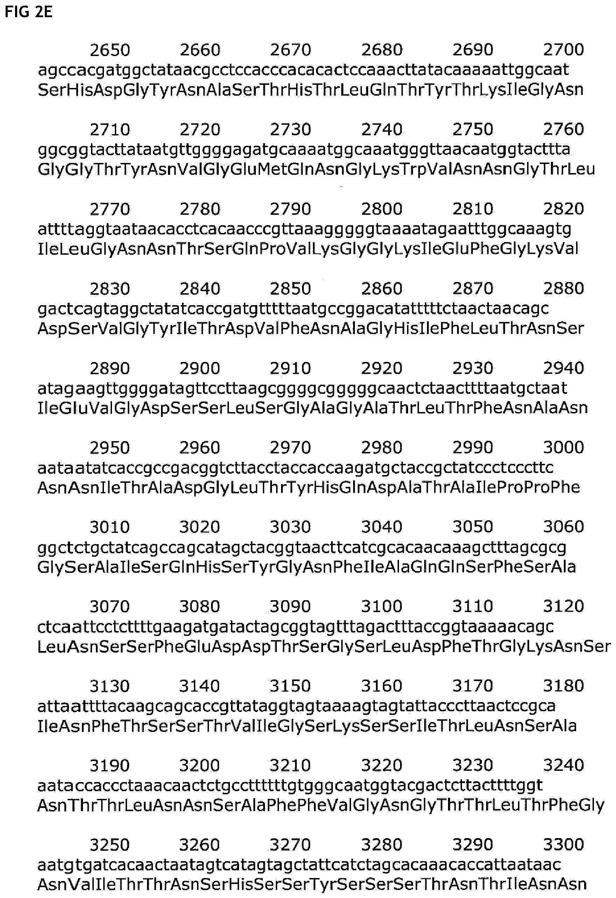

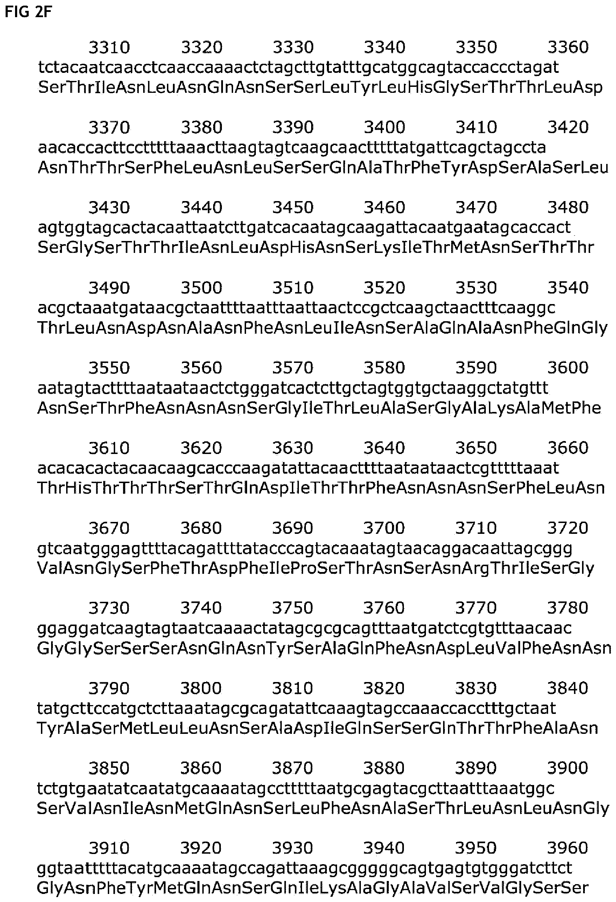

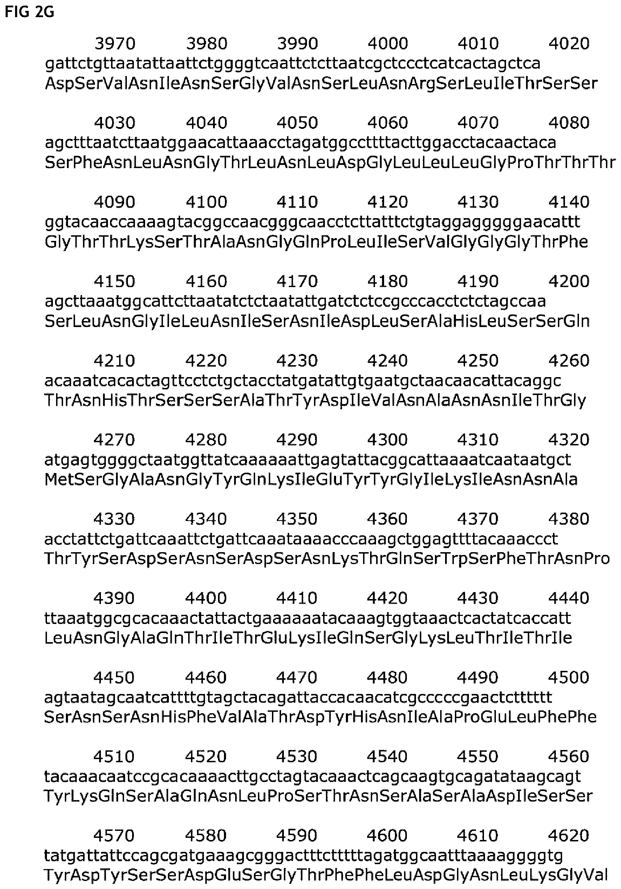

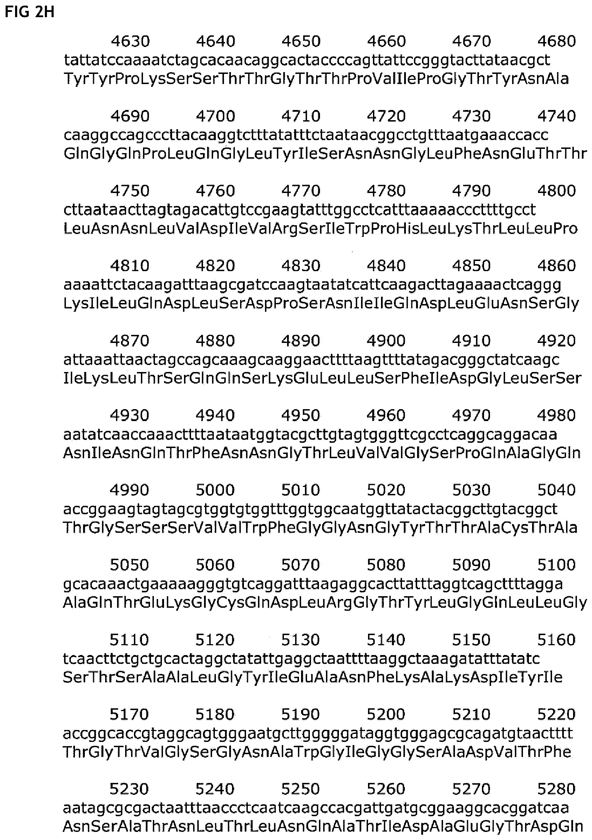

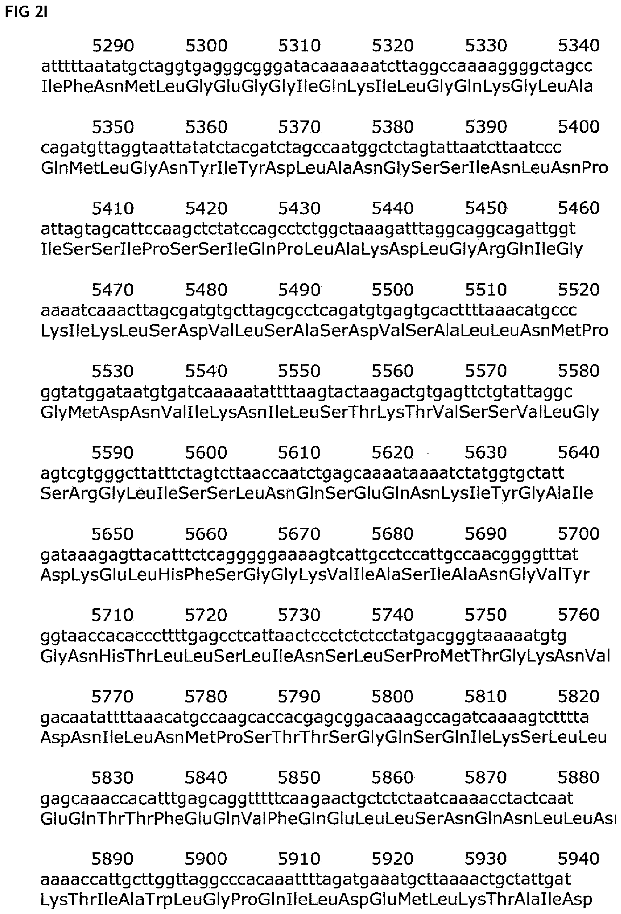

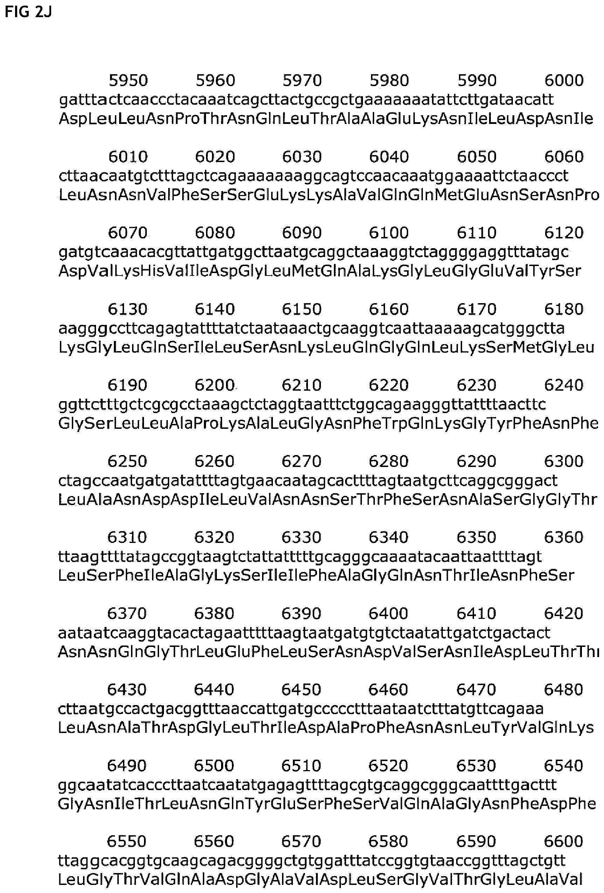

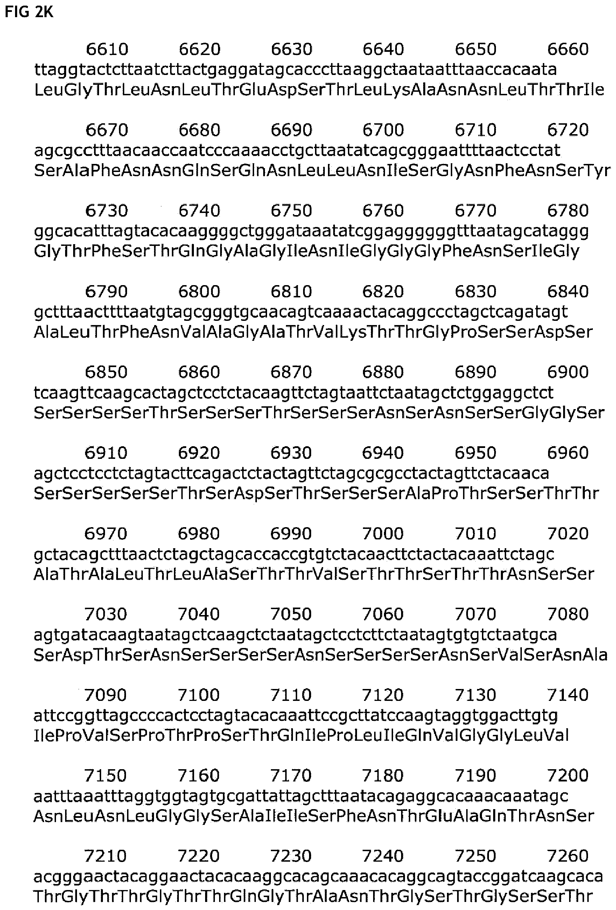

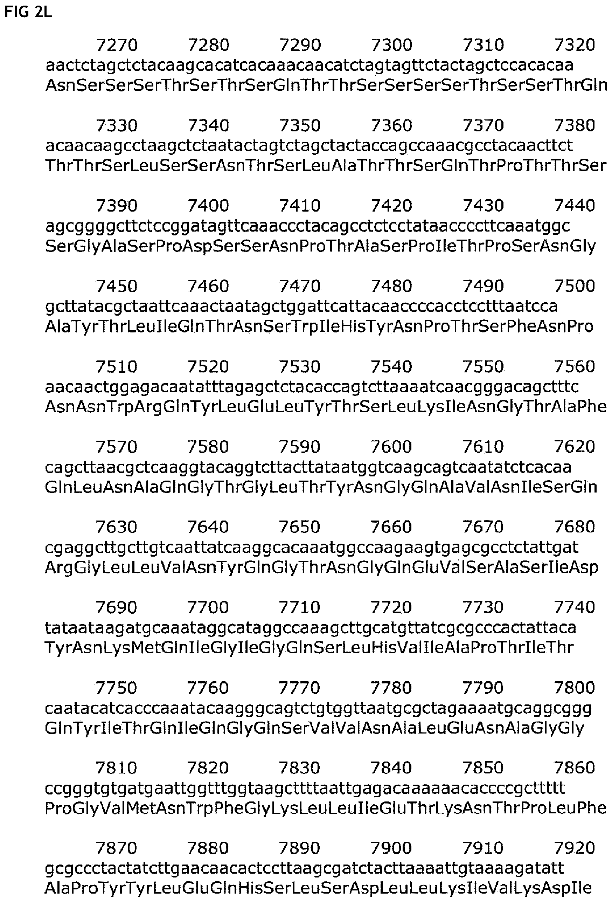

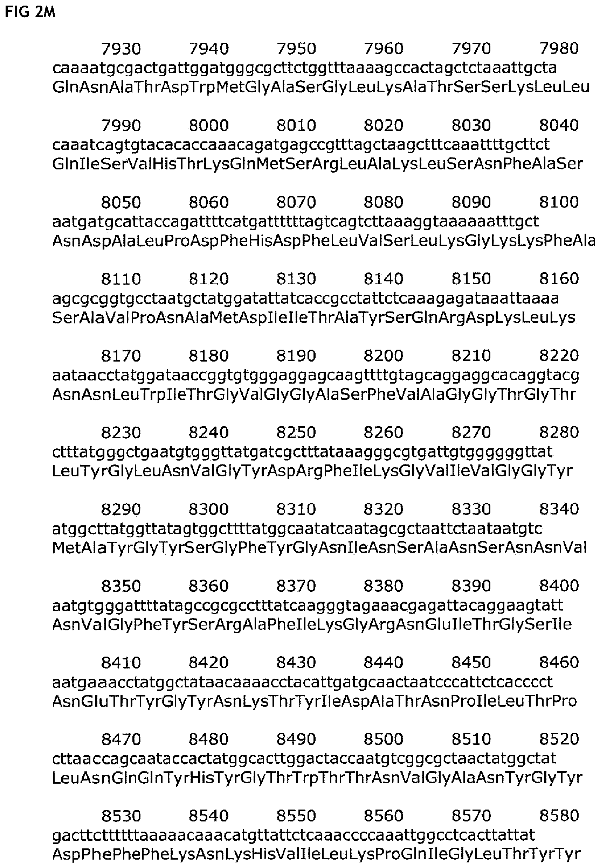

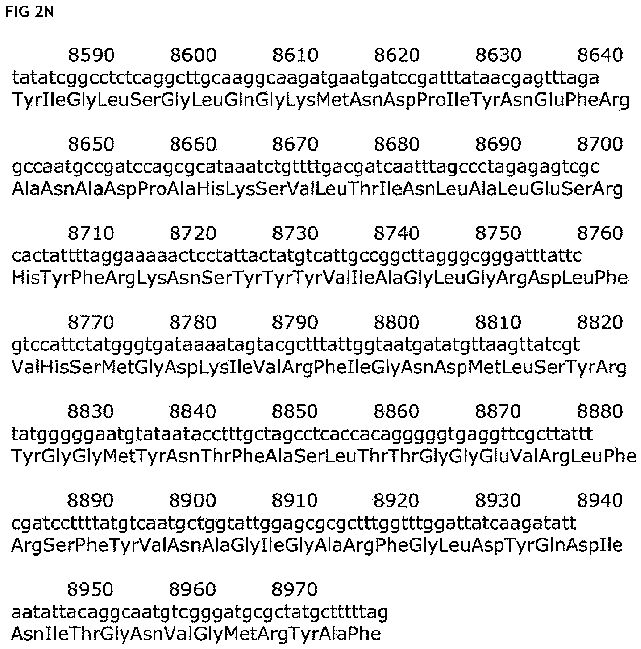

The terms "hsvA gene" and "HsvA protein" herein mean an *H. suis*-derived gene encoding or amino acids having an amino-terminal signal peptide region, a carboxy-terminal autotransporter (outer membrane transport mechanism of a protein) β-domain and a disordered region involved in the exertion of functions, in an outer membrane protein of a bacterium of the *Helicobacter* genus. One non-limiting example of the hsvA gene and the HsvA protein can include a gene having the nucleotide sequence set forth in SEQ ID NO: 1 (or the nucleotide sequence of Figure 2; the same holds true herein), and a protein having the amino acid sequence set forth in SEQ ID NO: 2 (or the amino acid sequence of Figure 2; the same holds true herein), respectively, both of which are derived from an *H. suis* SNTW101 strain. In the amino acid sequence set forth in SEQ ID NO: 2, amino acid positions 1 to 27 correspond to a signal sequence, amino acid positions 2273 to 2475 correspond to a disordered region, and amino acid positions 2686 to 2992 correspond to an autotransporter β-region. Accordingly, the HsvA protein herein includes a protein consisting of amino acid positions 28 to 2992 excluding the signal sequence in the amino acid sequence set forth in SEQ ID NO: 2. Other hsvA genes and HsvA proteins derived from other strains of *H. suis* are also included in the hsvA gene and the HsvA protein herein. As one example, the HsvA protein includes an *H. suis*-derived protein having an amino acid sequence having 80%, 85%, 90%, 95%, 98%, or 99% identity to the amino acid sequence set forth in SEQ ID NO: 2 (or an amino acid sequence consisting of amino acid positions 28 to 2992 in the amino acid sequence set forth in SEQ ID NO: 2). Likewise, the hsvA gene includes an *H. suis*-derived gene having a nucleotide sequence encoding an amino acid sequence having 80%, 85%, 90%, 95%, 98%, or 99% identity to the amino acid sequence set forth in SEQ ID NO: 2 (or an amino acid sequence consisting of amino acid positions 28 to 2992 in the amino acid sequence set forth in SEQ ID NO: 2). In this context, the identity of an amino acid sequence can be determined by a method usually used by those skilled in the art, for example, using a known program such as BLAST or FASTA. Alternatively, the hsvA gene includes an *H. suis*-derived gene comprising DNA hybridizing under stringent conditions to DNA having a nucleotide sequence complementary to the nucleotide sequence set forth in SEQ ID NO: 1. The HsvA protein includes a protein encoded by this gene. The HsvA protein further includes an *H. suis*-derived protein having an amino acid sequence derived from the amino acid sequence set forth in SEQ ID NO: 2 (or an amino acid sequence consisting of amino acid positions 28 to 2992 in the amino acid sequence set forth in SEQ ID NO: 2) by the substitution with other amino acids, deletion, addition, or insertion of 1 to 50 (which may be, for example, 1 to 40, 1 to 30, 1 to 25, 1 to 20, 1 to 15, 1 to 10, 1 to 8, 1 to 6, 1 to 5, 1 to 4, 1 to 3, or 1 to 2; the same holds true herein) amino acids. Likewise, the hsvA gene includes an *H. suis*-derived gene having a nucleotide sequence encoding an amino acid sequence derived from the amino acid sequence set forth in SEQ ID NO: 2 (or an amino acid sequence consisting of amino acid positions 28 to 2992 in the amino acid sequence set forth in SEQ ID NO: 2) by the substitution with other amino acids, deletion, addition, or insertion of 1 to 50 amino acids.

The hsvA gene and the HsvA protein include, for example, a gene having the nucleotide sequence set forth in SEQ ID NO: 27 or 29, and the amino acid sequence set forth in SEQ ID NO: 28 or 30, which are derived from an *H. suis* HS1 or HS5 strain, respectively.

Preferably, the HsvA protein has any one of the amino acid sequences set forth in SEQ ID NOs: 78 to 83; an amino acid sequence having 80%, 85%, 90%, 95%, 98%, or 99% identity to any one of the amino acid sequences set forth in SEQ ID NOs: 78 to 83; or an amino acid sequence derived from any one of the amino acid sequences set forth in SEQ ID NOs: 78 to 83 by the substitution with other amino acids, deletion, addition, or insertion of 1 to 50 amino acids.

(HsvA antigen peptide)

The term "HsvA antigen peptide" herein means a peptide having an amino acid sequence contained in the HsvA protein mentioned above, the peptide being capable of functioning as an antigen *in vivo.* Preferably, the peptide has an amino acid sequence conserved among a plurality of *H. suis* strains. Preferably, the HsvA antigen peptide consists of an *H. suis*-specific amino acid sequence absent in *H. pylori* or other *H. heilmannii.* Since HsvA is a membrane protein, preferably, the HsvA antigen peptide comprises an amino acid sequence constituting the extracellular domain of the HsvA protein. The HsvA antigen peptide consists of, for example, an amino acid sequence contained in a sequence from positions 1467 to 2992, positions 1467 to 2685, positions 1535 to 2135, or positions 2008 to 2135 in the amino acid sequence set forth in SEQ ID NO: 2 of the HsvA protein derived from an *H. suis* SNTW101 strain. As a result of analyzing HsvA-specific amino acid sequences that satisfy such conditions, the present inventors have successfully found the partial amino acid sequences set forth in SEQ ID NOs: 78 to 83. The amino acid sequences set forth in SEQ ID NOs: 78 to 83 are of HsvA proteins derived from different strains of *H. suis* infecting different species. These amino acid sequences are relatively conserved and have 76.7% identity. Accordingly, the HsvA protein antigen peptide of the present invention can have an amino acid sequence having 70%, 75%, 80%, 85%, 90%, 95%, 98%, or 99% identity to any one of the amino acid sequences set forth in SEQ ID NOs: 78 to 83; or an amino acid sequence derived from any one of the amino acid sequences set forth in SEQ ID NOs: 78 to 83 by the substitution with other amino acids, deletion, addition, or insertion of 1 to 50 amino acids, or can have an amino acid sequence contained in this amino acid sequence. The phrase "having a (particular) amino acid sequence" herein is meant to also include "consisting of the amino acid sequence".

The number of amino acids constituting the HsvA antigen peptide can be appropriately selected according to a purpose and may be, for example, 5 to 50 amino acids, 5 to 45 amino acids, 5 to 40 amino acids, 5 to 35 amino acids, 5 to 30 amino acids, 5 to 25 amino acids, 5 to 20 amino acids, 5 to 15 amino acids, 10 to 50 amino acids, 10 to 45 amino acids, 10 to 40 amino acids, 10 to 35 amino acids, 10 to 30 amino acids, 10 to 25 amino acids, or 10 to 20 amino acids. One example of the HsvA antigen peptide can include a peptide having the whole or a portion of an amino acid sequence contained in EKX1AVX2X3X4X5NSNX6X7 (peptide No. 11 (mix): SEQ ID NO: 24) (wherein X1 is K or D, X2 is Q, E or T, X3 is Q or S, X4 is M or L, X5 is E or K, X6 is P or S, and X7 is D or G), EKKAVQQMENSNPD (peptide No. 11 (SNTW101; HS1; HS5): SEQ ID NO: 3), EKKAVEQMENSNPD (peptide No. 11 (TKY): SEQ ID NO: 4), EKDAVTSLKNSNSG (peptide No. 11 (SH8): SEQ ID NO: 5), EKDAVTSLENSNSG (peptide No. 11 (SH10): SEQ ID NO: 6), NQGTLEFLSNDVSX8 (peptide No. 19: SEQ ID NO: 25) (wherein X8 is N or T), NQGTLEFLSNDVSN (peptide No. 19: SEQ ID NO: 7), NQGTLEFLSNDVST (peptide No. 19 (TKY): SEQ ID NO: 8), X9SX10KLQX11X12LKSX13X14X15 (peptide No. 33: SEQ ID NO: 26) (wherein X9 is L or F, X10 is N or D, X11 is G, D or N, X12 is Q or M, X13 is M or L, X14 is G or N, and X15 is L or M), LSNKLQGQLKSMGL (peptide No. 33: SEQ ID NO: 9), LSNKLQDQLKSMGL (peptide No. 33 (TKY): SEQ ID NO: 10), FSDKLQNMLKSLNM (peptide No. 33 (SH10): SEQ ID NO: 11), TNGQEVSASIDYNK (peptide No. 16: SEQ ID NO: 12), AKLSNFASNDALPD (peptide No. 23: SEQ ID NO: 13), PTTSSGASPDSSNP (peptide No. 10: SEQ ID NO: 14), GLGRDLFVHSMGDK (peptide No. 21: SEQ ID NO: 15), QIGKIKLSDVLSAS (peptide No. 15: SEQ ID NO: 16), YGAIDKELHFSGGK (peptide No. 34: SEQ ID NO: 17), NVDNILNMPSTTSG (peptide No. 20: SEQ ID NO: 18), GNLKGVYYPKSSTT (peptide No. 22: SEQ ID NO: 19), ITEKIQSGKLTITI (peptide No. 14: SEQ ID NO: 20), FHDFLVSLKGKKFA (peptide No. 26: SEQ ID NO: 21), TTGGEVRLFRSFYV (peptide No. 31: SEQ ID NO: 22), IGARFGLDYQDINI (peptide No. 35: SEQ ID NO: 23), KQLPQPKRSELKPK (peptide No. 8: SEQ ID NO: 86), TNIKQYMQNNHRSQ (peptide No. 31N: SEQ ID NO: 87), TLTLEGTETFAQNS (peptide No. 81: SEQ ID NO: 88), EAYAKNQGDIWSTI (peptide No. 63: SEQ ID NO: 89), VIGSKSSITLNSAN (peptide No. 73: SEQ ID NO: 90), and ADIQSSQTTFANSV (peptide No. 61: SEQ ID NO: 91). The HsvA antigen peptide may have one to several amino acids substituted, deleted, added or inserted in the peptide sequence as long as the peptide exerts functions as the HsvA antigen of interest. In the substitution of an amino acid, preferably, the amino acid is conservatively substituted with a similar amino acid residue. Examples thereof can include substitution between amino acids such as G and P, G and A or V, L and I, E and Q, D and N, C and T, T and S or A, and K and R.

The amino acid herein is described in a single-letter code. Specifically, C represents cysteine, A represents alanine, Y represents tyrosine, H represents histidine, R represents arginine, G represents glycine, E represents glutamic acid, L represents leucine, V represents valine, W represents tryptophan, T represents threonine, Y represents tyrosine, I represents isoleucine, F represents phenylalanine, D represents aspartic acid, N represents asparagine, Q represents glutamine, M represents methionine, K represents lysine, and P represents proline. Xn (n is a natural number) herein represents one type of amino acid selected from the group consisting of a plurality of amino acids defined respectively. For example, if "X1" is K or D in EKX1AVX2X3X4X5NSNX6X7 (SEQ ID NO: 24), that means the amino acid represented by X1 is either lysine or aspartic acid.

In one aspect, the present invention relates to an immunologically equivalent mutant of the HsvA antigen peptide. The term "Immunologically equivalent mutant of the peptide" herein means a peptide capable of binding to an antibody that binds to a peptide having a sequence derived from natural HsvA protein, but has an amino acid sequence different from that of the peptide having a sequence derived from natural HsvA protein. The mutant may be, for example, a peptide having one to several amino acids substituted with natural or artificial amino acids, deleted, added, or inserted in the peptide sequence. Alternatively, the mutant may be a peptide having some modified amino acids constituting the peptide having a sequence derived from natural HsvA protein. Alternatively, both the mutations, i.e., the amino acid mutation and the modification, may be introduced therein. The binding to an antibody that binds to a peptide having a sequence derived from natural HsvA protein means binding to an anti-HsvA antibody in other words. For example, a peptide capable of binding to an anti-HsvA antibody present in the blood of a patient infected with *H. suis* is included in the immunologically equivalent mutant of the peptide of the present invention. Whether or not a test peptide mutant is capable of binding to the antibody that binds to a peptide having a sequence derived from natural HsvA protein, or the anti-HsvA antibody present in the blood of a patient infected with *H. suis* can be determined by reacting the antibody with a solid phase bound with the peptide mutant, washing the solid phase, and then further reacting a labeled anti-IgG antibody therewith. When labeled IgG bound with the solid phase is detected after washing of labeled IgG, this peptide is determined as the immunologically equivalent mutant of the peptide of the present invention. The term "HsvA antigen peptide" herein includes the immunologically equivalent mutant of the HsvA antigen peptide, unless otherwise specified.

(Anti-HsvA antibody or immunoreactive fragment thereof)

In another aspect, the present invention relates to an antibody specifically recognizing HsvA protein (referred to as "anti-HsvA antibody" herein) or an immunoreactive fragment thereof. The anti-HsvA antibody of the present invention preferably binds to any of the HsvA protein antigen peptides defined above. The anti-HsvA antibody binds to, for example, a peptide having any one of the amino acid sequences set forth in SEQ ID NOs: 78 to 83; an amino acid sequence having 80%, 85%, 90%, 95%, 98%, or 99% identity to any one of the amino acid sequences set forth in SEQ ID NOs: 78 to 83; or an amino acid sequence derived from any one of the amino acid sequences set forth in SEQ ID NOs: 78 to 83 by the substitution with other amino acids, deletion, addition, or insertion of 1 to 50 amino acids. "Immunoreactive fragment of the antibody" means a portion of the antibody (partial fragment) or a peptide comprising a portion of the antibody, which retains the binding effect of the antibody to the antigen. Examples of such a fragment of the antibody can include $F(ab')_2$, Fab', Fab, single-chain Fv (hereinafter, referred to as "scFv"), disulfide-stabilized Fv (hereinafter, referred to as "dsFv") and their polymers, dimerized V region (hereinafter, referred to as "Diabody"), and a peptide comprising CDRs. Preferably, the antibody of the present invention or the immunoreactive fragment thereof specifically recognizes the HsvA antigen peptide. The phrase ""specifically" recognizes (binds)" herein for the antibody or the immunoreactive fragment thereof means that the antibody or the immunoreactive fragment thereof binds to the HsvA antigen peptide with substantially higher affinity than that for other amino acid sequences or conformations. The association constant (Ka) of the binding of the antibody of the present invention to the HsvA antigen can include, for example, at least $10^7$ $M^{-1}$, at least $10^8$ $M^{-1}$, at least $10^9$ $M^{-1}$, at least $10^{10}$ $M^{-1}$, at least $10^{11}$ $M^{-1}$, at least $10^{12}$ $M^{-1}$, and at least $10^{13}$ $M^{-1}$.

The antibody of the present invention may be polyclonal or monoclonal as long as the antibody can specifically recognize the HsvA antigen peptide. Preferably, the antibody of the present invention is monoclonal antibody. In this context, "monoclonal antibody" means an antibody derived from a single clone. The antibody of the present invention includes a complete antibody as well as a bispecific antibody and a single-chain antibody as long as the bispecific antibody and the single-chain antibody retain binding activity against the antigen. The complete antibody encompasses a nonhuman animal antibody, an antibody having the amino acid sequence of a nonhuman animal antibody and the amino acid sequence of a human-derived antibody, and a human antibody. Examples of the nonhuman animal antibody can include mouse, rat, hamster, and rabbit antibodies. The nonhuman animal antibody is preferably an antibody of an animal from which a hybridoma can be prepared, more preferably a mouse antibody. Examples of the antibody having the amino acid sequence of a nonhuman animal antibody and the amino acid sequence of a human-derived antibody can include a human-type chimeric antibody composed of a human antibody grafted with antigen binding domain Fv of an animal-derived monoclonal antibody, and a humanized antibody in which the CDR sequences of Fv domains directly involved in the antigen binding of an animal-derived monoclonal antibody are incorporated in the framework regions of a human antibody. The human antibody is a human antibody which is an expression product of a completely human-derived antibody gene. The immunoglobulin class of the antibody of the present invention is not particularly limited and may be any of immunoglobulin classes IgG, IgM, IgA, IgE, IgD, and IgY. Preferably, the immunoglobulin class of the antibody of the present invention is IgG. The antibody of the present invention also encompasses an antibody of any isotype.

The HsvA antigen peptide, or the anti-HsvA antibody or the immunoreactive fragment thereof may be labeled, if necessary. A detectable label such as a radiolabel, an enzyme, a fluorescent label, a bioluminescent label, a chemiluminescent label, metal can be used as the label. Examples of such a label can include, but are not limited to, detectable labels including: radiolabels such as 35S (sulfur 35), 32P (phosphorus 32), 3H (tritium), 1251 (iodine 125), 1311 (iodine 131), and 14C (carbon 14); enzymes such as β galactosidase, peroxidase, alkaline phosphatase, glucose oxidase, lactate oxidase, alcohol oxidase, monoamine oxidase, and horse radish peroxidase; coenzymes or prosthetic groups such as FAD, FMN, ATP, biotin, and hem; fluorescent labels such as fluorescein derivatives (fluorescein isothiocyanate (FITC), fluorescein thiofulbamil, etc.), rhodamine derivatives (tetramethylrhodamine, trimethylrhodamine (RITC), Texas Red, rhodamine 110, etc.), Cy dyes (Cy3, Cy5, Cy5.5, and Cy7), Cy-chrome, Spectrum Green, Spectrum Orange, propidium iodide, allophycocyanin (APC), R-phycoerythrin (R-PE), Alexa-Flour(R) fluorescent dyes such as Alexa-Flour(R) 488 and Alexa-Flour(R) 568 (Molecular Probes, Inc., USA), and Alexa-Flour(R) 568; bioluminescent labels such as luciferase; chemiluminescent labels such as luminol, luminol derivatives such as isoluminol and N-(4-aminobutyl)-N-ethyl isoluminol ester, acridinium derivatives such as N-methyl acridinium ester and N-methyl acridinium acylsulfonamide ester, lucigenin, adamantyl dioxetane, indoxyl derivatives, and ruthenium complexes; and metals such as metal colloids.

(Nucleic acid molecule, primer, and probe having hsvA gene-specific nucleotide sequence)

In an alternative aspect, the present invention relates to a nucleic acid molecule capable of specifically binding to hsvA gene. Preferably, the nucleic acid molecule capable of specifically binding to hsvA gene relates to a nucleic acid molecule having an hsvA gene-specific nucleotide sequence or a sequence complementary to the nucleotide sequence, or a nucleic acid molecule capable of binding under stringent conditions to an hsvA gene-specific nucleotide sequence or a sequence complementary to the nucleotide sequence (hereinafter, referred to as "nucleic acid molecule having an hsvA gene-specific nucleotide sequence, etc."). The hsvA-specific nucleic acid molecule may be a primer or a probe according to an application. In this context, the primer is a nucleic acid molecule that is used for the purpose of amplifying DNA by PCR or the like. The primer is a nucleic acid molecule that hybridizes to DNA having a sequence complementary to the primer so that the DNA can be elongated and thereby amplified. The DNA is detectable by detecting the amplified DNA. The probe is a nucleic acid molecule that is used for the purpose of detecting the presence of target DNA through binding to the target DNA. The nucleic acid molecule capable of binding to hsvA gene of the present invention may have a nucleotide sequence that is not derived from hsvA in the nucleotide sequence of the hsvA gene as long as the sequence is not identical or similar to that of *H. pylori* and *H. heilmannii* (except for *H. suis).* Whether or not a selected nucleotide sequence is specific for the hsvA gene can be determined, for example, by searching an already published database for this sequence, examining whether or not *H. pylori* and *H. heilmannii* (except for *H. suis)* have an identical or similar sequence, and determining the sequence to be not specific when the identical or similar sequence is present and to be specific when the identical or similar sequence is absent. In this context, the phrase "not similar" may mean that the identity is 10% or lower, 20% or lower, 30% or lower, 40% or lower, or 50% or lower. The nucleic acid molecule capable of specifically binding to hsvA gene can be selected as an arbitrary sequence, for example, from SEQ ID NO: 1, according to the method described above. The nucleic acid molecule capable of binding to hsvA gene of the present invention can be of, for example, 5 to 50 nucleotides, 5 to 45 nucleotides, 5 to 40 nucleotides, 5 to 35 nucleotides, 5 to 30 nucleotides, 5 to 25 nucleotides, 5 to 20 nucleotides, 5 to 15 nucleotides, 5 to 10 nucleotides, 10 to 50 nucleotides, 10 to 45 nucleotides, 10 to 40 nucleotides, 10 to 35 nucleotides, 10 to 30 nucleotides, 10 to 25 nucleotides, 10 to 20 nucleotides, 10 to 15 nucleotides, 15 to 50 nucleotides, 15 to 45 nucleotides, 15 to 40 nucleotides, 15 to 35 nucleotides, 15 to 30 nucleotides, 15 to 25 nucleotides, 15 to 20 nucleotides, or 17 to 25 nucleotides. Preferably, examples of the hsvA gene-specific nucleotide sequence can include CTTTTAGCGTGGTATCCTTC (SEQ ID NO: 31), TTACAAAGACCACCAACGGA (SEQ ID NO: 32), TACCATAGAAAGCGGAAAC (SEQ ID NO: 33), AGCTATAGCTTTGCACCTGA (SEQ ID NO: 34), ACTAACAGCATAGAAGTTGGGGA (SEQ ID NO: 35), AACAACATTACAGGCATGAG (SEQ ID NO: 36), CAAATAGTAACAGGACAATT (SEQ ID NO: 37), CAGGATTTAAGAGGCACTTA (SEQ ID NO: 38), TCTTAACCAATCTGAGCAA (SEQ ID NO: 39), GTCAAACACGTTATTGATGGCTT (SEQ ID NO: 40), GGGGTTTAATAGCATAGGG (SEQ ID NO: 41), TAGAGCTCTACACCAGTCTT (SEQ ID NO: 42), ATAAAGCCCATGAATTCTTAGGCATGCGTGCTCTG (SEQ ID NO: 43), TGCTATTGATAAAGAGTTACATTTCTCAGG (SEQ ID NO: 44), ATTTCTCAGGGGGAAAGTC (SEQ ID NO: 45), GGCTAATAATTTAACCACAATAAGCGCCTTTAA (SEQ ID NO: 46), GATGGGCGCTTCTGGTTTA (SEQ ID NO: 47), ATGAATTCTTAGGCATGCGTGCTCT (SEQ ID NO: 48), TTTTGGAGGTGTAGGAGTAGAT (SEQ ID NO: 49), AGCGCGCATTTAAAACAGGT (SEQ ID NO: 50), AGAAACGAGATTACAGGAAG (SEQ ID NO: 51), AGGAGCAAGTTTTGTAGCAG (SEQ ID NO: 52), AAAATGCGACTGATTGGATG (SEQ ID NO: 53), TTGAAATTTGGCCACGCT (SEQ ID NO: 54), TCACCCATAGAATGGACGAA (SEQ ID NO: 55), CTAGCGCATTAACCACAGACTG (SEQ ID NO: 56), TAGAAGTTGTAGACACGGT (SEQ ID NO: 57), GTGATATTGCCTTTCTGAAC (SEQ ID NO: 58), GCAAGTTTTGTGCGGATT (SEQ ID NO: 59), ATGTGATACACATCTGACC (SEQ ID NO: 60), AGTGCCGTTACCATCGTGAA (SEQ ID NO: 61), TATTCAAGGAAAGTCCCTGGAGAAACTCCAGAGAC (SEQ ID NO: 62), TTAAAGGCGCTTATTGTGGTTAAATTATTAGCC (SEQ ID NO: 63), ATTAGCCTTAAGGGTGCTATC (SEQ ID NO: 64), CTGGTAATGCATCATTAGAAGCAAA (SEQ ID NO: 65), TACGCGCAAAATAGGTTCTT (SEQ ID NO: 66), TGGAGAAACTCCAGAGACTA (SEQ ID NO: 67), TTGTTCGCTGTAGTGCCGTGG (SEQ ID NO: 68), GAAGGATACCACGCTAAAAG (SEQ ID NO: 69), AAGCTAGAGTTTTGGTTGAG (SEQ ID NO: 70), TTGCTCAGATTGGTTAAGA (SEQ ID NO: 71), ATCGAAATAAGCGAACCTCA (SEQ ID NO: 72), TTGAAAGCTTAGCTAAACGG (SEQ ID NO: 73), TGGTATTGCTGGTTAAGAGG (SEQ ID NO: 74), CAAACAGATGAGCCGT (SEQ ID NO: 75), ATGAAAAAGTTTAGTTCTCTCACATTGAAATTTGGCCACGCTC (SEQ ID NO: 76), and CTAAAAAGCATAGCGCATCCCGACATTGCCTGTAATATTAATATC (SEQ ID NO: 77).

In use of the nucleic acid molecule having an hsvA gene-specific nucleotide sequence as a primer, primers selected from the following primers can be used in combination as a forward primer and a reverse primer.

(Forward primer)

HSVANOFW-2: AGAAACGAGATTACAGGAAG (SEQ ID NO: 51)
HSVANOFW-3: AGGAGCAAGTTTTGTAGCAG (SEQ ID NO: 52)
HSVANOFW-5: AAAATGCGACTGATTGGATG (SEQ ID NO: 53)

(Reverse primer)

HSVANORV-1: ATCGAAATAAGCGAACCTCA (SEQ ID NO: 72)
HSVANORV-3: TTGAAAGCTTAGCTAAACGG (SEQ ID NO: 73)
HSVANORV-4: TGGTATTGCTGGTTAAGAGG (SEQ ID NO: 74).

In use of the nucleic acid molecule having an hsvA gene-specific nucleotide sequence as a primer, the combination of the forward primer and the reverse primer is preferably a combination HSVANOFW-2/HSVANORV-4 (101 bp), HSVANOFW-3/HSVANORV-4 (291 bp), HSVANOFW-5/HSVANORV-4 (556 bp), HSVANOFW-2/HSVANORV-1 (508 bp), or HSVANOFW-5/HSVANORV-3 (108 bp) (the length of a DNA fragment obtained by PCR amplification using the primers is indicated within the parentheses).

The phrase "hybridizing under stringent conditions" herein means hybridizing under hybridization conditions usually used by those skilled in the art. Whether or not to hybridize can be determined by a method described in, for example, Molecular Cloning, a Laboratory Manual, Fourth Edition, Cold Spring Harbor Laboratory Press (2012), or Current Protocols in Molecular Biology, Wiley Online Library. The hybridization conditions may be, for example, conditions involving hybridization at 42°C in 6 × SSC (0.9 M NaCl, 0.09 M trisodium citrate) or 6 × SSPE (3 M NaCl, 0.2 M $NaH_2PO_4$, 20 mM EDTA·2Na, pH 7.4) followed by washing with 0.5 × SSC at 42°C.

A "specific" sequence herein means that the sequence is characteristic of the protein, the peptide, or the nucleic acid molecule, and means that the sequence is substantially absent in other proteins, peptides, or nucleic acid molecules. In this context, the phrase "substantially absent" includes not only the case where a completely identical sequence is absent, as well as the case where similar sequence to that of the objective molecule (hsvA gene or HsvA protein) is present in the other molecules (e.g., an H. pylori-derived component) but the presence of the similar sequence still allows discrimination in biological binding reaction.

The phrase "specifically recognizing" or "specifically binding" means binding to the substance of interest but not substantially binding to other substances. Whether or not a candidate substance binds to the substance of interest and/or substantially does not bind to other substances can be confirmed by adopting a method well known to those skilled in the art such as Southern hybridization, PCR, Western blotting, or ELISA according to the types of the candidate substance (a nucleic acid, an antibody, etc.) and the substance of interest (a nucleic acid, a protein, etc.). In Western blotting, a protein can be confirmed, for example, by dissolving the protein in a solution of 2% SDS, 10% glycerol, 50 mM Tris-HCl (pH 6.8), and 100 mM dithiothreitol, boiling the solution, and conducting Western blot analysis using an anti-His antibody and a test antibody. In Southern hybridization, PCR, or ELISA, the substance can be confirmed using a method mentioned later. In this context, the phrase "not substantially binding" includes sufficiently discriminably low binding of a test substance to an allegedly non-bindable (or non-recognizable) nucleic acid, protein or peptide when compared with binding to the substance of interest. For example, the rate of cross-reaction with other substances compared with the substance of interest may be 1% or less, 0.5% or less, 0.3% or less, 0.1% or less, 0.05% or less, or 0.03% or less. The cross-reactivity can be obtained by calculation according to {(Actually measured value (concentration) for binding to other substances to be compared / Added sample concentration of the other substances to be compared) × 100%}. Particularly, the phrase " the antibody or the immunoreactive fragment thereof "specifically" recognizes (binds)" herein means that the antibody or the immunoreactive fragment thereof binds to the antigen with substantially high affinity compared with affinity for other amino acid sequences, or the antigen binds to the antibody or the immunoreactive fragment thereof with substantially high affinity compared with affinity for other antibodies or immunoreactive fragments thereof. The term "substantially high affinity" herein means high affinity detectable by discriminating the particular amino acid sequence from other amino acid sequences using a desired measurement apparatus or method. The substantially high affinity may be, for example, 3 or more times, 4 or more times, 5 or more times, 6 or more times, 7 or more times, 8 or more times, 9 or more times, or 10 or more times in terms of intensity (e.g., fluorescence intensity) detected by ELISA or EIA.

(Kit and composition)

In an alternative aspect, the present invention relates to a kit for determination of *H. suis* infection, a composition for determination of *H. suis* infection, and a pharmaceutical composition, comprising the HsvA antigen peptide or the nucleic acid molecule capable of specifically binding to hsvA gene. The kit and the composition of the present invention may further comprise a carrier selected from the group consisting of a solid phase, a hapten, and an insoluble carrier.

The kit and the composition of the present invention containing the nucleic acid molecule capable of specifically binding to hsvA gene can be based on a known method using a nucleic acid. Examples of such a method can include: a method using hybridization; a method using PCR; and a method known as Invader(R) assay (see, for example, Kwiatkowski, R.W. et al., Mol. Diagn. (1999) 4: 353-364).

The kit and the composition of the present invention may comprise, for example, a solid phase or a hapten on which a labeled nucleic acid molecule capable of specifically binding to hsvA gene is immobilized, and optionally, a solid phase on which a substance specifically binding to the hapten is immobilized. The kit and the composition of the present invention may comprise the nucleic acid molecule capable of specifically binding to hsvA gene as a primer (or a primer pair) or a probe. Alternatively, the kit and the composition of the present invention can have an allele-specific probe having an hsvA gene-specific nucleotide sequence, etc. and a sequence complementary to a portion of a quenching probe (flap), an Invader probe having the hsvA gene-specific nucleotide sequence, etc., triplex-specific DNase, and a fluorescently labeled universal probe having the quenching probe. The flap preferably differs among allele-specific probes. The fluorescent label can be appropriately selected for a probe well known to those skilled in the art and preferably differs among fluorescently labeled universal probes. For example, FAM and VIC can be used as fluorescent labels.

The kit and the composition containing the HsvA antigen peptide, or the antibody recognizing the peptide or the immunoreactive fragment thereof can be based on a known method using an antibody molecule. Examples of such a method can include: labeled immunoassay; immunoblotting; immunochromatography; chromatography; turbidimetric immunoassay (TIA); nanofluidic proteomic immunoassay (NIA); colorimetric method; latex immunoassay (LIA); counting immunoassay (CIA); chemiluminescent (enzyme) immunoassay (CLIA and CLEIA); precipitation reaction method; surface plasmon resonance (SPR) method; resonant mirror detector (RMD) method; and comparison interferometry. Whether or not the kit of the present invention is capable of carrying out the desired measurement can be confirmed by carrying out each measurement method by a method well known to those skilled in the art using the sample concerned or a sample of the concentration concerned, and thereby determining whether or not to be detectable.

The kit and the composition of the present invention can comprise, for example, (i) a solid phase or a hapten on which the HsvA antigen peptide, or the antibody recognizing the peptide or the immunoreactive fragment thereof is immobilized, and (ii) a labeled secondary antibody. The secondary antibody can be an anti-IgG antibody (in the case of immobilizing HsvA antigen peptide) or an anti-HsvA antibody ( in the case of immobilizing anti-HsvA antibody or immunoreactive fragment thereof ) according to the immobilized substance and an object to be detected. The kit and the composition of the present invention comprising a hapten may further comprise a solid phase on which a substance specifically binding to the hapten is immobilized.

In the kit and the composition of the present invention, the solid phase is not particularly limited as long as the solid phase can be used in immunochemical measurement. Examples thereof can include plates, tubes, chips (e.g., protein chips and Lab-on-$\alpha$-Chip), beads, membranes, absorbers and/or particles containing nitrocellulose, Sepharose, nylon, vinylon, polyester, acryl, polyolefin, polyurethane, rayon, polynosic, cupra, lyocell, acetate, polyvinylidene difluoride, silicone rubber, latex, polystyrene, polypropylene, polyethylene, polyvinyl chloride, polyvinylidene chloride, polystyrene, polyvinyl acetate, fluorinated resin, ABS resin, AS resin, acrylic resin, polymer alloy, glass fiber, carbon fiber, glass, gelatin, a polyamino acid and/or a magnetically sensitive material. A plate and magnetic beads are preferred. The term "insoluble carrier" herein means a suspendable insoluble solid phase such as beads. Examples thereof can include latex beads and magnetic beads.

The kit and the composition of the present invention may comprise, if necessary, a coloring reagent, a reagent for reaction termination, a standard antigen reagent, a reagent for sample pretreatment, a blocking reagent, or the like. The kit and the composition of the present invention comprising a labeled antibody may further comprise a substrate reactive with the label. The kit of the present invention may appropriately comprise an attached document, an instruction, and a container for housing of the kit or the composition.

## Advantageous Effects of Invention

An HsvA antigen peptide and a nucleic acid molecule capable of specifically binding to hsvA gene can be used for measuring the presence or absence or antibody titer of an anti-*H. suis* antibody in blood derived from a subject to be diagnosed with *H. suis* infection. Use of the HsvA antigen peptide as a peptide vaccine is possible for prevention or treatment of *H. suis* infection. The HsvA antigen peptide can further be used as an antigen for obtaining an anti-HsvA antibody. An anti-HsvA antibody or an immunoreactive fragment thereof can be used in the detection of an HsvA antigen peptide. The anti-HsvA antibody or the immunoreactive fragment thereof can further be used as a therapeutic drug for treatment of *H. suis* infection.

## Brief Description of Drawings

[Figure 1] Figure 1 is a schematic view of an hsvA gene-specific nucleic acid molecule.

[Figure 2A] Figure 2A shows the sequences of hsvA gene and HsvA protein derived from an SNTW101 strain of *H. suis*. Figures 2A to 2N show the consecutive sequences of one gene and a protein encoded thereby.

[Figure 2B] Figure 2B shows the sequences of the hsvA gene and the HsvA protein derived from an SNTW101 strain of *H. suis.*

[Figure 2C] Figure 2C shows the sequences of the hsvA gene and the HsvA protein derived from an SNTW101 strain of *H. suis.*

[Figure 2D] Figure 2D shows the sequences of the hsvA gene and the HsvA protein derived from an SNTW101 strain of *H. suis.*

[Figure 2E] Figure 2E shows the sequences of the hsvA gene and the HsvA protein derived from an SNTW101 strain of *H. suis.*

[Figure 2F] Figure 2F shows the sequences of the hsvA gene and the HsvA protein derived from an SNTW101 strain of *H. suis.*

[Figure 2G] Figure 2G shows the sequences of the hsvA gene and the HsvA protein derived from an SNTW101 strain of *H. suis.*

[Figure 2H] Figure 2H shows the sequences of the hsvA gene and the HsvA protein derived from an SNTW101 strain of *H. suis.*

[Figure 2I] Figure 2I shows the sequences of the hsvA gene and the HsvA protein derived from an SNTW101 strain of *H. suis.*

[Figure 2J] Figure 2J shows the sequences of the hsvA gene and the HsvA protein derived from an SNTW101 strain of *H. suis.*

[Figure 2K] Figure 2K shows the sequences of the hsvA gene and the HsvA protein derived from an SNTW101 strain of *H. suis.*

[Figure 2L] Figure 2L shows the sequences of the hsvA gene and the HsvA protein derived from an SNTW101 strain of *H. suis.*

[Figure 2M] Figure 2M shows the sequences of the hsvA gene and the HsvA protein derived from an SNTW101 strain of *H. suis.*

[Figure 2N] Figure 2N shows the sequences of the hsvA gene and the HsvA protein derived from an SNTW101 strain of *H. suis.*

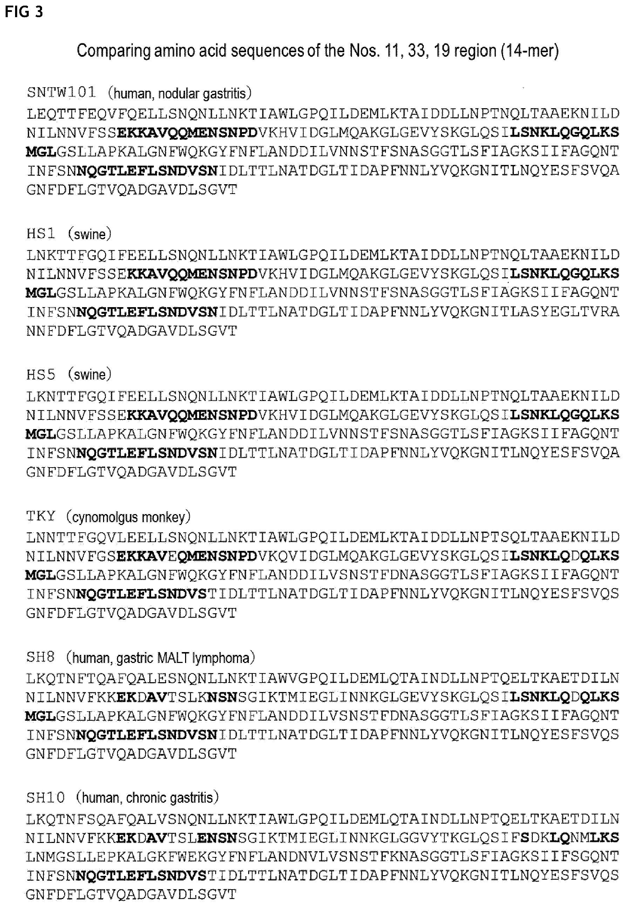

[Figure 3] Figure 3 is a diagram comparing the amino acid sequences of regions including peptide Nos. 11, 33, and 19 of SNTW101 (SEQ ID NO: 78), HS1 (SEQ ID NO: 79), HS5 (SEQ ID NO: 80), TKY (SEQ ID NO: 81), SH8 (SEQ ID NO: 82) and SH10 (SEQ ID NO: 83) strains of *H. suis.*

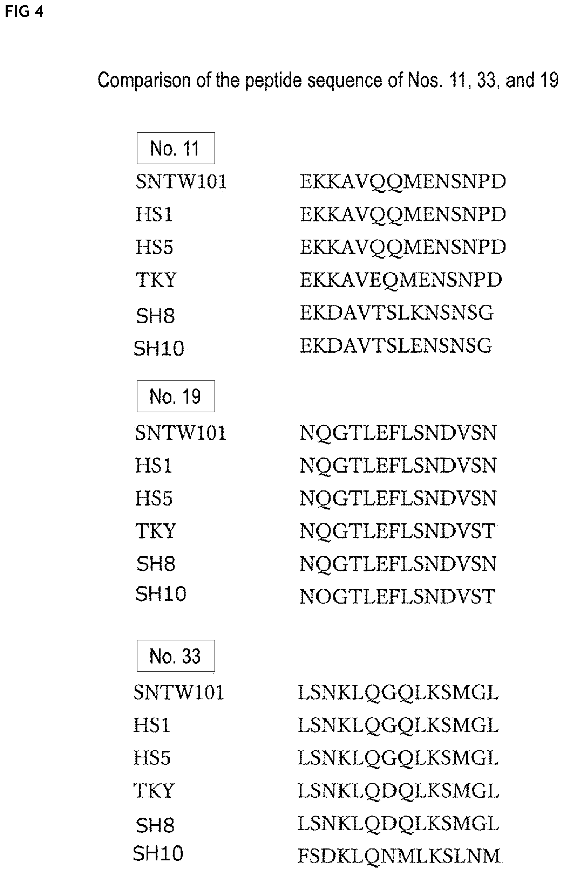

[Figure 4] Figure 4 is a diagram comparing the amino acid sequences of regions corresponding to peptide Nos. 11, 33, and 19 of SNTW101, HS1, HS5, TKY, SH8 and SH10 strains of *H. suis* (SEQ ID NOs: 3 to 11).

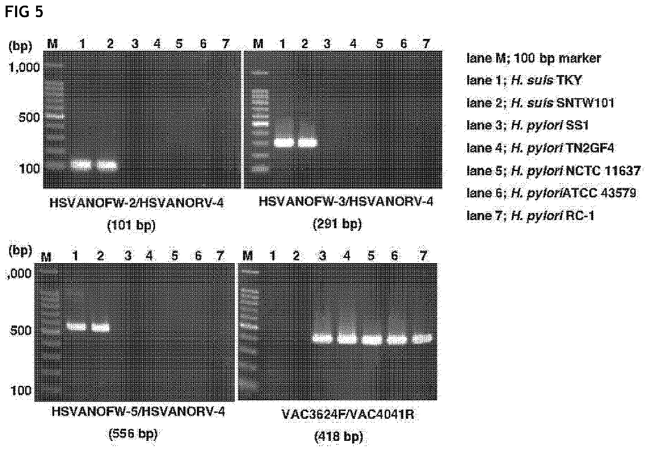

[Figure 5] Figure 5 shows the determination of *H. suis* by PCR. In photographs for *H. pylori* (SS1, TN2GF4, NCTC11637, ATCC43579, and RC-1 strains) and *H. suis* (SNTW101 and TKY strains), DNA was amplified by PCR using designed forward primer FW2, FW3, FW5, or VAC3624F (Non Patent Literature 7 described above) and reverse primer RV1, RV3, RV4, or VAC4041R (Non Patent Literature 7 described above), and the amplification product was confirmed by electrophoresis. Each lane depicts templated DNA prepared from 1) *H. suis* TKY, 2) *H. suis* SNTW101, 3) *H. pylori* SS1, 4) *H. pylori* TN2GF4, 5) *H. pylori* NCTC11637, 6) *H. pylori* ATCC43579, and 7) *H. pylori* RC-1 strains. The primer set used is shown below each photograph. For all the primer sets of the forward primer FW2, FW3, or FW5 and the reverse primer RV1, RV3, or RV4, the amplification product was confirmed only from DNA prepared from the gastric mucosa of an *H. suis*-infected mouse.

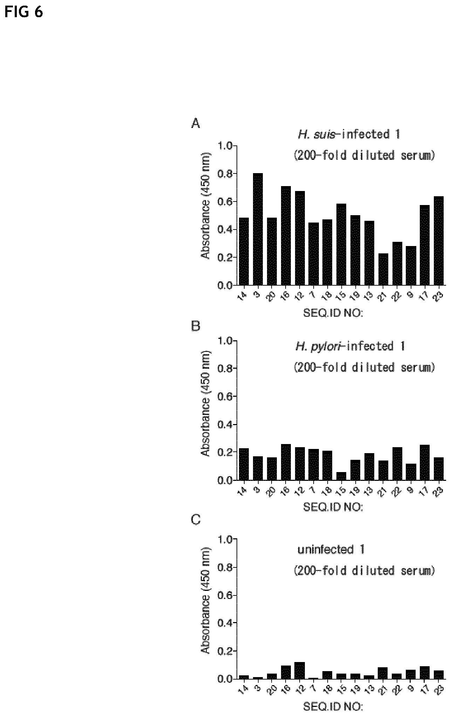

[Figure 6] Figure 6 is a graph showing results of measuring the binding titers of antibodies in serum obtained from A, an *H.* suis-infected human, B, an *H. pylori*-infected human, and C, a non-infected human against various peptides by ELISA. The abscissa depicts Nos of the peptides. The ordinate depicts absorbance at 450 nm. A mean from the experiment conducted in duplicate is shown. Synthesized 15 HsvA antigen peptides (Nos. 10 (SEQ ID NO: 14), 11 (SEQ ID NO: 3), 14 (SEQ ID NO: 20), 15 (SEQ ID NO: 16), 16 (SEQ ID NO: 12), 19 (SEQ ID NO: 7), 20 (SEQ ID NO: 18), 21 (SEQ ID NO: 15), 22 (SEQ ID NO: 19), 23 (SEQ ID NO: 13), 26 (SEQ ID NO: 21), 31 (SEQ ID NO: 22), 33 (SEQ ID NO: 9), 34 (SEQ ID NO: 17), and 35 (SEQ ID NO: 23)) specifically reacted with the serum of the subject infected with *H. suis.*

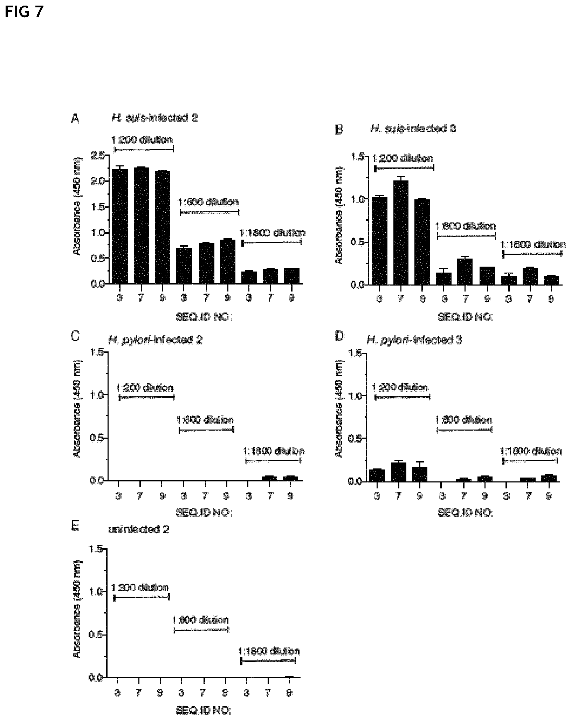

[Figure 7] Figure 7 is a graph showing results of measuring the binding titers of antibodies in the serum of 5 subjects against No. 11 peptide (SEQ ID NO: 3), No. 19 peptide (SEQ ID NO: 7), and No. 33 peptide (SEQ ID NO: 9) by ELISA. A and B show data from the serum of different *H. suis*-infected subjects, respectively. C and D show data from the serum of different *H. pylori*-infected subjects, respectively. E shows data from the serum of a non-infected subject. The abscissa depicts SEQ ID NOs of the antigen peptides and dilution ratios of the serum. The ordinate depicts absorbance at 450 nm. A mean from the experiment conducted in triplicate and standard deviation are shown. All the peptides strongly reacted with the *H. suis*-infected serum and weakly reacted with the *H. pylori*-infected or non-infected serum.

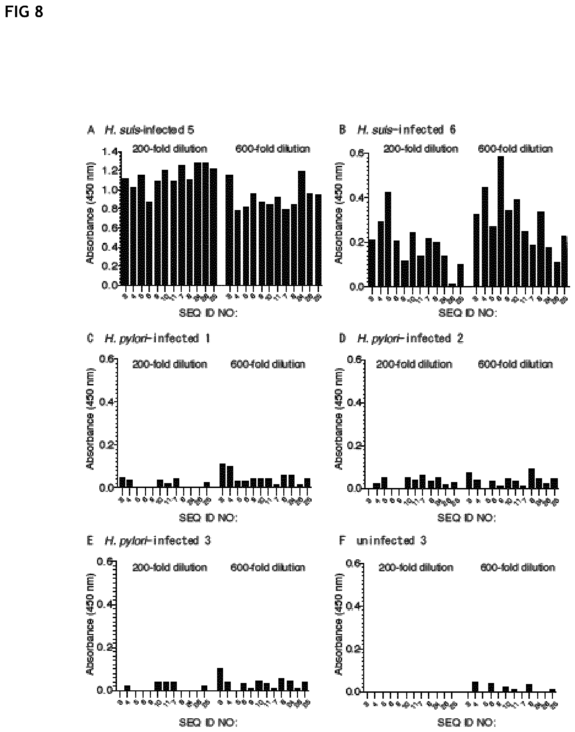

[Figure 8] Figure 8 is a graph showing results of measuring the binding titers of antibodies in the serum of 6 subjects (two *H. suis*-infected subjects, three *H. pylori*-infected subjects, and one non-infected subject) against No. 11 peptide (SEQ ID NOs: 3, 4, 5, 6, and 24), No. 19 peptide (SEQ ID NOs: 7, 8, and 25) and No. 33 peptide (SEQ ID NOs: 9, 10, 11, and 26) by ELISA. A and B show data from the serum of different *H. suis*-infected subjects, respectively. C, D and E show data from the serum of different *H. pylori*-infected subjects, respectively. F shows data from the serum of a non-infected subject. The abscissa depicts SEQ ID NOs of the antigen peptides and dilution ratios of the serum. The ordinate depicts absorbance at 450 nm. A mean from the experiment conducted in duplicate is shown. All the peptides strongly reacted with the *H. suis*-infected serum and weakly reacted with the *H. pylori*-infected or non-infected serum.

[Figure 9] Figure 9 is a graph showing results of measuring the binding titers of antibodies in the serum of 8 subjects (three *H.* suis-infected subjects, three *H.* pylori-infected subjects, and two non-infected subjects) against whole cells of an *H. pylori* TN2GF4 strain by ELISA. A and B show results obtained from serum diluted 600-fold and 1800-fold, respectively. The ordinate depicts absorbance at 450 nm. A mean from the experiment conducted in duplicate is shown. Only the *H. pylori*-infected serum strongly reacted with the bacterial cells of *H. pylori.*

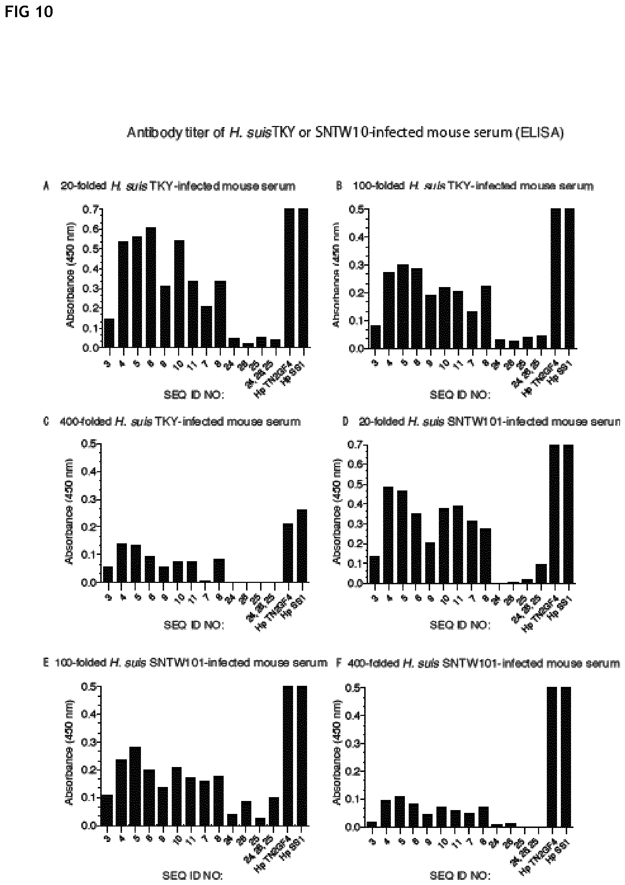

[Figure 10] Figure 10 is a graph showing results of measuring the binding titers of the serum of mice infected with an *H. suis* TKY or *H. suis* SNTW101 strain against No. 11 peptide (SEQ ID NOs: 3, 4, 5, 6, and 24), No. 19 peptide (SEQ ID NOs: 7, 8, and 25), No. 33 peptide (SEQ ID NOs: 9, 10, 11, and 26) and whole cells of *H. pylori* TN2GF4 and SS1 strains by ELISA. The abscissa depicts the types of the peptides or the bacterial cells used in ELISA. A, B and C show data from the serum of different *H. suis* TKY strain-infected mice, respectively. D, E and F show data from the serum of different *H. suis* SNTW101 strain-infected mice, respectively. A and D show results obtained from serum diluted 20-fold. B and E show results obtained from serum diluted 100-fold. C and F show results obtained from serum diluted 400-fold. The abscissa depicts SEQ ID NOs of the antigen peptides and dilution ratios of the serum. The ordinate depicts absorbance at 450 nm. A mean from the experiment conducted in duplicate is shown. The *H. suis*-infected mouse serum strongly reacted with the *H. pylori* whole cells, but also strongly reacted with all the peptides of Nos. 11, 19, and 33.

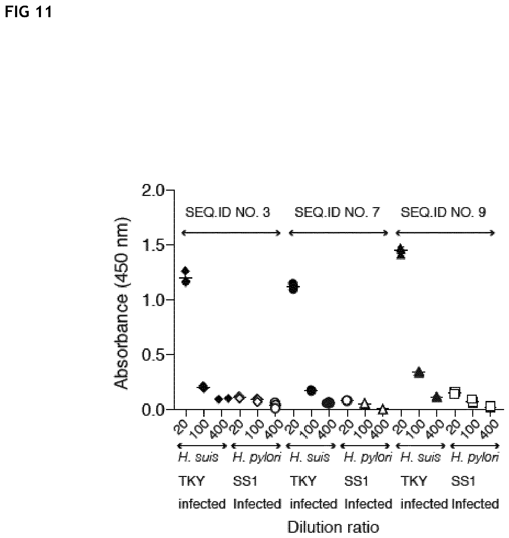

[Figure 11] Figure 11 is a graph showing results of measuring the serum of mice infected with an *H. suis* TKY or *H. pylori* SS1 strain by sandwich ELISA. The abscissa depicts the types of peptides or bacterial cells used in sandwich ELISA. The peptides and the bacterial cells used are as follows: SEQ ID NO: 3 (peptide No. 11 (SNTW101; HS1; HS5), SEQ ID NO: 7 (peptide No. 19 (SNTW101; HS1; HS5; SH8), and SEQ ID NO: 9 (peptide No. 33 (SNTW101; HS1; HS5). The ordinate depicts absorbance at 450 nm. A mean from the experiment conducted in triplicate and standard deviation are shown. Only the *H. suis*-infected mouse serum strongly reacted with all the peptides of Nos. 11, 19, and 33.

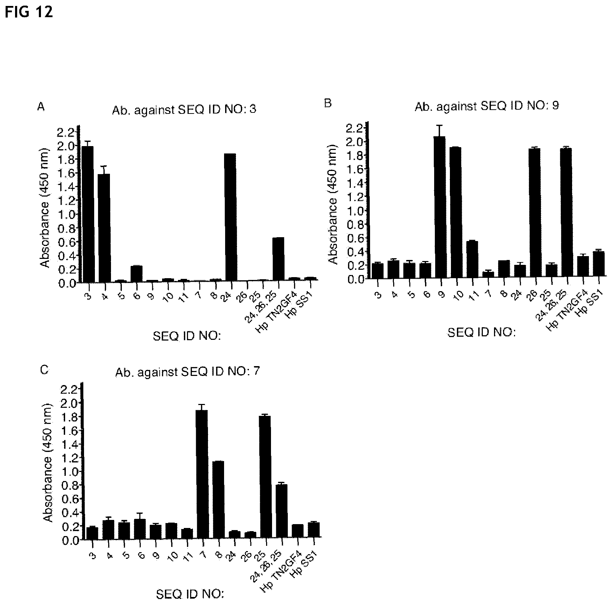

[Figure 12] Figure 12 is a graph showing results of ELISA about the binding of each rabbit antibody to various antigens (HsvA-derived peptides or *H. pylori* whole cells). The abscissa depicts various antigens (represented by the same numbers as in Figure 10) immobilized on a solid phase. The ordinate depicts absorbance at 450 nm. A shows the results obtained using a rabbit antibody against No. 11 peptide (SEQ ID NO: 3) prepared in Example 2. B shows the results obtained using a rabbit antibody against No. 33 peptide (SEQ ID NO: 9) prepared in Example 2. C shows the results obtained using a rabbit antibody against No. 19 peptide (SEQ ID NO: 7) prepared in Example 2. A mean from the experiment conducted in triplicate and standard deviation are shown. The antibody against each peptide strongly reacted with the peptide, but weakly reacted with the *H. pylori* whole cells.

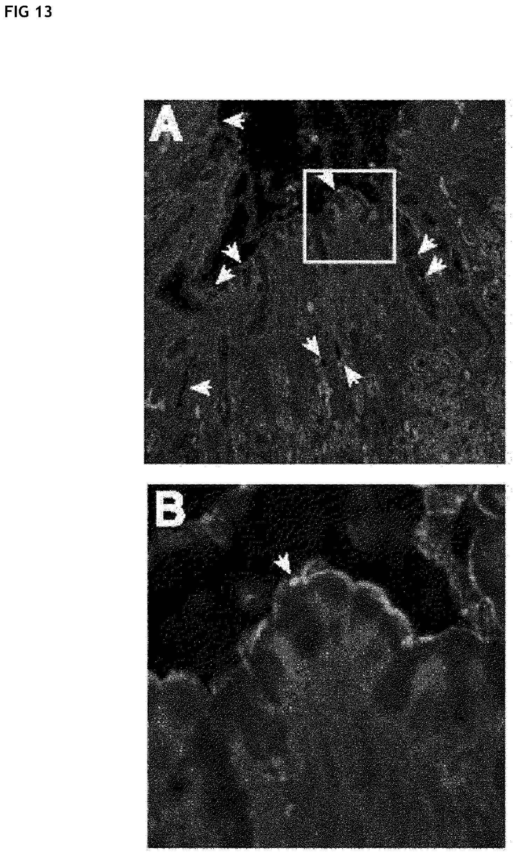

[Figure 13] Figure 13 shows immunohistochemical photographs obtained using a polyclonal antibody obtained by immunizing a rabbit with No. 11 peptide. A gastric tissue section of an *H. suis*-infected patient was reacted with a rabbit antibody against No. 11 peptide adjusted to 2 $\mu$g/mL with phosphate-buffered saline and then reacted with Alexa-Flour 488 anti-rabbit IgG diluted 400-fold with phosphate-buffered saline, and photographed under Leica confocal laser fluorescence microscope (TCS-SP5). Alexa-Flour 568 diluted 400-fold with phosphate-buffered saline was used in counterstaining. A is a photograph taken at a 200$\times$ magnification. B is a photograph of the boxed portion of A magnified 760 times. The arrows depict bacterial cells of *H. suis.*

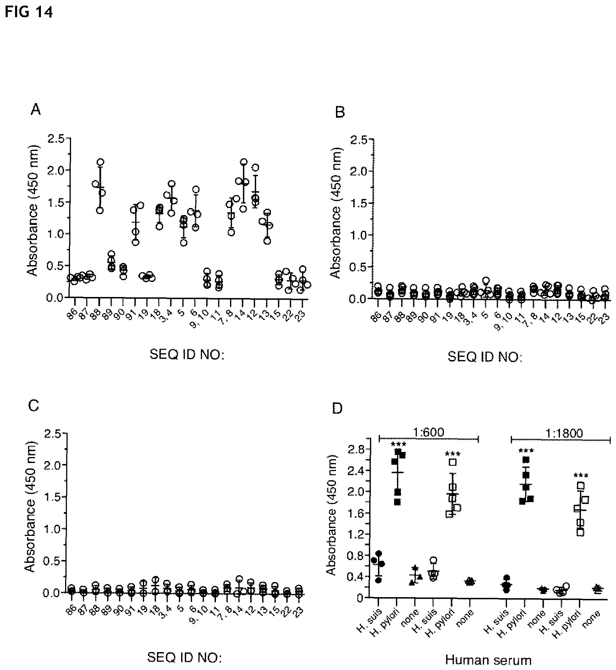

[Figure 14] Figure 14 is a graph showing results of measuring the binding activity of antibodies in serum collected from patients infected with *H. suis* or *H. pylori* against each peptide or bacterial cells by ELISA. The values are indicated by mean $\pm$ standard deviation (SD). A shows the binding of antibodies in serum collected from *H. suis*-infected patients (n = 4) to each peptide. The ordinate depicts absorbance at 450 nm. The abscissa depicts SEQ ID NOs of peptides used in immobilization on a solid phase. B shows the binding of antibodies in serum collected from *H. pylori*-infected patients (n = 5) to each peptide. The ordinate depicts absorbance at 450 nm. The abscissa depicts SEQ ID NOs of peptides used in immobilization on a solid phase. C shows the binding of antibodies in serum obtained from non-infected patients (n = 3) to each peptide. The ordinate depicts absorbance at 450 nm. The abscissa depicts SEQ ID NOs of peptides used in immobilization on a solid phase. D shows the binding of antibodies contained in *H. suis*-infected patient-derived serum, *H. pylori*-infected patient-derived serum, and non-infected patient-derived serum diluted 600-fold or 1800-fold to *H. pylori* TN2GF4 (filled marks) or SS1 (open marks). The ordinate depicts absorbance at 450 nm. The abscissa depicts the attributions of the patients from which the serum used was derived (H. suis: *H. suis*-infected patient, H. pylori: *H. pylori*-infected patient, none: non-infected patient). 1:600 shown in the upper part represents that the patient serum was diluted 600-fold and used as a sample. 1:1800 represents that the patient serum was diluted 1800-fold and used as a sample. In both "1:600" and "1:1800", the results indicated by filled marks on the left side depict binding to *H. pylori* TN2GF4, and the results indicated by open marks on the right side depict binding to *H. pylori* SS1. ***P < 0.0001 *(H. suis*-infected serum vs. *H.* pylori-infected serum or non-infected serum).

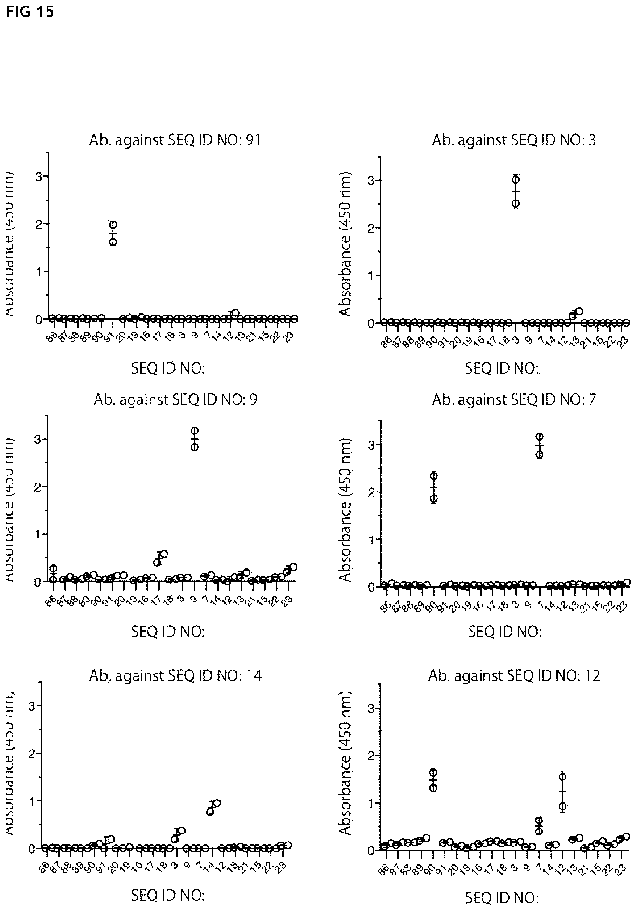

[Figure 15] Figure 15 is a graph showing results of examining the specificity of an antibody against each of peptides of Nos. 61, 11, 33, 19, 10, and 16 for the corresponding antigen peptide. The upper part of each graph describes the type of the peptide as an antigen with which the antibody measured in this graph was obtained (e.g., "Ab. against SEQ ID NO: 91" means the antibody obtained with the peptide of SEQ ID NO: 91 as an antigen). The ordinate depicts absorbance at 450 nm. The abscissa depicts SEQ ID NOs of the peptides used in immobilization on a solid phase. The values are indicated by mean ± standard deviation (SD).

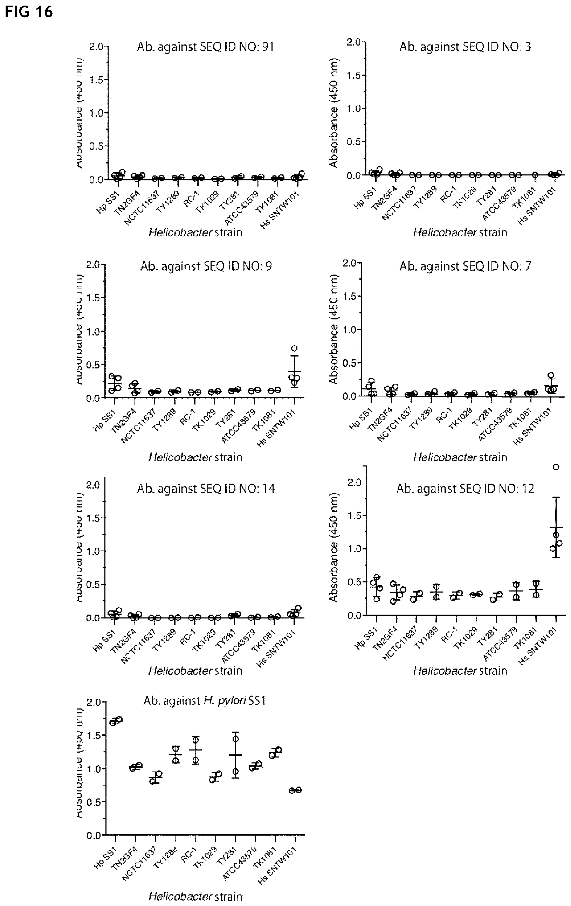

[Figure 16] Figure 16 is a graph showing results of measuring the binding activity of anti-peptide rabbit polyclonal antibodies obtained in Example 2 against bacterial cells of each *Helicobacter* species by ELISA. The upper part of each graph describes the type of the peptide as an antigen with which the antibody measured in this graph was obtained (e.g., "Ab. against SEQ ID NO: 91" means the antibody obtained with the peptide of SEQ ID NO: 91 as an antigen). The ordinate depicts absorbance at 450 nm. The abscissa depicts *Helicobacter* strains used in immobilization on a solid phase. The values are indicated by mean ± standard deviation (SD).

[Figure 17] Figure 17 shows photographs showing results of immunohistochemically staining (A) an *H. suis* SNTW101-infected mouse gastric section, (B) an enlarged view of the boxed portion of (A), and (C) a non-infected mouse gastric section using an anti-No. 16 peptide (SEQ ID NO: 12) antibody. The arrows depict stained bacterial cells.

## Description of Embodiments

In one aspect, the present invention relates to a method for determining the presence of *H. suis* in a sample, comprising detecting hsvA gene or a portion thereof, HsvA protein or a portion thereof, or an antibody against the HsvA protein in the sample. Particularly, the present invention relates to a method for determining infection of a subject with *H. suis,* comprising detecting hsvA gene or a portion thereof, HsvA protein or a portion thereof, or an antibody against the HsvA protein in a sample derived from the subject. In the present method, the sample or the subject in which the hsvA gene or a portion thereof, the HsvA protein or a portion thereof, or the antibody against the HsvA protein has been detected is determined to have *H. suis* or to be infected with *H. suis.*

When the method of the present invention is performed by measuring the binding of a polynucleotide, a protein or a portion thereof, or an antibody or a portion thereof in the sample to a test reagent, whether or not to "have been detected" in the determination does not require the absolute presence or absence of detection and may be determined by comparison with other samples. Specifically, the presence or absence of detection may be determined from measurement values, rather than being based on ± detection results. Specifically, in the method of the present invention, the "detecting" step is interchangeable, if necessary, with "measuring", and whether or not to "have been detected" may be determined by comparison with a negative control based on a measurement value of an intended substance. For example, even when the substance of interest is detected in a negative control, i.e., a sample containing no *H. suis* or a sample derived from a subject evidently having no *H. suis* infection, the substance detected in a very small amount is regarded as "having not been detected" in the method of the present invention as long as the measurement value of the subject is equivalent to that of the negative control. Thus, the sample or the subject is determined to have no *H. suis* or to be not infected with *H. suis.* On the other hand, when the measurement value of the subject is higher than that of the negative control, the substance is regarded as "having been detected". Thus, the sample or the subject is determined to have *H. suis* or to be infected with *H. suis.* Accordingly, in the method of the present invention, slight measurement value found in a negative control is acceptable by the present invention.

Preferably, the method for determining the presence of *H. suis* or the method for determining infection of a subject with *H. suis* according to the present invention employs at least one member selected from the group consisting of (i) a nucleic acid molecule capable of specifically binding to hsvA gene, (ii) an HsvA antigen peptide, and (iii) an anti-HsvA antibody or an immunoreactive fragment thereof in a sample derived from the subject.

(Method using nucleic acid molecule having hsvA gene-specific nucleotide sequence)

Whether or not a subject is infected with *H. suis* can be determined as the presence or absence of the infection by detecting hsvA gene present in a sample of the subject. The detection of *H. suis* using the nucleic acid molecule capable of specifically binding to hsvA gene can be performed by a method using hybridization; a method using PCR; or a method known as Invader(R) assay (see, for example, Kwiatkowski, R.W. et al., Mol. Diagn. (1999) 4: 353-364).

Examples of the method using hybridization can include methods such as Southern hybridization, Northern hybridization, dot hybridization, fluorescence in situ hybridization (FISH), DNA microarrays, and ASO. The method for determining the presence of *H. suis* in a sample according to the present invention may comprise, for example:

(a) contacting the sample with at least one nucleic acid molecule capable of specifically binding to hsvA gene;
(b) detecting binding of a polynucleotide in the sample to the nucleic acid molecule capable of specifically binding to hsvA gene; and
(c) determining the sample in which the binding has been detected, as a sample having *H. suis,* and determining the sample in which the binding has not been detected, as a sample having no *H. suis,* and thereby determining the presence of *H. suis.*

The method of the present invention may be, for example, a method for determining infection of a subject with *H. suis,* comprising:

(a) contacting a sample derived from the subject with at least one nucleic acid molecule capable of specifically binding to hsvA gene;
(b) detecting binding of a polynucleotide in the sample to the nucleic acid molecule capable of specifically binding to hsvA gene; and
(c) determining the subject from which the sample in which the binding has been detected is derived, to be infected with *H. suis,* and/or determining the sample in which the binding has not been measured or detected, as a sample having no *H. suis.*

The binding of a polynucleotide in the sample to the nucleic acid molecule capable of specifically binding to hsvA gene can be detected, for example, by labeling in advance the nucleic acid molecule capable of specifically binding to hsvA gene, contacting a sample derived from the subject with the nucleic acid molecule capable of specifically binding to hsvA gene, then performing washing, and detecting or measuring the label on the remaining nucleic acid molecule capable of specifically binding to hsvA gene. In this case, preferably, the sample derived from the subject is immobilized on a solid phase.

Examples of the method using PCR can include methods such as ARMS (amplification refractory mutation system), RT-PCR (reverse transcription-PCR), and nested PCR. The method for determining the presence of *H. suis* according to the present invention may comprise, for example, the step of:

(a) amplifying a portion of hsvA gene in a sample using a nucleic acid molecule capable of specifically binding to hsvA gene as a primer;
(b) detecting the amplified nucleic acid molecule; and
(c) determining the sample in which the amplified nucleic acid molecule has been detected, as a sample having *H. suis*, and determining the sample in which the amplified nucleic acid molecule has not been detected, as a sample having no *H. suis,* and thereby determining the presence of *H. suis.*

The present invention also relates to a method for determining *H. suis* infection, comprising:

(a) amplifying a portion of hsvA gene in a sample derived from a subject using a nucleic acid molecule capable of specifically binding to hsvA gene as a primer;
(b) detecting the amplified nucleic acid molecule; and
(c) determining the subject from which the sample in which the amplified nucleic acid molecule has been detected is derived, to be infected with *H. suis,* and determining the subject from which the sample in which the amplified nucleic acid molecule has not been detected is derived, to be not infected with *H. suis.*

In another aspect, the present invention relates to a method for determining *H. suis* infection, comprising:

(a) amplifying a portion of hsvA gene in a sample derived from a subject using a hsvA gene-specific nucleic acid molecule or the like as a primer;
(b) measuring a level of the amplified nucleic acid molecule; and
(c) determining the subject to be infected with *H. suis* when the measured level of the nucleic acid molecule is higher than that of the nucleic acid molecule measured for a negative control by a similar method, and determining the subject to be not infected with *H. suis* when the measured level of the nucleic acid molecule is equivalent to or lower than that of the nucleic acid molecule measured for a negative control by a similar method.

The amplification of a portion of the hsvA gene in the sample derived from the subject can be carried out through PCR reaction or the like using the sample derived from the subject as a template.

The amplified nucleic acid can be determined by dot blot hybridization, surface plasmon resonance (SPR) method, PCR-RFLP, in situ RT-PCR, PCR-SSO (sequence specific oligonucleotide), PCR-SSP, AMPFLP (amplifiable fragment length polymorphism), MVR-PCR, or PCR-SSCP (single strand conformation polymorphism).

The method for determining the presence of *H. suis* using the method known as Invader(R) assay may comprise, for example, the steps of:

(a) contacting a specimen with the following nucleic acids (i) and (ii) to form a triplex of DNA complementary to an allele probe:

(i) an allele-specific probe having an hsvA gene-specific nucleotide sequence or a sequence complementary to the sequence and a sequence complementary to a portion of a quenching probe (flap), and/or an allele-specific probe having an hsvA-specific nucleotide sequence or a sequence complementary to the sequence and a sequence complementary to a portion of a quenching probe (flap), and
(ii) an Invader probe having the hsvA gene-specific nucleotide sequence;

(b) contacting triplex-specific DNase with the nucleic acid specimen obtained by the step (a) to liberate the flap from the nucleic acid triplex;
(c) contacting the liberated flap with fluorescently labeled universal probes each having a sequence complementary to the flap and the quenching probe;
(d) contacting triplex-specific DNase with the nucleic acid specimen obtained by the step (c) to liberate the fluorescent label so that fluorescence is emitted;
(e) detecting the emitted fluorescence and thereby detecting the hsvA gene, wherein the sample in which the fluorescence emission has been detected is determined as a sample having *H. suis,* and the sample in which the fluorescence emission has not been detected is determined as a sample having no *H. suis,* thereby determining the presence of *H. suis.*

(Method using detection of HsvA protein-specific antibody)

Whether or not a subject is infected with *H. suis* can be determined by confirming the presence of an antibody against an *H. suis*-derived protein (particularly, HsvA protein) produced by the own immune system of the subject. The detection of *H. suis* using the HsvA antigen peptide, or the anti-HsvA antibody or the immunoreactive fragment thereof can be performed by, for example, labeled immunoassay such as enzyme immunoassay (EIA), simple EIA, enzyme-linked immunosorbent assay (ELISA), radioimmunoassay (RIA), or fluorescence immunoassay (FIA); immunoblotting such as Western blotting; immunochromatography such as metal colloid agglutination method; chromatography such as ionexchange chromatography or affinity chromatography; turbidimetric immunoassay (TIA); nanofluidic proteomic immunoassay (NIA); colorimetric method; latex immunoassay (LIA); counting immunoassay (CIA); chemiluminescent (enzyme) immunoassay (CLIA and CLEIA); precipitation reaction method; surface plasmon resonance (SPR) method; resonant mirror detector (RMD) method; or comparison interferometry.

In one aspect, the present invention relates to a method for determining *H. suis* infection, comprising:

(a) contacting a sample derived from a subject with the HsvA antigen of the present invention;
(b) detecting an antibody bound with the peptide, in the blood sample; and
(c) determining the subject in which the antibody bound with the peptide has been detected, to be infected with *H. suis,* and determining the subject in which the antibody bound with the peptide has not been detected, to be not infected with *H. suis.*

In another aspect, the present invention relates to a method for determining *H. suis* infection, comprising:

(a) contacting a sample derived from a subject with the HsvA antigen peptide of the present invention;
(b) measuring a level of an antibody bound with the peptide, in the blood sample; and
(c) determining the subject to be infected with *H. suis* when the measured level of the antibody is higher than that of the antibody measured for a negative control by a similar method, and determining the subject to be not infected with *H. suis* when the measured level of the antibody is equivalent to or lower than that of the antibody measured for a negative control by a similar method.

Whether or not the antibody in the sample derived from the subject is bound with the peptide can be confirmed, for example, by contacting the sample derived from the subject with the HsvA antigen peptide of the present invention, then washing the reaction solution to remove an unbound antibody, contacting a labeled anti-Ig antibody therewith so that a subject-derived antibody bound with the HsvA antigen peptide binds to the anti-Ig antibody, washing the reaction solution to remove an unbound antibody, then detecting the label on the anti-Ig antibody, and determining binding between the peptide and the antibody in the sample derived from the subject when the label on the anti-Ig antibody is detected. The binding level between the peptide and the antibody in the sample derived from the subject may be measured by measuring the label level of the anti-Ig antibody. In using the binding level, the amount of the antibody can be calculated from a measurement value by preparing a calibration curve using a standard solution having an abundance known in advance. Alternatively, the binding between the peptide and the antibody in the sample derived from the subject may be detected or measured using a surface plasmon resonance sensor.

(Diagnosis of *H. suis* infection using detection of HsvA antigen peptide)

Whether or not a subject is infected with *H. suis* can be determined by confirming the presence of an *H. suis*-derived protein (particularly, HsvA protein) in a sample derived from the subject. The detection of *H. suis* using the HsvA protein can be performed by a method similar to the aforementioned method using the detection of the HsvA protein-specific antibody.

The method for determining the presence of *H. suis* of the present invention may comprise, for example:

(a) contacting a sample with a labeled anti-HsvA antibody or immunoreactive fragment thereof;
(b) detecting the label on a bound antibody; and
(c) determining the sample in which the label has been detected, as a sample having *H. suis,* and determining the sample in which the binding has not been detected, as a sample having no *H. suis,* and thereby determining the presence of *H. suis.*

The method for determining infection of a subject with *H. suis* of the present invention may comprise, for example:

(a) contacting a sample derived from the subject with a labeled anti-HsvA antibody or immunoreactive fragment thereof;
(b) detecting the label on a bound antibody; and
(c) determining the subject from which the sample in which the label has been detected is derived, to be infected with *H. suis,* and/or determining the subject from which the sample in which the label has not been detected is derived, to be not infected with *H. suis.*

Alternatively, the method for determining the presence of *H. suis* of the present invention may comprise, for example:

(a) contacting a sample with an anti-HsvA antibody or an immunoreactive fragment thereof;
(b) contacting the sample obtained by the step (a) with a labeled anti-HsvA antibody or immunoreactive fragment thereof which is different from said anti-HsvA antibody or said immunoreactive fragment thereof;
(c) measuring or detecting the label on a bound antibody; and
(d) determining the sample in which the binding has been measured or detected, as a sample having *H. suis,* and determining the sample in which the binding has not been measured or detected, as a sample having no *H. suis,* and thereby determining the presence of *H. suis.*

The method for determining infection of a subject with *H. suis* of the present invention may comprise, for example:

(a) contacting a sample derived from the subject with an anti-HsvA antibody or an immunoreactive fragment thereof;
(b) contacting the sample obtained by the step (a) with a labeled anti-HsvA antibody or immunoreactive fragment thereof which is different from said anti-HsvA antibody or said immunoreactive fragment thereof;
(c) measuring or detecting the label on a bound antibody; and
(d) determining the subject from which the sample in which the binding has been measured or detected is derived, to be infected with *H. suis,* and/or determining the subject from which the sample in which the binding has not been measured or detected is derived, to be not infected with *H. suis.*

The labeling of the antibody or the fragment thereof can be performed by a general method in the art. For example, in fluorescently labeling a protein or a peptide, the protein or the peptide is washed with a phosphate buffer solution. Then, a dye prepared with DMSO, a buffer solution, or the like is added thereto and mixed. Then, the mixture can be left standing at room temperature for 10 minutes for binding. Alternatively, the labeling may be performed using a commercially available labeling kit such as a biotin labeling kit (Biotin Labeling Kit-NH2 or Biotin Labeling Kit-SH; Dojindo Laboratories), an alkaline phosphatase labeling kit (Alkaline Phosphatase Labeling Kit-NH2 or Alkaline Phosphatase Labeling Kit-SH; Dojindo Laboratories), a peroxidase labeling kit (Peroxidase Labeling Kit-NH2 or Peroxidase Labeling Kit-NH2; Dojindo Laboratories), a phycobiliprotein labeling kit (Allophycocyanin Labeling Kit-NH2, Allophycocyanin Labeling Kit-SH, B-Phycoerythrin Labeling Kit-NH2, B-Phycoerythrin Labeling Kit-SH, R-Phycoerythrin Labeling Kit-NH2, or R-Phycoerythrin Labeling Kit-SH; Dojindo Laboratories), a fluorescent labeling kit (Fluorescein Labeling Kit-NH2, HiLyte Fluor(R) 555 Labeling Kit-NH2, or HiLyte Fluor(R) 647 Labeling Kit-NH2; Dojindo Laboratories), DyLight 547, DyLight 647 (Techno Chemical Corp.), Zenon(R) Alexa Fluor(R) antibody labeling kit or Qdot(R) antibody labeling kit (Invitrogen Corp.), EZ-Label Protein Labeling Kit (Funakoshi Co., Ltd.). The labeled antibody or fragment thereof can be appropriately detected using an instrument suitable for the label.

*H. suis* infects a wide range of mammals. Therefore, the subject in the method for determining the presence of *H. suis* and the method for determining infection with *H. suis* described herein can be a mammal such as a human, a monkey, a pig, a cat, a dog, a rabbit, or sheep and is preferably a human, more preferably a human patient with gastritis, chronic gastritis, nodular gastritis, gastric MALT lymphoma, diffuse large B-cell lymphoma, stomach cancer, gastric or duodenal ulcer, idiopathic thrombocytopenic purpura, or functional dyspepsia. The method of the present invention can be performed qualitatively, quantitatively or semi-quantitatively.

For example, a tissue specimen collected from a subject by biopsy or a liquid collected from a subject can be used as the sample in the method for determining the presence of *H. suis* described herein. The sample is not particularly limited as long as the sample can be targeted by the method of the present invention. Examples thereof can include tissues, blood, plasma, serum, lymph, urine, feces, serous fluid, spinal fluid, joint fluid, aqueous humor, tears, saliva and fractionated or treated products thereof. When a nucleic acid is to be detected in the method for determining the presence of *H. suis* described herein, the sample is preferably a tissue (particularly, a gastric tissue (gastric biopsy sample)) or feces. When an antibody is to be detected in the method for determining the presence of *H. suis* described herein, the sample is preferably blood, plasma, serum, lymph, or urine.

The peptide, the nucleic acid molecule, and the antibody or the immunoreactive fragment thereof of the present invention can be appropriately prepared by methods well known to those skilled in the art with reference to the disclosure herein. The composition and the kit of the present invention can be appropriately produced by methods well known in the technical field.

The nucleic acid molecule and the antibody or the immunoreactive fragment thereof of the present invention specifically binds to *H. suis.* Therefore, for example, siRNA, antisense DNA or RNA, a neutralizing antibody or an immunoreactive fragment thereof, or a non-neutralizing antibody can be selected and thereby used as a pharmaceutical composition for removing *H. suis* from the body of a subject infected with *H. suis,* or for defending against *H. suis* infection. For example, the nucleic acid molecule and the antibody or the immunoreactive fragment thereof of the present invention can be purified, if necessary, then formulated according to a routine method, and thereby used as a pharmaceutical composition for treatment or prevention of a disease or disorder involving *H. suis* infection contributing to its development or exacerbation. The present invention also includes use of the nucleic acid molecule and the antibody or the immunoreactive fragment thereof of the present invention for producing an *H. suis* removing agent, or a therapeutic drug or a prophylactic drug for a disease or disorder involving *H. suis* infection contributing to its development or exacerbation. Alternatively, the present invention includes use of the nucleic acid molecule and the antibody or the immunoreactive fragment thereof of the present invention for removal of an *H. suis,* or a treatment or prevention of a disease or disorder involving *H. suis* infection contributing to its development or exacerbation. The present invention further relates to a method for removing *H. suis,* or a method for treating or preventing a disease or disorder involving *H. suis* infection contributing to its development or exacerbation, comprising administering the antibody of the present invention or the immunoreactive fragment thereof. The disease or the disorder involving *H. suis* infection contributing to its development or exacerbation herein includes gastritis, chronic gastritis, nodular gastritis, gastric MALT lymphoma, diffuse large B-cell lymphoma, stomach cancer, gastric or duodenal ulcer, idiopathic thrombocytopenic purpura, and functional dyspepsia. In the description above, "removal of *H. suis"* means that *H. suis* is removed from the body of a subject infected with *H. suis.* For use as a pharmaceutical composition, preferably, the antibody is humanized or a complete human antibody.

The pharmaceutical composition of the present invention may adopt any oral or parenteral preparation as long as the preparation can be administered to a patient. Examples of the composition for parenteral administration can include eye drops, injections, nasal drops, suppositories, patches, and ointments. An injection is preferred. Examples of the dosage form of the pharmaceutical composition of the present invention can include liquid preparations and freeze-dried preparations. For use as an injection, the pharmaceutical composition of the present invention can be supplemented, if necessary, with additives including solubilizers such as propylene glycol and ethylenediamine, buffers such as phosphate, tonicity agents such as sodium chloride and glycerin, stabilizers such as sulfite, preservatives such as phenol, and soothing agents such as lidocaine (see "Japanese Pharmaceutical Excipients Directory" Yakuji Nippo, Ltd. and "Handbook of Pharmaceutical Excipients Fifth Edition" APhA Publications). For use of the pharmaceutical composition of the present invention as an injection, examples of the storage container can include ampules, vials, prefilled syringes, pen-shaped cartridges for syringes, and bags for intravenous drips.

The pharmaceutical composition (therapeutic drug or prophylactic drug) of the present invention can be used as, for example, an injection which encompasses dosage forms such as intravenous injections, subcutaneous injections, intracutaneous injections, intramuscular injections, intravitreal injections, and drip injections. Such an injection can be prepared according to a known method, for example, by dissolving, suspending or emulsifying the antibody, etc. in a sterile aqueous or oily liquid usually used in injections. For example, physiological saline or an isotonic liquid containing glucose, sucrose, mannitol, or other pharmaceutical adjuvants can be used as the injectable aqueous liquid and can be used in combination with an appropriate solubilizer, for example, an alcohol (e.g., ethanol), a polyalcohol (e.g., propylene glycol and polyethylene glycol), or a nonionic surfactant [e.g., polysorbate 80, polysorbate 20, and HCO-50 (polyoxyethylene (50 mol) adduct of hydrogenated castor oil)]. For example, sesame oil or soybean oil can be used as the oily liquid and can be used in combination with a solubilizer such as benzyl benzoate or benzyl alcohol. The prepared injection liquid is filled into an appropriate ampule, vial, or syringe. Alternatively, an appropriate excipient may be added to the antibody of the present invention or the immunoreactive fragment thereof to prepare a freeze-dried preparation, which can be dissolved in injectable water, physiological saline, or the like in use and formulated as an injection liquid. In general, the oral administration of a protein such as an antibody is difficult because the protein is degraded by a digestive organ. However, the oral administration may be possible by making the best use of an antibody fragment or a modified antibody fragment and a dosage form. Examples of the preparation for oral administration can include capsules, tablets, syrups, and granules.

The pharmaceutical composition of the present invention is suitably prepared into a dosage form in a dosage unit adaptable to the amount of the active component administered. Examples of such a dosage form in a dosage unit include injections (ampules, vials, and prefilled syringes). Usually, 5 to 500 mg, 5 to 100 mg, or 10 to 250 mg of the antibody of the present invention or the immunoreactive fragment thereof may be contained per dosage unit of the dosage form.

The pharmaceutical composition (therapeutic drug or prophylactic drug) of the present invention may be administered locally or systemically. The administration method is not particularly limited, and the pharmaceutical composition is administered parenterally or orally as mentioned above. Examples of the parenteral administration route include intraocular administration, subcutaneous administration, intraperitoneal administration, injection or intravenous drips into blood (intravenous or intraarterial) or spinal fluid. Administration into blood is preferred. The pharmaceutical composition (therapeutic drug or prophylactic drug) of the present invention may be temporarily administered or may be continuously or intermittently administered. The administration may be, for example, continuous administration for 1 minute to 2 weeks.

The amount of the pharmaceutical composition of the present invention administered is not particularly limited as long as the amount produces the desired therapeutic effect or prophylactic effect. The amount of the pharmaceutical composition administered can be appropriately determined depending on symptoms, sex, age, etc. For example, the amount of the pharmaceutical composition of the present invention administered can be determined using, as an index, a therapeutic effect or a prophylactic effect on the disease or the disorder involving cAMP contributing to its development or exacerbation. For use in, for example, prevention and/or treatment of a patient with the disease or the disorder involving cAMP contributing to its development or exacerbation, the pharmaceutical composition of the present invention is conveniently administered approximately one to ten times a month, preferably approximately one to five times a month, by intravenous injection at usually approximately 0.01 to 20 mg/kg body weight, preferably approximately 0.1 to 10 mg/kg body weight, more preferably approximately 0.1 to 5 mg/kg body weight, in terms of a single dose of the active ingredient. For other parenteral administration and oral administration approaches, an amount conforming thereto can be administered. Particularly, for severe symptoms, the amount or the number of doses may be increased according to the symptoms.

Examples

Hereinafter, the present invention will be specifically described with reference to Examples. However, the present invention is not limited thereby. All literatures cited herein are incorporated herein by reference in their entirety.

(Example 1) Detection by PCR

(1) Preparation of DNA

*(1)-1. H. pylori*

Eleven strains (SS1, TN2GF4, RC-1, ATCC43579, NCTC11637, TK1029, TK1081, TY1289, and TY281) were used as *H. pylori.* A bacterial liquid preserved at -80°C was applied to Nissui Plate/Helicobacter Agar medium (Nissui Pharmaceutical Co., Ltd., Tokyo, Japan) and cultured at 37°C for 3 days under microaerophilic conditions (5% $O_2$, 10% $CO_2$, 85% $N_2$, humidity of 100%) . The resulting colonies were suspended in sterile distilled water, treated at 95°C for 5 minutes, and then centrifuged. The supernatant was used as crude purified chromosomal DNA. The crude purified chromosomal DNA was extracted with an equal amount of phenol:chloroform:isoamyl alcohol (25:24:1), then purified by ethanol precipitation, and dissolved in a small amount of sterile distilled water. The DNA concentration of the final product was determined according to the following expression by the measurement of absorbance at 260 nm (A260).

DNA concentration (μg/mL) = A260 × 50 (optical path length: 10 mm)

(1)-2. *H. suis*

Two strains (TKY and SNTW101) were used as *H. suis.* A female C57BL/6 mouse (Charles River Laboratories Japan, Inc., Yokohama, Japan) infected with *H. suis* was anatomized, and the greater curvature of the stomach was cut open. The contents were washed with PBS, and the mucosa was scraped using a glass slide. The mucosa was rubbed between opaque glass portions of two glass slides to prepare a suspension. DNA of the mouse gastric mucosa containing the bacterium was prepared using DNeasy Blood & Tissue kit (Qiagen N.V., Hilden, Germany). The DNA concentration of the final product was determined according to the following expression by the measurement of absorbance at 260 nm (A260).

DNA concentration (μg/mL) = A260 × 50 (optical path length: 10 mm)

(1)-3. *H. felis, H. mustelae, H. heilmannii s.s. (H. heilmannii sensu stricto), H. baculiformis, H. bizzozeronii, H. cynogastricus,* and *H. salmonis*

A bacterial liquid preserved at -80°C was suspended in sterile distilled water, treated at 95°C for 5 minutes, and then centrifuged. The supernatant was used as crude purified chromosomal DNA. The crude purified chromosomal DNA was extracted with an equal amount of phenol:chloroform:isoamyl alcohol (25:24:1), then purified by ethanol precipitation, and dissolved in a small amount of sterile distilled water.

(2) PCR

(2)-1. PCR

A reaction solution (25 μL in total) having the following composition was subjected to PCR reaction in duplicate using Dream Taq DNA Polymerase (Thermo Fisher Scientific Inc.): 2.5 μL of a 10 × buffer solution, 0.5 μL of 10 mM dNTP mix, 1 μL of a forward (FW) primer (5 μM), 1 μL of a reverse (RV) primer, 1 μL of template DNA (1 ng and 10 ng of each *H. pylori* (SS1, TN2GF4, RC-1, ATCC43579, NCTC11637, TK1029, TK1081, TY1289, and TY281 strains) DNA, and $10^{-3}$ ng, $10^{-2}$ ng, $10^{-1}$ ng, 1 ng, 10 ng, and 100 ng of each DNA prepared from the gastric biopsy of *H. suis* (SNTW101 and TKY strains)-infected mice), 0.25 μL of DNA polymerase, and 16.75 μL of sterile distilled water. The PCR reaction conditions was holding at 95°C for 1 minute, then 35 cycles of 95°C for 30 seconds, 55°C for 30 seconds and 72°C for 1 minute, and then keeping at 72°C for 5 minutes.

Primers were designed from regions having high identity among *H. suis* strains and lacking identity to other bacterial species by comparing the *H. suis* hsvA gene with an autotransporter protein similar to VacA present in *H. felis, H. bizzozeronii,* and *H. pylori.* The designed primers were as follows.

(Forward primer)

HSVANOFW-2: AGAAACGAGATTACAGGAAG (SEQ ID NO: 51)
HSVANOFW-3: AGGAGCAAGTTTTGTAGCAG (SEQ ID NO: 52)
HSVANOFW-5: AAAATGCGACTGATTGGATG (SEQ ID NO: 53)

(Reverse primer)

HSVANORV-1: ATCGAAATAAGCGAACCTCA (SEQ ID NO: 72)
HSVANORV-3: TTGAAAGCTTAGCTAAACGG (SEQ ID NO: 73)
HSVANORV4: TGGTATTGCTGGTTAAGAGG (SEQ ID NO: 74)

The following primers were used as primers amplifying the *H. pylori* vacA gene (Matsui H et al., Helicobacter. (2014) 19 (4): 260-71).

VAC3624F (forward): GAGCGAGCTATGGTTATGAC (SEQ ID NO: 84)

VAC4041R (reverse): CATTCCTAAATTGGAAGCGAA (SEQ ID NO: 85)

10 $\mu$L of each obtained amplification product was electrophoresed on 1.5% agarose gel and stained with ethidium bromide to confirm an amplified band.

(2)-2. Real-time PCR

A double quencher probe (PrimeTime(R) qPCR Probes) having the sequence given below was synthesized by outsourcing to Integrated DNA Technologies, Inc. (IDT). Since the primers FW5 and RW3 mentioned above have high identity among *H. suis* strains, new primers were designed from the same regions.

Probe: FAM/TGTACACAC/ZEN/CAAACAGATGAGCCGT (SEQ ID NO: 75)/3IABkFQ
Forward:
GATGGGCGCTTCTGGTTTA (SEQ ID NO: 47)
Reverse:
CTGGTAATGCATCATTAGAAGCAAA (SEQ ID NO: 65)

PCR reaction was performed under the following conditions using the probe (final concentration: 0.25 $\mu$M), the primers (final concentration: 0.5 $\mu$M), and PCR master mix: PrimeTime Gene Expression Master Mix (Integrated DNA Technologies, Inc. (IDT)) as well as QuantStudio (Applied Biosystems, Inc.) according to the IDT attached protocol.
95°C for 3 minutes, 1 cycle
95°C for 15 seconds - 60°C for 1 minute, 40 cycles

(3) Results

A calibration curve was prepared beforehand using plasmids into which a target region was cloned. As a result, favorable amplification efficiency and quantitativeness were exhibited from $10^2$ copies to $10^7$ copies (not shown). Results of PCR using *H. pylori* DNA and gastric biopsied tissue DNA of *H. suis*-infected mouse are shown in Figure 5. All the combinations of designed primers HSVANOFW-2/HSVANORV-4, HSVANOFW-3/HSVANORV-4, and HSVANOFW-5/HSVANORV-4 specifically amplified *H. suis* DNA and did not amplify *H. pylori* DNA. On the other hand, primers designed for *H. pylori* vacA amplified only *H. pylori* DNA and did not amplify *H. suis* DNA.

Results of quantitative PCR using gastric biopsied tissue DNA of an *H. suis*-infected mouse showed favorable amplification efficiency in amounts of DNA from 100 ng to $10^{-2}$ ng (not shown) . $6.9 \times 10^5$ copies per 10 ng of DNA from the *H. suis* SNTW101 strain, $3.7 \times 10^5$ copies per 10 ng of DNA from the *H. suis* TKY strain, $1.5 \times 10^3$ copies per 10 ng of DNA from the *H. suis* SH8 strain, and $4.2 \times 10^3$ copies per 10 ng of DNA from the *H. suis* SH10 strain were obtained. On the other hand, no amplification was observed in *H. pylori* DNA (SS1, TN2GF4, RC-1, ATCC43579, NCTC11637, TK1029, TK1081, TY1289, and TY281 strains). Likewise, *H. felis, H. mustelae, H. heilmannii s.s.* ASB1.4, *H. baculiformis, H. bizzozeronii, H. cynogastricus,* or *H. salmonis* chromosomal DNA used as a template was not amplified. No amplification was found for *H. pylori* (SS1, TN2GF4, NCTC11637, ATCC43579, RC-1, TK1029, TK1081, TY1289, and TY281 strains), *H. felis, H. mustelae, H. heilmannii sensu stricto (H. heilmannii s.s.* ASB1.4), *H. baculiformis, H. bizzozeronii, H. cynogastricus,* and *H. salmonis.* Only the *H. suis*-infected mouse-derived gastric mucosa DNA was amplified.

These results demonstrated that all the designed primer sets enable *H. suis* infection to be diagnosed by discrimination from *H. pylori, H. felis, H. mustelae, H. heilmannii s.s.* ASB1.4, *H. baculiformis, H. bizzozeronii, H. cynogastricus,* and *H. salmonis.*

(Example 2) Peptide synthesis and antibody preparation

*H. suis* was successfully detected specifically by PCR. Therefore, in consideration of the possibility that HsvA was expressed, a study was conducted on whether to be able to diagnose *H. suis* infection by targeting HsvA.

(1) Peptide synthesis

From the putative amino acid sequence of HsvA (SEQ ID NO: 2; Figure 2), presumably antigenic peptide sequences were selected and synthesized based on the specificity of the bacterial species and conservation among strains.

No. 11: EKKAVQQMENSNPD (SEQ ID NO: 3)
No. 11 (TKY): EKKAVEQMENSNPD (SEQ ID NO: 4)
No. 11 (SH8): EKDAVTSLKNSNSG (SEQ ID NO: 5)
No. 11 (SH10): EKDAVTSLENSNSG (SEQ ID NO: 6)
No. 19: NQGTLEFLSNDVSN (SEQ ID NO: 7)
No. 19 (TKY): NQGTLEFLSNDVST (SEQ ID NO: 8)
No. 33: LSNKLQGQLKSMGL (SEQ ID NO: 9)
No. 33 (TKY): LSNKLQDQLKSMGL (SEQ ID NO: 10)
No. 33 (SH10): FSDKLQNMLKSLNM (SEQ ID NO: 11)
No. 16: TNGQEVSASIDYNK (SEQ ID NO: 12)
No. 23: AKLSNFASNDALPD (SEQ ID NO: 13)
No. 10: PTTSSGASPDSSNP (SEQ ID NO: 14)
No. 21: GLGRDLFVHSMGDK (SEQ ID NO: 15)
No. 15: QIGKIKLSDVLSAS (SEQ ID NO: 16)
No. 34: YGAIDKELHFSGGK (SEQ ID NO: 17)
No. 20: NVDNILNMPSTTSG (SEQ ID NO: 18)
No. 22: GNLKGVYYPKSSTT (SEQ ID NO: 19)
No. 14: ITEKIQSGKLTITI (SEQ ID NO: 20)
No. 26: FHDFLVSLKGKKFA (SEQ ID NO: 21)
No. 31: TTGGEVRLFRSFYV (SEQ ID NO: 22)
No. 35: IGARFGLDYQDINI (SEQ ID NO: 23)
No. 8: KQLPQPKRSELKPK (SEQ ID NO: 86)
No. 31N: TNIKQYMQNNHRSQ (SEQ ID NO: 87)
No. 81: TLTLEGTETFAQNS (SEQ ID NO: 88)
No. 63: EAYAKNQGDIWSTI (SEQ ID NO: 89)
No. 73: VIGSKSSITLNSAN (SEQ ID NO: 90)
No. 61: ADIQSSQTTFANSV (SEQ ID NO: 91)

(2) Antibody preparation

Cysteine (C) was added to the amino terminus of each 14-mer peptide of No. 10, No. 11, No. 16, No. 19, No. 33, or No. 61 among the sequences selected above to synthesize peptides. A rabbit (New Zealand White) was immunized with using keyhole limpet hemocyanin (KLH) as a carrier to obtain an ELISA titer of 1:512,000 or more. The immunized rabbit serum was purified through an affinity column bound with Protein G. A recovered IgG fraction was used as an anti-peptide rabbit antibody (polyclonal antibody) in the following experiment.

(4) Preparation of *H. pylori* antigen for ELISA

*H. pylori* was shake-cultured at a temperature of 37°C and a humidity of 100% for 48 hours under microaerophilic conditions (5% $O_2$, 10% $CO_2$, 85% $N_2$) in brucella broth containing 10% fetal calf serum (FCS). Then, the culture solution was centrifuged to collect bacterial cells. The bacterial cells were washed three times with sterilized distilled water to remove lipopolysaccharide components. A small amount of the bacterial cells was suspended in sterilized distilled water.

(5) Quantification of protein

Proteins were quantified using Bio-Rad protein assay kit and a 96-well plate. Bovine serum albumin (BSA) was used as a standard protein in calibration curve preparation.

(Example 3) Measurement of anti-*H. suis* antibody titer in infected subject (human) using ELISA (enzyme-linked immunosorbent assay; enzyme immunoassay)

Each peptide dissolved in a 0.2 M carbonate-bicarbonate buffer solution (pH 9.4) or the *H. pylori* whole cells (4 μg/mL) prepared in Example 2(4) were added dropwise at 100 μL/well to 96-well NUNC-Immuno Plate #439454 and left overnight at 4°C. On the next day, the peptide solution was discarded, and the plate was washed three times with 200 μL/well of PBS-T (phosphate-buffered saline containing 0.05% (V/V) Tween 20, pH 7.4). Nacalai Tesque blocking solution (PBS-based, pH 7.2) diluted 5-fold with sterile distilled water was added dropwise at 200 μL/well and left at 37°C for 1 hour. The blocking solution was discarded, and the plate was washed three times with 200 μL/well of PBS-T (phosphate-buffered saline containing 0.05% (V/V) Tween 20, pH 7.4).

Serum was collected from each of subjects infected with *H. pylori* and subjects infected with *H. suis*. Also, serum was collected as a control from uninfected healthy persons (non-infected). Each human serum diluted using Nacalai Tesque blocking solution (PBS-based, pH 7.2) diluted 10-fold with sterile distilled water was added dropwise at 100 μL/well and left at 37°C for 1 hour. The serum solution was discarded, and the plate was washed three times with 200 μL/well of PBS-T (phosphate-buffered saline containing 0.05% (V/V) Tween 20, pH 7.4). A horseradish peroxidase-labeled secondary antibody (Goat anti-Human IgG, Jackson ImmunoResearch Inc.) diluted 100,000-fold using Nacalai Tesque blocking solution (PBS-based, pH 7.2) diluted 10-fold with sterile distilled water was added dropwise at 100 μL/well and left at 37°C for 1 hour. The secondary antibody solution was discarded, and the plate was washed three times with 200 μL/well of PBS-T (phosphate-buffered saline containing 0.05% (V/V) Tween 20, pH 7.4). SuperBlue TMB Microwell Peroxidase Substrate (1-Component) from Kirkegaard & Perry Laboratories, Inc. (KPL) was added dropwise at 100 μL/well for development of blue color, which was then turned into yellow color by the dropwise addition of 1 N hydrochloric acid at 100 μL/well. Absorbance at 450 nm was measured using a plate reader.

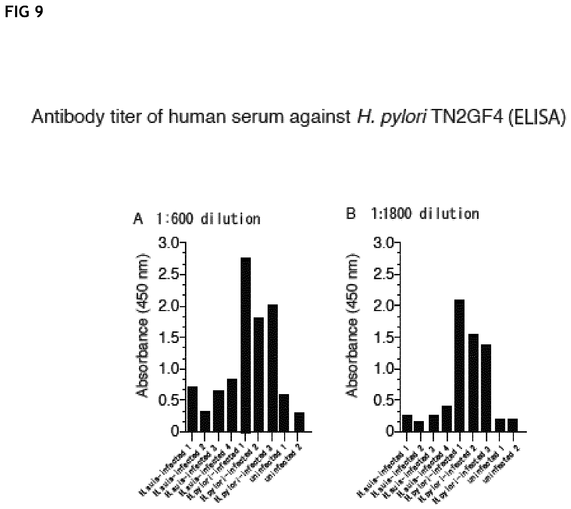

The results of ELISA using the human serum are shown in Figures 6 to 9. The *H. suis*-positive subjects had high antibody titers against the HsvA antigen peptide, whereas the subjects infected with *H. pylori* and non-infected subjects without *H. suis* infection had low antibody titers against the HsvA antigen peptide. The antibody titers against the *H. pylori* whole cells were high in the subjects infected with *H. pylori* and were low in the subjects infected with *H. suis* and the non-infected subjects. This showed for the first time that: *H. suis* expresses the hsvA gene in the human body; and an antibody against the HsvA protein is produced in the blood of a human infected with *H. suis*. This demonstrated that use of the HsvA antigen peptide enables *H. suis* infection to be diagnosed by discrimination from *H. pylori* infection in humans.

(Example 4) Measurement of anti-*H. suis* antibody titer in infected mouse using sandwich ELISA

The antibody against each peptide of Nos. 11, 19, and 33 (1 μg/mL) dissolved in a 0.2 M carbonate-bicarbonate buffer solution (pH 9.4) was added dropwise at 100 μL/well to 96-well NUNC-Immuno Plate #439454 and left overnight at 4°C. On the next day, the antibody solution was discarded, and the plate was washed three times with 200 μL/well of PBS-T (phosphate-buffered saline containing 0.05% (V/V) Tween 20, pH 7.4). Nacalai Tesque blocking solution (phosphate buffer solution-based, pH 7.2) diluted 5-fold with sterile distilled water was added dropwise at 200 μL/well and left at 37°C for 1 hour. The blocking solution was discarded, and the plate was washed three times with 200 μL/well of PBS-T (phosphate-buffered saline containing 0.05% (V/V) Tween 20, pH 7.4). Each peptide of Nos. 11, 19, and 33 (4 μg/mL) diluted using Nacalai Tesque blocking solution (phosphate buffer solution-based, pH 7.2) diluted 10-fold with sterile distilled water was added dropwise at 100 μL/well and left at 37°C for 1 hour. The peptide solution was discarded, and the plate was washed three times with 200 μL/well of PBS-T (phosphate-buffered saline containing 0.05% (V/V) Tween 20, pH 7.4). Mouse serum diluted using Nacalai Tesque blocking solution (PBS-based, pH 7.2) diluted 10-fold with exchange water was added dropwise at 100 μL/well and left at 37°C for 1 hour. The serum solution was discarded, and the plate was washed three times with 200 μL/well of PBS-T (phosphate-buffered saline containing 0.05% (V/V) Tween 20, pH 7.4). A horseradish peroxidase-labeled secondary antibody (Donkey Anti-Mouse IgG, Jackson ImmunoResearch Inc.) diluted 100,000-fold using Nacalai Tesque blocking solution (phosphate buffer solution-based, pH 7.2) diluted 10-fold with sterile distilled water was added dropwise at 100 μL/well and left at 37°C for 1 hour. The secondary antibody solution was discarded, and the plate was washed three times with 200 μL/well of PBS-T (phosphate-buffered saline containing 0.05% (V/V) Tween 20, pH 7.4). SuperBlue TMB Microwell Peroxidase Substrate (1-Component) from Kirkegaard & Perry Laboratories, Inc. (KPL) was added dropwise at 100 μL/well for development of blue color, which was then turned into yellow color by the dropwise addition of 1 N hydrochloric acid at 100 μL/well. Absorbance at 450 nm was measured using a plate reader.

The results of measuring the binding titers of antibodies in the serum of mice infected with an *H. suis* TKY or *H. pylori* SS1 strain against the peptides by ELISA and sandwich ELISA are shown in Figures 10 and 11, respectively. In both the systems, high antibody titers specific for the HsvA protein-derived peptides were found in the *H. suis*-infected mouse serum. This showed for the first time that: *H. suis* expresses the HsvA protein in the mouse body; and an antibody against the HsvA protein is produced in the blood of a mouse infected with *H. suis*. This demonstrated that use of the HsvA-specific antigen peptide also enables *H. suis* infection to be diagnosed by discrimination from *H. pylori* infection in mice.

(Example 5) Measurement of specificity of peptide antibody using ELISA (enzyme-linked immunosorbent assay; enzyme immunoassay)

Each peptide of Nos. 11, 19 and 33 (4 μg/mL) dissolved in a 0.2 M carbonate-bicarbonate buffer solution (pH 9.4) or *H. pylori* whole cells were added dropwise at 100 μL/well to 96-well NUNC-Immuno Plate #439454 and left overnight at 4°C. On the next day, the peptide solution was discarded, and the plate was washed three times with 200 μL/well of PBS-T (phosphate-buffered saline containing 0.05% (V/V) Tween 20, pH 7.4). Nacalai Tesque blocking solution (phosphate buffer solution-based, pH 7.2) diluted 5-fold with sterile distilled water was added dropwise at 200 μL/well and left at 37°C for 1 hour. The blocking solution was discarded, and the plate was washed three times with 200 μL/well of PBS-T (phosphate-buffered saline containing 0.05% (V/V) Tween 20, pH 7.4). Each anti-peptide antibody (1 μg/mL) obtained in Example 2(2) diluted using Nacalai Tesque blocking solution (phosphate buffer solution-based, pH 7.2) diluted 10-fold with exchange water was added dropwise at 100 μL/well and left at 37°C for 1 hour. The serum solution was discarded, and the plate was washed three times with 200 μL/well of PBS-T (phosphate-buffered saline containing 0.05% (V/V) Tween 20, pH 7.4). A horseradish peroxidase-labeled secondary antibody (Goat anti-Rabbit IgG, Jackson ImmunoResearch Inc.) diluted 100,000-fold using Nacalai Tesque blocking solution (PBS-based, pH 7.2) diluted 10-fold with exchange water was added dropwise at 100 μL/well and left at 37°C for 1 hour. The secondary antibody solution was discarded, and the plate was washed three times with 200 μL/well of PBS-T (phosphate-buffered saline containing 0.05% (V/V) Tween 20, pH 7.4). SuperBlue TMB Microwell Peroxidase Substrate (1-Component) from Kirkegaard & Perry Laboratories, Inc. (KPL) was added dropwise at 100 μL/well for development of blue color, which was then turned into yellow color by the dropwise addition of 1 N hydrochloric acid at 100 μL/well. Absorbance at 450 nm was measured using a plate reader.

The results of measuring the binding of the anti-peptide rabbit antibodies prepared in Example 2 onto wells bound with each antigen *(H. suis* HsvA antigen peptide (Nos. 11, 19 and 33) or *H. pylori* whole cells (TN2GF4 and SS1 strains)) are shown in Figure 12. The antibody against peptide No. 11 (SEQ ID NO: 3), peptide No. 33 (SEQ ID NO: 9), or peptide No. 19 (SEQ ID NO: 7) specifically exhibited strong binding to the peptide used as antigen in the preparation of this antibody, and on the other hand, exhibited only weak binding to the *H. pylori* whole cells. Particularly, the antibody against No. 11 peptide exhibited low nonspecific binding strength against the *H. pylori* whole cells.

(Example 6) Immunostaining using anti-No. 11 peptide antibody

A gastric paraffin section of an *H. suis*-infected human was deparaffinized by dipping in xylene three times, dehydrated ethanol once, 90% ethanol once, 80% ethanol once, and pure water once (5 minutes each) in order. Phosphate-buffered saline containing 1% (W/V) skimmed milk, pH 7.2 was added dropwise to the deparaffinized glass slide and left at room temperature for 30 minutes for blocking treatment. Subsequently, the glass slide was washed three times with phosphate-buffered saline, pH 7.2 at room temperature for 5 minutes. The anti-No. 11 peptide antibody was diluted into 2 μg/mL with phosphate-buffered saline, pH 7.2, added dropwise to the glass slide, and reacted at room temperature for 3 hours. The antibody solution was removed from the glass slide, which was then washed three times with phosphate-buffered saline, pH 7.2 at room temperature for 5 minutes. Alexa-Fluor 488 anti-rabbit IgG (Thermo Fisher Scientific Inc.) (secondary antibody) diluted 400-fold with phosphate-buffered saline, pH 7.2 was added dropwise to the glass slide and reacted at room temperature for 3 hours. The secondary antibody solution was removed from the glass slide, which was then washed three times with phosphate-buffered saline, pH 7.2 at room temperature for 5 minutes. Alexa-Fluor 568 phalloidin (Thermo Fisher Scientific Inc.) diluted 400-fold with phosphate-buffered saline, pH 7.2 was added dropwise to the glass slide and reacted at room temperature for 30 minutes for counterstaining (F actin staining). The secondary antibody solution was removed from the glass slide, which was then washed three times with phosphate-buffered saline, pH 7.2 at room temperature for 5 minutes. The glass slide was mounted to Permaflow (Therma Fisher Scientific Inc.). The glass slide was observed under confocal laser fluorescence microscope Leica TCS-SP5. Photograph A was taken at a 200× magnification. Photograph B was a photograph of the boxed portion of A magnified 760 times. The arrows depict bacterial cells of *H. suis.*

The results are shown in Figure 13. The bacterial cells in the gastric tissue section of the *H. suis*-infected patient were stained with the anti-No. 11 peptide antibody. This demonstrated that *H. suis* infection can be diagnosed by immunohistochemistry using the anti-No. 11 peptide antibody.

(Example 7)

Each peptide of peptide Nos. 8, 31N, 81, 63, 73, 61, 11 + 11 (TKY), 11 (SH8), 11 (SH10), 19 + 19 (TKY), 33 + 33 (TKY), 33 (SH10), 16, 23, 10, 21, 20, 22, 31, and 35 prepared in Example 2 or *H. pylori* TN2GF4 or SS1 whole cells (4 μg/mL) were dissolved in a 0.1 M carbonate-bicarbonate buffer solution (pH 9.4), added dropwise at 100 μL/well to a 96-well plate (NUNC-Immuno plate #439454), and left standing overnight at 4°C. On the next day, the peptide solution was discarded, and the plate was washed three times with 200 μL/well of PBS-T (phosphate-buffered saline containing 0.05% (V/V) Tween 20, pH 7.4). A blocking solution (BSA, 1% (W/V) phosphate buffer solution-based, pH 7.4) was added dropwise at 200 μL/well and left standing at 37°C for 1 hour. The blocking solution was discarded, and the plate was washed three times with 200 μL/well of PBS-T.

The serum samples used were serum obtained from *H. suis*-infected patients (n = 4), serum obtained from *H. pylori*-infected patients (n = 5), and serum obtained from non-infected subjects (n = 3). Each serum was diluted 1,800-fold (diluted 600-fold and 1800-fold only for reaction with *H. pylori* test specimens) using Nacalai Tesque blocking solution (phosphate buffer solution-based, pH 7.2) diluted 10-fold with distilled water, added dropwise at 50 μL/well to the plate prepared by the method mentioned above, and left standing at 37°C for 1 hour. The serum solution was discarded, and the plate was washed three times with 200 μL/well of PBS-T. A horseradish peroxidase-labeled secondary antibody (Peroxidase-conjugated AffiniPure Goat Anti-Human IgG (H + L), Jackson ImmunoResearch Inc.) diluted 100,000-fold using Nacalai Tesque blocking solution (phosphate buffer solution-based, pH 7.2) diluted 10-fold with distilled water was added dropwise at 50 μL/well and left standing at 37°C for 1 hour. The secondary antibody solution was discarded, and the plate was washed three times with PBS-T. SuperBlue TMB Microwell Peroxidase Substrate (1-Component) (Kirkegaard & Perry Laboratories, Inc. (KPL)) was added dropwise at 50 μL/well for development of blue color, which was then turned into yellow color by the dropwise addition of 1 N hydrochloric acid at 50 μL/well. Absorbance at 450 nm (reference: 630 nm) was measured using a plate reader.

The results are shown in Figure 14. The present ELISA method was able to discriminate positivity (absorbance: 0.5 or more) from negativity (absorbance: 0.5 or less) as to the human serum (*H. suis*-positive 4 test specimens (patients 5, 6, 7, and 8); *H. pylori*-positive 5 test specimens (patients 4, 5, 6, 7, and 8); non-infected 3 test specimens (healthy persons 4, 5, and 6) diluted 1800-fold. Ten peptides of Nos. 81, 61, 20, 11 + 11 (TKY), 11 (SH8), 11 (SH10), 19 + 19 (TKY), 10, 16, and 23 were found to have high reactivity with the *H. suis*-positive patient serum (A). On the other hand, the *H. pylori-positive* patient serum (B) and the non-infected serum (C) reacted with none of the peptides. The *H. pylori*-infected serum exhibited strong reactivity with the *H. pylori* whole cells, whereas the *H. suis*-infected serum did not exhibit reaction therewith (D). This showed that these peptides are exceedingly specific for antibodies against *H. suis* without reacting with antibodies against *H. pylori.* Accordingly, these peptides are useful in specific infection diagnosis using antibodies in serum.

(Example 8) Specificity of anti-peptide antibody against each peptide of Nos. 61, 11, 33, 19, 10, and 16 for corresponding antigen peptide

Each peptide of Nos. 8, 31N, 81, 63, 73, 61, 11 + 11 (TKY), 11 (SH8), 11 (SH10), 19 + 19 (TKY), 33 + 33 (TKY), 33 (SH10), 16, 23, 10, 21, 20, 22, 31, and 35 prepared in Example 2 was dissolved (4 μg/mL) in a 0.1 M carbonate-bicarbonate buffer solution (pH 9.4), added dropwise at 100 μL/well to a 96-well plate (NUNC-Immuno plate #439454), and left standing overnight at 4°C. On the next day, the peptide solution was discarded, and the plate was washed three times with 200 μL/well of PBS-T (phosphate-buffered saline containing 0.05% (V/V) Tween 20, pH 7.4). A blocking solution (BSA, 1% (W/V) phosphate buffer solution-based, pH 7.4) was added dropwise at 200 μL/well and left standing at 37°C for 1 hour. The blocking solution was discarded, and the plate was washed three times with 200 μL/well of PBS-T. The anti-peptide rabbit polyclonal antibody (IgG, 0.2 μg/mL) of Example 2 diluted using Nacalai Tesque blocking solution (phosphate buffer solution-based, pH 7.2) diluted 10-fold with distilled water was added dropwise at 50 μL/well and left standing at 37°C for 1 hour (n = 2). The serum solution was discarded, and the plate was washed three times with 200 μL/well of PBS-T. A horseradish peroxidase-labeled secondary antibody (Peroxidase-conjugated AffiniPure Goat Anti-Rabbit IgG (H + L), Jackson ImmunoResearch Inc.) diluted 100,000-fold using Nacalai Tesque blocking solution (phosphate buffer solution-based (pH 7.2) diluted 10-fold with distilled water was added dropwise at 50 μL/well and left standing at 37°C for 1 hour. The secondary antibody solution was discarded, and the plate was washed three times with PBS-T. SuperBlue TMB Microwell Peroxidase Substrate (1-Component) (Kirkegaard & Perry Laboratories, Inc. (KPL)) was added dropwise at 50 μL/well for development of blue color, which was then turned into yellow color by the dropwise addition of 1 N hydrochloric acid at 50 μL/well. Absorbance at 450 nm (reference: 630 nm) was measured using a plate reader.

The results are shown in Figure 15. The antibody against each peptide was shown to specifically react with only the corresponding peptide used as an antigen.

(Example 9) Specificity of anti-peptide antibody against each peptide of Nos. 61, 11, 33, 19, 10, and 16 for bacterial cells

*H. pylori* SS1, *H. pylori* TN2GF4, *H. pylori* NCTC11637, *H. pylori* TY1289, *H. pylori* RC-1, *H. pylori* TK1029, *H. pylori* TY281, *H. pylori* ATCC43579, *H. pylori* TK1081, or *H. suis* SNTW101 (4 μg/mL) dissolved in a 0.1 M carbonate-bicarbonate buffer solution (pH 9.4) was added dropwise at 100 μL/well to a 96-well plate (NUNC-Immuno plate #439454), and left standing overnight at 4°C. On the next day, the peptide solution was discarded, and the plate was washed three times with 200 μL/well of PBS-T (phosphate-buffered saline containing 0.05% (V/V) Tween 20, pH 7.4). A blocking solution (BSA, 1% (W/V) phosphate buffer solution-based, pH 7.4) was added dropwise at 200 μL/well and left standing at 37°C for 1 hour. The blocking solution was discarded, and the plate was washed three times with 200 μL/well of PBS-T. The anti-peptide rabbit polyclonal antibody (IgG, 0.2 μg/mL) prepared in Example 2 or a polyclonal antibody against *H. pylori* SS1 (rabbit IgG) (described in Non Patent Literature 4) diluted using Nacalai Tesque blocking solution (phosphate buffer solution-based, pH 7.2) diluted 10-fold with distilled water was added dropwise at 50 μL/well and left standing at 37°C for 1 hour (n = 2 to 4). The serum solution was discarded, and the plate was washed three times with 200 μL/well of PBS-T. A horseradish peroxidase-labeled secondary antibody (Peroxidase-conjugated AffiniPure Goat Anti-Rabbit IgG (H + L), Jackson ImmunoResearch Inc.) diluted 100,000-fold using Nacalai Tesque blocking solution (phosphate buffer solution-based, pH 7.2) diluted 10-fold with distilled water was added dropwise at 50 μL/well and left standing at 37°C for 1 hour. The secondary antibody solution was discarded, and the plate was washed three times with PBS-T. SuperBlue TMB Microwell Peroxidase Substrate (1-Component) (Kirkegaard & Perry Laboratories, Inc. (KPL)) was added dropwise at 50 μL/well for development of blue color, which was then turned into yellow color by the dropwise addition of 1 N hydrochloric acid at 50 μL/well. Absorbance at 450 nm (reference: 630 nm) was measured using a plate reader.

The results are shown in Figure 16. The anti-peptide antibody did not react with the bacterial cells of *H. pylori.* On the other hand, the anti-*H. pylori* polyclonal antibody reacted with the bacterial cells of *H. pylori* and *H. suis.*

(Example 10) Method for culturing *H. suis* SNTW101

(1) Separation of *H. suis* SNTW101 from gastric mucosa of infected mouse and culture

Culture was performed using a modified medium based on the method of Smet A., et al., International Journal of Systematic and Evolutionary Microbiology (2012), 62, 299-306. 0.05% (V/V) hydrochloric acid and 10 mg/L linezolid were added to Brucella agar plate (2 vials/L Skirrow, 10 mg/L vancomycin, 5 mg/L trimethoprim, 2500 IU/L polymyxin B, 2 vials/L vitox, 5 mg/L amphotericin B, 20% (V/V) fetal calf serum). Shake culture was performed for 2 weeks under conditions of 37°C, a humidity of 100%, 12% $CO_2$, 5% $O_2$, and 83% $N_2$. 150 μL of Brucella broth (2 vials/L Skirrow, 2 vials/L vitox, 5 mg/L linezolid, 20% (V/V) fetal calf serum, pH 5) was added thereto every other day. The culture solution was centrifuged, and the precipitates were washed three times with water and used as bacterial cells of *H. suis* SNTW101. Proteins were quantified using Bio-Rad protein assay kit. A calibration curve was prepared using bovine serum albumin (BSA) as a standard protein.

(Example 11) Immunohistochemistry using anti-No. 16 peptide (SEQ ID NO: 12) antibody

The stomach of an *H. suis* SNTW101-infected C57BL/6 mouse was cut open along the greater curvature. The contents were washed with PBS, and the stomach was excised in the perpendicular direction and fixed in 10% neutral buffered formalin. A gastric tissue was embedded in paraffin, and 4 μm sections were prepared. The sections were deparaffinized, then washed with water, and heat-treated at 95°C for 20 minutes using a citrate buffer solution (pH 6.0) for antigen retrieval. The sections were washed with water and then reacted with 0.3% hydrogen peroxide water at room temperature for 10 minutes. The sections were washed twice with PBS and then reacted with the anti-No. 16 peptide antibody (2 μg/mL PBS) at room temperature for 1 hour. The sections were washed twice with PBS and then reacted with Histofine Simple Stain Mouse MAX-PO(R) (Nichirei Corp., Code. 414341) as a secondary antibody at room temperature for 30 minutes. The sections were washed twice with PBS, followed by color development at room temperature for 5 minutes using a DAB (3,3'-diaminobenzidine tetrahydrochloride) solution. The sections were washed twice with PBS, then poststained with hematoxylin, washed with water, dehydrated, immersed, and mounted for microscopic observation.

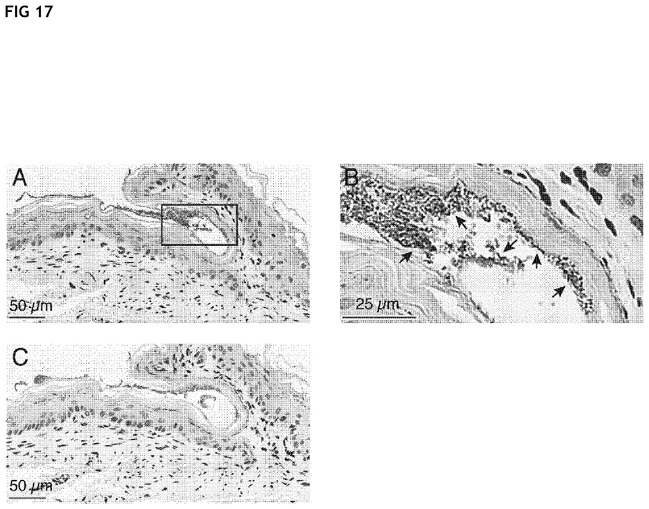

The results are shown in Figure 17. The antibody against No. 16 peptide (SEQ ID NO: 12) was shown to be able to immunostain bacterial cells of *H. suis.*

## Claims

1. An HsvA antigen peptide or an immunologically equivalent mutant thereof.

2. The antigen peptide or the immunologically equivalent mutant thereof of claim 1, which is derived from *H. suis.*

3. The antigen peptide or the immunologically equivalent mutant thereof of claim 1, wherein the HsvA antigen peptide has a sequence consisting of 5 to 50 amino acids contained in any one of the amino acid sequences set forth in SEQ ID NO: 2, SEQ ID NO: 28, SEQ ID NO: 30, and SEQ ID NOs: 78 to 83.

4. The antigen peptide or the immunologically equivalent mutant thereof of claim 3, wherein the HsvA antigen peptide has a sequence consisting of 5 to 50 amino acids contained in any one of the amino acid sequences set forth in SEQ ID NOs: 78 to 83.

5. The antigen peptide or the immunologically equivalent mutant thereof of claim 1, wherein the HsvA antigen peptide is a peptide having any one amino acid sequence selected from the group of the following:

   EKX1AVX2X3X4X5NSNX6X7 (peptide No. 11: SEQ ID NO: 24);
   NQGTLEFLSNDVSX8 (peptide No. 19: SEQ ID NO: 25);
   X9SX10KLQX11X12LKSX13X14X15 (peptide No. 33: SEQ ID NO: 26) ;
   TNGQEVSASIDYNK (peptide No. 16: SEQ ID NO: 12);
   AKLSNFASNDALPD (peptide No. 23: SEQ ID NO: 13);
   PTTSSGASPDSSNP (peptide No. 10: SEQ ID NO: 14);

GLGRDLFVHSMGDK (peptide No. 21: SEQ ID NO: 15);
QIGKIKLSDVLSAS (peptide No. 15: SEQ ID NO: 16);
YGAIDKELHFSGGK (peptide No. 34: SEQ ID NO: 17);
NVDNILNMPSTTSG (peptide No. 20: SEQ ID NO: 18);
GNLKGVYYPKSSTT (peptide No. 22: SEQ ID NO: 19);
ITEKIQSGKLTITI (peptide No. 14: SEQ ID NO: 20);
FHDFLVSLKGKKFA (peptide No. 26: SEQ ID NO: 21);
TTGGEVRLFRSFYV (peptide No. 31: SEQ ID NO: 22);
IGARFGLDYQDINI (peptide No. 35: SEQ ID NO: 23);
KQLPQPKRSELKPK (peptide No. 8: SEQ ID NO: 86);
TNIKQYMQNNHRSQ (peptide No. 31N: SEQ ID NO: 87);
TLTLEGTETFAQNS (peptide No. 81: SEQ ID NO: 88);
EAYAKNQGDIWSTI (peptide No. 63: SEQ ID NO: 89);
VIGSKSSITLNSAN (peptide No. 73: SEQ ID NO: 90); and
ADIQSSQTTFANSV (peptide No. 61: SEQ ID NO: 91); or an immunologically equivalent mutant thereof,

wherein X1 is K or D; X2 is Q, E or T; X3 is Q or S; X4 is M or L; X5 is E or K; X6 is P or S; X7 is D or G; X8 is N or T; X9 is L or F; X10 is N or D; X11 is G, D or N; X12 is Q or M; X13 is M or L; X14 is G or N; and X15 is L or M.

**6.** The antigen peptide or the immunologically equivalent mutant thereof of claim 5, wherein the HsvA antigen peptide is a peptide having any one amino acid sequence selected from the group of the following:

EKKAVQQMENSNPD (peptide No. 11: SEQ ID NO: 3);
EKKAVEQMENSNPD (peptide No. 11 (TKY): SEQ ID NO: 4);
EKDAVTSLKNSNSG (peptide No. 11 (SH8): SEQ ID NO: 5);
EKDAVTSLENSNSG (peptide No. 11 (SH10): SEQ ID NO: 6);
NQGTLEFLSNDVSN (peptide No. 19: SEQ ID NO: 7);
NQGTLEFLSNDVST (peptide No. 19 (TKY): SEQ ID NO: 8);
LSNKLQGQLKSMGL (peptide No. 33: SEQ ID NO: 9);
LSNKLQDQLKSMGL (peptide No. 33 (TKY): SEQ ID NO: 10);
FSDKLQNMLKSLNM (peptide No. 33 (SH10): SEQ ID NO: 11);
TNGQEVSASIDYNK (peptide No. 16: SEQ ID NO: 12);
AKLSNFASNDALPD (peptide No. 23: SEQ ID NO: 13);
PTTSSGASPDSSNP (peptide No. 10: SEQ ID NO: 14);
GLGRDLFVHSMGDK (peptide No. 21: SEQ ID NO: 15);
QIGKIKLSDVLSAS (peptide No. 15: SEQ ID NO: 16);
YGAIDKELHFSGGK (peptide No. 34: SEQ ID NO: 17);
NVDNILNMPSTTSG (peptide No. 20: SEQ ID NO: 18);
GNLKGVYYPKSSTT (peptide No. 22: SEQ ID NO: 19);
ITEKIQSGKLTITI (peptide No. 14: SEQ ID NO: 20);
FHDFLVSLKGKKFA (peptide No. 26: SEQ ID NO: 21);
TTGGEVRLFRSFYV (peptide No. 31: SEQ ID NO: 22);
IGARFGLDYQDINI (peptide No. 35: SEQ ID NO: 23);
KQLPQPKRSELKPK (peptide No. 8: SEQ ID NO: 86);
TNIKQYMQNNHRSQ (peptide No. 31N: SEQ ID NO: 87);
TLTLEGTETFAQNS (peptide No. 81: SEQ ID NO: 88);
EAYAKNQGDIWSTI (peptide No. 63: SEQ ID NO: 89);
VIGSKSSITLNSAN (peptide No. 73: SEQ ID NO: 90); and
ADIQSSQTTFANSV (peptide No. 61: SEQ ID NO: 91); or an immunologically equivalent mutant thereof.

**7.** An antibody or an immunoreactive fragment thereof that specifically binds to the antigen peptide or the immunologically equivalent mutant thereof of any one of claims 1 to 6.

**8.** A nucleic acid molecule consisting of 5 to 40 nucleotides, wherein the nucleic acid molecule is capable of binding to hsvA gene under stringent conditions.

**9.** The nucleic acid molecule of claim 8, wherein the hsvA gene has the nucleotide sequence set forth in SEQ ID NO: 1, SEQ ID NO: 27 or SEQ ID NO: 29.

10. The nucleic acid molecule of claim 9 having any one nucleotide sequence selected from the group of the following:

CTTTTAGCGTGGTATCCTTC (SEQ ID NO: 31);
TTACAAAGACCACCAACGGA (SEQ ID NO: 32); TACCATAGAAAGCGGAAAC (SEQ ID NO: 33); AGCTATAGCTTTGCACCTGA (SEQ ID NO: 34);
ACTAACAGCATAGAAGTTGGGGA (SEQ ID NO: 35);
AACAACATTACAGGCATGAG (SEQ ID NO: 36);
CAAATAGTAACAGGACAATT (SEQ ID NO: 37);
CAGGATTTAAGAGGCACTTA (SEQ ID NO: 38); TCTTAACCAATCTGAGCAA (SEQ ID NO: 39); GTCAAACACGTTATTGATGGCTT (SEQ ID NO: 40); GGGGTTTAATAGCATAGGG (SEQ ID NO: 41); TAGAGCTCTACACCAGTCTT (SEQ ID NO: 42); ATAAAGCCCATGAATTCTTAGGCATGCGTGCTCTG (SEQ ID NO: 43); TGCTATTGATAAAGAGTTACATTTCTCAGG (SEQ ID NO: 44); ATTTCTCAGGGGGAAAGTC (SEQ ID NO: 45);
GGCTAATAATTTAACCACAATAAGCGCCTTTAA (SEQ ID NO: 46);
GATGGGCGCTTCTGGTTTA (SEQ ID NO: 47);
ATGAATTCTTAGGCATGCGTGCTCT (SEQ ID NO: 48);
TTTTGGAGGTGTAGGAGTAGAT (SEQ ID NO: 49);
AGCGCGCATTTAAAACAGGT (SEQ ID NO: 50);
AGAAACGAGATTACAGGAAG (SEQ ID NO: 51);
AGGAGCAAGTTTTGTAGCAG (SEQ ID NO: 52);
AAAATGCGACTGATTGGATG (SEQ ID NO: 53); TTGAAATTTGGCCACGCT (SEQ ID NO: 54); TCACCCATAGAATGGACGAA (SEQ ID NO: 55); CTAGCGCATTAACCACAGACTG (SEQ ID NO: 56);
TAGAAGTTGTAGACACGGT (SEQ ID NO: 57); GTGATATTGCCTTTCTGAAC (SEQ ID NO: 58); GCAAGTTTTGTGCGGATT (SEQ ID NO: 59);
ATGTGATACACATCTGACC (SEQ ID NO: 60); AGTGCCGTTACCATCGTGAA (SEQ ID NO: 61); TATTCAAGGAAAGTCCCTGGAGAAACTCCAGAGAC (SEQ ID NO: 62); TTAAAGGCGCTTATTGTGGTTAAATTATTAGCC (SEQ ID NO: 63); ATTAGCCTTAAGGGTGCTATC (SEQ ID NO: 64);
CTGGTAATGCATCATTAGAAGCAAA (SEQ ID NO: 65);
TACGCGCAAAATAGGTTCTT (SEQ ID NO: 66);
TGGAGAAACTCCAGAGACTA (SEQ ID NO: 67);
TTGTTCGCTGTAGTGCCGTGG (SEQ ID NO: 68);
GAAGGATACCACGCTAAAAG (SEQ ID NO: 69);
AAGCTAGAGTTTTGGTTGAG (SEQ ID NO: 70); TTGCTCAGATTGGTTAAGA (SEQ ID NO: 71); ATCGAAATAAGCGAACCTCA (SEQ ID NO: 72);
TTGAAAGCTTAGCTAAACGG (SEQ ID NO: 73);
TGGTATTGCTGGTTAAGAGG (SEQ ID NO: 74), CAAACAGATGAGCCGT (SEQ ID NO: 75);
ATGAAAAAGTTTAGTTCTCTCACATTGAAATTTGGCCACGCTC (SEQ ID NO: 76); and CTAAAAAGCATAGCGCATCCCGACATTGCCTGTAATATTAATATC (SEQ ID NO: 77).

11. The nucleic acid molecule of any one of claims 8 to 10, wherein the nucleic acid molecule is a primer capable of amplifying the whole or a portion of the hsvA gene.

12. The nucleic acid molecule of any one of claims 8 to 10, wherein the nucleic acid molecule is a probe for detecting the hsvA gene.

13. A method for determining the presence of *H. suis,* comprising detecting the whole or a portion of hsvA gene in a sample,
wherein the sample in which the whole or a portion of the hsvA gene has been detected is determined that *H. suis.* is present.

14. The method for determining the presence of *H. suis* of claim 13, comprising:

amplifying the whole or a portion of the hsvA gene using DNA in the sample as a template and using the primer of claim 11;
detecting the amplified DNA; and
determining that *H. suis* is present in the sample in which the DNA amplification has been detected.

15. The method for determining the presence of *H. suis* of claim 13, comprising:

contacting DNA in the sample with the probe of claim 12;
detecting DNA bound with the probe of claim 12; and
determining that *H. suis* is present in the sample in which the DNA bound with the probe of claim 11 has been detected.

**16.** A method for determining the presence of *H. suis,* comprising: detecting HsvA protein or a fragment thereof in a sample; and determining that *H. suis* is present in the sample in which the HsvA protein or the fragment thereof has been detected.

**17.** The method for determining the presence of *H. suis* of claim 16, comprising:

contacting the sample with the antibody or an immunoreactive fragment thereof of claim 7;
detecting the HsvA protein or the fragment thereof in the sample bound with the antibody or the immunoreactive fragment thereof; and
determining that *H. suis* is present in the sample in which the HsvA protein or the fragment thereof bound with the antibody or the immunoreactive fragment thereof has been detected.

**18.** A method for determining infection of a subject with *H. suis,* comprising:

performing a method of any one of claims 12 to 17 using a sample derived from the subject as a sample; and
determining the subject is infected with *H. suis,* when a sample derived from whom is determined that *H. suis* is present by the method.

**19.** A method for determining infection of a subject with *H. suis,* comprising:

detecting an antibody that binds to an HsvA antigen peptide in a blood sample derived from the subject; and
determining the subject is infected with *H. suis* when the antibody that binds to the peptide has been detected.

**20.** The method for determining infection with *H. suis* of claim 19, comprising the steps of:

contacting the sample derived from the subject with the peptide of any one of claims 1 to 6;
detecting an antibody bound with the peptide, in the blood sample; and
determining the subject is infected with *H. suis* when the antibody bound with the peptide has been detected.

**21.** A composition for determination of *H. suis* infection, comprising any one selected from a peptide of any one of claims 1 to 6, a nucleic acid molecule of any one of claims 8 to 10, and an antibody or an immunoreactive fragment thereof of claim 7.

**22.** A pharmaceutical composition comprising any one selected from a peptide of any one of claims 1 to 6, a nucleic acid molecule of any one of claims 8 to 10, and an antibody or an immunoreactive fragment thereof of claim 7.

**23.** The pharmaceutical composition of claim 22 for *H. suis* eradication therapy.

**24.** The pharmaceutical composition of claim 22 for treatment or prevention of gastritis, gastric ulcer, duodenal ulcer, stomach cancer, chronic gastritis, gastric MALT lymphoma, nodular gastritis, idiopathic thrombocytopenic purpura, functional dyspepsia, or diffuse large B-cell lymphoma.

FIG 1

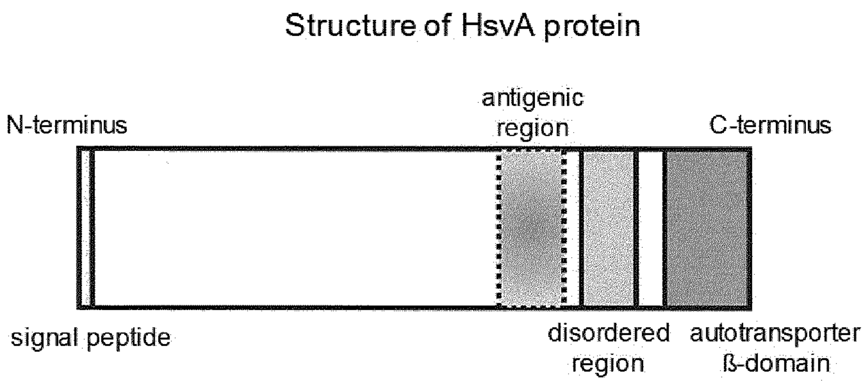
Structure of HsvA protein
antigenic region
N-terminus
C-terminus
signal peptide
disordered region
autotransporter ß-domain

FIG 2A

```
        10        20        30        40        50        60
atgaaaaagtttagttctctcacattgaaatttggccacgctctagcacggcgcattaaa
MetLysLysPheSerSerLeuThrLeuLysPheGlyHisAlaLeuAlaArgArgIleLys

        70        80        90       100       110       120
gctaaccaagctaggcgtgcgggtacttatgtctttaaaaaacaactcccccaacccaag
AlaAsnGlnAlaArgArgAlaGlyThrTyrValPheLysLysGlnLeuProGlnProLys

       130       140       150       160       170       180
cgttctgagttgaaacctaaattaatcaagcaggggacttttattttaggtgtaatgagc
ArgSerGluLeuLysProLysLeuIleLysGlnGlyThrPheIleLeuGlyValMetSer

       190       200       210       220       230       240
caaccgcttttagcgtggtatccttcatggattagtggcacacacacactcaacacaaca
GlnProLeuLeuAlaTrpTyrProSerTrpIleSerGlyThrHisThrLeuAsnThrThr

       250       260       270       280       290       300
aatattaagcagtatatgcaaaataaccatagaagccaaaattggttatggactggtggc
AsnIleLysGlnTyrMetGlnAsnAsnHisArgSerGlnAsnTrpLeuTrpThrGlyGly

       310       320       330       340       350       360
tctaatgtcttttatgaagctagtaatggaagttatttttgtactaactggaattgtaac
SerAsnValPheTyrGluAlaSerAsnGlySerTyrPheCysThrAsnTrpAsnCysAsn

       370       380       390       400       410       420
ggctcggttacattaattggtagtggctctaccacttacacgcttagcaatctcaattac
GlySerValThrLeuIleGlySerGlySerThrThrTyrThrLeuSerAsnLeuAsnTyr

       430       440       450       460       470       480
gaaggcggtagcctcaatctacaaattaatggtaatggcacacaaggcgcgcttaatatt
GluGlyGlySerLeuAsnLeuGlnIleAsnGlyAsnGlyThrGlnGlyAlaLeuAsnIle

       490       500       510       520       530       540
agcaatgtcaacatggatagctatcaaggaaggcaatttacagatacatggaatgcccaa
SerAsnValAsnMetAspSerTyrGlnGlyArgGlnPheThrAspThrTrpAsnAlaGln

       550       560       570       580       590       600
agtgttagtatcagcggaaatctacaagtagggcaaaatcatatcactatcaacgcaaac
SerValSerIleSerGlyAsnLeuGlnValGlyGlnAsnHisIleThrIleAsnAlaAsn

       610       620       630       640       650       660
cacggcactacagcgaacaacgctaccattaacgcggatcaaggcaacgggattttaaat
HisGlyThrThrAlaAsnAsnAlaThrIleAsnAlaAspGlnGlyAsnGlyIleLeuAsn
```

FIG 2B

```
        670        680        690        700        710        720
atcaatgataaaacaagcgagagtttcacaaacaccacctttaaaggtacggggcagatc
IleAsnAspLysThrSerGluSerPheThrAsnThrThrPheLysGlyThrGlyGlnIle

        730        740        750        760        770        780
aatttacagagcaacaacatcacctttaacaatgttacttttaatgatagcaacactggt
AsnLeuGlnSerAsnAsnIleThrPheAsnAsnValThrPheAsnAspSerAsnThrGly

        790        800        810        820        830        840
tcacatgttacggacaacggcactcttacccttgagggcactgagacctttgcacaaaac
SerHisValThrAspAsnGlyThrLeuThrLeuGluGlyThrGluThrPheAlaGlnAsn

        850        860        870        880        890        900
tcgccccttatcaatctaggcgccaatgttactatccaagccaacaccatttttaacatt
SerProLeuIleAsnLeuGlyAlaAsnValThrIleGlnAlaAsnThrIlePheAsnIle

        910        920        930        940        950        960
acagaagatcttacaaagaccaccaacggagcctataatcttgatacccttgtaagtaca
ThrGluAspLeuThrLysThrThrAsnGlyAlaTyrAsnLeuAspThrLeuValSerThr

        970        980        990       1000       1010       1020
agtggtaataaaagtatcaacgatagtagctatgcaagccatttgtgggatcttatccgc
SerGlyAsnLysSerIleAsnAspSerSerTyrAlaSerHisLeuTrpAspLeuIleArg

       1030       1040       1050       1060       1070       1080
tatcaaggccaaacaggtagcctttttaacgggcaactcagtaatggcactactttaagt
TyrGlnGlyGlnThrGlySerLeuPheAsnGlyGlnLeuSerAsnGlyThrThrLeuSer

       1090       1100       1110       1120       1130       1140
aatcctagctctggcaatgggatttactatgtgaaatatacttttggcaatggtgattgg
AsnProSerSerGlyAsnGlyIleTyrTyrValLysTyrThrPheGlyAsnGlyAspTrp

       1150       1160       1170       1180       1190       1200
gatattcttaaagaagattttgaaaataactctctatcggcgcaacttgaggcttacgct
AspIleLeuLysGluAspPheGluAsnAsnSerLeuSerAlaGlnLeuGluAlaTyrAla

       1210       1220       1230       1240       1250       1260
aaaaatcagggtgatatttggagtacaatccacaacattaattctacttggaattttaat
LysAsnGlnGlyAspIleTrpSerThrIleHisAsnIleAsnSerThrTrpAsnPheAsn

       1270       1280       1290       1300       1310       1320
gtcggagagggtagtgcctatatcaaccccgggaatggaacagatcaagcttgggcacaa
ValGlyGluGlySerAlaTyrIleAsnProGlyAsnGlyThrAspGlnAlaTrpAlaGln
```

FIG 2C

```
       1330      1340      1350      1360      1370      1380
agcgataaaaattttaaagttactttagataatgggagtggtgggacgcttattcttggt
SerAspLysAsnPheLysValThrLeuAspAsnGlySerGlyGlyThrLeuIleLeuGly

       1390      1400      1410      1420      1430      1440
aatagcacagaaacccccagttcaagcggtaaaattctctttggaggcacaggggtaat
AsnSerThrGluThrProSerSerSerGlyLysIleLeuPheGlyGlyThrGlyGlyAsn

       1450      1460      1470      1480      1490      1500
ccttttgctttaaatggcaattacggcggagatcttggctatatgacaggggaatttgac
ProPheAlaLeuAsnGlyAsnTyrGlyGlyAspLeuGlyTyrMetThrGlyGluPheAsp

       1510      1520      1530      1540      1550      1560
gcgggtaaaatttatcttaccggtaccatagaaagcggaaactcttttcacgatggtaac
AlaGlyLysIleTyrLeuThrGlyThrIleGluSerGlyAsnSerPheHisAspGlyAsn

       1570      1580      1590      1600      1610      1620
ggcactaacattagctacaacgctattacaaacattacagctaatggtttgcactatctc
GlyThrAsnIleSerTyrAsnAlaIleThrAsnIleThrAlaAsnGlyLeuHisTyrLeu

       1630      1640      1650      1660      1670      1680
aacgataacgccggagcatggcatagcaacgctaattttagagctaccaatggcagtatc
AsnAspAsnAlaGlyAlaTrpHisSerAsnAlaAsnPheArgAlaThrAsnGlySerIle

       1690      1700      1710      1720      1730      1740
aacatctcaaactcccaattccaagatcaaagtagcgggcaatttacctttaactctcaa
AsnIleSerAsnSerGlnPheGlnAspGlnSerSerGlyGlnPheThrPheAsnSerGln

       1750      1760      1770      1780      1790      1800
aatcaaacttttagtagcacaacttttactggcaatagtcatacgatcactctacacgct
AsnGlnThrPheSerSerThrThrPheThrGlyAsnSerHisThrIleThrLeuHisAla

       1810      1820      1830      1840      1850      1860
accaacaacctaaccctcaccaacacaagctttaatgattcaaatgctagcttgacacta
ThrAsnAsnLeuThrLeuThrAsnThrSerPheAsnAspSerAsnAlaSerLeuThrLeu

       1870      1880      1890      1900      1910      1920
gaggcagataagggtagtttacgagataccgataaccaaactagccaaatcacagctaaa
GluAlaAspLysGlySerLeuArgAspThrAspAsnGlnThrSerGlnIleThrAlaLys

       1930      1940      1950      1960      1970      1980
aacctccaagtaattgctaatcaagctagttttagtaacacgatctttaatgcacaaaac
AsnLeuGlnValIleAlaAsnGlnAlaSerPheSerAsnThrIlePheAsnAlaGlnAsn
```

FIG 2D

```
        1990      2000      2010      2020      2030      2040
agctcttttaaaactaaccaattgaccctgacaaatgatacatttaataatggtagctat
SerSerPheLysThrAsnGlnLeuThrLeuThrAsnAspThrPheAsnAsnGlySerTyr

        2050      2060      2070      2080      2090      2100
agctttgcacctgaaaacaacggcaaccacacaaccacatttggcggtaccaccacgatt
SerPheAlaProGluAsnAsnGlyAsnHisThrThrThrPheGlyGlyThrThrThrIle

        2110      2120      2130      2140      2150      2160
aatactagcagtagcccctttgctaatttaggcggaagcattagtttaaacaacggcgct
AsnThrSerSerSerProPheAlaAsnLeuGlyGlySerIleSerLeuAsnAsnGlyAla

        2170      2180      2190      2200      2210      2220
atttttaatcttaataatattttaagttctttacaaattggtacaacttataacatctta
IlePheAsnLeuAsnAsnIleLeuSerSerLeuGlnIleGlyThrThrTyrAsnIleLeu

        2230      2240      2250      2260      2270      2280
ggcggaagcggggctaatattctttacaagaacgatacacaatatgcctcagcactttgg
GlyGlySerGlyAlaAsnIleLeuTyrLysAsnAspThrGlnTyrAlaSerAlaLeuTrp

        2290      2300      2310      2320      2330      2340
caactcattaaaatcaacgggactactattaactcagaaacagagataagtgataataac
GlnLeuIleLysIleAsnGlyThrThrIleAsnSerGluThrGluIleSerAspAsnAsn

        2350      2360      2370      2380      2390      2400
gatacgcaagtttggaatgtagtctttaacatagatggcatgcccattaagatacaagaa
AspThrGlnValTrpAsnValValPheAsnIleAspGlyMetProIleLysIleGlnGlu

        2410      2420      2430      2440      2450      2460
acctttgctagtagtggacttagctttaaagttattagccaagctaaagatatttggtca
ThrPheAlaSerSerGlyLeuSerPheLysValIleSerGlnAlaLysAspIleTrpSer

        2470      2480      2490      2500      2510      2520
gatgtgtatcacatgactactaattgtgcatacaatgccttagccagcccaccgggctgt
AspValTyrHisMetThrThrAsnCysAlaTyrAsnAlaLeuAlaSerProProGlyCys

        2530      2540      2550      2560      2570      2580
tatggttatcaaaatggtacacacaacatattgaagcactttaataagtacggctttgaa
TyrGlyTyrGlnAsnGlyThrHisAsnIleLeuLysHisPheAsnLysTyrGlyPheGlu

        2590      2600      2610      2620      2630      2640
gcttacaatgaaagtgtaggcgcagacgggattgcctatatcaaccctttaaactcacaa
AlaTyrAsnGluSerValGlyAlaAspGlyIleAlaTyrIleAsnProLeuAsnSerGln
```

FIG 2E

2650 2660 2670 2680 2690 2700
agccacgatggctataacgcctccacccacacactccaaacttatacaaaaattggcaat
SerHisAspGlyTyrAsnAlaSerThrHisThrLeuGlnThrTyrThrLysIleGlyAsn

2710 2720 2730 2740 2750 2760
ggcggtacttataatgttggggagatgcaaaatggcaaatgggttaacaatggtacttta
GlyGlyThrTyrAsnValGlyGluMetGlnAsnGlyLysTrpValAsnAsnGlyThrLeu

2770 2780 2790 2800 2810 2820
attttaggtaataacacctcacaacccgttaaaggggggtaaaatagaatttggcaaagtg
IleLeuGlyAsnAsnThrSerGlnProValLysGlyGlyLysIleGluPheGlyLysVal

2830 2840 2850 2860 2870 2880
gactcagtaggctatatcaccgatgtttttaatgccggacatatttttctaactaacagc
AspSerValGlyTyrIleThrAspValPheAsnAlaGlyHisIlePheLeuThrAsnSer

2890 2900 2910 2920 2930 2940
atagaagttggggatagttccttaagcggggcgggggcaactctaacttttaatgctaat
IleGluValGlyAspSerSerLeuSerGlyAlaGlyAlaThrLeuThrPheAsnAlaAsn

2950 2960 2970 2980 2990 3000
aataatatcaccgccgacggtcttacctaccaccaagatgctaccgctatccctcccttc
AsnAsnIleThrAlaAspGlyLeuThrTyrHisGlnAspAlaThrAlaIleProProPhe

3010 3020 3030 3040 3050 3060
ggctctgctatcagccagcatagctacggtaacttcatcgcacaacaaagctttagcgcg
GlySerAlaIleSerGlnHisSerTyrGlyAsnPheIleAlaGlnGlnSerPheSerAla

3070 3080 3090 3100 3110 3120
ctcaattcctcttttgaagatgatactagcggtagtttagactttaccggtaaaaacagc
LeuAsnSerSerPheGluAspAspThrSerGlySerLeuAspPheThrGlyLysAsnSer

3130 3140 3150 3160 3170 3180
attaattttacaagcagcaccgttataggtagtaaaagtagtattacccttaactccgca
IleAsnPheThrSerSerThrValIleGlySerLysSerSerIleThrLeuAsnSerAla

3190 3200 3210 3220 3230 3240
aataccaccctaaacaactctgcctttttgtgggcaatggtacgactcttacttttggt
AsnThrThrLeuAsnAsnSerAlaPhePheValGlyAsnGlyThrThrLeuThrPheGly

3250 3260 3270 3280 3290 3300
aatgtgatcacaactaatagtcatagtagctattcatctagcacaaacaccattaataac
AsnValIleThrThrAsnSerHisSerSerTyrSerSerSerThrAsnThrIleAsnAsn

FIG 2F

3310 3320 3330 3340 3350 3360
tctacaatcaacctcaaccaaaactctagcttgtatttgcatggcagtaccaccctagat
SerThrIleAsnLeuAsnGlnAsnSerSerLeuTyrLeuHisGlySerThrThrLeuAsp

3370 3380 3390 3400 3410 3420
aacaccacttcctttttaaacttaagtagtcaagcaactttttatgattcagctagccta
AsnThrThrSerPheLeuAsnLeuSerSerGlnAlaThrPheTyrAspSerAlaSerLeu

3430 3440 3450 3460 3470 3480
agtggtagcactacaattaatcttgatcacaatagcaagattacaatgaatagcaccact
SerGlySerThrThrIleAsnLeuAspHisAsnSerLysIleThrMetAsnSerThrThr

3490 3500 3510 3520 3530 3540
acgctaaatgataacgctaattttaatttaattaactccgctcaagctaactttcaaggc
ThrLeuAsnAspAsnAlaAsnPheAsnLeuIleAsnSerAlaGlnAlaAsnPheGlnGly

3550 3560 3570 3580 3590 3600
aatagtacttttaataataactctgggatcactcttgctagtggtgctaaggctatgttt
AsnSerThrPheAsnAsnAsnSerGlyIleThrLeuAlaSerGlyAlaLysAlaMetPhe

3610 3620 3630 3640 3650 3660
acacacactacaacaagcacccaagatattacaacttttaataataactcgtttttaaat
ThrHisThrThrThrSerThrGlnAspIleThrThrPheAsnAsnAsnSerPheLeuAsn

3670 3680 3690 3700 3710 3720
gtcaatgggagttttacagattttatacccagtacaaatagtaacaggacaattagcggg
ValAsnGlySerPheThrAspPheIleProSerThrAsnSerAsnArgThrIleSerGly

3730 3740 3750 3760 3770 3780
ggaggatcaagtagtaatcaaaactatagcgcgcagtttaatgatctcgtgtttaacaac
GlyGlySerSerSerAsnGlnAsnTyrSerAlaGlnPheAsnAspLeuValPheAsnAsn

3790 3800 3810 3820 3830 3840
tatgcttccatgctcttaaatagcgcagatattcaaagtagccaaaccacctttgctaat
TyrAlaSerMetLeuLeuAsnSerAlaAspIleGlnSerSerGlnThrThrPheAlaAsn

3850 3860 3870 3880 3890 3900
tctgtgaatatcaatatgcaaaatagcctttttaatgcgagtacgcttaatttaaatggc
SerValAsnIleAsnMetGlnAsnSerLeuPheAsnAlaSerThrLeuAsnLeuAsnGly

3910 3920 3930 3940 3950 3960
ggtaatttttacatgcaaaatagccagattaaagcgggggcagtgagtgtgggatcttct
GlyAsnPheTyrMetGlnAsnSerGlnIleLysAlaGlyAlaValSerValGlySerSer

FIG 2G

3970 3980 3990 4000 4010 4020
gattctgttaatattaattctggggtcaattctcttaatcgctccctcatcactagctca
AspSerValAsnIleAsnSerGlyValAsnSerLeuAsnArgSerLeuIleThrSerSer

4030 4040 4050 4060 4070 4080
agctttaatcttaatggaacattaaacctagatggccttttacttggacctacaactaca
SerPheAsnLeuAsnGlyThrLeuAsnLeuAspGlyLeuLeuLeuGlyProThrThrThr

4090 4100 4110 4120 4130 4140
ggtacaaccaaaagtacggccaacgggcaacctcttatttctgtaggagggggaacattt
GlyThrThrLysSerThrAlaAsnGlyGlnProLeuIleSerValGlyGlyGlyThrPhe

4150 4160 4170 4180 4190 4200
agcttaaatggcattcttaatatctctaatattgatctctccgcccacctctctagccaa
SerLeuAsnGlyIleLeuAsnIleSerAsnIleAspLeuSerAlaHisLeuSerSerGln

4210 4220 4230 4240 4250 4260
acaaatcacactagttcctctgctacctatgatattgtgaatgctaacaacattacaggc
ThrAsnHisThrSerSerSerAlaThrTyrAspIleValAsnAlaAsnAsnIleThrGly

4270 4280 4290 4300 4310 4320
atgagtggggctaatggttatcaaaaaattgagtattacggcattaaaatcaataatgct
MetSerGlyAlaAsnGlyTyrGlnLysIleGluTyrTyrGlyIleLysIleAsnAsnAla

4330 4340 4350 4360 4370 4380
acctattctgattcaaattctgattcaaataaaacccaaagctggagttttacaaaccct
ThrTyrSerAspSerAsnSerAspSerAsnLysThrGlnSerTrpSerPheThrAsnPro

4390 4400 4410 4420 4430 4440
ttaaatggcgcacaaactattactgaaaaaatacaaagtggtaaactcactatcaccatt
LeuAsnGlyAlaGlnThrIleThrGluLysIleGlnSerGlyLysLeuThrIleThrIle

4450 4460 4470 4480 4490 4500
agtaatagcaatcattttgtagctacagattaccacaacatcgcccccgaactctttttt
SerAsnSerAsnHisPheValAlaThrAspTyrHisAsnIleAlaProGluLeuPhePhe

4510 4520 4530 4540 4550 4560
tacaaacaatccgcacaaaacttgcctagtacaaactcagcaagtgcagatataagcagt
TyrLysGlnSerAlaGlnAsnLeuProSerThrAsnSerAlaSerAlaAspIleSerSer

4570 4580 4590 4600 4610 4620
tatgattattccagcgatgaaagcgggactttctttttagatggcaatttaaaaggggtg
TyrAspTyrSerSerAspGluSerGlyThrPhePheLeuAspGlyAsnLeuLysGlyVal

FIG 2H

4630 4640 4650 4660 4670 4680
tattatccaaaatctagcacaacaggcactaccccagttattccgggtacttataacgct
TyrTyrProLysSerSerThrThrGlyThrThrProValIleProGlyThrTyrAsnAla

4690 4700 4710 4720 4730 4740
caaggccagcccttacaaggtctttatatttctaataacggcctgtttaatgaaaccacc
GlnGlyGlnProLeuGlnGlyLeuTyrIleSerAsnAsnGlyLeuPheAsnGluThrThr

4750 4760 4770 4780 4790 4800
cttaataacttagtagacattgtccgaagtatttggcctcatttaaaaaccctttttgcct
LeuAsnAsnLeuValAspIleValArgSerIleTrpProHisLeuLysThrLeuLeuPro

4810 4820 4830 4840 4850 4860
aaaattctacaagatttaagcgatccaagtaatatcattcaagacttagaaaactcaggg
LysIleLeuGlnAspLeuSerAspProSerAsnIleIleGlnAspLeuGluAsnSerGly

4870 4880 4890 4900 4910 4920
attaaattaactagccagcaaagcaaggaacttttaagttttatagacgggctatcaagc
IleLysLeuThrSerGlnGlnSerLysGluLeuLeuSerPheIleAspGlyLeuSerSer

4930 4940 4950 4960 4970 4980
aatatcaaccaaacttttaataatggtacgcttgtagtgggttcgcctcaggcaggacaa
AsnIleAsnGlnThrPheAsnAsnGlyThrLeuValValGlySerProGlnAlaGlyGln

4990 5000 5010 5020 5030 5040
accggaagtagtagcgtggtgtggtttggtggcaatggttatactacggcttgtacggct
ThrGlySerSerSerValValTrpPheGlyGlyAsnGlyTyrThrThrAlaCysThrAla

5050 5060 5070 5080 5090 5100
gcacaaactgaaaaagggtgtcaggatttaagaggcacttatttaggtcagcttttagga
AlaGlnThrGluLysGlyCysGlnAspLeuArgGlyThrTyrLeuGlyGlnLeuLeuGly

5110 5120 5130 5140 5150 5160
tcaacttctgctgcactaggctatattgaggctaattttaaggctaaagatatttatatc
SerThrSerAlaAlaLeuGlyTyrIleGluAlaAsnPheLysAlaLysAspIleTyrIle

5170 5180 5190 5200 5210 5220
accggcaccgtaggcagtgggaatgcttggggggataggtgggagcgcagatgtaactttt
ThrGlyThrValGlySerGlyAsnAlaTrpGlyIleGlyGlySerAlaAspValThrPhe

5230 5240 5250 5260 5270 5280
aatagcgcgactaatttaaccctcaatcaagccacgattgatgcggaaggcacggatcaa
AsnSerAlaThrAsnLeuThrLeuAsnGlnAlaThrIleAspAlaGluGlyThrAspGln

FIG 2I

5290 5300 5310 5320 5330 5340
atttttaatatgctaggtgagggcgggatacaaaaaatcttaggccaaaaggggctagcc
IlePheAsnMetLeuGlyGluGlyGlyIleGlnLysIleLeuGlyGlnLysGlyLeuAla

5350 5360 5370 5380 5390 5400
cagatgttaggtaattatatctacgatctagccaatggctctagtattaatcttaatccc
GlnMetLeuGlyAsnTyrIleTyrAspLeuAlaAsnGlySerSerIleAsnLeuAsnPro

5410 5420 5430 5440 5450 5460
attagtagcattccaagctctatccagcctctggctaaagatttaggcaggcagattggt
IleSerSerIleProSerSerIleGlnProLeuAlaLysAspLeuGlyArgGlnIleGly

5470 5480 5490 5500 5510 5520
aaaatcaaacttagcgatgtgcttagcgcctcagatgtgagtgcacttttaaacatgccc
LysIleLysLeuSerAspValLeuSerAlaSerAspValSerAlaLeuLeuAsnMetPro

5530 5540 5550 5560 5570 5580
ggtatggataatgtgatcaaaaatattttaagtactaagactgtgagttctgtattaggc
GlyMetAspAsnValIleLysAsnIleLeuSerThrLysThrValSerSerValLeuGly

5590 5600 5610 5620 5630 5640
agtcgtgggcttatttctagtcttaaccaatctgagcaaaataaaatctatggtgctatt
SerArgGlyLeuIleSerSerLeuAsnGlnSerGluGlnAsnLysIleTyrGlyAlaIle

5650 5660 5670 5680 5690 5700
gataaagagttacatttctcaggggggaaaagtcattgcctccattgccaacggggtttat
AspLysGluLeuHisPheSerGlyGlyLysValIleAlaSerIleAlaAsnGlyValTyr

5710 5720 5730 5740 5750 5760
ggtaaccacacccttttgagcctcattaactccctctctcctatgacgggtaaaaatgtg
GlyAsnHisThrLeuLeuSerLeuIleAsnSerLeuSerProMetThrGlyLysAsnVal

5770 5780 5790 5800 5810 5820
gacaatattttaaacatgccaagcaccacgagcggacaaagccagatcaaaagtctttta
AspAsnIleLeuAsnMetProSerThrThrSerGlyGlnSerGlnIleLysSerLeuLeu

5830 5840 5850 5860 5870 5880
gagcaaaccacatttgagcaggtttttcaagaactgctctctaatcaaaacctactcaat
GluGlnThrThrPheGluGlnValPheGlnGluLeuLeuSerAsnGlnAsnLeuLeuAsı

5890 5900 5910 5920 5930 5940
aaaaccattgcttggttaggcccacaaattttagatgaaatgcttaaaactgctattgat
LysThrIleAlaTrpLeuGlyProGlnIleLeuAspGluMetLeuLysThrAlaIleAsp

FIG 2J

5950 5960 5970 5980 5990 6000
gatttactcaaccctacaaatcagcttactgccgctgaaaaaaatattcttgataacatt
AspLeuLeuAsnProThrAsnGlnLeuThrAlaAlaGluLysAsnIleLeuAspAsnIle

6010 6020 6030 6040 6050 6060
cttaacaatgtctttagctcagaaaaaaaggcagtccaacaaatggaaaattctaaccct
LeuAsnAsnValPheSerSerGluLysLysAlaValGlnGlnMetGluAsnSerAsnPro

6070 6080 6090 6100 6110 6120
gatgtcaaacacgttattgatggcttaatgcaggctaaaggtctaggggaggtttatagc
AspValLysHisValIleAspGlyLeuMetGlnAlaLysGlyLeuGlyGluValTyrSer

6130 6140 6150 6160 6170 6180
aagggccttcagagtattttatctaataaactgcaaggtcaattaaaaagcatgggctta
LysGlyLeuGlnSerIleLeuSerAsnLysLeuGlnGlyGlnLeuLysSerMetGlyLeu

6190 6200 6210 6220 6230 6240
ggttctttgctcgcgcctaaagctctaggtaatttctggcagaagggttattttaacttc
GlySerLeuLeuAlaProLysAlaLeuGlyAsnPheTrpGlnLysGlyTyrPheAsnPhe

6250 6260 6270 6280 6290 6300
ctagccaatgatgatattttagtgaacaatagcacttttagtaatgcttcaggcgggact
LeuAlaAsnAspAspIleLeuValAsnAsnSerThrPheSerAsnAlaSerGlyGlyThr

6310 6320 6330 6340 6350 6360
ttaagttttatagccggtaagtctattatttttgcagggcaaaatacaattaattttagt
LeuSerPheIleAlaGlyLysSerIleIlePheAlaGlyGlnAsnThrIleAsnPheSer

6370 6380 6390 6400 6410 6420
aataatcaaggtacactagaattttttaagtaatgatgtgtctaatattgatctgactact
AsnAsnGlnGlyThrLeuGluPheLeuSerAsnAspValSerAsnIleAspLeuThrThr

6430 6440 6450 6460 6470 6480
cttaatgccactgacggtttaaccattgatgcccccctttaataatctttatgttcagaaa
LeuAsnAlaThrAspGlyLeuThrIleAspAlaProPheAsnAsnLeuTyrValGlnLys

6490 6500 6510 6520 6530 6540
ggcaatatcacccttaatcaatatgagagttttagcgtgcaggcgggcaattttgacttt
GlyAsnIleThrLeuAsnGlnTyrGluSerPheSerValGlnAlaGlyAsnPheAspPhe

6550 6560 6570 6580 6590 6600
ttaggcacggtgcaagcagacggggctgtggatttatccggtgtaaccggtttagctgtt
LeuGlyThrValGlnAlaAspGlyAlaValAspLeuSerGlyValThrGlyLeuAlaVal

FIG 2K

```
        6610      6620      6630      6640      6650      6660
ttaggtactcttaatcttactgaggatagcacccttaaggctaataatttaaccacaata
LeuGlyThrLeuAsnLeuThrGluAspSerThrLeuLysAlaAsnAsnLeuThrThrIle

        6670      6680      6690      6700      6710      6720
agcgcctttaacaaccaatcccaaaacctgcttaatatcagcgggaattttaactcctat
SerAlaPheAsnAsnGlnSerGlnAsnLeuLeuAsnIleSerGlyAsnPheAsnSerTyr

        6730      6740      6750      6760      6770      6780
ggcacatttagtacacaaggggctgggataaatatcggagggggtttaatagcataggg
GlyThrPheSerThrGlnGlyAlaGlyIleAsnIleGlyGlyGlyPheAsnSerIleGly

        6790      6800      6810      6820      6830      6840
gctttaacttttaatgtagcgggtgcaacagtcaaaactacaggccctagctcagatagt
AlaLeuThrPheAsnValAlaGlyAlaThrValLysThrThrGlyProSerSerAspSer

        6850      6860      6870      6880      6890      6900
tcaagttcaagcactagctcctctacaagttctagtaattctaatagctctggaggctct
SerSerSerSerThrSerSerSerThrSerSerSerAsnSerAsnSerSerGlyGlySer

        6910      6920      6930      6940      6950      6960
agctcctcctctagtacttcagactctactagttctagcgcgcctactagttctacaaca
SerSerSerSerSerThrSerAspSerThrSerSerSerAlaProThrSerSerThrThr

        6970      6980      6990      7000      7010      7020
gctacagctttaactctagctagcaccaccgtgtctacaacttctactacaaattctagc
AlaThrAlaLeuThrLeuAlaSerThrThrValSerThrThrSerThrThrAsnSerSer

        7030      7040      7050      7060      7070      7080
agtgatacaagtaatagctcaagctctaatagctcctcttctaatagtgtgtctaatgca
SerAspThrSerAsnSerSerSerSerAsnSerSerSerSerAsnSerValSerAsnAla

        7090      7100      7110      7120      7130      7140
attccggttagccccactcctagtacacaaattccgcttatccaagtaggtggacttgtg
IleProValSerProThrProSerThrGlnIleProLeuIleGlnValGlyGlyLeuVal

        7150      7160      7170      7180      7190      7200
aatttaaatttaggtggtagtgcgattattagctttaatacagaggcacaaacaaatagc
AsnLeuAsnLeuGlyGlySerAlaIleIleSerPheAsnThrGluAlaGlnThrAsnSer

        7210      7220      7230      7240      7250      7260
acgggaactacaggaactacacaaggcacagcaaacacaggcagtaccggatcaagcaca
ThrGlyThrThrGlyThrThrGlnGlyThrAlaAsnThrGlySerThrGlySerSerThr
```

FIG 2L

```
       7270      7280      7290      7300      7310      7320
aactctagctctacaagcacatcacaaacaacatctagtagttctactagctccacacaa
AsnSerSerSerThrSerThrSerGlnThrThrSerSerSerSerThrSerSerThrGln

       7330      7340      7350      7360      7370      7380
acaacaagcctaagctctaatactagtctagctactaccagccaaacgcctacaacttct
ThrThrSerLeuSerSerAsnThrSerLeuAlaThrThrSerGlnThrProThrThrSer

       7390      7400      7410      7420      7430      7440
agcggggcttctccggatagttcaaaccctacagcctctcctataacccccttcaaatggc
SerGlyAlaSerProAspSerSerAsnProThrAlaSerProIleThrProSerAsnGly

       7450      7460      7470      7480      7490      7500
gcttatacgctaattcaaactaatagctggattcattacaaccccacctcctttaatcca
AlaTyrThrLeuIleGlnThrAsnSerTrpIleHisTyrAsnProThrSerPheAsnPro

       7510      7520      7530      7540      7550      7560
aacaactggagacaatatttagagctctacaccagtcttaaaatcaacgggacagctttc
AsnAsnTrpArgGlnTyrLeuGluLeuTyrThrSerLeuLysIleAsnGlyThrAlaPhe

       7570      7580      7590      7600      7610      7620
cagcttaacgctcaaggtacaggtcttacttataatggtcaagcagtcaatatctcacaa
GlnLeuAsnAlaGlnGlyThrGlyLeuThrTyrAsnGlyGlnAlaValAsnIleSerGln

       7630      7640      7650      7660      7670      7680
cgaggcttgcttgtcaattatcaaggcacaaatggccaagaagtgagcgcctctattgat
ArgGlyLeuLeuValAsnTyrGlnGlyThrAsnGlyGlnGluValSerAlaSerIleAsp

       7690      7700      7710      7720      7730      7740
tataataagatgcaaataggcataggccaaagcttgcatgttatcgcgcccactattaca
TyrAsnLysMetGlnIleGlyIleGlyGlnSerLeuHisValIleAlaProThrIleThr

       7750      7760      7770      7780      7790      7800
caatacatcacccaaatacaagggcagtctgtggttaatgcgctagaaaatgcaggcggg
GlnTyrIleThrGlnIleGlnGlyGlnSerValValAsnAlaLeuGluAsnAlaGlyGly

       7810      7820      7830      7840      7850      7860
ccgggtgtgatgaattggtttggtaagcttttaattgagacaaaaaacaccccgcttttt
ProGlyValMetAsnTrpPheGlyLysLeuLeuIleGluThrLysAsnThrProLeuPhe

       7870      7880      7890      7900      7910      7920
gcgccctactatcttgaacaacactccttaagcgatctacttaaaattgtaaaagatatt
AlaProTyrTyrLeuGluGlnHisSerLeuSerAspLeuLeuLysIleValLysAspIle
```

FIG 2M

```
     7930       7940       7950       7960       7970       7980
caaaatgcgactgattggatgggcgcttctggtttaaaagccactagctctaaattgcta
GlnAsnAlaThrAspTrpMetGlyAlaSerGlyLeuLysAlaThrSerSerLysLeuLeu

     7990       8000       8010       8020       8030       8040
caaatcagtgtacacaccaaacagatgagccgtttagctaagctttcaaattttgcttct
GlnIleSerValHisThrLysGlnMetSerArgLeuAlaLysLeuSerAsnPheAlaSer

     8050       8060       8070       8080       8090       8100
aatgatgcattaccagattttcatgatttttagtcagtcttaaaggtaaaaaatttgct
AsnAspAlaLeuProAspPheHisAspPheLeuValSerLeuLysGlyLysLysPheAla

     8110       8120       8130       8140       8150       8160
agcgcggtgcctaatgctatggatattatcaccgcctattctcaaagagataaattaaaa
SerAlaValProAsnAlaMetAspIleIleThrAlaTyrSerGlnArgAspLysLeuLys

     8170       8180       8190       8200       8210       8220
aataacctatggataaccggtgtgggaggagcaagttttgtagcaggaggcacaggtacg
AsnAsnLeuTrpIleThrGlyValGlyGlyAlaSerPheValAlaGlyGlyThrGlyThr

     8230       8240       8250       8260       8270       8280
ctttatggctgaatgtgggttatgatcgctttataaagggcgtgattgtgggggttat
LeuTyrGlyLeuAsnValGlyTyrAspArgPheIleLysGlyValIleValGlyGlyTyr

     8290       8300       8310       8320       8330       8340
atggcttatggttatagtggcttttatggcaatatcaatagcgctaattctaataatgtc
MetAlaTyrGlyTyrSerGlyPheTyrGlyAsnIleAsnSerAlaAsnSerAsnAsnVal

     8350       8360       8370       8380       8390       8400
aatgtgggattttatagccgcgcctttatcaagggtagaaacgagattacaggaagtatt
AsnValGlyPheTyrSerArgAlaPheIleLysGlyArgAsnGluIleThrGlySerIle

     8410       8420       8430       8440       8450       8460
aatgaaacctatggctataacaaaacctacattgatgcaactaatcccattctcacccct
AsnGluThrTyrGlyTyrAsnLysThrTyrIleAspAlaThrAsnProIleLeuThrPro

     8470       8480       8490       8500       8510       8520
cttaaccagcaataccactatggcacttggactaccaatgtcggcgctaactatggctat
LeuAsnGlnGlnTyrHisTyrGlyThrTrpThrThrAsnValGlyAlaAsnTyrGlyTyr

     8530       8540       8550       8560       8570       8580
gacttctttttaaaaacaaacatgttattctcaaaccccaaattggcctcacttattat
AspPhePhePheLysAsnLysHisValIleLeuLysProGlnIleGlyLeuThrTyrTyr
```

FIG 2N

```
        8590      8600      8610      8620      8630      8640
tatatcggcctctcaggcttgcaaggcaagatgaatgatccgatttataacgagtttaga
TyrIleGlyLeuSerGlyLeuGlnGlyLysMetAsnAspProIleTyrAsnGluPheArg

        8650      8660      8670      8680      8690      8700
gccaatgccgatccagcgcataaatctgttttgacgatcaatttagccctagagagtcgc
AlaAsnAlaAspProAlaHisLysSerValLeuThrIleAsnLeuAlaLeuGluSerArg

        8710      8720      8730      8740      8750      8760
cactattttaggaaaaactcctattactatgtcattgccggcttagggcgggatttattc
HisTyrPheArgLysAsnSerTyrTyrTyrValIleAlaGlyLeuGlyArgAspLeuPhe

        8770      8780      8790      8800      8810      8820
gtccattctatgggtgataaaatagtacgctttattggtaatgatatgttaagttatcgt
ValHisSerMetGlyAspLysIleValArgPheIleGlyAsnAspMetLeuSerTyrArg

        8830      8840      8850      8860      8870      8880
tatgggggaatgtataatacctttgctagcctcaccacagggggtgaggttcgcttattt
TyrGlyGlyMetTyrAsnThrPheAlaSerLeuThrThrGlyGlyGluValArgLeuPhe

        8890      8900      8910      8920      8930      8940
cgatccttttatgtcaatgctggtattggagcgcgctttggtttggattatcaagatatt
ArgSerPheTyrValAsnAlaGlyIleGlyAlaArgPheGlyLeuAspTyrGlnAspIle

        8950      8960      8970
aatattacaggcaatgtcgggatgcgctatgctttttag
AsnIleThrGlyAsnValGlyMetArgTyrAlaPhe
```

## FIG 3

### Comparing amino acid sequences of the Nos. 11, 33, 19 region (14-mer)

SNTW101 (human, nodular gastritis)

LEQTTFEQVFQELLSNQNLLNKTIAWLGPQILDEMLKTAIDDLLNPTNQLTAAEKNILD
NILNNVFSS**EKKAVQQMENSNPD**VKHVIDGLMQAKGLGEVYSKGLQSI**LSNKLQGQLKS**
**MGL**GSLLAPKALGNFWQKGYFNFLANDDILVNNSTFSNASGGTLSFIAGKSIIFAGQNT
INFSN**NQGTLEFLSNDVSN**IDLTTLNATDGLTIDAPFNNLYVQKGNITLNQYESFSVQA
GNFDFLGTVQADGAVDLSGVT

HS1 (swine)

LNKTTFGQIFEELLSNQNLLNKTIAWLGPQILDEMLKTAIDDLLNPTNQLTAAEKNILD
NILNNVFSSE**KKAVQQMENSNPD**VKHVIDGLMQAKGLGEVYSKGLQSI**LSNKLQGQLKS**
**MGL**GSLLAPKALGNFWQKGYFNFLANDDILVNNSTFSNASGGTLSFIAGKSIIFAGQNT
INFSN**NQGTLEFLSNDVSN**IDLTTLNATDGLTIDAPFNNLYVQKGNITLASYEGLTVRA
NNFDFLGTVQADGAVDLSGVT

HS5 (swine)

LKNTTFGQIFEELLSNQNLLNKTIAWLGPQILDEMLKTAIDDLLNPTNQLTAAEKNILD
NILNNVFSSE**KKAVQQMENSNPD**VKHVIDGLMQAKGLGEVYSKGLQSI**LSNKLQGQLKS**
**MGL**GSLLAPKALGNFWQKGYFNFLANDDILVNNSTFSNASGGTLSFIAGKSIIFAGQNT
INFSN**NQGTLEFLSNDVSN**IDLTTLNATDGLTIDAPFNNLYVQKGNITLNQYESFSVQA
GNFDFLGTVQADGAVDLSGVT

TKY (cynomolgus monkey)

LNNTTFGQVLEELLSNQNLLNKTIAWLGPQILDEMLKTAIDDLLNPTSQLTAAEKNILD
NILNNVFGS**EKKAV**E**QMENSNPD**VKQVIDGLMQAKGLGEVYSKGLQSI**LSNKLQ**D**QLKS**
**MGL**GSLLAPKALGNFWQKGYFNFLANDDILVSNSTFDNASGGTLSFIAGKSIIFAGQNT
INFSN**NQGTLEFLSNDVS**TIDLTTLNATDGLTIDAPFNNLYVQKGNITLNQYESFSVQS
GNFDFLGTVQADGAVDLSGVT

SH8 (human, gastric MALT lymphoma)

LKQTNFTQAFQALESNQNLLNKTIAWVGPQILDEMLQTAINDLLNPTQELTKAETDILN
NILNNVFKK**EK**D**AV**TSLK**NSN**SGIKTMIEGLINNKGLGEVYSKGLQSI**LSNKLQ**D**QLKS**
**MGL**GSLLAPKALGNFWQKGYFNFLANDDILVSNSTFDNASGGTLSFIAGKSIIFAGQNT
INFSN**NQGTLEFLSNDVSN**IDLTTLNATDGLTIDAPFNNLYVQKGNITLNQYESFSVQS
GNFDFLGTVQADGAVDLSGVT

SH10 (human, chronic gastritis)

LKQTNFSQAFQALVSNQNLLNKTIAWLGPQILDEMLQTAINDLLNPTQELTKAETDILN
NILNNVFKK**EK**D**AV**TSL**ENSN**SGIKTMIEGLINNKGLGGVYTKGLQSIF**S**DK**LQ**NM**LKS**
LNMGSLLEPKALGKFWEKGYFNFLANDNVLVSNSTFKNASGGTLSFIAGKSIIFSGQNT
INFSN**NQGTLEFLSNDVS**TIDLTTLNATDGLTIDAPFNNLYVQKGNITLNQYESFSVQS
GNFDFLGTVQADGAVDLSGVT

FIG 4

Comparison of the peptide sequence of Nos. 11, 33, and 19

**No. 11**

| | |
|---|---|
| SNTW101 | EKKAVQQMENSNPD |
| HS1 | EKKAVQQMENSNPD |
| HS5 | EKKAVQQMENSNPD |
| TKY | EKKAVEQMENSNPD |
| SH8 | EKDAVTSLKNSNSG |
| SH10 | EKDAVTSLENSNSG |

**No. 19**

| | |
|---|---|
| SNTW101 | NQGTLEFLSNDVSN |
| HS1 | NQGTLEFLSNDVSN |
| HS5 | NQGTLEFLSNDVSN |
| TKY | NQGTLEFLSNDVST |
| SH8 | NQGTLEFLSNDVSN |
| SH10 | NOGTLEFLSNDVST |

**No. 33**

| | |
|---|---|
| SNTW101 | LSNKLQGQLKSMGL |
| HS1 | LSNKLQGQLKSMGL |
| HS5 | LSNKLQGQLKSMGL |
| TKY | LSNKLQDQLKSMGL |
| SH8 | LSNKLQDQLKSMGL |
| SH10 | FSDKLQNMLKSLNM |

FIG 5

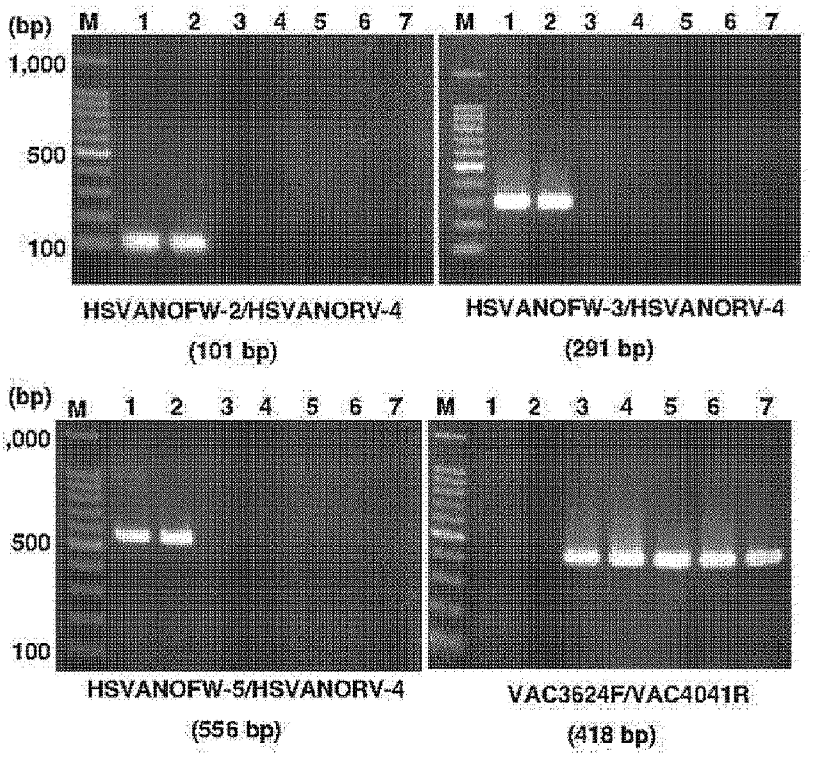


lane M; 100 bp marker
lane 1; *H. suis* TKY
lane 2; *H. suis* SNTW101
lane 3; *H. pylori* SS1
lane 4; *H. pylori* TN2GF4
lane 5; *H. pylori* NCTC 11637
lane 6; *H. pylori*ATCC 43579
lane 7; *H. pylori* RC-1

FIG 6

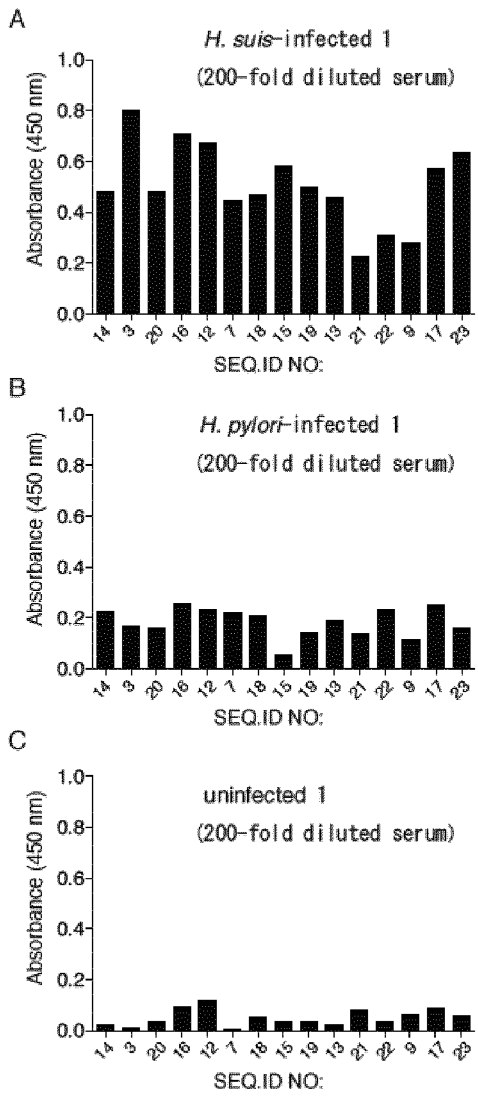
A
H. suis-infected 1
(200-fold diluted serum)
Absorbance (450 nm)
1.0
0.8
0.6
0.4
0.2
0.0
14 3 20 16 12 7 18 15 19 13 21 22 9 17 23
SEQ.ID NO:
B
H. pylori-infected 1
(200-fold diluted serum)
Absorbance (450 nm)
1.0
0.8
0.6
0.4
0.2
0.0
14 3 20 16 12 7 18 15 19 13 21 22 9 17 23
SEQ.ID NO:
C
uninfected 1
(200-fold diluted serum)
Absorbance (450 nm)
1.0
0.8
0.6
0.4
0.2
0.0
14 3 20 16 12 7 18 15 19 13 21 22 9 17 23
SEQ.ID NO:

FIG 7

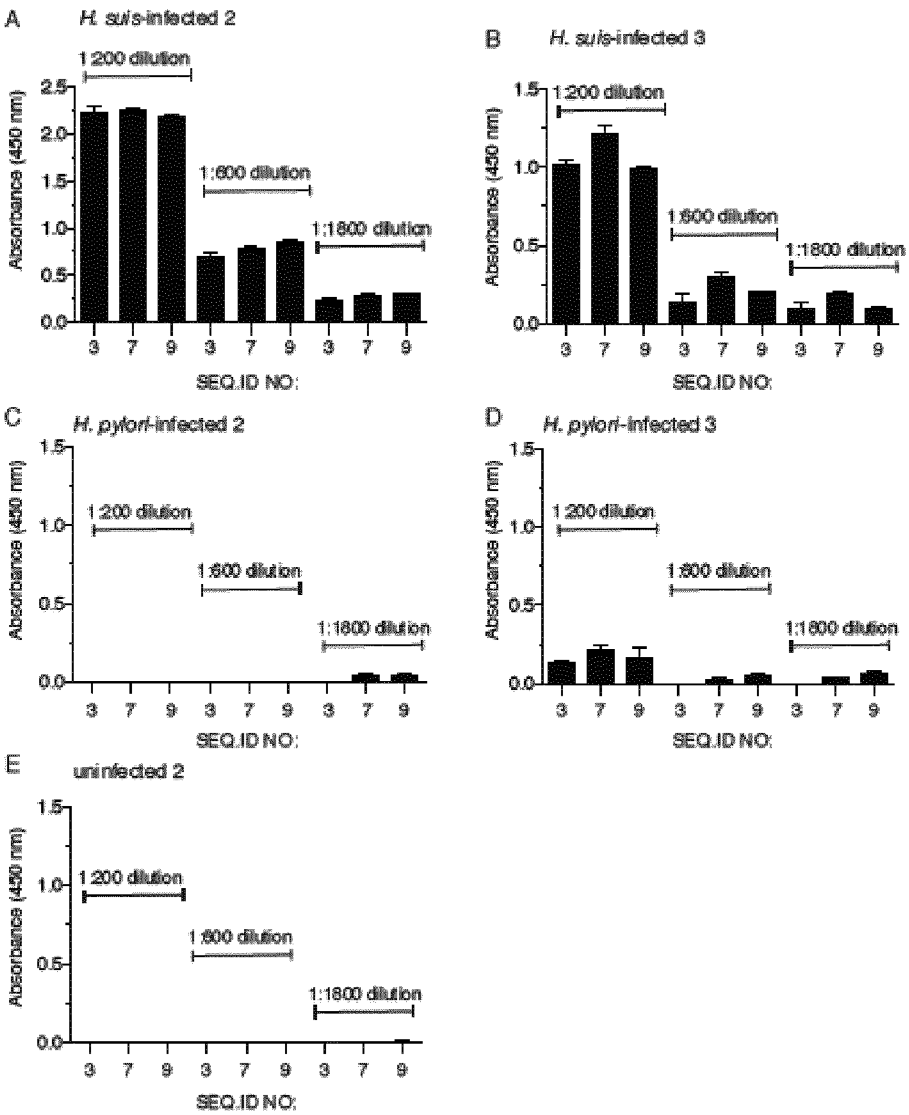
A
H. suis-infected 2
1:200 dilution
1:600 dilution
1:1800 dilution
Absorbance (450 nm)
SEQ.ID NO:
B
H. suis-infected 3
C
H. pylori-infected 2
D
H. pylori-infected 3
E
uninfected 2

FIG 8

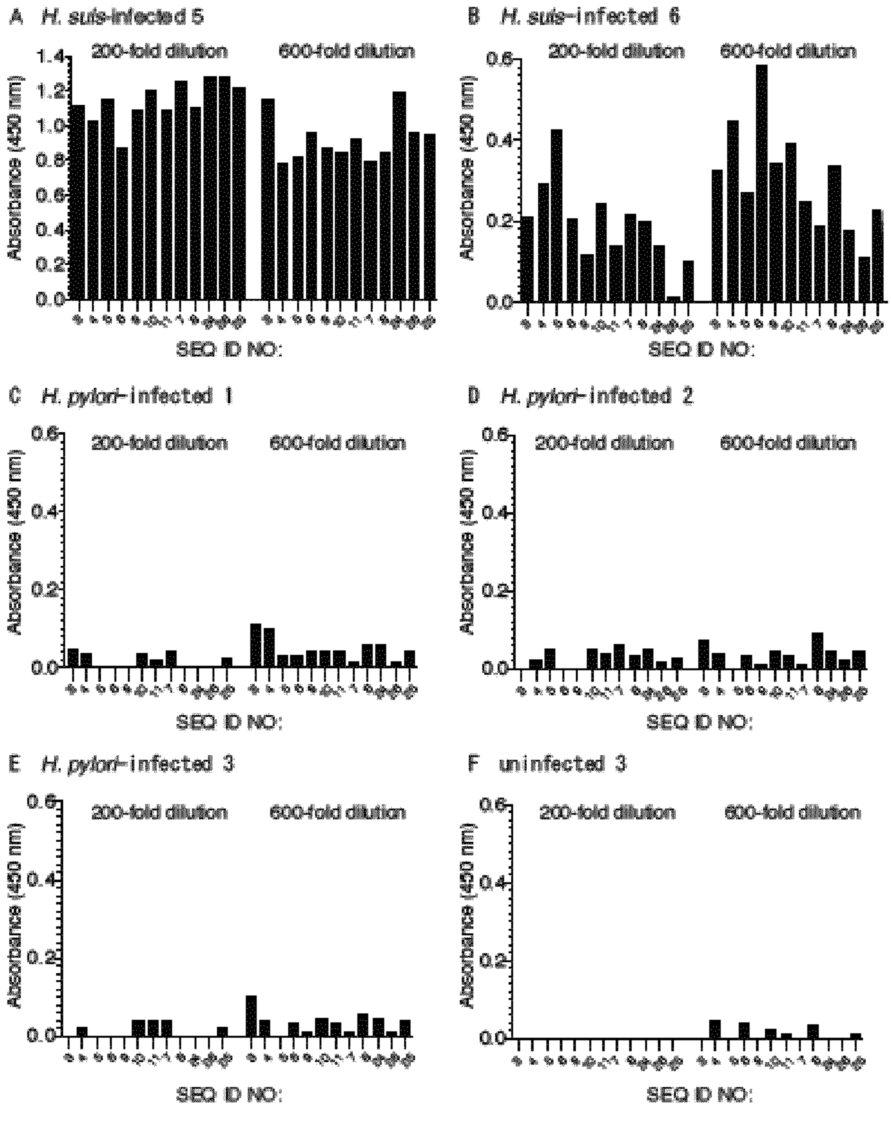
A H. suis-infected 5
B H. suis-infected 6
C H. pylori-infected 1
D H. pylori-infected 2
E H. pylori-infected 3
F uninfected 3
200-fold dilution
600-fold dilution
Absorbance (450 nm)
SEQ ID NO:

FIG 9

Antibody titer of human serum against *H. pylori* TN2GF4 (ELISA)

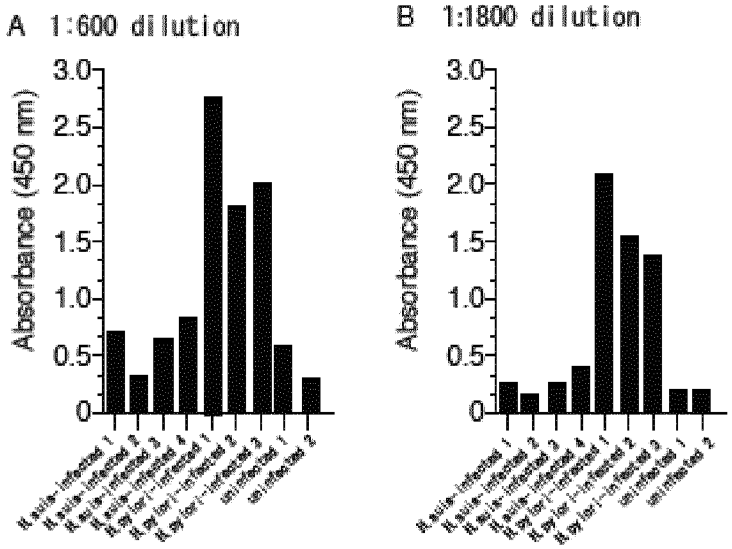

FIG 10

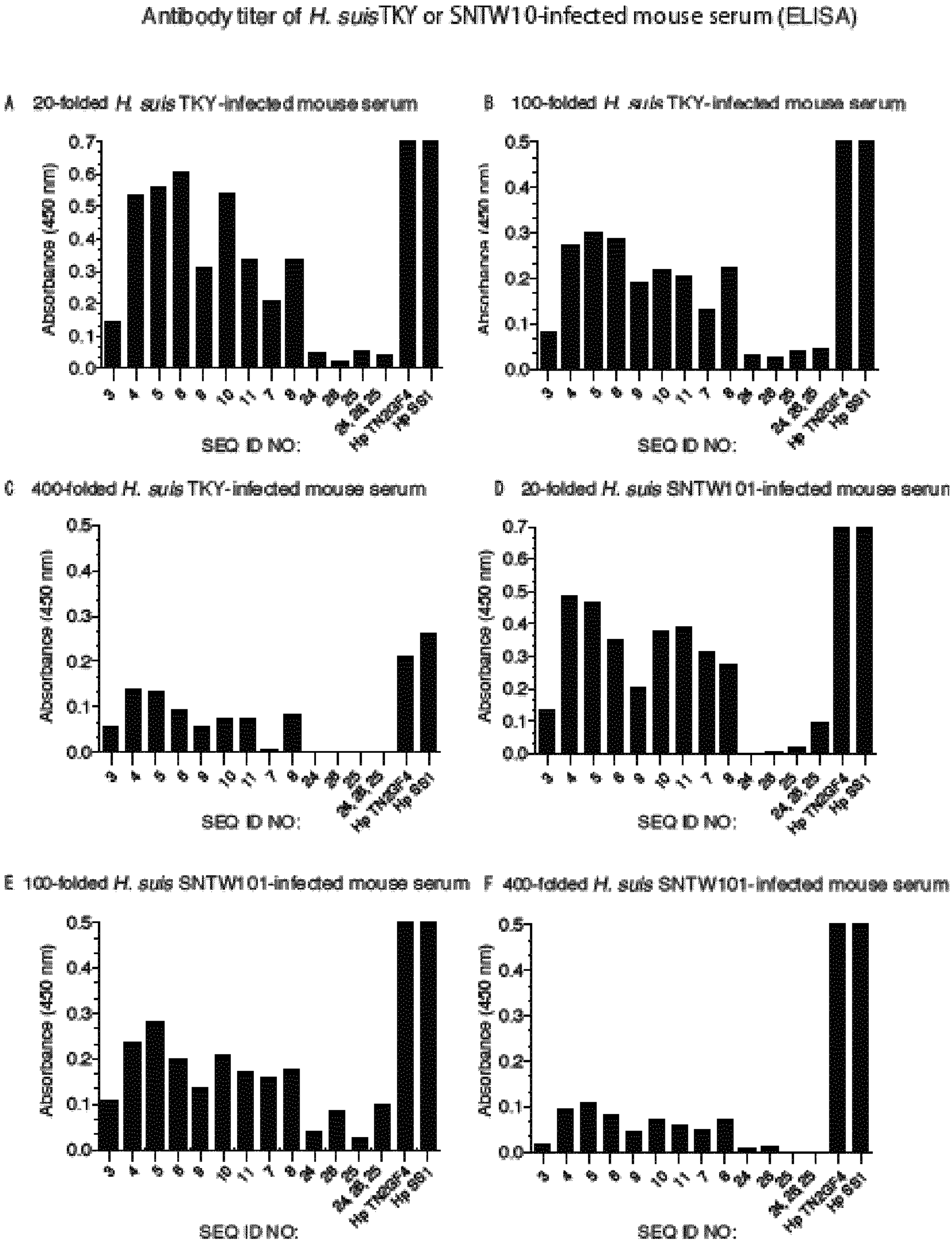
Antibody titer of H. suis TKY or SNTW10-infected mouse serum (ELISA)
A 20-folded H. suis TKY-infected mouse serum
B 100-folded H. suis TKY-infected mouse serum
C 400-folded H. suis TKY-infected mouse serum
D 20-folded H. suis SNTW101-infected mouse serum
E 100-folded H. suis SNTW101-infected mouse serum
F 400-folded H. suis SNTW101-infected mouse serum
Absorbance (450 nm)
3 4 5 6 9 10 11 7 8 24 28 25 24, 28, 25 Hp TN2GF4 Hp SS1
SEQ ID NO:

FIG 11

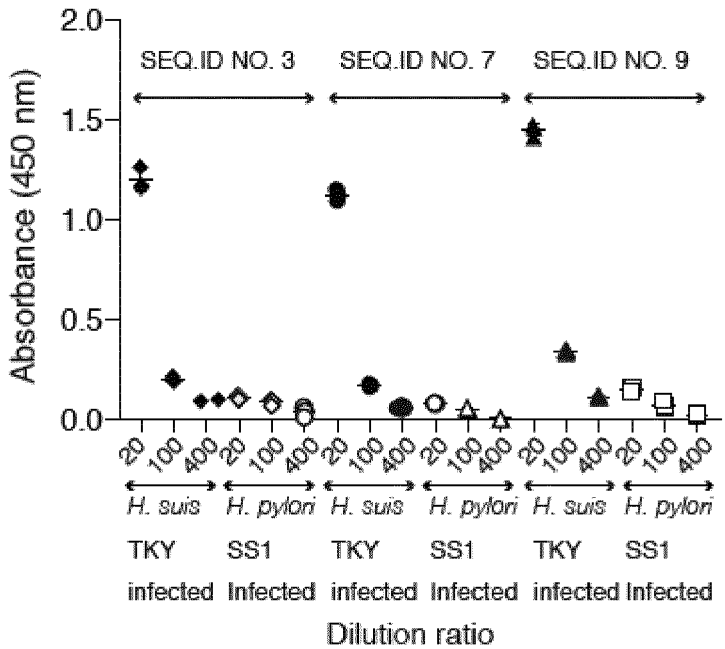
2.0
1.5
1.0
0.5
0.0
Absorbance (450 nm)
SEQ.ID NO. 3
SEQ.ID NO. 7
SEQ.ID NO. 9
20
100
400
H. suis TKY infected
H. pylori SS1 Infected
Dilution ratio

FIG 12

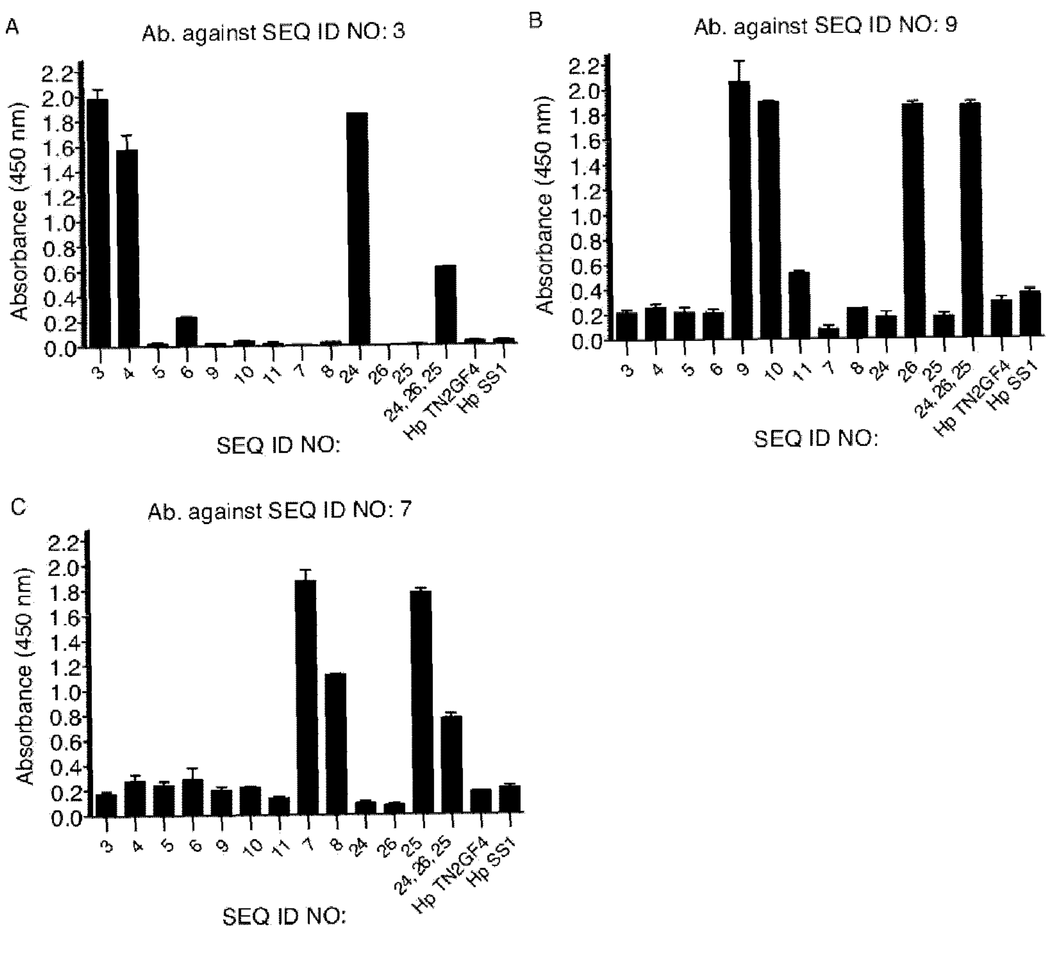
A
Ab. against SEQ ID NO: 3
Absorbance (450 nm)
2.2
2.0
1.8
1.6
1.4
1.2
1.0
0.8
0.6
0.4
0.2
0.0
3
4
5
6
9
10
11
7
8
24
26
25
24, 26, 25
Hp TN2GF4
Hp SS1
SEQ ID NO:
B
Ab. against SEQ ID NO: 9
Absorbance (450 nm)
SEQ ID NO:
C
Ab. against SEQ ID NO: 7
Absorbance (450 nm)
SEQ ID NO:

FIG 13

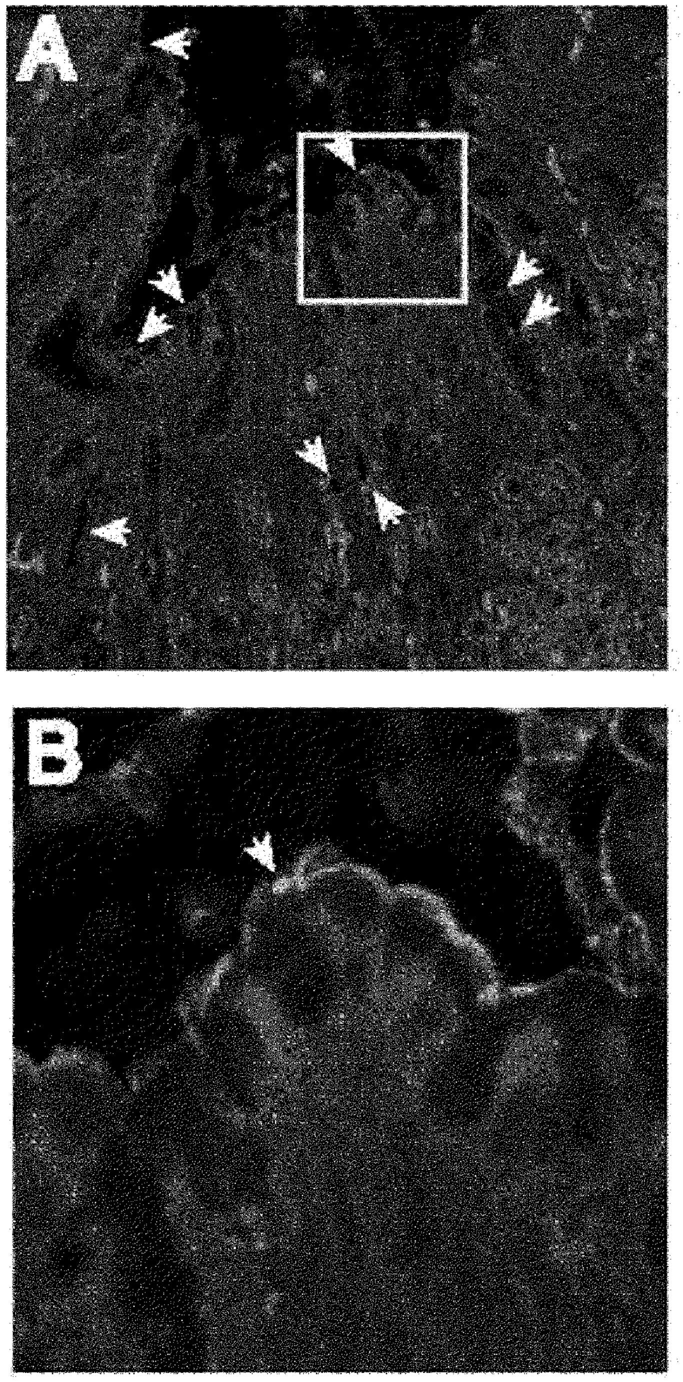

FIG 14

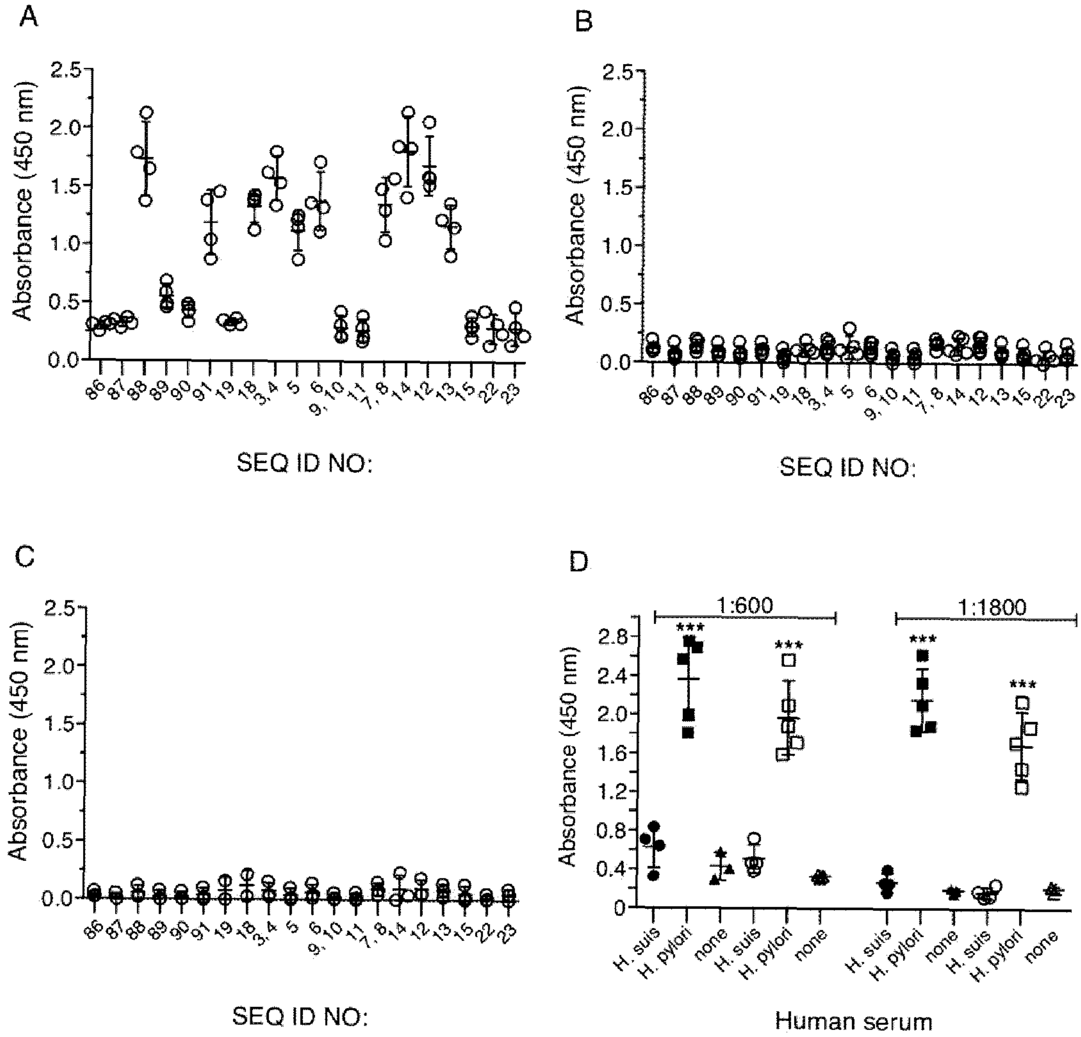
A
Absorbance (450 nm)
2.5
2.0
1.5
1.0
0.5
0.0
86 87 88 89 90 91 19 18 3, 4 5 6 9, 10 11 7, 8 14 12 13 15 22 23
SEQ ID NO:
B
Absorbance (450 nm)
SEQ ID NO:
C
Absorbance (450 nm)
SEQ ID NO:
D
1:600
1:1800
***
Absorbance (450 nm)
2.8
2.4
2.0
1.6
1.2
0.8
0.4
0
H. suis
H. pylori
none
Human serum

FIG 15

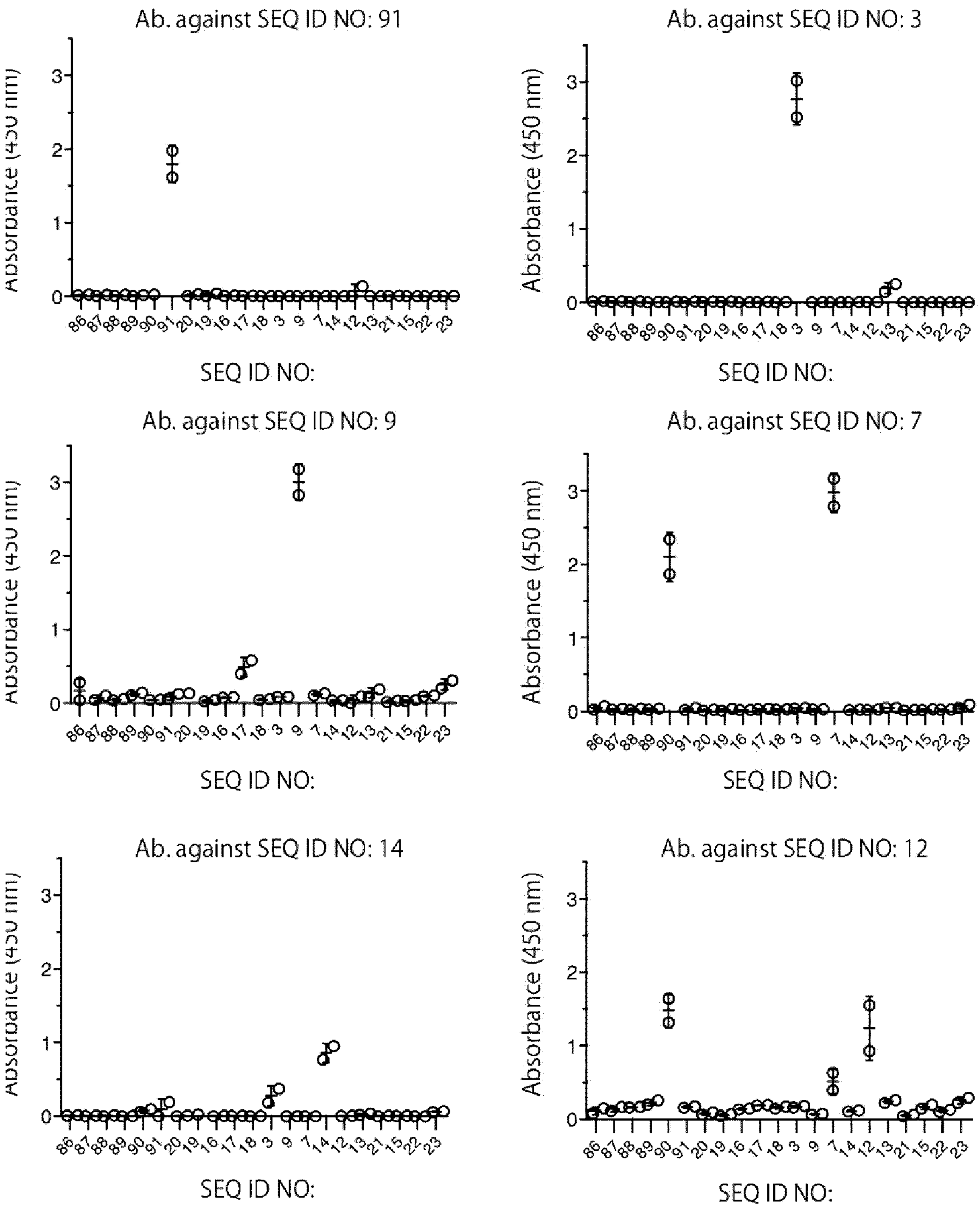
Ab. against SEQ ID NO: 91
Ab. against SEQ ID NO: 3
Ab. against SEQ ID NO: 9
Ab. against SEQ ID NO: 7
Ab. against SEQ ID NO: 14
Ab. against SEQ ID NO: 12
Absorbance (450 nm)
0
1
2
3
86 87 88 89 90 91 20 19 16 17 18 3 9 7 14 12 13 21 15 22 23
SEQ ID NO:

FIG 16

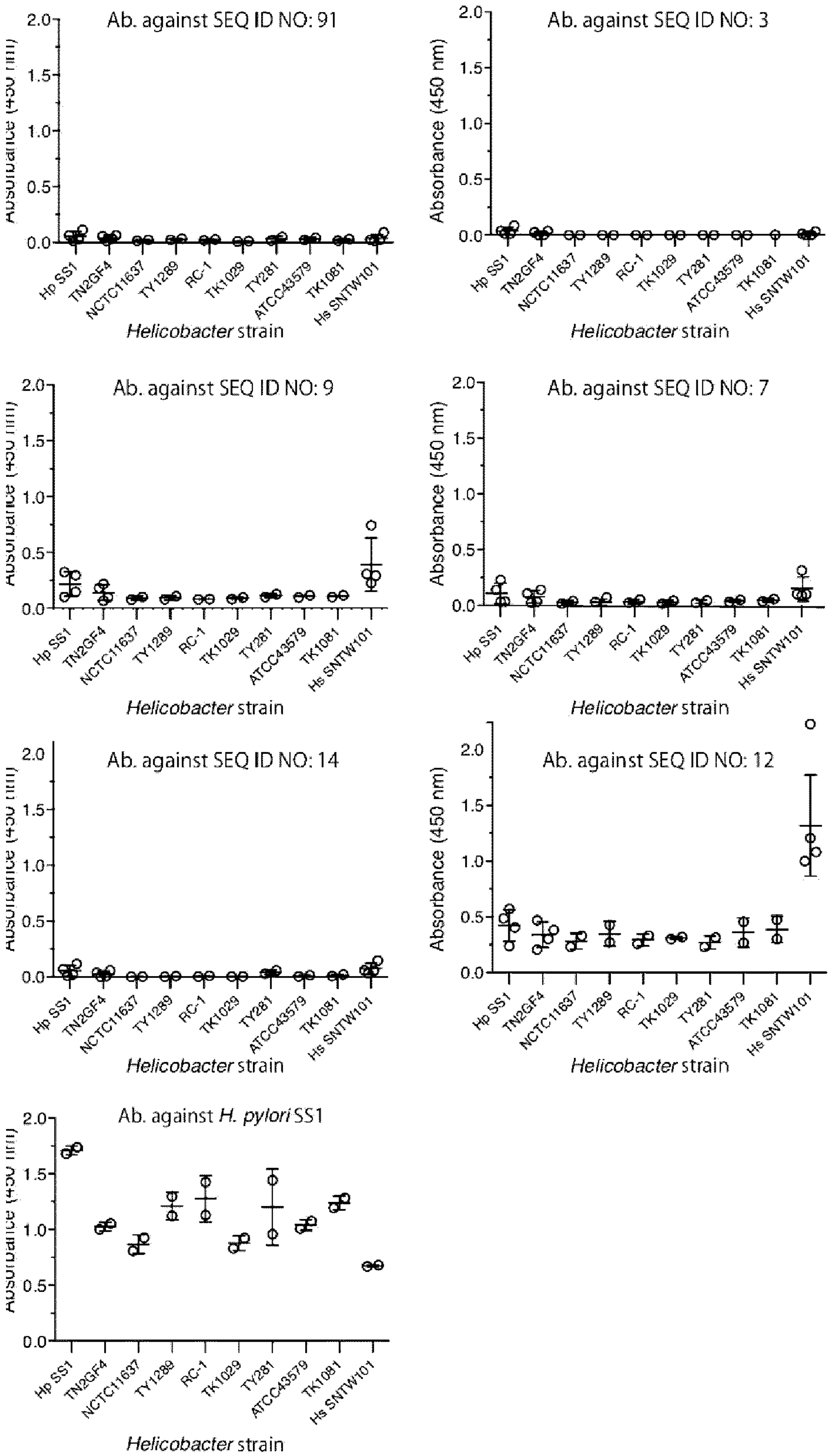
Ab. against SEQ ID NO: 91
Ab. against SEQ ID NO: 3
Ab. against SEQ ID NO: 9
Ab. against SEQ ID NO: 7
Ab. against SEQ ID NO: 14
Ab. against SEQ ID NO: 12
Ab. against H. pylori SS1
Absorbance (450 nm)
0.0
0.5
1.0
1.5
2.0
Hp SS1
TN2GF4
NCTC11637
TY1289
RC-1
TK1029
TY281
ATCC43579
TK1081
Hs SNTW101
Helicobacter strain

FIG 17

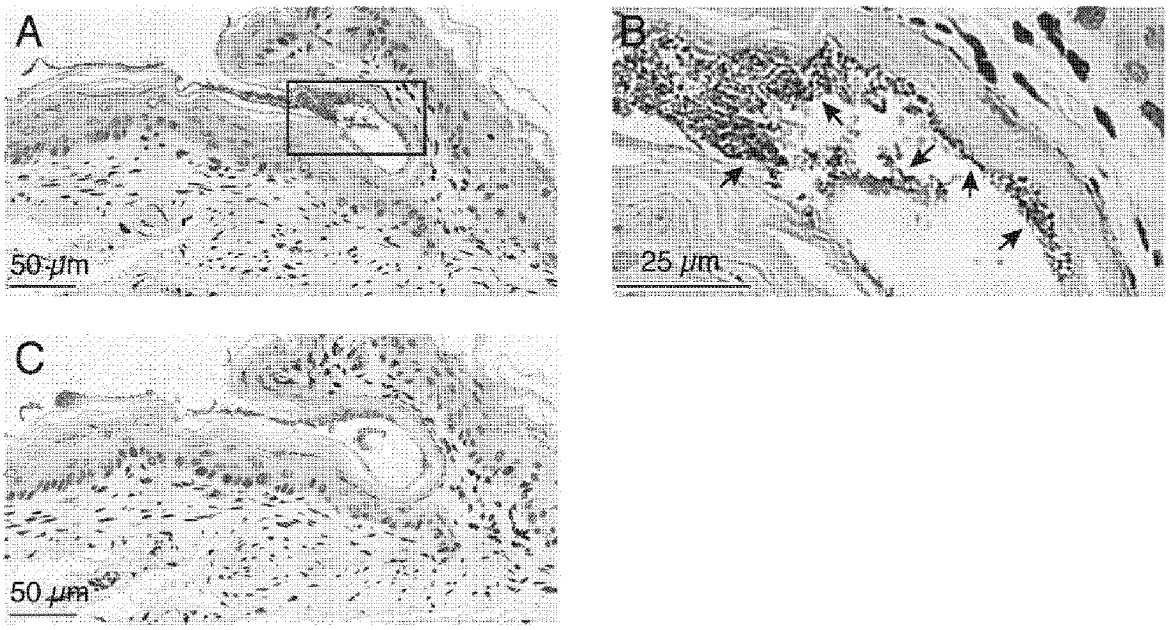

**INTERNATIONAL SEARCH REPORT**

International application No.
PCT/JP2019/020248

A. CLASSIFICATION OF SUBJECT MATTER
Int.Cl. [see extra sheet]

According to International Patent Classification (IPC) or to both national classification and IPC

B. FIELDS SEARCHED

Minimum documentation searched (classification system followed by classification symbols)
Int.Cl. C12N15/31, A61K31/7088, A61K31/711, A61K39/106, A61K48/00, A61P1/04, A61P7/04, A61P31/04, A61P35/00, A61P35/02, A61P37/04, C07K14/205, C12N15/11, C12Q1/686, C12Q1/689, G01N33/569

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580(JDreamIII), CAplus/WPIDS/MEDLINE/EMBASE/BIOSIS(STN), UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X<br>A | OMP1394 [Helicobacter suis], accession no. SFZ72639, [retrieved on 16 August 2019], GenBank[online], 05 January 2017, retrieved from the Internet: <URL:https://www.ncbi.nlm.nih.gov/protein/1127910160>, particularly, SOURCE column, PROTEIN column, ORIGIN column | 1-18, 21<br>19-20, 22-24 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>16.08.2019 | Date of mailing of the international search report<br>27.08.2019 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2019/020248

C (Continuation). DOCUMENTS CONSIDERED TO BE RELEVANT

| Category* | Citation of document, with indication, where appropriate, of the relevant passages | Relevant to claim No. |
|---|---|---|
| X<br>A | OMP1181 [Helicobacter suis], accession no. SFZ72414, [retrieved on 16 August 2019], GenBank[online], 05 January 2017, retrieved from the Internet: <URL:https://www.ncbi.nlm.nih.gov/protein/SFZ72414>, particularly, SOURCE column, PROTEIN column, ORIGIN column | 1-18, 21<br>19-20, 22-24 |
| X<br>A | OMP1114 [Helicobacter suis], accession no. SFZ72295, [retrieved on 16 August 2019], GenBank [online], 05 January 2017, retrieved from the Internet: <URL:https://www.ncbi.nlm.nih.gov/protein/1127909236>, particularly, SOURCE column, PROTEIN column, ORIGIN column | 1-18, 21<br>19-20, 22-24 |
| X<br>A | OMP539 [Helicobacter suis], accession no. SFZ72530, [retrieved on 16 August 2019], GenBank [online], 05 January 2017, retrieved from the Internet: <URL:https://www.ncbi.nlm.nih.gov/protein/1127909862>, particularly, SOURCE column, PROTEIN column, ORIGIN column | 1-18, 21<br>19-20, 22-24 |
| X<br>A | putative vacuolating cytotoxin [Helicobacter suis HS5], accession no. EFX41822, [retrieved on 16 August 2019], GenBank [online], 01 February 2011, retrieved from the Internet: <URL:https://www.ncbi.nlm.nih.gov/protein/EFX41822.1>, particularly, SOURCE column, PROTEIN column, ORIGIN column | 1-18, 21<br>19-20, 22-24 |
| A | JP 2016-010331 A (THE KITASATO INSTITUTE) 21 January 2016<br>(Family: none) | 1-24 |
| P, X | 松井英則 他, WS1-07 ヘリコバクター・スイス感染の迅速検査法の開発, 第 24 回日本ヘリコバクター学会学術集会プログラム・抄録集, 31 May 2018, p. 93, (MATSUI, Hidenori et al.), non-official translation (Development of rapid detection method for WS1-07 Helicobacter suis infection, Programs and Proceedings of the 24th Annual Meeting of the Japanese Society for Helicobacter Research) | 1-24 |

Form PCT/ISA/210 (continuation of second sheet) (January 2015)

**INTERNATIONAL SEARCH REPORT**
Information on patent family members

International application No.
PCT/JP2019/020248

(Continuation of Box No. A)

CLASSIFICATION OF SUBJECT MATTER
C12N15/31(2006.01)i, A61K31/7088(2006.01)i, A61K31/711(2006.01)i, A61K39/106(2006.01)i, A61K48/00(2006.01)i, A61P1/04(2006.01)i, A61P7/04(2006.01)i, A61P31/04(2006.01)i, A61P35/00(2006.01)i, A61P35/02(2006.01)i, A61P37/04(2006.01)i, C07K14/205(2006.01)i, C12N15/11(2006.01)i, C12Q1/686(2018.01)i, C12Q1/689(2018.01)i, G01N33/569(2006.01)i

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011015664 A
- US 20060078919 A
- JP 2016010331 A


### Non-patent literature cited in the description


- **BLAECHER C et al.** *Helicobacter,* 2016, vol. 22 (3), e12369
- **OVERBY A et al.** *Digestion,* 2016, vol. 93 (4), 260-5
- **VERMOOTE M et al.** *Vet Res.,* 2011, vol. 42, 51
- **NAKAMURA M et al.** *Infect Immun.,* 2007, vol. 75 (3), 1214-22
- **OVERBY A et al.** *Digestion,* 2017, vol. 95 (1), 61-6
- **MASAHIKO NAKAMURA et al.** *Modern Medical Laboratory,* 2016, vol. 44 (4), 278-84
- **MATSUI H et al.** *Helicobacter.,* 2014, vol. 19 (4), 260-71
- **SMET A et al.** *Genome Announcements,* 2013, vol. 1 (1), e00033-12
- **SCHOTT T et al.** *Journal of Bacteriology,* 2011, vol. 193 (17), 4565-6
- **VERMOOTE M et al.** *Veterinary Research,* 2011, vol. 42, 51
- **ARNOLD I C et al.** *Genome Biol. Evol.,* 2011, vol. 3, 302-8
- **MATSUI H et al.** *Genome Announcements,* 2016, vol. 4 (5), e00934-16
- **BENTO-MIRANDA M et al.** *World J Gastroenterol,* 2014, vol. 20 (47), 17779-87
- **COVER TL et al.** *Nat Rev Microbiol.,* 2005, vol. 3 (4), 320-32
- Molecular Cloning, a Laboratory Manual. Cold Spring Harbor Laboratory Press, 2012
- Current Protocols in Molecular Biology. Wiley Online Library
- **KWIATKOWSKI, R.W. et al.** *Mol. Diagn.,* 1999, vol. 4, 353-364
- Japanese Pharmaceutical Excipients Directory. Yakuji Nippo, Ltd,
- Handbook of Pharmaceutical Excipients. APhA Publications
- **MATSUI H et al.** *Helicobacter,* 2014, vol. 19 (4), 260-71
- **SMET A. et al.** *International Journal of Systematic and Evolutionary Microbiology,* 2012, vol. 62, 299-306